# EUROPÄISCHE PATENTSCHRIFT

(11) **EP 3 298 148 B1**
(45) Veröffentlichungstag und Bekanntmachung des Hinweises auf die Patenterteilung: **26.07.2023**
(21) Anmeldenummer: 16724394.8
(22) Anmeldetag: 19.05.2016
(51) Int. Cl.: C12N 15/82, C12N 15/113, A01H 5/00

(54) **VERFAHREN UND KONSTRUKTE ZUR GEZIELTEN NUKLEINSÄURE EDITIERUNG IN PFLANZEN**
METHOD AND HYBRIDS FOR TARGETED NUCLEIC ACID EDITING IN PLANTS
PROCÉDÉ ET HYBRIDES POUR L'ÉDITION CIBLÉE D'ACIDE NUCLÉIQUE DANS LES VÉGÉTAUX

(30) Priorität: 19.05.2015 DE 102015006335; 05.11.2015 DE 102015014252
(43) Veröffentlichungstag der Anmeldung: 28.03.2018
(73) Patentinhaber: KWS SAAT SE & Co. KGaA, 37574 Einbeck (DE)
(72) Erfinder: HARLING, Hinrich, 37120 Bovenden (DE); MARTIN-ORTIGOSA, Susana, 37574 Einbeck (DE); NIESSEN, Markus, 30880 Laatzen (DE); STREITNER, Corinna, 49328 Melle (DE); SCHUMANN, Nadine, 37555 Einbeck (DE); JONGEDIJK, Erik, 9160 Lokeren (BE)
(74) Vertreter: Eisenführ Speiser
(86) Internationale Anmeldenummer: PCT/EP2016/061237
(87) Internationale Veröffentlichungsnummer: WO 2016/184955

(56) Entgegenhaltungen:
- EP-A1- 2 274 973
- WO-A1-2014/144155
- WO-A1-2014/194190
- US-A1- 2015 059 010
- BALTES N J ET AL: "DNA replicons for plant genome engineering", THE PLANT CELL, AMERICAN SOCIETY OF PLANT BIOLOGISTS, US, Bd. 26, Nr. 1, 17. Januar 2014 (2014-01-17), Seiten 151-163, XP002752140, ISSN: 1040-4651, DOI: 10.1105/TPC.113.119792 [gefunden am 2014-01-17]
- Thomas P Quinn ET AL: "A Streamlined Method for the Production, Screening, and Application of sgRNAs for CRISPR/Cas Gene Editing", , 2014, XP055290306, Mountain View, CA, USA Gefunden im Internet: URL:http://info.clontech.com/rs/clontech/i mages/633702_Guide_It_PS_0514_web.pdf [gefunden am 2016-07-21]
- TAKESHI MARUYAMA ET AL: "Increasing the efficiency of precise genome editing with CRISPR-Cas9 by inhibition of nonhomologous end joining", NATURE BIOTECHNOLOGY, Bd. 33, Nr. 5, 20. April 2015 (2015-04-20) , Seiten 538-542, XP055290186, ISSN: 1087-0156, DOI: 10.1038/nbt.3190
- ALI ZAHIR ET AL: "Efficient Virus-Mediated Genome Editing in Plants Using the CRISPR/Cas9 System", MOLECULAR PLANT, Bd. 8, Nr. 8, 6. März 2015 (2015-03-06), Seiten 1288-1291, XP055290127,
- HUI-LI XING ET AL: "A CRISPR/Cas9 toolkit for multiplex genome editing in plants", BMC PLANT BIOLOGY, BIOMED CENTRAL, LONDON, GB, Bd. 14, Nr. 1, 29. November 2014 (2014-11-29), Seite 327, XP021205803, ISSN: 1471-2229, DOI: 10.1186/S12870-014-0327-Y
- HYUN YOUBONG ET AL: "Site-directed mutagenesis in Arabidopsis thaliana using dividing tissue-targeted RGEN of the CRISPR/Cas system to generate heritable null alleles", PLANTA, SPRINGER VERLAG, DE, Bd. 241, Nr. 1, 1. Oktober 2014 (2014-10-01), Seiten 271-284, XP035417502, ISSN: 0032-0935, DOI: 10.1007/S00425-014-2180-5 [gefunden am 2014-10-01]
- THOMAS B JACOBS ET AL: "Targeted genome modifications in soybean with CRISPR/Cas9", BMC BIOTECHNOLOGY, BIOMED CENTRAL LTD. LONDON, GB, Bd. 15, Nr. 1, 12. März 2015 (2015-03-12), Seite 16, XP021219338, ISSN: 1472-6750, DOI: 10.1186/S12896-015-0131-2
- W. JIANG ET AL: "Demonstration of CRISPR/Cas9/sgRNA-mediated targeted gene modification in Arabidopsis, tobacco, sorghum and rice", NUCLEIC ACIDS RESEARCH, Bd. 41, Nr. 20, 2. September 2013 (2013-09-02), Seiten e188-e188, XP055219328, GB ISSN: 0305-1048, DOI: 10.1093/nar/gkt780
- HUI ZHANG ET AL: "The CRISPR/Cas9 system produces specific and homozygous targeted gene editing in rice in one generation", PLANT BIOTECHNOLOGY JOURNAL, Bd. 12, Nr. 6, 23. Mai 2014 (2014-05-23), Seiten 797-807, XP055269351, GB ISSN: 1467-7644, DOI: 10.1111/pbi.12200
- LUISA BORTESI ET AL: "The CRISPR/Cas9 system for plant genome editing and beyond", BIOTECHNOLOGY ADVANCES, Bd. 33, Nr. 1, 20. Dezember 2014 (2014-12-20), Seiten 41-52, XP055217852, ISSN: 0734-9750, DOI: 10.1016/j.biotechadv.2014.12.006
- ANONYMOUS: "A Streamlined Method for the Production, Screening, and Application of sgRNAs for CRISPR/Cas9 Gene Editing", BIOTECHNIQUES, Bd. 57, Nr. 3, September 2014 (2014-09), Seite 157, XP055290305,
- BERND ZETSCHE ET AL: "Cpf1 Is a Single RNA-Guided Endonuclease of a Class 2 CRISPR-Cas System", CELL, Bd. 163, Nr. 3, 25. September 2015 (2015-09-25), Seiten 759-771, XP055267511, US ISSN: 0092-8674, DOI: 10.1016/j.cell.2015.09.038 in der Anmeldung erwähnt
- BENT ANDREW F: "Arabidopsis in planta transformation. Uses, mechanisms, and prospects for transformation of other species", PLANT PHYSIOLOGY, AMERICAN SOCIETY OF PLANT PHYSIOLOGISTS, ROCKVILLE, MD, USA, vol. 124, no. 4, 1 December 2000 (2000-12-01), pages 1540-1547, XP002416813, ISSN: 0032-0889, DOI: 10.1104/PP.124.4.1540

## Beschreibung

### Technisches Gebiet

Die vorliegende Erfindung betrifft insbesondere Verfahren zur Herstellung einer Maispflanze, umfassend die Bereitstellung und Einbringung mindestens einer gRNA sowie einer CRISPR Nuklease oder eines katalytisch aktiven Fragments davon und/oder einer Effektor-Domäne oder mindestens eines rekombinanten Konstruktes, umfassend eine gRNA sowie eine CRISPR Nuklease oder ein katalytisch aktives Fragment davon und/oder eine Effektor-Domäne, oder die dafür kodierenden Sequenzen, sowie mindestens eine regulatorische Sequenz und/oder eine Lokalisationssequenz, in eine pflanzliche Zielstruktur, umfassend mindestens eine meristematische Zelle, wodurch direkt eine Maispflanze gewonnen werden kann, die eine gezielte Veränderung in einer Nukleinsäure-Zielregion umfasst, wobei das mindestens eine rekombinante Konstrukt nicht chromosomal oder extrachromosomal integriert wird. Zudem werden geeignete rekombinante Konstrukte und Vektoren sowie Verfahren zur Einbringung dieser Konstrukte und Vektoren in eine pflanzliche Zielstruktur von Interesse offenbart. Schließlich wird die Verwendung eines rekombinanten Konstrukts zur gezielten Veränderung einer Nukleinsäure-Zielregion in einer pflanzlichen Zelle offenbart, sowie Pflanzen, Pflanzenmaterial oder eine pflanzliche Zelle, die durch die erfindungsgemäßen Verfahren erhältlich oder erhalten sind. Ferner wird ein *in vitro* Screening Verfahren offenbart, um als Vorabtest die Funktionalität einer gRNA oder einer für eine gRNA kodierende Sequenz bezüglich der gezielten Modifikation einer spezifischen Nukleinsäure-Zielregion in einer pflanzlichen Zelle zusammen mit einer CRISPR Nuklease, umfassend u.a. Cas und/oder Cpf1 Nuklease oder Varianten oder katalytisch aktive Fragmente davon, oder einem katalytisch aktiven Fragment davon im hohen Durchsatz leicht bestimmen zu können. Die hierein offenbarten Verfahren sind insbesondere dafür geeignet, gezielt ein gewünschtes Merkmal in eine Pflanze einzubringen, zu modifizieren oder zu deletieren, insbesondere im Rahmen der gezielten Merkmalsentwicklung (trait development), um eine hochspezifische und effiziente Genom-Editierung zu gewährleisten.

### Hintergrund der Erfindung

Genom Editierung stellt ein molekularbiologisches Verfahren dar, durch welches gezielte Modifikationen wie Insertionen, Deletionen oder Punktmutationen oder Kombinationen davon in das Genom eines lebenden Organismus eingebracht werden können. Hierfür werden gezielte molekulare Instrumente benötigt, welche zum einen Nukleaseaktivität besitzen, darüber hinaus aber auch mit ausreichender Spezifität zu der zu verändernden Zielsequenz geleitet werden können, um eine gezielte und ortsgerichtete Mutagenese planen und durchführen zu können. In der Pflanzenbiotechnologie hat sich die gezielte Genome Editierung in den letzten Jahren zu einer Alternative im Vergleich zur klassischen Züchtung und zu transgenen Ansätzen entwickelt. Die bisher verfügbaren Tools wie Zink-Finger Nukleasen (ZFNs) oder "transcription activator-like effector nucleases" (TALENs) sind im Bereich der Pflanzenbiotechnologie allerdings nur begrenzt eingesetzt worden, was an der teilweise niedrigen Effizienz, aber auch an schwierigem und aufwendigem Design der Konstrukte liegt.

Ein weiteres molekulares Werkzeug, welches in den letzten Jahren gehäuft zur präzisen und ortsgerichteten Genommodifikation eingesetzt wurde, sind CRISPR Nuklease-basierte Systeme. Diese Nukleasen, umfassend u.a. Cas (CRISPR-assoziierte Gene) Nuklease und Cpf1 Nukleasen, sind Teil des nunmehr in der Literatur als "CRISPR" bezeichneten Systems (Clustered regularly interspaced short palindromic repeat). Dieses System wurde ursprünglich 1987 bei Analyse des *iap* Gens von *E. coli* identifiziert, wobei natürlich vorkommende Wiederholungssequenzen im bakteriellen Genom identifiziert wurden. Später wurde herausgefunden, dass diese palindromischen DNA-Widerholungssequenzen von 20 bis 50 Nukleotiden einem Muster folgen. Hierauf wurde das Akronym CRISPR geprägt (Jansen, R. et al., "Identification of genes that are associated with DNA repeats in prokaryotes", Mol. Microbiol., 2002, 43(6), 1565-1575), wobei sich die Forschung nach wie vor auf Bakterien fokussierte. Schließlich wurde beschrieben, dass der CRISPR-Lokus eine Art bakterielles Immunsystem darstellt und erworbene Immunität gegen Phagen verleihen kann (Barrangou et al. "CRISPR provides acquired resistance against viruses in procaryotes" Science 2007, 315:1709.1712), indem die invadierende Phagen-DNA zunächst als Protospacer in einen CRISPR Lokus eingebaut, der Lokus dann transkribiert und schließlich der CRISPR-vermittelte Silencing Mechanismus aktiviert wird.

Durch die funktionelle Charakterisierung erfolgte auch die schrittweise Nutzung des Systems als universelles Werkzeug zur Genommodifikation höherer Organismen. Zwischenzeitlich ist eine Vielzahl von CRISPRSystemen beschrieben (siehe etwa Van der Oost et al. "Unravelling the structural and mechanistic basis of CRISPR-Cas systems" Nature 2014, 482:331-338, Makarova et al., "An updated evolutionary classification of CRISPR-Cas systems", Nature Reviews Microbiology 13, 722-736), wobei die Analysen bei weitem noch nicht abgeschlossen sind.

Durch die Entdeckung und Nutzbarmachung des bakteriellen Typ-II CRISPR Systems steht ein weiteres "Genome Editing" System mit großem Potential zur Verfügung.

Fünf Typen (I-V) von CRISPR Systemen wurden bis dato beschrieben (Barrangou et al., 2007, Science, 315(5819):1709-12.; Brouns et al., 2008, Science, 321(5891):960-4.; Marraffini und Sontheimer, 2008, Science, 322(5909):1843-5; Makarova et al., Nature Rev. Microbiol., 13, 722-736, 2015), wobei jedes System ein Cluster von CRISPR-assoziierten (cas oder auch anderen) Genen und einen dazugehörigen CRISPR-Array umfasst. Diese charakteristischen CRISPR Arrays bestehen aus repetitiven Sequenzen (direkte Wiederholungen, sog. Repeats), in die kurze Stücke von nicht-repetitiven Sequenzen ("Spacer") eingelagert sind, wobei die Spacer von kurzen Fragmenten fremden Genmaterials abstammen (Protospacer). Die CRISPR-Arrays werden anschließend in kurze CRISPR RNAs (crRNAs) transkribiert, wobei die crRNAs die Cas Proteine oder andere Effektor-Nukleasen eines CRISPR Systems zu den jeweiligen Ziel-Nukleinsäuremolekülen, die es zu schneiden gilt, mittels Watson-Crick Basenpaarung dirigieren. Die Type I und Typ III CRISPR Systeme verwenden Komplexe aus Cas Proteinen sowie crRNAs, um hierdurch die Erkennung und anschließende Spaltung von Ziel-Nukleinsäure zu erzielen (Wiedenheft et al., 2011, Nature, 477(7365):486-9). Hingegen erkennen und spalten Typ II CRISPR Systeme in ihrer natürlichen Form Ihre Ziel-DNA im Zusammenspiel der RNA-dirigierten Nuklease Cas9 mit zwei nicht-kodierenden RNAs, der crRNA sowie einer trans-aktivierenden RNA (tracrRNA) (Garneau et al., 2010; Sapranauskas et al., 2011, Nucleic Acids Res., 39(21):9275-82; Deltcheva et al., 2011, Nature, 471(7340):602-7). Ein mögliches Typ IV CRISPR System wurde ebenfalls vorgeschlagen (Makarova et al., Biol. Direct, 6 (38), 2011).

Die in natürlichen Systemen durch CRISPR/Cas vermittelte Immunantwort benötigt somit eine sog. CRISPR-RNA (crRNA), wobei die Maturierung dieser Leit-RNA, welche die gezielte Aktivierung der CRISPR Nuklease steuert, zwischen den unterschiedlichen derzeit charakterisierten CRISPR Systemen deutlich variiert. Zunächst wird die invadierende DNA, welche auch "Spacer" genannt wird, zwischen zwei aneinander liegenden Repeat-Regionen am proximalen Ende des CRISPR Lokus integriert. Typ-II CRISPR Systeme kodieren als Schlüsselenzym für den Interferenzschritt eine Cas9-Nuklease, welche sowohl eine crRNA als auch eine trans-aktivierende RNA (tracrRNA) als Leitmotiv benötigt. Diese hybridisieren und bilden doppelsträngige (ds) RNA Regionen, welche von RNAselll erkannt und gespalten werden können, um reife crRNAs zu bilden. Diese wiederum assoziieren dann mit dem Cas-Molekül, um die Nuklease gerichtet zur Nukleinsäure-Zielregion zu führen.

Ein rekombinantes CRISPR/Cas System, oder allgemein ein CRISPR/Nuklease System, erlaubt es, durch eine kleine individuell anpassbare nicht kodierende RNA (guide RNA bzw. gRNA) in Kombination mit einer möglicherweise modifizierten Nuklease eine(n) gezielte(n) DNA Erkennung und/oder Bindung und optional einen Einzel- oder Doppelstrangbruch zu erzeugen. Rekombinante gRNA Moleküle können sowohl die variable DNA-Erkennungs- als auch die Nuklease-Interaktionsregion umfassen und somit abhängig von der konkreten Ziel-Nukleinsäure und der gewünschten Nuklease gezielt entworfen werden (Jinek et. al., 2012, supra). Als weiterer Sicherheitsmechanismus müssen in der Nukleinsäure-Zielregion zudem PAMs (protospacer adjacent motifs), DNA-Sequenzen, welche im Typ II CRISPR System etwa direkt vom Cas9/RNA-Komplex erkannter DNA folgen, vorhanden sein. So lautet die PAM-Sequenz für die Cas9 aus *Streptococcus pyogenes* etwa "NGG" oder "NAG" (Standard IUPAC Nukleotid-Code) (Jinek et al., "A programmable dual-RNA-guided DNA endonuclease in adaptive bacterial immunity", Science 2012, 337: 816-821). Die PAM-Sequenz für Cas9 aus *Staphylococcus aureus* lautet "NNGRRT" oder "NNGRR(N)". Weitere variante CRISPR/Cas9 Systeme sind bekannt. So schneidet eine *Neisseria meningitidis* Cas9 bei der PAM Sequenz NNNNGATT. Eine *Streptococcus thermophilus* Cas9 schneidet bei der PAM-Sequenz NNAGAAW. Durch Einsatz von modifizierten Cas-Polypeptiden lassen sich ferner gezielte Einzelstrangbrüche erzielen. Der kombinierte Einsatz von Cas-Nickasen mit unterschiedlichen rekombinanten gRNAs kann durch doppeltes DNA-Nicking ebenfalls hochgezielte DNA-Doppelstrangbrüche induzieren. Durch den Einsatz von zwei gRNAs kann darüber hinaus die Spezifität der DNA-Bindung und dadurch der DNA-Spaltung optimiert werden.

Zusätzlich zum CRISPR/Cas System wurden jüngst auch sog. CRISPR/Cpf1 Systeme beschrieben, die sich analog zum CRISPR/Cas System als Werkzeuge für die gezielte Genom-Editierung eignen (s. Zetsche et al., "Cpf1 Is a Singel RNA-Guides Endonuclease of a Class 2 CRISPR-Cas System", Cell, 163, pp. 1-13, Oktober 2015). Das CRISPR/Cpf1 System wird auch als Typ V CRISPR System bezeichnet (Makarova et al., Nature Rev. Microbiol., 2015, supra). Anders als etwa eine Cas9 Nuklease eines Typ II CRISPR/Cas Systems benötigt eine Cpf1 Nuklease keine zusätzlichen trans-aktivierenden tracr-RNAs. Cpf1 erkennt T-reiche PAM Sequenzen und schneidet die Ziel-DNA unter Entstehung sog. "sticky ends", d.h. von Überhängen, wohingegen Cas9 sog. "blunt ends" hinterlässt. Wie auch Cas Nukleasen enthalten Cpf1 Nukelasen eine RuvC-ähnliche Endonukleasedomäne, wohingegen ihnen eine zweite HNH Endonukleasedomäne fehlt (Makarova & Koonin, Methods Mol. Biol., 1311, 47-75, 2015). Während Typ I, III und IV CRISPR Systeme derzeit als Klasse 1 Systeme bezeichnet werden, ordnet man Typ II und Typ V Systeme der Klasse 2 zu (vgl. Makarova et al., Nature Rev. Microbiol.,2015, supra).

Ein DNA Doppelstrangbruch innerhalb einer pflanzlichen Zelle wird entweder durch "nonhomologous end joining" (NHEJ) oder "homologe Rekombination ((HR), auch genannt "homology-directed repair" (HDR)) repariert. Darüber hinaus wurden in Pflanzen auch sogenannte Alternative end-joining Wege (AEJ) beschrieben (Charbonnel C, Allain E, Gallego ME, White CI (2011) Kinetic analysis of DNA double-strand break repair pathways in Arabidopsis. DNA Repair (Amst) 10: 611-619).

In US 2015/082478 A1 wird daher etwa vorgeschlagen, einen separaten HDR DNA Reparaturvektor zu verwenden, um einen Doppelstrangbruch, der zuvor durch ein rekombinantes CRISPR/Cas System erzielt wurde, einzuführen.Anders als die genetische Veränderung bakterieller Genome stellt die Modifikation von komplexen eukaryontischen Genomen eine größere Herausforderung dar, da auf Grund der Komplexität dieser Genome molekulare Werkzeuge bereitgestellt werden müssen, welche eine zielgenaue Genommodifikation ohne unerwünschte Off-Target-Effekte, d.h. unerwünschte Mutationen oder Modifikationen innerhalb des Genoms oder der nicht genomischen DNA der Zielzelle, bewirken.

Aus US 8,697,359 B1 ist bekannt, dass unter Einsatz der CRISPR/Cas Technologie eukaryontische Genome, speziell Säugergenome und diese bevorzugt zu therapeutischen Zwecken, verändert werden können. Hierbei wird die Expression eines Zielgens durch gezielte Einführung einer Cas9 Endonuklase sowie einer guide RNA (gRNA) programmierbar unterdrückt. Diese gRNA ist ein wesentliches Element eines jeden Cas9-CRISPR-Systems, da sie die eigentliche Cas-Nuklease gerichtet zur (genomischen) Ziel-DNA leitet. Hierzu wird für Cas9-CRISPR-Systeme zudem eine tracr (trans-activating CRISPR RNA) Sequenz und eine tracr mate Sequenz offenbart, welche von der gRNA umfasst sein kann, wobei die tracr Sequenzen hybridisieren, um so erkannt zu werden. Der Einsatz der CRISPR-Technologie zur Modifikation von komplexen pflanzlichen Genomen sowie dafür erforderliche molekulare Werkzeuge werden jedoch nicht offenbart.

WO 2015/026885 A1 hingegen widmet sich speziell der Anwendung der CRISPR/Cas Technologie in Pflanzen. Jedoch wird/werden hier ausschließlich eine Herangehensweise und geeignete molekulare Werkzeuge offenbart, welche zwingend die Subkultivierung, Selektion und Regeneration von Pflanzenkalli nach erfolgter Einführung der CRISPR/Cas Werkzeuge benötigen und somit nicht die direkte Gewinnung einer Pflanze oder von Pflanzenmaterial, enthaltend die gewünschte DNA-Veränderung ermöglicht, welche/welches die gewünschte DNA-Veränderung enthält.

Eine Übersicht zum aktuellen Status der Entwicklung des Einsatzes der CRISPR/Cas Technologie zur Genom Editierung von Pflanzengenomen findet sich in Bortesi & Fischer ("The CRISPR/Cas9 system for plant genome editing and beyond", Biotechnology Advances, 33, Seiten 41-52, 20.12.2014). Hier wird u.a. über die Problematik der Bereitstellung spezifischer gRNAs für das Targeting in Mais berichtet. Ferner wird die Problematik der hohen "Off-target" Mutationsraten thematisiert, welche nicht nur beim Einsatz von CRISPR/Cas bei Säugerzellen, sondern auch beim Einsatz in Pflanzenzellen beobachtet wird, wobei hier das Design der einzelnen CRISPR/Cas Werkzeuge entscheidend ist, um in der jeweiligen Zielzelle/dem jeweiligen Zielorganismus eine ortsgerichtete gezielte Veränderung ohne "Off-target" Wirkungen zu erzielen. Ferner erkennt Bortesi & Fischer, dass das CRISPR/Cas System auch zur epigenetischen Veränderung von DNA eingesetzt werden kann, da das CRISPR/Cas System auch zur Spaltung methylierter DNA verwendet werden kann, stellt jedoch fest, dass hierzu noch keine Anwendungen in Pflanzen bekannt sind.

Weiterhin beschreibt Guilinger *et al.* Fokl-Nukleasen, welche wie Nickasen verwendet werden, und damit eine höhere Spezifität erzeugen (Guilinger et al.; Fusion of catalytically inactive Cas9 to Fokl nuclease improves the specificity of genome modification, doi:10.1038/nbt.2909). Guilinger et al. präsentieren jedoch ausschließlich Daten für humane Zellen, nicht für Pflanzenzellen.

Mali *et al* 2013 bezieht sich auf die Verwendung des CRISPR/Cas Systems in humanen Zellen, wobei hier Nuklease-Null Varianten von Cas9 oder Aptamer-gekoppelte gRNAs zum Einsatz kommen, welche an transkriptionelle Aktivatordomänen fusioniert sein können (Mali, P., et al. (2013). "CAS9 transcriptional activators for target specificity screening and paired nickases for cooperative genome engineering). Mali *et al.* erwähnt jedoch nicht, wie und in welchem Umfang ein entsprechendes System in Pflanzenzellen zum Einsatz kommen könnte.

Zusammenfassend sind viele der heute verwendeten Ansätze im pflanzlichen Bereich nur temporär und/oder räumlich begrenzt (transient) dahingehend, dass Mutationen in bereits ausdifferenzierten Zellen, z.B. in Blättern, bewirkt werden, diese Mutationen jedoch nicht über die Keimbahn weitervererbt werden. Des Weiteren gibt es Ansätze, die eine stabile Integration der kodierenden Sequenzen inklusive der hierfür erforderlichen regulatorischen Sequenzen wie Promotoren und Terminatoren ins Genom voraussetzen, über diese dann eine stabile Mutation erzeugt werden kann, die von Generation zu Generation vererbt wird. Hierbei sind jedoch die CRISPR/Cas Werkzeuge nach wie vor im Genom der Pflanze und dadurch auch potentieller Pflanzenprodukte wie Früchte und Samen enthalten, was bezüglich der Risikobewertung der entsprechenden Produkte unerwünscht ist.

Demnach ist der CRISPR/Cas Ansatz in der Pflanzenbiotechnologie nach wie vor von einer niedrigen Effizienz, aber auch einem schwierigen und aufwendigen Design der Konstrukte gekennzeichnet, sowie dem häufigen Auftreten von Off-Target Effekten. Zudem basieren viele der derzeitigen Ansätze darauf, die CRISPR/Cas Werkzeuge entweder stabil in das Genom einer Pflanzenzelle zu integrieren oder in Zellen eines ausdifferenzierten Gewebes, etwa in Blätter, einzuführen. Folglich werden bei dem stabilen Ansatz zum einen auch die einzelnen Werkzeuge wie beispielsweise die Cas9, und nicht nur die von ihnen bewirkten gezielten DNA-Veränderungen, an die Nachkommen vererbt. Bei der Transformation in ausdifferenzierte Zellen und Gewebe wird die durch die CRISPR/Cas Werkzeuge eingeführte Mutation nur in den jeweiligen Zellen wirksam, kann jedoch nicht über die Keimbahn weitervererbt werden. Gerade im Hinblick auf die gezielte Entwicklung von positiven Merkmalen in Pflanzen, umfassend Resistenzen, insbesondere gegenüber Schädlingen und Umwelteinflüssen, wie z.B. Kälte, Trockenheit, Salzgehalt, einem erhöhten Ertrag, oder Herbizidresistenzen, ist die Bereitstellung verlässlicher Verfahren zur gezielten An- und Abschaltung oder zur Modifikation von genomischem Material, wie auch zum Silencing von RNA innerhalb einer pflanzlichen Zelle von großem wirtschaftlichen Interesse.

Im Hinblick auf die Auswahl geeigneter gRNAs existieren zwar bereits erste *in silico* Werkzeuge, welche es erlauben, geeignete gRNAs zu identifizieren und danach herzustellen (siehe etwa: https://www.dna20.com/eCommerce/cas9/input), jedoch gibt es derzeit keine spezifischen Tools, welche für die Anwendung in wichtigen Nutzpflanzen, welche stets über komplexe Genome verfügen, einsetzbar wären. Ferner sagen die verfügbaren Tools nichts über die tatsächliche Effektivität einer *in silico* bestimmten gRNA im anschließenden Test *in vitro* oder *in vivo* innerhalb einer pflanzlichen Zelle aus.

Es besteht somit ein anhaltendes Bedürfnis nach der Etablierung von transienten und zudem optional induzierbaren Verfahren und Konstrukten, basierend u.a. auf gRNAs und CRISPR Nukleasen oder gRNAs und anderen Effektor-Domänen, zur Erwirkung einer gewünschten Veränderung einer Zielsequenz in einer pflanzlichen Zielzelle, wobei nur die Veränderung der Zielsequenz, jedoch nicht die Konstrukte an die Nachkommen weitergegeben werden. Zudem besteht ein großes Bedürfnis danach, ein CRISPR-basiertes Verfahren bereitzustellen, das die Möglichkeit eröffnet, direkt eine Keimbahnveränderung in einer pflanzlichen Zelle oder einem pflanzlichen Gewebe durchzuführen, sodass die Veränderung vererbbar ist und an der Pflanze, die aus der veränderten Pflanzenzelle oder dem Gewebe resultiert, unmittelbar Saatgut geerntet werden kann, welches die gezielte Genommodifikation enthält, ohne aufwendige und kostspielige Zwischenschritte beschreiten zu müssen. Schließlich besteht ein Bedürfnis, das RNA-dirigierte DNA-Modifikationssystem, welches durch die CRISPR/Cas Werkzeuge bereitgestellt wird, gezielt zu erweitern, indem nicht nur genomische Zielstrukturen, sondern jegliche Nukleinsäure als Zielstruktur, nicht nur im Genom einer Zelle, sondern etwa auch im Zytosol oder in Plastiden, kontrolliert modifiziert werden kann. Hierzu existiert derzeit zudem ein Bedürfnis, geeignete Einbringungssysteme bereitzustellen, welche die gezielte Einbringung der CRISPR/Cas Werkzeuge und dadurch die gezielte Modifikation einer Zielregion innerhalb einer pflanzlichen Zielstruktur erlauben.

Weiterhin besteht ein Bedürfnis nach effizienten *in vitro* Screening Verfahren, welche es ermöglichen, die Effektivität einer gRNA innerhalb einer pflanzlichen Zelle in einem *in vitro* Assay im hohen Durchsatz zu überprüfen und verlässlich vorherzusagen, um dadurch kostspielige und langwierige Ansätze mit pflanzlichem Material zu vermeiden.

Schließlich gilt es, insbesondere bei der Modifikation großer eukaryontischer Genome, umfassend Pflanzengenome und Genome von tierischen Organismen, die Präzision eines Genom Editierungsansatzes zu optimieren, um geringere Off-Target Effekte zu erzielen und idealerweise eine optimale Reparatur eines gezielt eingeführten Doppelstrangbruches durch Bereitstellung einer Reparatur-Matrize zusammen mit den eigentlichen Genom-Modifikationswerkzeugen zu erzielen.

### Zusammenfassung der Erfindung

Der vorliegenden Erfindung liegt daher die Aufgabe zu Grunde, Verfahren und molekulare Werkzeuge bereitzustellen, welche die transiente Transformation von pflanzlichem Gewebe oder von pflanzlichen Zellen, speziell von meristematischen Zellen, und dadurch die kontrollierte Veränderung einer beliebigen Nukleinsäure-Zielregion in einem beliebigen pflanzlichen zellulären Kompartiment zu erlauben. Ebenso war es eine Aufgabe, geeignete Einbringungsverfahren für die molekularen Werkzeuge gemäß der vorliegenden Offenbarung bereitzustellen. Zudem sollten geeignete *in vitro* Screening Assays bzw. Tests bereitgestellt werden, sodass geeignete funktionale gRNAs vorab bereits *in vitro* identifiziert werden können, um den anschließenden Aufwand *in planta* oder *in vitro* in pflanzlichem Gewebe effizient reduzieren zu können.

Es war Ziel, die oben genannten Vorteile in einem System, welches breit in der Pflanzenbiotechnologie anwendbar ist, zu vereinigen.

Insbesondere galt es, Werkzeuge bereitzustellen, die die Pflanzenzüchtung diverser mono- und dikotyler Pflanzen erleichtern, sodass innerhalb kurzer Zeit Merkmale von Interesse in ein pflanzliches Genom eingebracht oder ebenso entfernt oder modifiziert werden können.

Diese Aufgabe wird durch die hierin bereitgestellten Verfahren gemäß dem Gegenstand der beigefügten Ansprüche und ferner wie hierin in der Beschreibung, den Figuren und dem beigefügten Sequenzprotokoll detailliert dargelegt, gelöst. Die Aufgabe wird speziell durch die Bereitstellung eines Verfahrens umfassend die Transformation oder Transfektion von mindestens einer meristematischen Zelle gelöst. Darüber hinaus wird die Aufgabe durch die Bereitstellung von rekombinanten Konstrukten gelöst, welche gezielt modifizierte CRISPR-Werkzeuge und/oder weitere Effektor-Domänen umfassen. Schließlich wird die Aufgabe durch die Bereitstellung von geeigneten regulatorischen Sequenzen und Lokalisationssequenzen gelöst, welche es erlauben, das rekombinante Konstrukt von Interesse kontrolliert in ein beliebig ansteuerbares Kompartiment einer pflanzlichen Zielstruktur von Interesse zu dirigieren.

Hierdurch kann mindestens eine gezielte Veränderung in jeglicher Nukleinsäure-Zielregion in einem beliebigen Kompartiment einer pflanzlichen Zelle, insbesondere einer meristematischen pflanzlichen Zelle, erzielt werden. Da die so veränderte mindestens eine meristematische Zelle die gezielte Veränderung in der Nukleinsäure-Zielregion durch die anschließende Zellteilung und Differenzierung unmittelbar und direkt an ihre Nachkommen weitergeben kann und/oder sie das Potential besitzt, dass aus der meristematischen Zelle ein ganzer veränderter pflanzlicher Organismus heranreift, kann so die Bereitstellung einer Pflanze oder von Pflanzenmaterial oder einer pflanzlichen Zelle erreicht werden, ohne komplexe weitere Kultivierungs- oder Kreuzungs- und Selektionsschritte (vor allem komplexe Rückkreuzungsregime) einschlagen zu müssen. Vielmehr kann aus der mindestens einen so veränderten meristematischen Zielzelle unmittelbar und direkt eine Pflanze, Pflanzenmaterial oder eine pflanzliche Zelle gewonnen werden. Hierdurch ist es möglich, gRNA(s) und/oder CRISPR Nuklease(n) oder ein oder mehrere katalytisch aktive(s) Fragment(e) davon und/oder weitere Effektor-Domäne(n) nur transient in den Meristemen zu produzieren bzw. bereitzustellen bzw. aktivieren, wobei diese rekombinanten Makromoleküle vorzugsweise anschließend, d.h. nachdem gRNA(s) und/oder CRISPR Nuklease(n) oder ein oder mehrere katalytisch aktive(s) Fragment(e) davon und/oder weitere Effektor-Domäne(n)die gewünschte Wirkung erfüllt haben, degradiert werden, da keine Integration davon ins Genom oder endogene extrachromosomale DNA stattfindet, was auf Grund regulatorischer Aspekte und der Risikobewertung des Pflanzenprodukts vorteilhaft sein kann. Die hierin verwendeten CRISPR Nukleasen oder katalytisch aktiven Fragmente davon können auch eine oder mehrere Mutation(en) in der katalytischen Domäne, welche für die DNA-(Doppel- bzw. Einzelstrang)spaltung verantwortlich ist, enthalten. Dies resultiert in einem breiten Anwendungsspektrum der CRISPR Nuklease oder des katalytisch aktiven Fragments davon und, etwa im Fall von Cas-basierten Nickasen, auf einer höheren Spezifität der Bindung, da zwei CRISPR/Cas Konstrukte zum Einsatz kommen, um beide Einzelstränge des DNA-Doppelstrangs an der gewünschten Stelle zu spalten. Auch Cas-null Varianten werden hierin vorgeschlagen sowie deren kombinierter Einsatz mit weiteren Effektor-Domänen zur Optimierung einer gezielten Nukleinsäure-Editierung.

Darüber hinaus wurde gefunden, dass durch die Ausnutzung des Wirkmechanismus der CRISPR-Werkzeuge auch weitere Effektor-Domänen wie DNA- bzw. RNA- bzw. Histonmodifizierende oder DNA- bzw. RNA- bzw. Histon-bindende Polypeptide oder Nukleinsäuren, umfassend zum Beispiel alle Arten von monomeren, dimeren oder multimeren Nukleasen, umfassend Nickasen, Aktivatoren und Repressoren der Transkription, Phosphatasen, Glykosylasen, oder Enzyme, welche epigenetische Veränderungen bewirken können, wie Methylasen, Acetylasen, Methyltransferasen oder Histon-Deacetylasen, Aptamere, umfassend einzelsträngige DNA- oder RNA-Sequenzen sowie Peptide, fluoreszierende Proteine, Proteine zur Biolumineszenz, Markernukleinsäure- oder Markeraminosäuresequenzen, und dergleichen, und Kombinationen davon gemäß den hierin bereitgestellten Verfahren eingesetzt werden können, wodurch sich das Spektrum der bekannten gezielten Genom-Editierung hin zu einer allgemeinen Nukleinsäure-Editierung, die nicht auf genomische DNA *per se* beschränkt ist, verbreitern lässt.

Im Hinblick auf den hierein offenbarten Aspekt der Editierung von genomischer DNA ist auch die Bereitstellung einer DNA-Reparatur-Matrize oder HDR Matrize vorgesehen, die gezielt zusammen mit den CRISPR Werkzeugen in eine Zielzelle oder Zielstruktur von Interesse eingebracht werden kann, um hierdurch zum einen das fehleranfällige endogene NHEJ Reparatursystem auszukompetitieren und darüber hinaus eine gewünschte Nukleinsäure an den Ort eines Doppelstrangbruches einführen zu können. Die Begriffe DNA-Reparatur-Matrize und HDR Matrize werden hierein austauschbar verwendet.

Die vorliegende Offenbarung bietet somit die Möglichkeit, den CRISPR/Cas-Mechanismus dahingehend auszuweiten, dass nicht nur die nukleolytische Spaltung von DNA, sondern auch eine beliebige Modifikation von genomischer DNA, z.B. die epigenetische Veränderung, sowie von RNA in pflanzlichen Zellen (wie beispielsweise mRNA) ermöglicht wird.

Durch den Einsatz weiterer regulatorischer Sequenzen, umfassend Promotoren, Teminatoren, Transkriptionsfaktorbindestellen oder Introns, und/oder von Lokalisationssequenzen, umfassend Kern-, Mitochondrien- und Plastidenlokalisationssequenzen, stellt die vorliegende Offenbarung zudem die Möglichkeit bereit, jegliche Nukleinsäure-Zielregion einer pflanzlichen Zielstruktur gezielt zu modifizieren, wodurch zum Beispiel auch mitochondriale und Plastiden-DNA als Ziel der Editierung fungieren kann. Darüber hinaus eröffnet sich auch die Möglichkeit der gezielten Veränderung von RNA, z.B. von mRNA, wobei auch hier die gRNA-dirigierte Sequenzerkennung, welche dem CRISPR/Cas-System zu Grunde liegt, ausgenutzt und gemäß der vorliegenden Offenbarung "umprogrammiert" wird, um den Anwendungsbereich der CRISPR/Cas-Technologie zu erweitern.

Hierin werden auch Verfahren und Konstrukte bereitgestellt, bei welchen gRNA und/oder CRISPR Nuklease oder das katalytisch aktive Fragment davon bereits verknüpft mit einer weiteren Effektor-Domäne auf einem rekombinanten Konstrukt bereitgestellt werden.

Weiterhin wird ein Verfahren bereitgestellt, bei welchem die mindestens eine gRNA sowie die mindestens eine CRISPR Nuklease oder das katalytisch aktive Fragment davon und/oder die mindestens eine weitere Effektor-Domäne separat auf unterschiedlichen rekombinanten Konstrukten bereitgestellt werden. Gemäß dieses Verfahrens kann die gRNA-Komponente als DNA oder RNA bereitgestellt werden, die CRISPR Nuklease oder Variante davon oder das katalytisch aktive Fragment davon kann als DNA oder RNA oder als Polypeptidsequenz bereitgestellt werden und die Effektor-Domäne kann als DNA oder RNA oder als Polypeptidsequenz bereitgestellt werden.

Zudem war es eine Aufgabe, durch die Etablierung pflanzenspezifischer Konstrukte und Verfahren die Möglichkeit bereitzustellen, die Konstrukte gezielt, etwa induzierbar oder gewebe- oder organellspezifisch, bereitzustellen und unerwünschte Off-Target Effekte zu minimieren. Schließlich stellte es eine Aufgabe dar, Verfahren und Konstrukte zu entwerfen, die nicht nur die Möglichkeit effizienter Gen-Knock-Ins, sondern beispielweise auch gerade die Möglichkeit spezifischer Gen-Knock-Outs, von Insertionen von genetischen Fragmenten, spezifischer epigenetischer Modifikationen, der Einführung von Punktmutationen, Acetylierungen, Methylierungen, Phosphorylierung, Glykosylierungen, Markierungen durch Resistenzmarker oder fluoreszierende Proteine, Aktivierung oder Reprimierung der Transkription, gezielter Spaltung von doppelsträngiger oder einzelsträngiger Nukleinsäuren, der Bindung von Nukleinsäuren und dergleichen bieten, sodass ein breiter Einsatzbereich in der Pflanzenzüchtung eröffnet wird. Aus züchterischer wie regulatorischer Sicht ist es wünschenswert, sowohl eine stabile Vererbbarkeit eines durch die Modifikation bewirkten Merkmals über mindestens eine Generation hinweg bei gleichzeitiger Abwesenheit der hierfür erforderlichen Konstrukte des CRISPR/Cas-Systems in der resultierenden Pflanze oder den resultierenden pflanzlichen Zellen zu ermöglichen.

Schließlich war es eine Aufgabe, ein *in vitro* Screening Verfahren zur Identifizierung einer gRNA oder einer für eine gRNA kodierende Sequenz in einem *in vitro* Assay, zur Identifizierung einer gRNA oder einer für eine gRNA kodierende Sequenz, die geeignet ist, zusammen mit einer CRISPR Nuklease oder einem katalytisch aktiven Fragment davon eine Nukleinsäure-Zielregion in einer pflanzlichen Zelle gezielt zu modifizieren.

Konkrete Aspekte und Ausführungsformen der vorliegenden Erfindung ergeben sich aus der folgenden detaillierten Beschreibung und den Beispielen, den Figuren, dem Sequenzprotokoll sowie insbesondere den beigefügten Patentansprüchen.

### Kurze Beschreibung der Zeichnungen

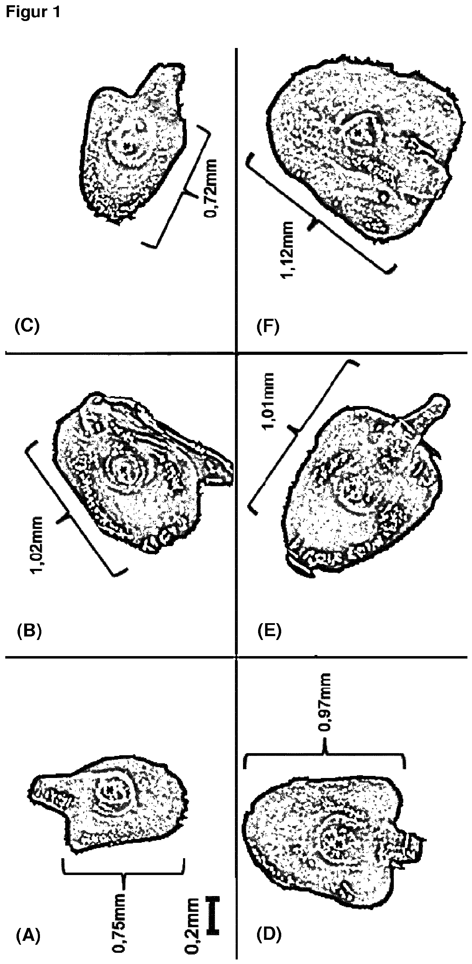

Figur 1 A-F (Fig. 1 A-F) zeigen Mais-Embryonen von unterschiedlicher Größe. In den hier analysierten Embryonen ist das Meristem eindeutig als scheibenförmige Struktur im Zentrum des Embryos zu erkennen. Abhängig von der Größe und des Entwicklungsstadiums der Embryonen ist das Meristem unterschiedlich entwickelt und unterschiedlich gut erkennbar. Das Meristem ist zusätzlich noch durch einen Stern (*) markiert.

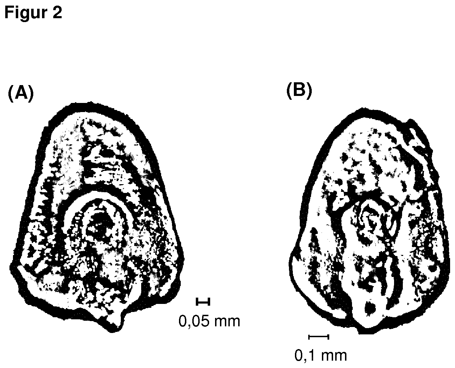

Figur 2 A und B (Fig. 2 A und B) zeigen einen direkten Vergleich der Meristeme eines 0,5 mm großen (A) und eines 1 mm großen (B) Mais-Embryos. In beiden Fällen ist das Meristem als scheibenförmige Struktur im Zentrum des Embryos zu erkennen, allerdings ist in dem 1 mm großen Embryo das Meristem bereits sehr stark von Blattgewebe umgeben. Dies erschwert die Zugänglichkeit des Meristems, so dass kleinere Embryonen mit einem exponierteren Meristem zu bevorzugen sind.

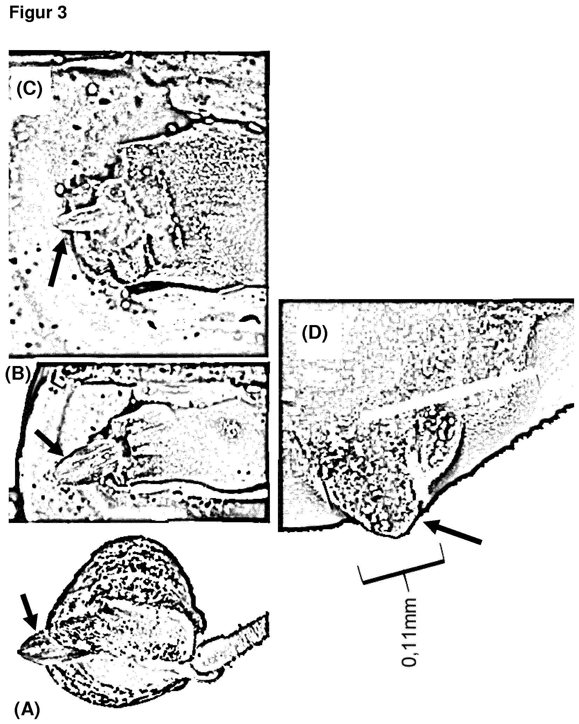

Figur 3 A-D (Fig. 3 A-D) geben präparierte Meristeme in Mais-Keimlingen wieder. Da das Meristem in Keimlingen komplett von Blättern umgeben ist, muss es freipräpariert werden, um für einen Beschuss, Mikroinjektion etc. zugänglich zu sein. Dazu werden die äußeren Gewebestrukturen vollständig entfernt, so dass das Meristem (Pfeile) frei liegt.

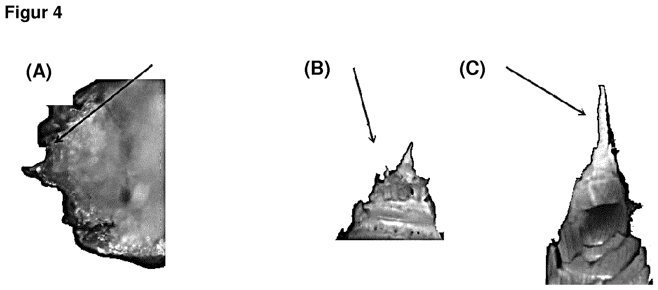

Figur 4 A-C (Fig. 4 A-C) zeigen präparierte Meristeme in älteren Maispflanzen. Da das Meristem in älteren Pflanzen, wie auch bei Keimlingen komplett von Blättern umgeben ist, muss es präpariert werden, um für einen Beschuss, Mikroinjektion etc. zugänglich zu sein. Dazu werden die äußeren Blätter vollständig entfernt, so dass das Meristem (Pfeile) frei liegt.

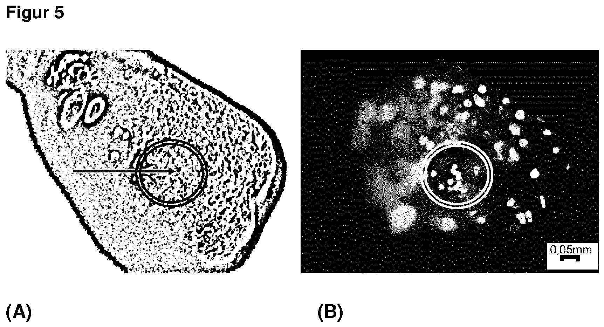

Figur 5 A und B (Fig. 5 A und B) zeigt den biolistischen Testbeschuss der Mais-Embryo-Meristeme. In Figur 5 (A) ist ein schematisches Bild eines Embryos mit der scheibenförmigen Meristemstruktur (hervorgehoben durch einen doppelten Ring und einen Pfeil) wiedergegeben. Figur 5 (B) zeigt die Fluoreszenz (weiße Regionen in der schwarz/weiß Abbildung) nach Testbeschuss. Für den Testbeschuss wurde ein für ein fluoreszierendes Protein kodierendes Gen verwendet. Es ist eine eindeutige Expression des Proteins in den meristematischen Regionen (doppelter Ring) detektierbar.

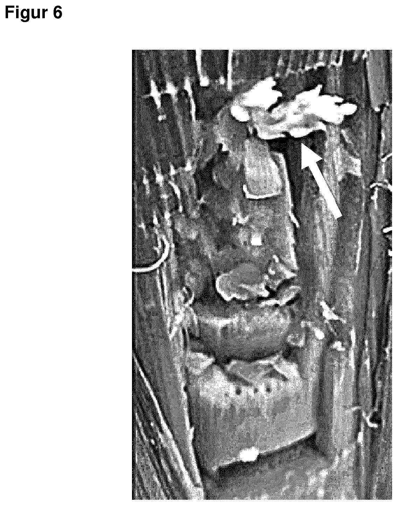

Figur 6 verdeutlicht die Präparation von "Tassel"-Meristemen an adulten Maispflanzen. Durch eine fensterartige Öffnung werden die Meristeme (Pfeil) halbseitig freigelegt. Die rekombinanten Konstrukte können dann z.B. über Bombardment oder Mikroinjektion und dergleichen eingebracht werden. Vorteilhaft ist, dass die Pflanze nicht stark geschädigt wird und die Meristeme nicht vollständig frei liegen (etwa 1-2 Tage später ist das "Tassel-Meristem" bereits nicht mehr durch die Öffnung zu sehen, da es weiter nach oben geschoben wird) und dadurch eine Oxidation und weiterer Schaden reduziert wird.

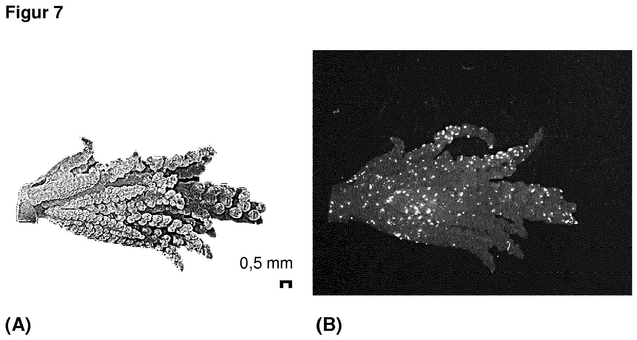

Figur 7 A und B (Fig. 7 A und B) zeigt den biolistischen Testbeschuss eines freigelegten "Tassel"-Meristems aus Mais. In Figur 7 (A) ist ein schematisches Bild der Meristemgewebe der Maisfahne ("Tassel") wiedergegeben. Figur 7 (B) zeigt die Fluoreszenz (weiße Regionen in der schwarz/weiß Abbildung) nach Testbeschuss. Für den Testbeschuss wurde ein für ein fluoreszierendes Protein kodierendes Gen verwendet. Es ist eine eindeutige Expression des Proteins in den meristematischen Geweben detektierbar.

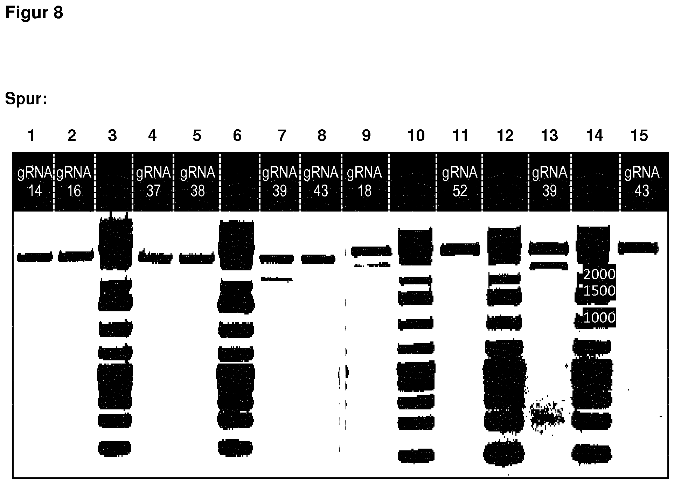

Figur 8 zeigt das Ergebnis eines *in vitro* Assays zur Beurteilung der Effizienz einer gRNA von Interesse. Ausgangspflanze ist hier eine Maispflanze, das Zielgen das hmg13-Gen (HMG-transcription factor 13; GRMZM2G066528). Die Figur stellt das Ergebnis einer Auftrennung in einem 1 %igen Gel mit dem Standardlaufparameter 100 V und Visualisierung über Ethidiumbromid-vermittelter Fluoreszenz dar. In Spuren 3, 6 10, 12 und 14 befindet sich der molekulare Größen-Marker (Angabe in Basenpaaren; GeneRuler 1kb plus DNA Ladder (Thermo Fisher Scientific Inc, USA; SM1331) 20000, 10000, 7000, 5000, 4000, 3000, 2000, 1500, 1000, 700, 500, 400, 300, 200, 75 bp). In den anderen Spuren sind von links nach rechts sind die Ergebnisse für die gRNA 14 (SEQ ID NO:41), die gRNA 16 (SEQ ID NO:42), molekularer Marker, gRNA 37 (SEQ ID NO:43), gRNA 38 (SEQ ID NO: 44), molekularer Marker, gRNA 39 (SEQ ID NO: 45), gRNA 43 (SEQ ID NO: 46), gRNA 18 (SEQ ID NO: 47), molekularer Marker, gRNA 52 (SEQ ID NO: 48), molekularer Marker, gRNA 39 (SEQ ID NO: 45) und gRNA 43 (SEQ ID NO: 46) gezeigt. Angegebene SEQ ID NOs geben bei den gRNAs jeweils den individuell unterschiedlichen Protospacer-Bereich an, der übrige Bereich der gRNA ist bei allen hier verwendeten gRNAs identisch.

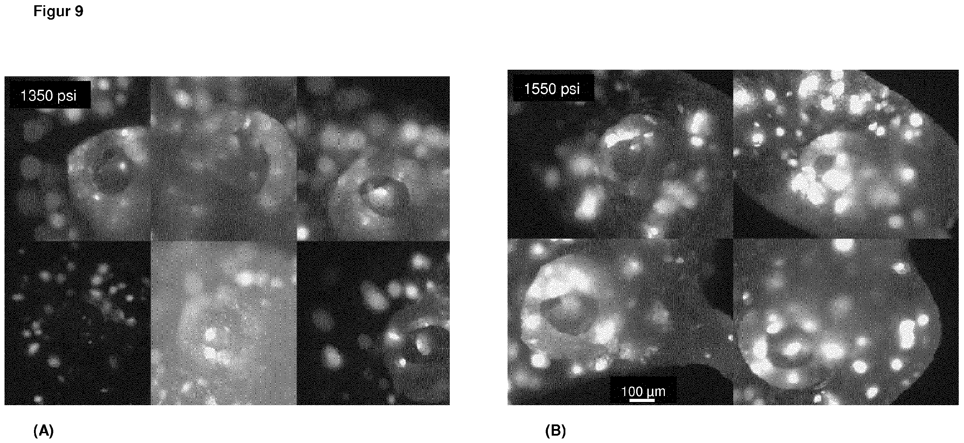

Figur 9 A und B (Fig. 9 A und B) zeigt das Ergebnis eines Testbeschusses von Mais-Embryonen unter Verwendung unterschiedlicher Drücke (angegeben in psi=pound per square inch). Die Maisembryonen wurden 7-10 Tage nach Pollination beschossen und nach weiteren 2 Tagen wurden die mikroskopischen Analysen durchgeführt. Als Plasmid wurde ein Expressionsvektor verwendet, welcher u.a. einen Fluoreszenzmarker kodiert. Figur 9 (A) zeigt in den sechs Einzelabbildungen den Beschuss mit 1350 psi. Figur 9 (B) zeigt in den vier Einzelabbildungen den Beschuss mit 1550psi. Im Vergleich sieht man in den unteren Abbildungen deutlich mehr Fluoreszenz, hier in der schwarz-weiß Abbildung entsprechend einer intensiveren Helligkeit. Eine erhöhte Fluoreszenz/Helligkeit, d.h. eine erhöhte Effizienz der Einbringung, kann jedoch mit einer reduzierten Keimung der Embryonen einhergehen.

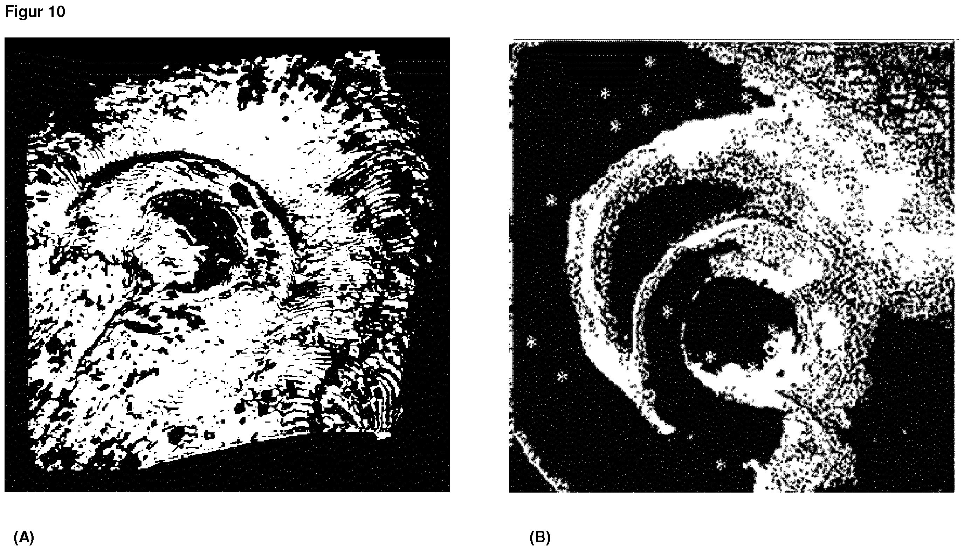

Figur 10 A und B (Fig. 10 A und B) zeigt zwei Ansichten eines Mais-Embryos sowie darin lokalisiertes meristematisches Gewebe. Initial wurden diese Daten durch einen Fluoreszenzmarker visualisiert. Die Akkumulation an Fluoreszenz im ursprünglichen Assay ist in Figur 10 in (A) auch (B) Abbildung durch Sterne gekennzeichnet. Figur 10 (A) ist die Aufsicht auf den Embryo, Figur 10 (B) zeigt eine tiefere Schicht, in der auch im meristematischen Bereich die Fluoreszenz detektierbar ist (markiert mit Sternen). Aufgenommen wurden die Bilder mit einem Laser-Scanning-Mikroskop, der verwendete Vektor für den Beschuss war ein Expressionsvektor, welcher u.a. ein fluoreszierendes Protein kodiert. Die Embryoschichten wurden mit einem geeigneten Farbstoff gefärbt.

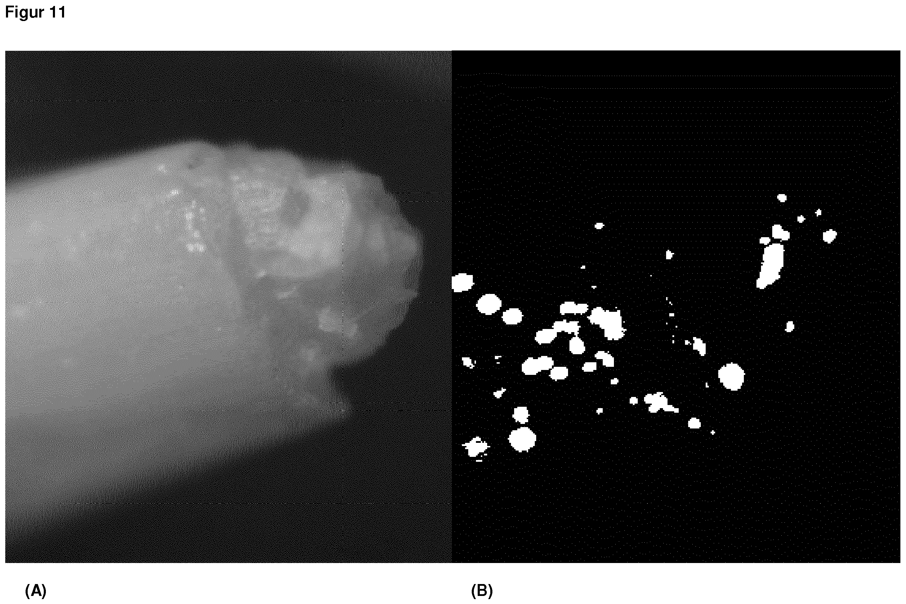

Figur 11 A und B (Fig. 11 A und B) zeigt den horizontalen Beschuss der frei präparierten Meristeme in älteren Maispflanzen (5-10 Tage alte Keimlinge) gemäß Beispiel 3. Da das Meristem in älteren Pflanzen, wie auch bei Keimlingen komplett von Blättern umgeben ist, musste es präpariert werden, um für einen Beschuss etc. zugänglich zu sein. Dazu wurden die äußeren Blätter vollständig entfernt. Aufgenommen wurden die Bilder einen Tag nach Beschuss mit einem Laser-Scanning-Mikroskop, der verwendete Vektor für den Beschuss war ein Fluoreszenzprotein kodierender Expressionsvektor. Figur 11 (A) zeigt eine mikroskopische Aufnahme des präparierten Meristems in der Seitenansicht. Figur 11 (B) zeigt die detektierte Fluoreszenz in dieser Seitenansicht (weiße Punkte). Die Embryoschichten wurden mit einem geeigneten Farbstoff gefärbt.

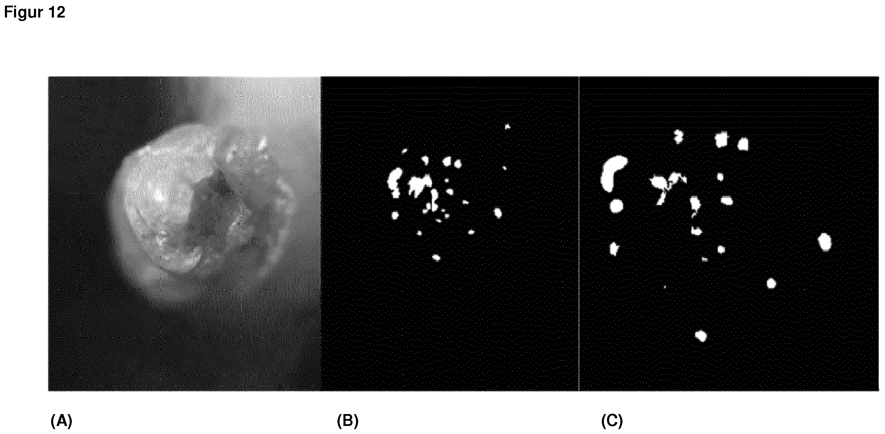

Figur 12 A-C (Fig. 12 A-C) zeigt den vertikalen Beschuss der frei präparierten Meristeme in älteren Maispflanzen (5-10 Tage alte Keimlinge) gemäß Beispiel 3. Da das Meristem in älteren Pflanzen, wie auch bei Keimlingen komplett von Blättern umgeben ist, musste es präpariert werden, um für einen Beschuss etc. zugänglich zu sein. Dazu wurden die äußeren Blätter vollständig entfernt. Aufgenommen wurden die Bilder einen Tag nach Beschuss mit einem Laser-Scanning-Mikroskop, der verwendete Vektor für den Beschuss war Fluoreszenzprotein kodierenden Expressionsvektor. Figur 12 (A) zeigt eine mikroskopische Aufnahme des präparierten Meristems in der Aufsicht. Figur 12 (B) zeigt die detektierte Fluoreszenz in dieser Aufsicht (weiße Punkte). Figur 12 (C) zeigt den Bereich der detektierten Fluoreszenz in etwa 2-facher Vergrößerung. Die Embryoschichten wurden mit einem geeigneten Farbstoff gefärbt.

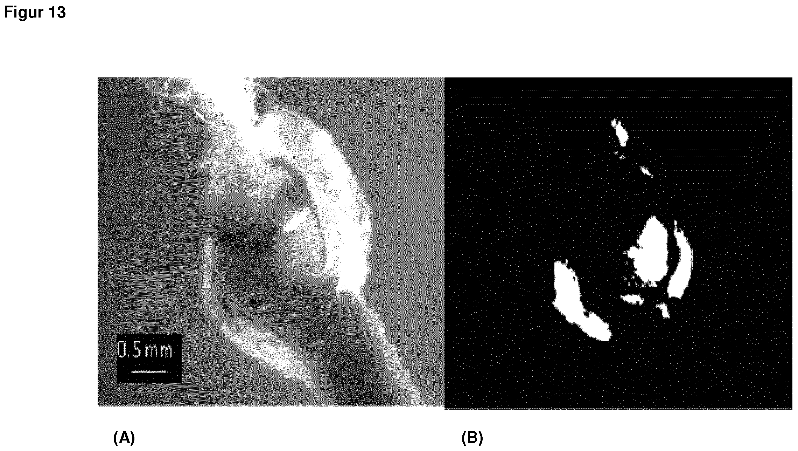

Figur 13 A und B (Fig. 13 A und B) zeigt einen auskeimenden Embryo mit transienter Fluoreszenz, auch in den meristematischen Bereichen. In Figur 13 (A) ist die embryonale Zielstruktur zuerkennen, in Figur 13 (B) dieselbe Zielstruktur, worin die Fluoreszenz des eingebrachten Markes mittels Fluoreszenzmikroskopie visualisiert wurde. Die Fluoreszenz wurde hier durch Einbringung eines geeigneten Fluoreszenzmarkers erzielt. In der Abbildung in schwarz-weiß entsprechen die weißen Regionen in Figur 13 (B) den Regionen, in denen Fluoreszenz nachgewiesen wurde.

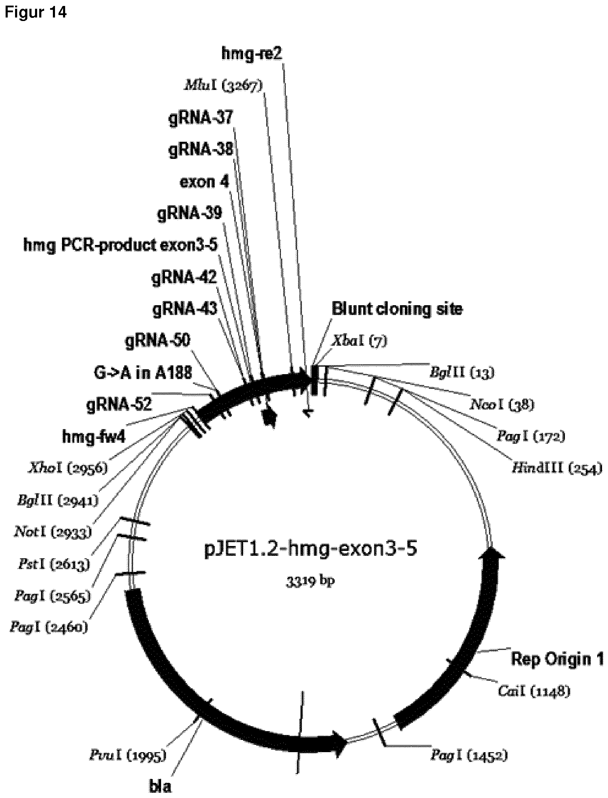

Figur 14 zeigt eine exemplarische Vektorkarte des Plasmids pJET1.2-hmg-exon3-5 gemäß Beispiel 1.

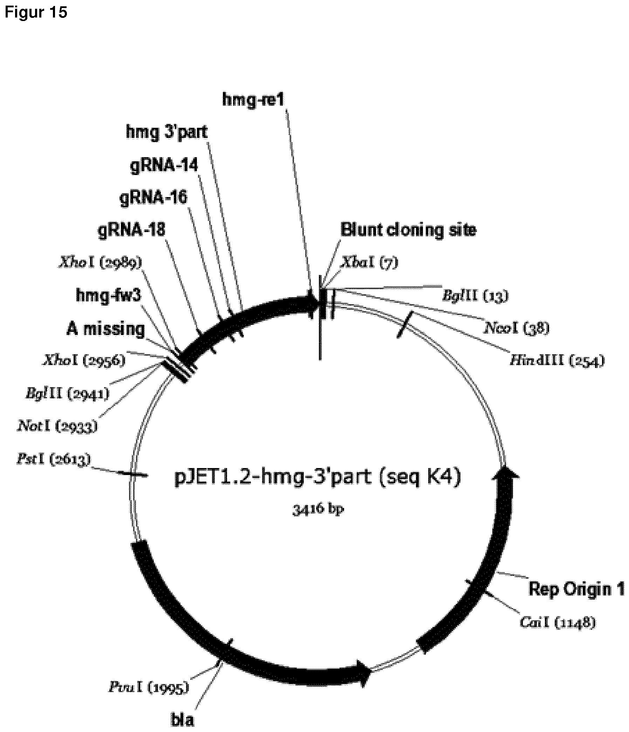

Figur 15 zeigt eine exemplarische Vektorkarte des Plasmids pJET1.2-hmg-3'part/Teil gemäß Beispiel 1.

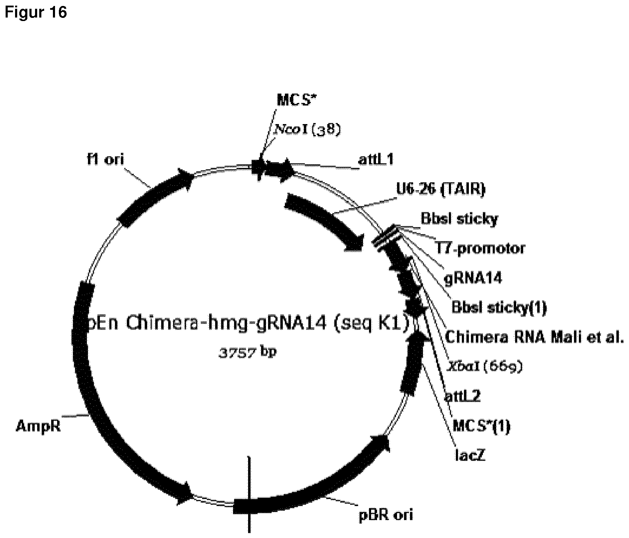

Figur 16 zeigt eine exemplarische Vektorkarte des Plasmids pEn Chimera-hmg-gRNA14 gemäß Beispiel 1.

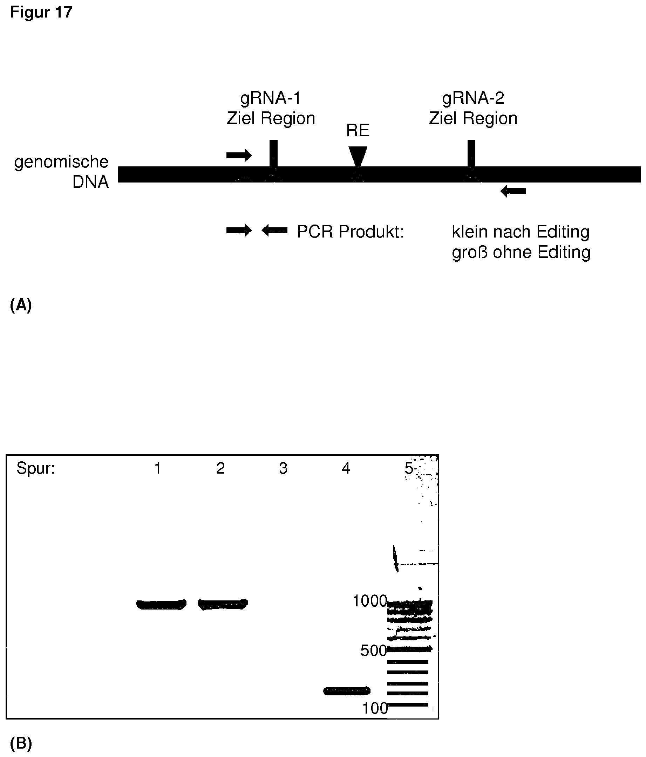

Figur 17 A und B (Fig. 17 A und B) zeigt eine 2-gRNA Strategie, wie sie zum Zwecke der hierein offenbarten Verfahren eingesetzt wurde. Fig. 17 A zeigt die Anwendung von zwei gRNAs, gRNA-1 und gRNA-2, die einen Bereich der genomischen DNA ansteuern mit dem Ziel den zwischen ihnen liegenden Bereich durch eine CRISPR Nuklease, z.B. eine Cas Nuklease oder eine beliebige andere CRISPR Nuklease, aus einer genomischen DNA Region herauszuschneiden. RE: Restriktionsenzym. Fig. 17 B zeigt das Ergebnis der Analyse auf ein Editing Ereignis nach Anwendung der 2-gRNA Strategie auf das Genom einer Maispflanze. Hierzu wurde aus Maispflanzen die genomische DNA isoliert und das Zielgen hmg13-Gen (HMG-transcription factor 13; GRMZM2G066528) mit PCR amplifiziert. Die Figur 17 B stellt das Ergebnis einer Auftrennung in einem 1%igen Gel mit dem StandardLaufparameter 100 V und die anschließende Visualisierung über Ethidiumbromid-vermittelte Fluoreszenz dar. In Spur 5 befindet sich der molekulare Größen-Marker (Angabe in Basenpaaren; GeneRuler 50 bp DNA Ladder (Thermo Fisher Scientific Inc, USA; SM0373) 1000, 900, 800, 700, 600, 500, 400, 300, 250, 200, 150, 100, 50 bp). In den Spuren 1 und 2 ist jeweils das Ergebnis für nicht-editierte Maispflanzen zu sehen, in der Spur 4 das Ergebnis nach erfolgreichem Editing. Das PCR-Produkt ist kleiner, da der Bereich zwischen den beiden gRNA Ziel-Regionen herausgeschnitten wurde.

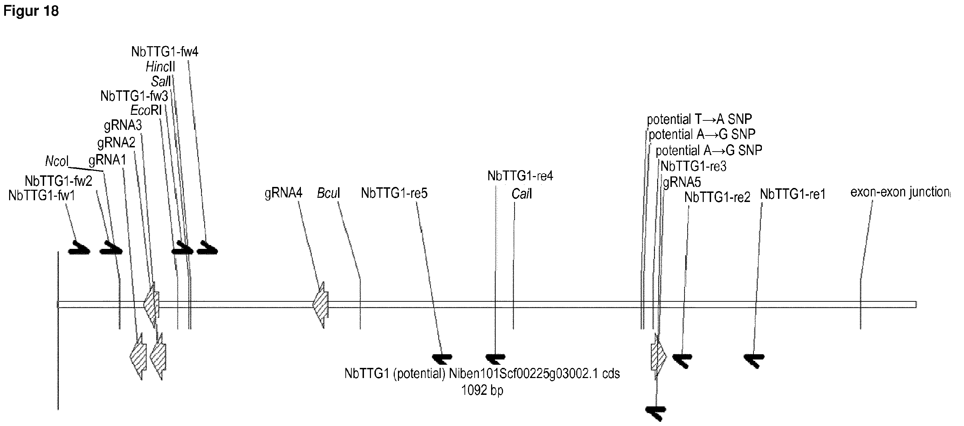

Figur 18 zeigt einen Teil des *Nicotiana benthamiana* NbTTG1 Gens. Die gRNA Ziel-Regionen sind als schraffierte Pfeile dargestellt, Primer Bindestelle (fw: forward, re: reverse) als schwarze Pfeile. Des Weiteren sind Schnittstellen für Restriktionsenzyme eingezeichnet, die im Rahmen der Analyse auf ein Editing Ergebnis hin zum Einsatz kommen.

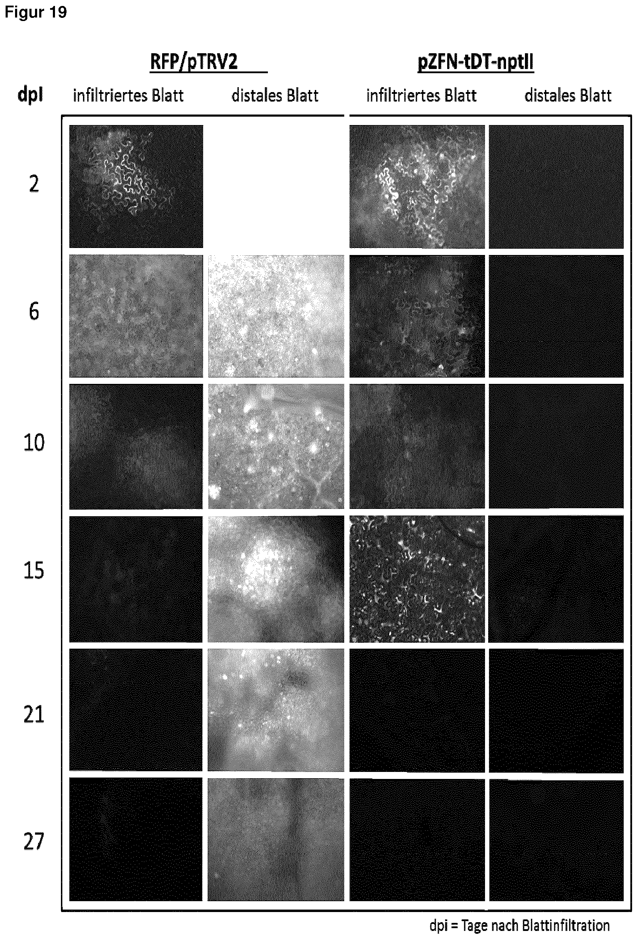

Figur 19: TRV erreicht in *Nicotiana benthamina* distales, nicht-inokuliertes Blattgewebe. Figur 19 zeigt die Blattinokulation von *N. benthamiana* mit einem rot fluoreszierenden Proteinkonstrukt (RFP) sowie einem Kontrollkonstrukt unter Verwendung von Tobacco rattle virus (TRV)-vermittelter Expression. Das Konstrukt pZFN-tDT-nptll fungierte als Kontrolle, die ausschließlich die Expression des RFP in den inokulierten, jedoch nicht in den distalen Blättern erlaubte. In der schwarz-weiß Abbildung von Fig. 19 entsprechen helle(re) Bereiche der ursprünglich detektierten roten Fluoreszenz, wohingegen schwarze Bereiche diejenigen Regionen wiederspiegeln, in denen keine Fluoreszenz detektiert werden konnte.

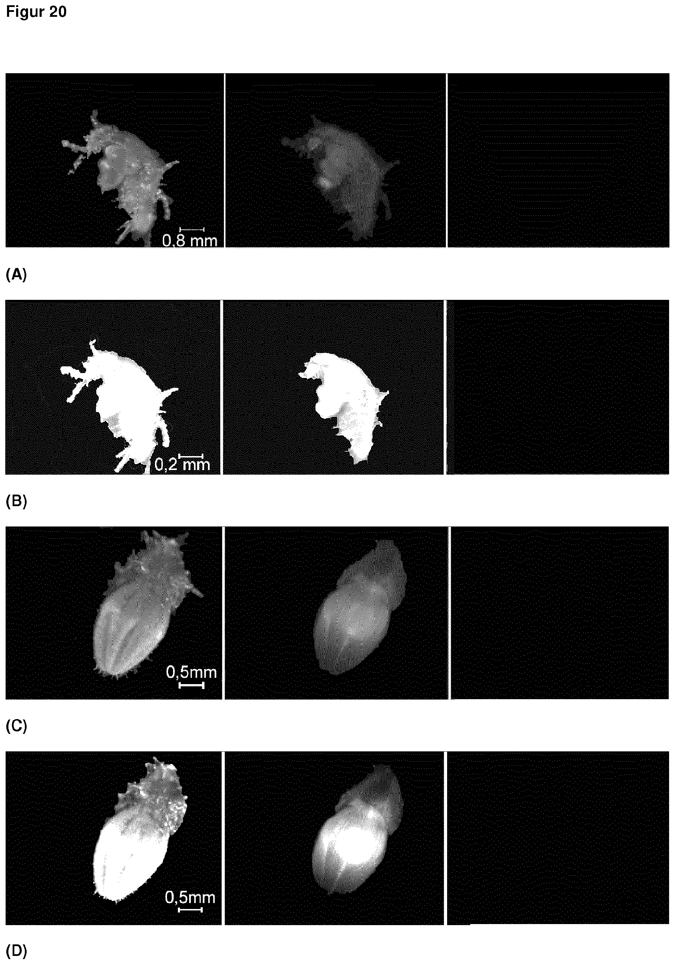

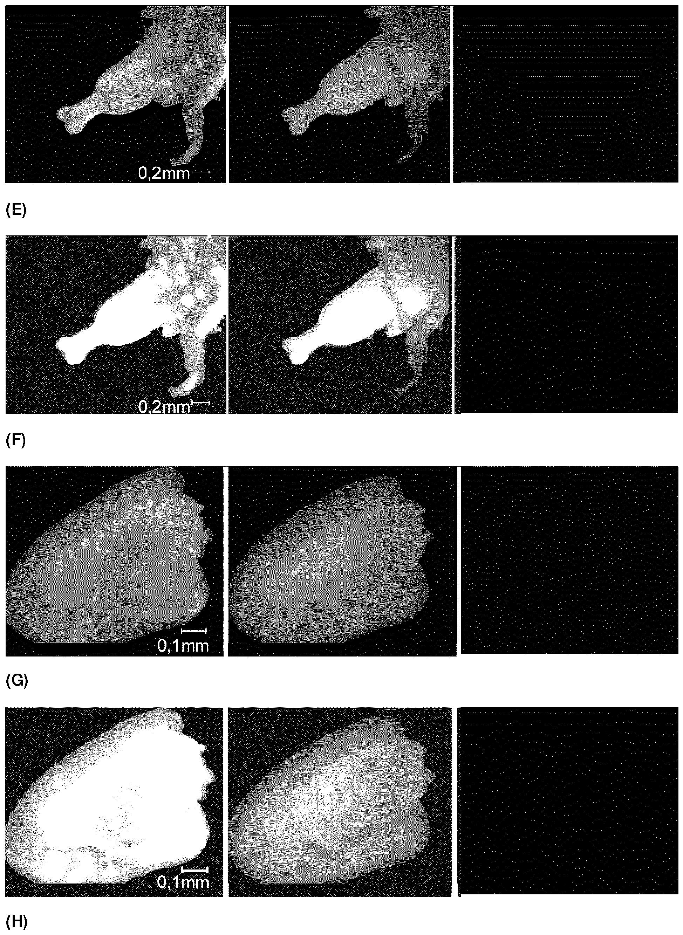

Figur 20 A-H (Fig. 20 A-H) zeigt fluoreszenzmikroskopische Aufnahmen von verschiedenen Blütenstrukturen, die mit TRV infiziert wurden, welcher ein rot fluoreszierendes Protein exprimieren kann. Alle Figuren A bis H zeigen links eine Hellfeldaufnahme, in der Mitte eine Aufnahme mit Rot-Filter sowie rechts eine Aufnahme mit Grün (GFP) Filter. Letzterer dient zur Kontrolle der Autofluoreszenz. In Fig. 20 A (Originalaufnahme in schwarz/weiß) und B (Pendant zu A mit Anpassung Kontrast/Helligkeit) ist ein Blütenmeristem zu sehen. In Fig. 20 C (Originalaufnahme in schwarz/weiß) und D (Pendant zu C mit Anpassung Kontrast/Helligkeit) ist ein Blütenknospe zu sehen. In Fig. 20 E (Originalaufnahme in schwarz/weiß) und F (Pendant zu E mit Anpassung Kontrast/Helligkeit) ist die Aufnahme eines Stempels zu sehen. In Fig. 20 G (Originalaufnahme in schwarz/weiß) und H (Pendant zu G mit Anpassung Kontrast/Helligkeit) ist ein präparierter Stempel mit offen liegenden Ovarien zu sehen.

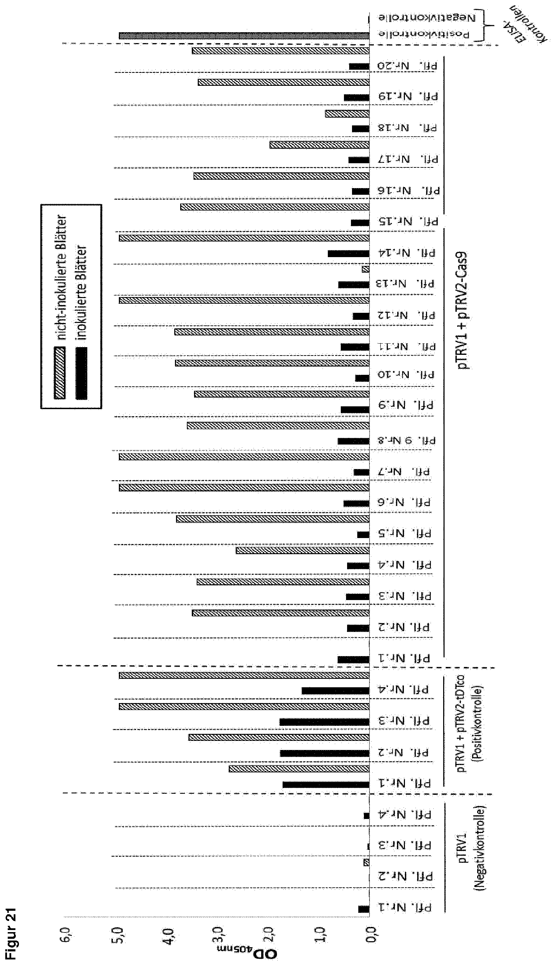

Figur 21 zeigt die Quantifizierung von TRV-Titern in *N*. *benthamiana* inokuliert mit pTRV1 (= Negativkontrolle), pTRV1 + pTRV2-tDTco (= Positivkontrolle) bzw. pTRV1 + pTRV2-Cas9 10 dpl.

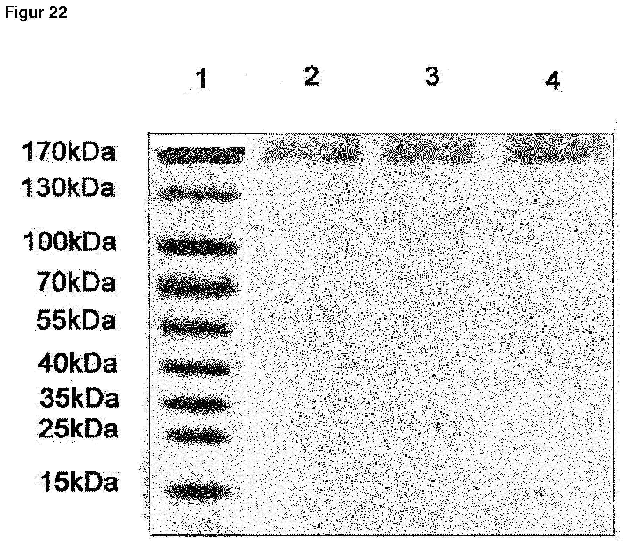

Figur 22 zeigt den Proteinnachweis von Cas9 (160 kDa) exprimiert in und anschließend isoliert aus Blattmaterial transgener Maispflanzen. Als Größenstandard kam der PageRuler Prestained Protein Ladder (10-170 kDa; von oben nach unten 170, 130, 100, 70, 55, 40, 35, 25, 15, 10 kDa) zum Einsatz. Belichtungzeit war 30 Minuten. Spur 1: vorgefärbter Proteinmarker; Spur 2: 10µg Protein aus Cas9 exprimierendem Mais; Spur 3: 15µg Protein aus Cas9 exprimierendem Mais; Spur 4: 20µg Protein aus Cas9 exprimierendem Mais.

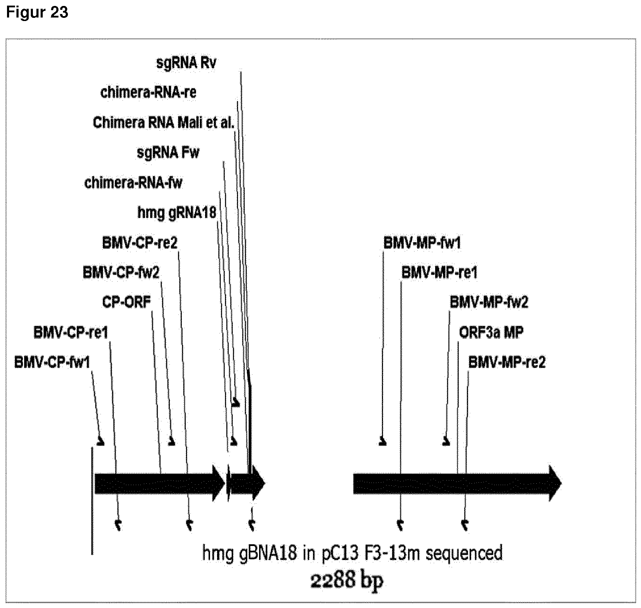

Figur 23 zeigt exemplarisch eine Virussequenz (BMV). Die verschiedenen Primer-Kombination, die für das Quantifizierungssystem der gRNA verwendet werden, sind als Pfeile eingezeichnet. "Fw" bezeichnet forward, "re" reverse Primer, die eine Sequenz von Interesse flankieren. Die "hmg gRNA" bezeichnet eine spezifische gRNA für das HMG-transcription factor Gen integriert in das zu analysierende Konstrukt von Interesse. Die abgebildete chimäre RNA ("Chimera RNA Mali et al.") beschreibt ein chimäres künstliches RNA Kosntrukt angelehnt an die Offenbarung von Mali et al., 2013 *supra,* wobei die hierein beschriebenen gRNAs wie im Beispielteil erläutert spezifisch für die Verwendung in pflanzlichen Zellen angepasst wurden.

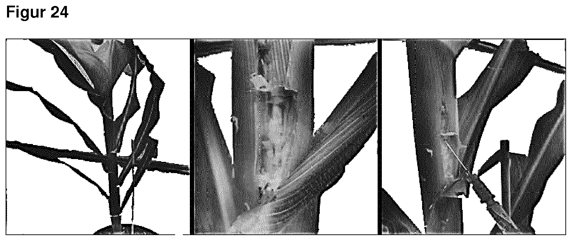

Figur 24 zeigt eine Mais-Pflanze des Genotyps A188 im V7-Stadium (linkes Bild), dieselbe Pflanze nach Einführung eines künstlichen Fensters in die Region, die das Tassel-Gewebe schützt (Mitte) sowie die anschließende Injektion *einer Agrobacterium* Lösung in den Bereich des freigelegten Tassels (rechtes Bild).

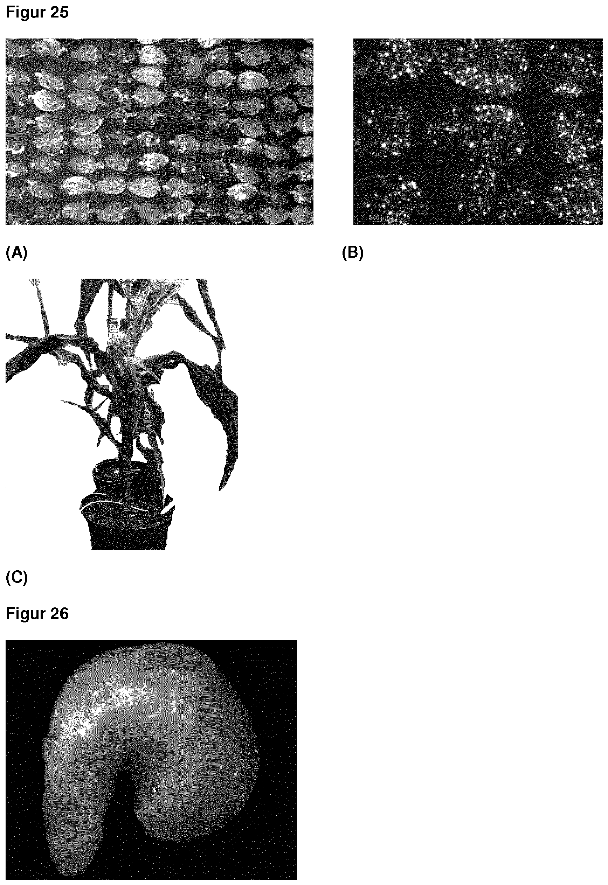

Figur 25 A-C (Fig. A-C) zeigt unreife Embryonen (Fig. 25 A) einer Maispflanze, die isoliert und anschließend mit einem CRISPR/Cas9 Konstrukt sowie einem ein rot fluoreszierendes Protein exprimierenden Plasmids durch Partikelbeschuss bombadiert wurden. Fig. 25 B zeigt die Fluoreszenzentwicklung (weiße und helle Regionen) an Tag 1 nach dem Beschuss. Fig. 25 C zeigt eine ausgereifte Maispflanze, die aus den derart bomardierten und anschließend bis zur Reife weitergezüchteten Embryonen aus Fig. 25 A und B gewonnen werden konnte.
Figur 26 zeigt einen unreifen *Beta vulgaris* Embryo, gewonnen nach den unten näher beschriebenen Verfahren.

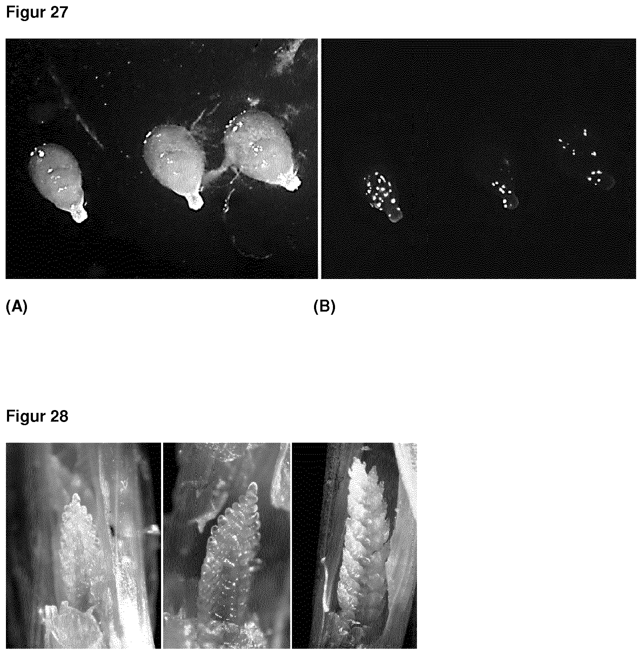

Figur 27 A und B (Fig. 27 A und B): Fig. 27 A: Unreife Weizenkörner nach Meristem-Transformation. Fig. 27 B: Entspricht der Fluoreszenzaufnahme von Fig. 27 A. Die hellen Bereiche entsprechen der detektierten Fluoreszenz (helle/weiße Bereiche in der schwarz/weiß Aufnahme). Nach der Meristem-Transformation konnten direkt keimende Weizenpflänzchen aus den behandelten Weizenkörner (A und B) erhalten werden.
Figur 28 zeigt im linken Bild die Lokalisation der unreifen Blütenstände in Weizen. Die mittleren Bilder sowie das rechte Bild zeigen von links nach rechts die weitere Entwicklung der wie unten beschrieben transformierten meristematischen Gewebe.

### Ausführliche Beschreibung

### Definitionen

Der Begriff Pflanze oder pflanzliche Zelle, wie hierin verwendet, bezieht sich auf pflanzliche Organismen, Pflanzenorgane, differenzierte und undifferenzierte Pflanzengewebe, Pflanzenzellen, Samen und deren Abkömmlinge oder Nachkommen. Pflanzliche Zellen umfassen z.B. Zellen von Samen, reifen und unreifen Embryonen, meristematischen Geweben, Keimlingen, Kallusgeweben, Blättern, Blüten, Wurzeln, Pflanzensprossen, Gametophyten, Sporophyten, Pollen und Mikrosporen, Protoplasten, Makroalgen und Mikroalgen.

Der Begriff fertile Pflanze wie hierein verwendet, bezieht sich auf eine fertile Pflanze, die dazu in der Lage ist, Nachkommen zu produzieren, d.h. eine fertile Pflanze ist eine Pflanze, die lebensfähige männliche und weibliche Gameten produzieren kann. Eine männlich sterile Pflanze ist demnach eine Pflanze, die keine lebensfähigen männlichen Gameten produzieren kann, die jedoch weiblich-fertil sein kann. Eine weiblich sterile Pflanze ist eine Pflanze, die keine lebensfähigen weiblichen Gameten produzieren kann, wobei die Pflanze nach wie vor männlich fertil sein kann.

Pflanzenmaterial, wie hierin verwendet, bezieht sich auf jegliches Material, das von einer Pflanze in einem beliebigen Entwicklungsstadium gewonnen werden kann. Der Begriff umfasst somit pflanzliche Zellen, Gewebe und Organe sowie ausgebildete Pflanzenstrukturen sowie ebenfalls subzelluläre Komponenten wie Nukleinsäuren, Polypeptide, sowie alle chemischen Pflanzenstoffe, die innerhalb einer Pflanzenzelle vorliegen und/oder von dieser produziert werden können.

Der Begriff chromosomal oder extrachromosomal integriert, wie hierin verwendet, bezieht sich auf die transiente Einbringung und/oder die Gestaltung des einen bzw. der mehreren rekombinanten Konstrukts/Konstrukte gemäß der vorliegenden Offenbarung und damit auf das anschließende Schicksal des einen bzw. der mehreren rekombinanten Konstrukts/Konstrukte in einer pflanzliche Zielstruktur, z.B. einer Zelle, wobei sowohl das eine bzw. die mehreren rekombinanten Konstrukt(e) als auch die Bedingungen zur Einbringung davon derart gehalten sind, dass keine Integration des mindestens einen rekombinanten Konstrukts in das endogene Nukleinsäurematerial einer pflanzlichen Zielstruktur, umfassend das Genom oder extrachromosomale Nukleinsäuren der pflanzlichen Zielstruktur, z.B. einer Zelle, stattfindet, sodass das mindestens eine rekombinante Konstrukt nicht chromosomal oder extrachromosomal integriert in die endogene DNA/RNA der Zielzelle und somit nicht an die Nachkommen der Zelle weitergegeben wird. Das eine bzw. die mehreren rekombinante(n) Konstrukt(e) oder dessen Transkriptions- bzw. Translationsprodukte liegen somit nur vorübergehend, d.h. transient, konstitutiv oder induzierbar, aktiv in der Zielzelle vor, sind aber nicht an die Nachkommen der Zielzelle vererbbar, d.h. sie liegen auch nicht in den Nachkommen einer Zielzelle aktiv vor.

Der Begriff "homologe Rekombination" wie hierin verwendet, bezeichnet einen Vorgang, wie er bei allen Organismen vorkommt. Voraussetzung hierfür sind homologe, doppelsträngige DNA-Abschnitte. Homolog bedeutet daher, dass es eine große Ähnlichkeit in der Nukleotidsequenz zweier Sequenzen gibt. Bei natürlich vorkommenden Doppelstrangbrüchen kann durch homologe Rekombination der Schaden dadurch ausgebessert werden, dass die Informationen auf dem unbeschädigten Chromatid im Genom eines Organismus als Vorlage genutzt werden. Sofern, wie auch gemäß der vorliegenden Offenbarung, ein gezielter und präziser Doppelstrangbruch im Rahmen der Genom Editierung in eine Nukleinsäure-Zielregion von Interesse eingefügt wird, kann auch hier die homologe Rekombination dazu genutzt werden, den entstandenen Bruch zu reparieren, wobei hierdurch das gezielte Entwerfen einer DNA-Reparatur-Matrize zudem zielgerichtet Einfluss auf die zu reparierende Nukleinsäure-Zielregion von Interesse genommen werden kann. Unterschiedliche Organismen unterscheiden sich im Hinblick auf das Verhältnis von homologer und nicht-homologer Rekombination, wie es natürlicherweise vorkommt (s.o. NHEJ). Im Allgemeinen beeinflusst die Länge der Homologieregion die Frequenz von Ereignissen homologer Rekombination: je länger die Homologieregion, desto größer die Häufigkeit. Die Länge der Homologieregion, die benötigt wird, um homologe Rekombination zu erzielen ist zudem speziesabhängig. In manchen Fällen kann es notwendig sein, mindestens fünf Kilobasen (kb) an Homologie zu verwenden, aber homologe Rekombination wurde auch bereits bei einer Homologieregion von nur etwa 25 Basenpaaren (bp) beobachtet.

Homologie-gerichtete Reparatur (HDR) bezeichnet einen zellulären Mechanismus, um doppel- wie einzelsträngige DNA-Brüche zu reparieren. HDR umfasst somit Elemente der homologen Rekombination sowie des sogenannten Single-Strand Annealings (SSA) (Lieber Michael et al., Annu.Rev.Biochem.79:181-211, 2010). Die häufigste Form von HDR in einer Zelle ist die homologe Rekombination, wobei diese Art der Reparatur auch die höchste Sequenzhomologie zwischen Donor- und Akzeptor-DNA erfordert. Andere Formen von HDR umfassen Single-Strand Annealing (SSA). SSA ist nicht-konservativ und tritt natürlicherweise zwischen direkten Wiederholungen von > 30 bp und resultiert in Deletionen. HDR bei Nicks d.h. bei Einzelstrangbrüchen, erfolgt über einen anderen Mechanismus als HDR bei Doppelstrangbrüchen (Davis und Maizels PNAS, 2014 E924-32). Da gemäß der vorliegenden Offenbarung sowohl Doppelstrang- als auch Einzelstrang-Bruch-induzierende CRISPR-Nukleasen vorgeschlagen werden, bezieht sich der Begriff HDR oder homologe Rekombination daher auf die Reparatur von einem gezielt eingeführten Einzelstrang- wie auch Doppelstrang-Bruch unter Verwendung einer geeigneten Reparatur-Matrize.

Herbizidresistenz oder Herbizidtoleranz wie hierein verwendet bezeichnet die Fähigkeit einer Pflanze oder einer pflanzlichen Zelle resistent oder toleranter gegenüber der Wirkung eines Herbizids oder Pestizids zu sein. Diese Eigenschaft wird üblicherweise duch mindestens ein Protein oder eine RNA vermittelt, das/die entweder künstlich, z.B. als Transgen in eine pflanzliche Zelle eingebracht wurde, oder, das durch (gezielte) Modifikation eines endogenen Gens erlangt werden kann. Der Begriff Abkömmling oder Nachkomme, wie hierin verwendet, bezeichnet im Kontext eines rekombinanten Mikroorganismus, einer Pflanze oder einer Zelle gemäß der vorliegenden Offenbarung die Nachkommen eines solchen Organismus oder einer solchen Zelle, welche durch natürliche reproduktive ungeschlechtliche Zellteilungs- und Differenzierungsvorgänge aus dem Ursprungsorganismus oder der Ursprungszelle hervorgehen. Es ist dem Fachmann auf dem Gebiet bekannt, dass im Zuge der Zellteilung und Differenzierung auf natürliche Weise Mutationen ins Genom eines Organismus eingeführt werden können, wodurch sich der Abkömmling oder der Nachkomme genomisch vom Elternorganismus unterscheidet, jedoch nach wie vor der gleichen (Sub-)Spezies zugeordnet werden kann. Auch derartige durch natürliche Vorgänge veränderte Abkömmlinge, welche neben der gezielt eingebrachten Veränderung weitere Veränderungen in anderen DNA-Regionen tragen sind daher vom Begriff Abkömmling oder Nachkomme gemäß der vorliegenden Offenbarung umfasst.

Der Begriff CRISPR Nuklease, wie hierin verwendet, bezieht sich allgemein auf eine Nuklease, wie sie in einem natürlich vorkommenden CRISPR System vorkommt sowie Modifikationen und Mutanten und katalytisch aktive Fragmente davon. Im natürlich vorkommenden CRISPR Lokus stellt die CRISPR Nuklease das Molekül dar, das das Effektormolekül bildet und im Zusammenspiel mit einer crRNA und optional einer tracr RNA, bzw. zusammen mit einer künstlichen gRNA eine Nukleinsäure-Zielstruktur erkennen und/oder spalten kann. CRISPR Nukleasen umfassen daher Cas-Nuklease, Cpf1-Nukleasen oder weitere CRISPR Effektor- und/oder Nukleasedomänen, umfassend Csf1 und Kombinationen und Varianten davon. Darüber hinaus umfasst dieser Begriff auch gezielt modifizierte Nukleasen, die etwa zu Nickasen, zur Erzielung von Einzelstrangbrüchen, oder Nuklease-null Varianten, die zu Bindungs- und Erkennungszwecken, nicht jedoch zur Erzielung eines Doppelstrangbruches, umgewandelt wurden. Da sich in der Literatur zwischenzeitlich der Begriff CRISPR/Cas als Synonym für alle Typen von CRISPR Systemen etabliert hat, soll sich dieser Begriff gemäß der vorliegenden Offenbarung, wenn nicht spezifisch anderes angegeben, auch auf jegliches CRISPR Typ I-V System sowie das zugehörige Effektorprotein beziehen.

Der Begriff Vektor oder Vektorsystem, wie hierin verwendet, bezieht sich auf ein Transportmittel, um ein rekombinantes Konstrukt, umfassend Nukleinsäuren oder auch Polypeptide sowie gegebenenfalls weitere Sequenzen wie regulatorische Sequenzen oder Lokalisationssequenzen, direkt oder indirekt in eine gewünschte Zielzelle bzw. pflanzliche Zielstruktur in das erwünschte zelluläre Kompartiment einbringen zu können. Die direkte Einbringung erfolgt direkt in eine pflanzliche Zielzelle bzw. pflanzliche Zielstruktur, welche Nukleinsäuren enthält, die gemäß der vorliegenden Offenbarung gezielt verändert werden sollen. Die indirekte Einbringung umfasst die Einbringung in eine Struktur, z.B. Zellen von Blätter oder weitere pflanzliche Organe und Gewebe, welche zwar nicht direkt die pflanzliche Zielzelle von Interesse umfassen, wodurch jedoch die systemische Ausbreitung und Weiterleitung des Vektors, umfassend ein rekombinantes Konstrukt gemäß der vorliegenden Offenbarung, in die pflanzliche Zielstruktur, z.B. meristematische Gewebe oder Zellen oder Stammzellen, gewährleistet wird. Der Begriff Vektor oder Vektorsystem, wie hierin verwendet, umfasst im Kontext der Transfektion von Aminosäuresequenzen geeignete Agenzien zur Peptid- oder Proteintransfektion wie z.B. ionische Lipidmischungen oder Agenzien, die zur Transfektion einer Nukleinsäure geeignet sind, wie z.B. Trägermaterialen, durch welche Nukleinsäure- und Aminosäuresequenzen mittels Partikel-Beschuss in eine Zelle eingeführt werden können, wie z.B. Gold- und Wolframpartikel. Ferner umfasst dieser Begriff speziell bei der Anwendung der hierin offenbarten Verfahren und Konstrukte auch virale Vektoren, d.h. modifizierte Viren, wie zum Beispiel solche, welche aus einem der folgenden Viren stammen: Maize Streak Virus (MSV), Barley Stripe Mosaic Virus (BSMV), Brome Mosaic virus (BMV, Zugangsnummern: RNA1: X58456; RNA2: X58457; RNA3: X58458), Maize stripe virus (MSpV), Maize rayado fino virus (MYDV), Maize yellow dwarf virus (MYDV), Maize dwarf mosaic virus (MDMV), Positivstrang RNA Viren der Familien *Benyviridae,* z.B. *Beet necroticyellow vein virus* (Zugangsnummern: RNA1: NC_003514; RNA2: NC_003515; RNA3: NC_003516; RNA4: NC_003517) oder der Familie *Bromoviridae,* z.B. Viren der Gattung *Alfalfa mosaic virus* (Zugangsnummern: RNA1: NC_001495; RNA2: NC_002024; RNA3: NC_002025) oder der Gattung *Bromovirus,* z.B. BMV (s.o.), oder der Gattung *Cucumovirus,* z.B. *Cucumbermosaic virus* (Zugangsnummern: RNA1: NC_002034; RNA2: NC_002035; RNA3: NC_001440), oder der Gattung *Oleavirus,* dsDNA Viren der Familie *Caulimoviridae,* insbesondere der Familien *Badnavirus* oder *Caulimovirus,* z.B. verschiedene *Banana* streak Viren (s. z.B. Zugangsnummern: NC_007002, NC_015507, NC_006955 oder NC_003381) oder *Cauliflower mosaic virus* (Zugangsnummer: NC_001497), oder Viren der Gattung *Cavemovirus, Petuvirus, Rosadnavirus, Solendovirus, Soymovirus* oder *Tungrovirus,* Positivstrang RNA Viren der Familie *Closteroviridae,* z.B. der Gattung *Ampelovirus, Crinivirus, z.B. Lettuce infectious yellows virus* (Zugangsnummer: RNA1: NC_003617; RNA2: NC_003618) oder *Tomato chlorosis virus* (Zugangsnummer: RNA1: NC_007340; RNA2: NC_007341), *Closterovirus, z.B. Beet yellows virus* (Zugangsnummer: NC_001598), oder *Velarivirus,* einzelsträngige DNA (+/-) Viren der Familie *Geminiviridae,* z.B. Viren der Familie *Becurtovirus, Begomovirus, z.B. Bean golden yellow mosaic virus, Tobacco curly shoot virus, Tobacco mottle leaf curl virus, Tomato chlorotic mottle virus, Tomato dwarf leaf virus, Tomato golden mosaic virus, Tomato leaf curl virus, Tomato mottle virus,* oder *Tomato yellow spot virus,* oder *Geminiviridae* der Gattung *Curtovirus,* z.B. *Beet curly top virus,* oder *Geminiviridae* der Gattung Topocuvirus, Turncurtvirus oder *Mastrevirus,* z.B *Maize streak virus* (s.o.), *Tobacco yellow dwarf virus, Wheat dwarf virus,* Positivstrang RNA Viren der Familie *Luteoviridae,* z.B. der Gattung *Luteovirus,* z.B. *Barley yellow dwarf virus-PAV* (Zugnagsnummer: NC_004750), oder der Gattung *Polerovirus,* z.B. *Potato leafroll virus* (Zugangsnummer: NC_001747), Einzelstrang DNA Viren Familie *Nanoviridae,* umfassen die Gattungen *Nanovirus* oder *Babuvirus,* doppelsträngige RNA Viren der Familie *Partiviridae,* umfassend *interalia* die Familien *Alphapartitivirus, Betapartitivirus* oder *Deltapartitivirus,* Viroide der Familie *Pospiviroidae,* Positivstrang RNA Viren der Familie *Potyviridae,* z.B. umfassend die Gattungen *Brambyvirus, Bymovirus, Ipomovirus, Macluravirus, Poacevirus,* z.B. *Triticum mosaic virus* (Zugangsnummer: NC_012799), oder *Potyviridae* der Gattung *Potyvirus,* z.B. *Beet mosaic virus* (Zugangsnummer: NC_005304), *Maize dwarf mosaic virus* (Zugangsnummer: NC_003377), *Potato virus Y* (Zugangsnummer: NC_001616), oder Zea *mosaic virus* (Zugangsnummer: NC_018833), oder *Potyviridae* der Gattung *Tritimovirus,* z.B. *Brome streak mosaic virus* (Zugangsnummer: NC_003501) oder *Wheat streak mosaic virus* (Zugangsnummer: NC_001886), Einzelstrang RNA Viren der Familie *Pseudoviridae,* z.B. der Gattungen *Pseudovirus,* oder *Sirevirus,* doppelsträngige RNA Viren der Familie *Reoviridae,* z.B. Rice dwarf virus (Zugangsnummern: RNA1: NC_003773; RNA2: NC_003774; RNA3: NC_003772; RNA4: NC_003761; RNA5: NC_003762; RNA6: NC_003763; RNA7: NC_003760; RNA8: NC_003764; RNA9: NC_003765; RNA10: NC_003766; RNA11: NC_003767; RNA12: NC_003768), Positivstrang RNA Viren der Familie *Tombusviridae,* z.B. umfassend die Gattungen *Alphanecrovirus, Aureusvirus, Betanecrovirus, Carmovirus, Dianthovirus, Gallantivirus, Macanavirus, Machlomovirus, Panicovirus, Tombusvirus, Umbravirus* oder *Zeavirus,* z.B. *Maize necrotic streak virus* (Zugangsnummer: NC_007729), oder Positivstrang RNA Viren der Familie *Virgaviridae,* z.B. Viren der Gattung *Furovirus, Hordeivirus,* z.B. *Barley stripe mosaic virus* (Zugangsnummern: RNA1: NC_003469; RNA2: NC_003481; RNA3: NC_003478), oder der Gattung *Pecluvirus, Pomovirus, Tobamovirus* oder *Tobravirus,* z.B. *Tobacco rattle virus* (Zugangsnummern: RNA1: NC_003805; RNA2: NC_003811), sowie Negativstrang RNA Viren der Ordnung *Mononegavirales,* insbesondere der Familie *Rhabdoviridae,* z.B. *Barley yellow striate mosaic virus* (Zugangsnummer: KM213865) oder *Lettuce necrotic yellows virus* (Zugangsnummer/Exemplar: NC_007642/ AJ867584), Positivstrang RNA Viren der Ordnung *Picornavirales,* insbesondere der Familie *Secoviridae,* z.B. der Gattungen *Comovirus, Fabavirus, Nepovirus, Cheravirus, Sadwavirus, Sequivirus, Torradovirus,* oder *Waikavirus,* Positivstrang RNA Viren der Ordnung *Tymovirales,* insbesondere der Familie *Alphaflexiviridae,* z.B. Viren der Gattung *Allexivirus, Lolavirus, Mandarivirus,* oder *Potexvirus, Tymovirales,* insbesondere der Familie *Betaflexiviridae,* z.B. Viren der Gattung *Capillovirus, Carlavirus, Citrivirus, Foveavirus, Tepovirus,* oder *Vitivirus,* Positivstrang RNA Viren der Ordnung *Tymovirales,* insbesondere der Familie *Tymoviridae,* z.B. Viren der Gattung *Maculavirus, Marafivirus,* oder *Tymovirus,* und bakterielle Vektoren, wie zum Beispiel *Agrobacterium* spp., wie zum Beispiel *Agrobacterium tumefaciens.* Schließlich umfasst der Begriff auch geeignete Transportmittel zur Einführung von linearen Nukleinsäuren (einzel- oder doppelsträngig) in eine Zielzelle. Dem Fachmann auf dem Gebiet sind in Kenntnis der hierin offenbarten Konstrukte alle weiteren Sequenzen bekannt, über welche ein Vektor verfügen muss, um in einer erwünschten Zielzelle funktional zu sein. Auch die gängige Herstellung, Aufarbeitung und Anwendung derartiger Vektoren ist dem Fachmann auf dem Gebiet bekannt.

Der Begriff Vektorsystem, wie hierin verwendet, bezeichnet ein System, das aus mindestens einem oder mehreren Vektor(en) besteht oder diese(n) enthält. So kann ein Vektorsystem einen Vektor aufweisen, welcher zwei unterschiedliche rekombinante Konstrukte, umfassend Nukleinsäure- und/oder Aminosäuresequenzen, enthält/kodiert. Ein Vektorsystem kann darüber hinaus auch mehrere Vektoren enthalten, die ihrerseits mindestens eine Nukleinsäure- oder Aminosäuresequenz gemäß der vorliegenden Offenbarung enthalten/kodieren.

Der Begriff Quantitativer Merkmals-Locus oder QTL, wie hierin verwendet, bezeichnet eine DNA-Region, die mit der differenziellen Expression eines quantitativen phänotypischen Merkmals in mindestens einem definierten genetischen Hintergrund assoziiert ist, z.B. in mindestens einer Züchtungspopulation. Die QTL-Region umfasst, oder ist eng verknüpft mit, dem Gen oder den Genen, die das fragliche Merkmal beeinflussen. Ein Allel eines QTL kann demnach mehrere Gene oder auch andere genetische Faktoren innerhalb einer zusammenhängenden genomischen Region, oder einer Linkage-Gruppe, z.B. einem Haplotyp, umfassen. Ein Allel eines QTL kann einen Haplotyp innerhalb eines festgelegten Fensters bezeichnen, wobei dieses Fenster eine zusammenhängende genomische Region darstellt, die mit einem Satz an einem oder mehreren polymorphen Markern definiert und (rück)verfolgt werden kann. Ein Haplotyp kann durch die einzigartigen Fingerabdrücke von Allelen bei jedem Marker innerhalb des festgelegten Fensters definiert werden.

Wie unten noch näher ausgeführt, stehen dem Fachmann eine Vielzahl von Verfahren zur Verfügung, um diejenigen pflanzlichen Zielstrukturen, umfassend mindestens eine meristematische Zelle oder eine ganze Pflanze, oder ein Pflanzenmaterial oder eine pflanzliche Zelle davon zu identifizieren, die eine gezielte Veränderung in ihrer genomischen DNA in oder nahe bei einer Nukleinsäure-Zielregion trägt, ohne dass überprüfbare Marker-Phänotypen verwendet würden. Solche Verfahren basieren auf der direkten Analyse einer Nukleinsäure-Zielregion oder -Zielsequenz von Interesse, um eine beliebige Änderung in dieser Nukleinsäure-Region oder-Sequenz nachzuweisen und umfassen, ohne darauf beschränkt zu sein, PCR-Verfahren, Sequenzierungsverfahren, Nuklease-Verdau, Southern Blots, Northern Blots und jede beliebige Kombination davon.

Der Begriff Nukleinsäure oder Nukleinsäuresequenz, wie hierin verwendet, bezieht sich auf natürliche wie synthetische Desoxyribonukleinsäuren (DNA) und Ribonukleinsäuren (RNA), welche auch synthetische Nukleotidanaloga enthalten können. Die Nukleinsäuren, welche gemäß der vorliegenden Erfindung zur Synthese eines gewünschten Produkts wie Protein oder RNA oder zur gezielten Steuerung davon verwendet werden, z.B. eine CRISPR Nuklease, umfassend u.a. eine Cas-Nuklease oder eine Cpf1-Nuklease, oder eine gRNA, können gegebenenfalls "auf die Anwendung in einer pflanzlichen Zielstruktur angepasst" sein. In einer Ausführungsform können die vorgenannten Sequenzen Codon-optimiert sein, d.h. die Codon-Verwendung eines Genes oder einer RNA wird spezifisch auf die der Zielzelle/Zielorganismus angepasst. Es ist dem Fachmann auf dem Gebiet bekannt, dass ein gewünschtes Zielgen, welches ein Protein von Interesse kodiert, ohne Änderung der translatierten Proteinsequenz modifiziert werden kann, um der spezifischen Speziesabhängigen Codon-Verwendung Rechnung zu tragen. So sind/können die Nukleinsäuren der vorliegenden Offenbarung spezifisch auf die Codon-Verwendung von *Hordeum vulgare, Sorghum bicolor, Secale cereale,* Triticale, *Saccharum officinarium, Zea mays, Setaria italic, Oryza sativa, Oryza minuta, Oryza australiensis, Oryza alta, Triticum aestivum, Triticum durum, Hordeum bulbosum, Brachypodium distachyon, Hordeum marinum, Aegilops tauschii, Malus domestica, Beta vulgaris, Helianthus annuus, Daucus glochidiatus, Daucus pusillus, Daucus muricatus, Daucus carota, Eucalyptus grandis, Erythranthe guttata, Genlisea aurea, Nicotiana sylvestris, Nicotiana tabacum, Nicotiana tomentosiformis, Solanum lycopersicum, Solanum tuberosum,* Coffea *canephora, Vitis vinifera, Cucumis sativus, Morus notabilis, Arabidopsis thaliana, Arabidopsis lyrata, Arabidopsis arenosa, Crucihimalaya himalaica, Crucihimalaya wallichii, Cardamine flexuosa, Lepidium virginicum, Capsella bursa-pastoris, Olmarabidopsis pumila, Arabis hirsuta, Brassica napus, Brassica oleracea, Brassica rapa, Brassica juncacea, Brassica nigra, Raphanus sativus, Eruca vesicaria sativa, Citrus sinensis, Jatropha curcas, Glycine max, Gossypium ssp.* oder *Populus trichocarpa* angepasst (werden). Ferner muss gemäß der vorliegenden Offenbarung die Sequenz der gRNA oder der die gRNA kodierenden Sequenz an die Nukleinsäure-Zielregion innerhalb einer pflanzlichen Zielstruktur angepasst werden. In einer weiteren Ausführungsform kann die gRNA oder die die gRNA kodierende Sequenz zudem in der Region angepasst werden, welche für die Interaktion oder Kopplung mit einer Cas-Nuklease und/oder einer Effektor-Domäne verantwortlich ist.

Der Begriff Sequenzen, wie hierin verwendet, bezieht sich auf Nukleinsäuresequenzen sowie Aminosäuresequenzen, wobei die jeweilige Sequenz neben natürlichen Nukleotiden und Aminosäuren auch synthetische Analoga oder synthetische Verknüpfungen als Bausteine enthalten können.

Die Begriffe Polypeptid, Polypeptidsequenz, Proteinsequenz und Aminosäuresequenz werden hierin austauschbar verwendet.

Der Begriff katalytisch aktives Fragment wie hierin verwendet, insbesondere im Bezug auf CRISPR Nukleasen oder Varianten davon, bezieht sich auf eine Aminosäure-Kernsequenz, die von einer gegebenen Aminosäure-Templatesequenz abgeleitet ist, mit der Maßgabe, dass das resultierende katalytisch aktive Fragment das ganze oder Teile des aktive(n) Zentrum(s) der Template-Sequenz umfasst und dadurch nach wie vor die gleiche enzymatische Funktion erfüllt, wie die Templatesequenz. Derartige Modifikationen, d.h. Trunkierungen, sind dem Fachmann auf dem Gebiet wohlbekannt und speziell bei sterisch anspruchsvollen Enzymen sinnvoll, um vielseitige und stabilere trunkierte Enzyme, umfassend das katalytisch aktive Fragment, bereitzustellen.

Der Begriff regulatorische Sequenz, wie hierin verwendet, bezieht sich auf eine Nukleinsäure- oder eine Protein-Sequenz, welche die Transkription und/oder Translation der einer offenbarten Nukleinsäure-Sequenzen in cis oder trans steuern kann.

Der Begriff Konstrukt oder rekombinantes Konstrukt (hierin austauschbar verwendet) wie hierin verwendet bezieht sich auf ein Konstrukt, umfassend unter anderen Plasmide oder Plasmidvektoren, Cosmide, künstliche Hefe- oder bakterielle Chromosomen (YACs und BACs), Phagemide, Bakteriophagenvektoren, eine Expressionskassette, einzelsträngige oder lineare Nukleinsäuresequenzen oder Aminosäuresequenzen, und virale Vektoren, d.h. modifizierte Viren, welche in eine Zielzelle gemäß der vorliegenden Offenbarung eingebracht werden können. Ein rekombinantes Konstrukt kann CRISPR/Cas Werkzeuge oder Teile davon, umfassend mindestens eine gRNA oder mindestens eine CRISPR Nuklease Variante und/oder mindestens eine weitere Effektor-Domänen entweder in der Form einer Nukleinsäure oder einer Aminosäuresequenz, umfassen. Ferner kann das rekombinante Konstrukt regulatorische Sequenzen und/oder Lokalisationssequenzen umfassen. Das rekombinante Konstrukt kann etwa in einen Plasmidvektor integriert vorliegen und/oder isoliert von einem Plasmidvektor vorliegen, z.B. in der Form einer Polypeptidsequenz oder einer nicht-Plasmidvektor verknüpften einzel- oder doppelsträngigen Nukleinsäure vorliegen. Nach Einbringung liegt das Konstrukt vorzugsweise extrachromosomal und nicht in das Genom integriert und gewöhnlich in der Form einer doppelsträngigen oder einzelsträngigen DNA, einer doppelsträngigen oder einzelsträngigen RNA oder eines Polypeptids vor. Plasmidvektor wie hierin verwendet, bezieht sich auf ein Konstrukt, welches ursprünglich aus einem Plasmid gewonnen wurde. Hierbei handelt es sich in der Regel um zirkuläre autonom replizierende extrachromosomale Elemente in der Form einer doppelsträngigen Nukleinsäuresequenz. In der Gentechnik werden diese ursprünglichen Plasmide gezielt verändert, indem unter anderem Resistenzgene, Ziel-Nukleinsäuren, Lokalisationssequenzen, regulatorische Sequenzen und dergleichen eingebracht werden. Die strukturellen Bestandteile des ursprünglichen Plasmids, wie der Replikationsursprung, werden hierbei beibehalten. Eine Vielzahl von Plasmidvektoren zur Anwendung in einer Zielzelle von Interesse ist kommerziell erhältlich und die Modifikation davon für eigene Klonierungsstrategien ist dem Fachmann auf dem Gebiet wohlbekannt. Derartige bekannte Plasmidvektoren werden hierin auch als Standardvektoren bezeichnet, wobei dies implizieren soll, dass der Basisvektor kommerziell erhältlich ist und leicht vom Fachmann auf dem entsprechenden technischen Gebiet den Bedürfnissen des jeweiligen Experiments angepasst werden kann.

Der Begriff eines Enhancers oder eines Enhancer-Elements bezeichnet eine Basen-/Nukleotidabfolge mit charakteristischer Sequenz. Ein Enhancer gehört zu den cisregulatorischen Elementen und kann die Anlagerung eines Transkriptionskomplexes an einen Promotor und damit die Transkriptionsaktivität eines Gens beeinflussen. Ein Promotor wiederum bezeichnet eine DNA-Sequenz, die die Expression einer kodierenden Sequenz oder einer funktionalen RNA kontrollieren kann. Die Promotor-Sequenz besteht aus proximalen sowie distalen Elementen in Relation zur regulierten Sequenz, wobei Letztere häufig als Enhancer bezeichnet werden. Promotoren können ein breites Aktivitätsspektrum besitzen, sie können jedoch auch gewebe- oder entwicklungsspezifisch aktiv/aktivierbar sein, z.B. in Zellen von Wurzeln, Samen, meristematischen Zellen etc. Ebenso gibt es konstitutiv aktive wie auch induzierbare Promotoren, wobei die Induktion durch eine Vielzahl von Umwelteinflüssen stimuliert werden kann. Es sind starke Promotoren, welche eine hohe Transkription der regulierten Sequenz aktivieren können, sowie schwache Promotoren beschrieben. Oft sind Promotoren stark reguliert. Ein Promotor gemäß der vorliegenden Offenbarung kann einen endogenen nativ vorliegenden Promotor bezeichnen, oder einen künstlichen (synthetischen/chimär) oder transgenen Promotor, der entweder aus einer anderen Spezies gewonnen wurde, oder der künstlich oder synthetisch/chimär ist, d.h. so nicht in der Natur vorkommt bzw. aus verschiedenen Promotor-Elementen zusammengesetzt ist.

Die Begriffe 3' nicht-kodierende Sequenz, Transkriptionsterminator, Terminator oder Terminationssequenz, wie hierin verwendet, beziehen sich auf DNA-Sequenzen, die stromabwärts, das heißt in 3'-Richtung einer kodierenden Sequenz lokalisiert sind und umfassen Polyadenylierungserkennungssequenzen und andere Sequenzen, die regulatorische Signale kodieren, die dazu in der Lage sind, die mRNA-Prozessierung und/oder die Genexpression zu beeinflussen. Das Polyadenylierungssignal ist üblicherweise dadurch gekennzeichnet, dass es das Hinzufügen von poly-A-Nukleotiden an das 3'-Ende eines mRNA-Vorläufers bewirkt.

Der Begriff funktional verknüpft, wie hierin verwendet, bezieht sich auf die Verbindung von Nukleinsäure-Sequenzen auf einem einzigen Nukleinsäure-Fragment, sodass die einzelnen Fragmente Gene oder regulatorischen Sequenzen oder andere Regionen physikalisch miteinander verbunden sind und sich die einzelnen Sequenzen oder Segmente gegenseitig regulieren oder hybridisieren oder beeinflussen können. Ein Promotor ist etwa dann funktional verknüpft mit einer kodierenden Sequenz, sofern er dazu in der Lage ist, die Expression dieser kodierenden Sequenz zu regulieren, das heißt, die kodierende Sequenz steht dann unter der transkriptionellen Kontrolle des entsprechenden Promotors. Darüber hinaus können kodierende Sequenzen funktional mit regulatorischen Sequenzen, entweder im Sinn- oder Anti-Sinn-Orientierung, verknüpft sein. Komplementäre RNA-Regionen können etwa, entweder direkt oder indirekt, 5' mit der Ziel-mRNA, oder 3' mit der Ziel-mRNA, oder innerhalb der Ziel-mRNA verknüpft sein, oder eine erste Region der Komplementarität ist 5' und ihr Komplement ist 3' mit der Ziel-mRNA funktional verknüpft.

Der Begriff stabile Transformation oder stabile Integration, wie hierin verwendet, bezieht sich auf das Einbringen einer Nukleinsäure-Sequenz von Interesse, z.B. in der Form einer DNA-Reparatur-Matrize, oder eines Teils davon, oder eines geeigneten Vektors, in das Genom einer pflanzlichen Zielstruktur von Interesse, wobei das Genom sowohl das nukleäre als auch das extranukleäre Genom, etwa das Genom von Organellen, umfasst, was in einer genetisch stabilen und dadurch vererbbaren Veränderung des Genoms resultiert. Im Gegensatz dazu bezieht sich der Begriff transiente Transformation oder transientes Einbringen oder transiente Integration, wie hierin verwendet, auf das Einbringen einer Nukleinsäure-Sequenz von Interesse in ein pflanzliches Kompartiment von Interesse, umfassend den Kern, Organellen oder das Zytoplasma oder ein weiteres Kompartiment innerhalb einer pflanzlichen Zelle, wodurch entweder die Transkription und/oder Translation oder, für den Fall das Direkteffektor-Moleküle (DNA, RNA, oder Protein) die eingebrachten Moleküle oder Komplexe ihre Wirkung innerhalb der pflanzlichen Zelle entfalten können, es jedoch nicht zu einer stabilen Integration in das Genom der Zelle und damit nicht zu einer Vererbung der entsprechenden Sequenzen und/oder Effektor-Moleküle kommt.

Der Begriff Genom, wie hierin verwendet, bezieht sich auf die Gesamtheit des genetischen Materials, umfassend Gene sowie nicht-kodierende Sequenzen, die in einer Zelle eines Organismus oder eines Virus oder eine Organells vorhanden sind und umfasst daher sowohl das nukleäre (falls vorhanden) als auch das extra-nukleäre (falls vorhanden) Genom. Zudem bezieht sich der Begriff Genom, wie hierin verwendet, auf den vollständigen Satz an Chromosomen, der als (haploide) Einheit von einem Eltern-Organismus vererbt wird.

Eine Motivation zur Entwicklung immer neuer molekularer Marker in Pflanzenspezies ist das Potenzial, eine erhöhte Effizienz der gezielten Pflanzenzüchtung durch Marker-gestützte Selektion (Marker Assisted Selection (MAS)) zu erzielen. Genetische Marker-Allele, oder alternativ die oben geschilderten quantitativen Merkmals-Loci (QTL)Allele werden dafür verwendet, um Pflanzen oder Pflanzenmaterial oder eine pflanzliche Zelle zu identifizieren, die einen gewünschten Genotyp an einem oder mehreren Locus/Loci enthalten, und von denen angenommen wird, dass sie den erwünschten Genotyp zusammen mit einem gewünschten Phänotyp an ihre Nachkommen weitergeben können. Genetische Marker-Allele (oder QTL-Allele) können auch verwendet werden, um Pflanzen zu identifizieren, die einen gewünschten Genotyp an einem Locus, oder an mehreren nicht-verknüpften oder verknüpften Loci, z.B. einem Haplotyp, enthalten, von denen man annimmt, dass sie den gewünschten Genotyp zusammen mit einem gewünschten Phänotyp an ihre Nachkommen weitergeben können. Zum Zwecke der Marker-gestützten Selektion kann der Begriff Marker, wie hierin verwendet, daher sowohl Marker als auch QTL-Loci umfassen. Sobald für einen gewünschten Phänotyp und einen polymorphen chromosomalen Locus, z.B. ein Marker-Locus oder einen QTL, bestimmt wurde, dass sie gemeinsam segregieren, ist es möglich, diese polymorphen Loci zu verwenden, um auf Allele hin zu selektionieren, die dem gewünschten Phänotyp entsprechen. Dieses Vorgehen wird als Marker-gestützte Selektion (MAS) bezeichnet. Hierzu wird eine Nukleinsäure-Sequenz, die der Marker-Nukleinsäure entspricht, in einer biologischen Probe von einer Pflanze, die analysiert werden soll, nachgewiesen. Dieser Nachweis kann in der Form von Hybridisierung einer Nukleinsäure-Sonde an einen Marker, z.B. unter Verwendung von Allel-spezifischer Hybridisierung, Southern Blot Analyse, Northern Blot Analyse, *in situ* Hybridisierung, Hybridisierung von Primern gefolgt von PCR-Amplifizierung einer Region des Markers, oder dergleichen, oder durch jegliche beliebige Kombination davon erfolgen. Eine Vielzahl von Verfahren zum Nachweis von Markern ist dem Fachmann auf dem Gebiet bekannt. Nachdem die Anwesenheit oder Abwesenheit eines spezifischen Markers in der biologischen Probe von Interesse, umfassend mindestens eine pflanzliche Zelle, bevorzugt eine meristematische Zelle, bestätigt wurde, wird die entsprechende Pflanze ausgewählt, und kann im Anschluss verwendet werden, um Nachkommen- Pflanzen durch selektive Züchtung zu gewinnen.

Ebenso können die Verfahren gemäß der vorliegenden Erfindung verwendet werden, um eine *in planta* gezielt veränderte meristematische Zelle auf die Anwesenheit oder Abwesenheit eines spezifischen Markers hin zu analysieren. Aus dieser meristematischen Zelle können dann bevorzugt entweder weibliche oder männliche Gameten oder Keimzellen gewonnen werden, wobei insbesondere die Pollen einer derart *in planta* veränderten Pflanze unmittelbar für die anschließende selektive Züchtung verwendet werden können. Da in der klassischen Pflanzenzüchtung ein Bedarf danach besteht, Merkmale von Interesse in eine Zielpflanze einzuführen, die eine hohe Ausbeute und/oder andere wünschenswerte Merkmale kodieren, um hierdurch verbesserte Pflanzensorten zu entwickeln, besteht ein großer Bedarf nach Marker-gestützter Selektion, um hierdurch die Zeit für aufwendige und teure Überprüfung einer großen Anzahl von Proben verkürzen zu können.

Gemäß den Verfahren der vorliegenden Offenbarung können auch gezielt phänotypische Marker in eine pflanzliche Zielstruktur von Interesse eingebracht werden. Ein phänotypischer Marker, wie hierin verwendet, bezeichnet einen selektionierbaren Marker der die Überprüfung und Auffindbarkeit einer pflanzlichen Zelle oder Zielstruktur von Interesse erleichtert. Phänotypische Marker umfassen allgemein entweder positiv oder negativ selektionierbare Marker, etwa optische Marker oder (Antibiotika)Resistenz-Gene. Jeglicher pflanzliche Marker, der für eine pflanzliche Zielstruktur von Interesse, insbesondere eine meristematische Zelle, nützlich ist, kann verwendet werden. Selektionierbare oder auffindbare Marker umfassen üblicherweise DNA-Segmente, die es gestatten eine Zelle, oder auch ein durch ein "Tag" markiertes Molekül innerhalb einer Zelle von Interesse, oft unter bestimmten Bedingungen, zu identifizieren. Solche Marker können eine Aktivität kodieren, die ausgewählt ist aus, aber nicht beschränkt ist auf, die Produktion von RNA, Peptid oder Protein, oder die Marker können eine Bindungsstelle für RNA, Peptide, Proteine, anorganische und organische Verbindungen oder Zusammensetzungen und dergleichen bereitstellen. Beispielhafte selektionierbare Marker umfassen, ohne darauf beschränkt zu sein, DNA-Segmente, die Restriktionsenzymschnittstellen umfassen, DNA-Segmente, die eine fluoreszierende Sonde umfassen, DNA-Segmente, die Produkte kodieren, die Resistenz gegenüber ansonsten toxischen Verbindungen vermitteln, umfassend Antibiotika, wie zum Beispiel Spectinomycin, Ampicillin, Kanamycin, Tetracyclin, BASTA, Neomycin-Phosphotransferase II (NEO) und Hygromycin-Phosphotransferase (HPT), DNA-Segmente, die Produkte kodieren, die einer pflanzlichen Zielzelle von Interesse unter natürlichen Umständen fehlen würden, z.B. tRNA-Gene, auxotrophe Marker und dergleichen, DNA-Segmente, die Produkte kodieren, die leicht identifizierbar sind, insbesondere optisch wahrnehmbare Marker, z.B. phänotypische Marker wie β-Galaktosidase, GUS, fluoreszierende Proteine, wie zum Beispiel grün-fluoreszierendes Protein (GFP) sowie weitere fluoreszierende Proteine wie zum Beispiel blau (CFP), gelb (YFP), oder rot (RFP)-fluoreszierende Proteine sowie Oberflächenproteine, wobei insbesondere solche fluoreszierenden Proteine von Interesse sind, die eine hohe Fluoreszenz-Intensität zeigen, da diese Proteine auch in tieferen Gewebeschichten identifiziert werden können, sofern keine einzelne Zelle, sondern eine komplexe pflanzliche Zielstruktur oder ein Pflanzenmaterial oder eine Pflanze umfassend mehrere Gewebe- oder Zell-Typen analysiert werden soll, neue Primer-Stelle für PCR, die Aufnahme von DNA-Sequenzen, die nicht durch Restriktions-Endonukleasen oder andere DNA-modifizierende Enzyme oder Effektor-Domänen gemäß der vorliegenden Offenbarung modifiziert werden können, DNA-Sequenzen, die für spezifische Modifikationen, z.B. epigenetische Modifikationen, z.B. Methylierungen, benötigt werden sowie DNA-Sequenzen, die ein PAM-Motiv tragen, das von einem geeigneten CRISPR-System gemäß der vorliegenden Offenbarung erkannt werden kann, sowie DNA-Sequenzen, denen ein PAM-Motiv, wie es in einer natürlichen endogenen Pflanzengenom-Sequenz vorkommt, fehlt.

Die Verfahren gemäß der vorliegenden Erfindung können speziell für die Züchtung von Pflanzen eingesetzt werden, um ein oder mehrere transgene Merkmale in eine Pflanze, der mindestens einen pflanzliche Zielstruktur von Interesse, umfassend mindestens eine meristematische Zelle, einzubringen. Derzeit werden transgene Merkmale zufällig durch Transformationssysteme in das Pflanzengenom eingebracht, wobei dies mit physikalisch/mechanischen Verfahren, oder auf biologischem Wege, umfassend etwa den biolistischen Beschuss von Pflanzenmaterial oder die Transformation mit *Agrobacterium* und/oder virale Vektoren erfolgt. Im Laufe der letzten Jahre wurden mehr und mehr spezifische Protokolle zur gezielten Einbringung von Transgenen in das Genom von pflanzlichen Zellen etabliert. Eine wichtige Technologie ist etwa die ortsspezifische Integration (site-specific integration (SSI)), die die gerichtete Einbringung eines Transgens an denselben Ort in einem Chromosom erlaubt, an den auch ein bereits zuvor eingeführtes Transgen eingebracht wurde. Darüber hinaus wurden im Laufe der letzten Jahre mehr und mehr gezielt entworfene ziel-spezifische Nukleasesysteme etabliert, um die Spaltung einer chromosomalen Zielststelle durch diese Nukleasen zu unterstützen. Die derzeit häufig verwendeten Nukleasen zur Genomeditierung in eukaryontischen Genomen umfassen z.B. Mega-Nukleasen, Zink-Finger-Meganukleasen, Zink-Finger-Nukleasen, Transcription activator-like effector nuclease (TALENs) sowie eine stetig wachsende Familie an CRISPR-Nukleasen sowie gezielt veränderten Varianten und katalytisch aktiven Fragmenten davon. Gerade CRISPR-basierte Nuklease-Systeme haben sich als äußerst zweckdienlich für eine hochpräzise ziel-spezifische und programmierbare Veränderung von Nukleinsäure-Zielregionen von Interesse erwiesen. Da das CRISPR-System durch eine, oftmals chimäre, gRNA geführt wird und nicht rein protein-basierte Targetierung und Ziel-Selektion erlaubt, kann hierdurch auch ein hohes Maß an Sicherheit und eine Reduzierung von unerwünschten Off-Target-Effekten erzielt werden. Darüber hinaus bietet die vorliegende Offenbarung einen weiteren Vorteil des intrinsisch aus zwei Komponenten bestehenden CRISPR-Systemen, nämlich dahingehend, dass entweder eine gRNA und/oder eine CRISPR-Nuklease, oder eine Variante, oder ein katalytisch aktives Fragment davon, gezielt mit einer weiteren Effektor-Domäne versehen werden kann, wodurch die Variabilität und der Einsatzbereich des CRISPR-Systems deutlich erweitert werden kann. Durch eine Reprogrammierung einer CRISPR-Nuklease kann etwa eine Nuklease-Null-Variante erzeugt werden, die ihre katalytische Aktivität bezüglich der Spaltung von DNA verloren hat, aber ihre DNA-Erkennungsfunktion beibehält. Durch die Kombination eines derartig veränderten Moleküls mit einer Effektor-Domäne, insbesondere einer Effektor-Domäne, welche die epigenetische Modulation des Genoms einer Zielzelle von Interesse gestattet, können daher durch das transiente Einbringen eines CRISPR-Systems, umfassend mindestens eine gRNA, mindestens eine CRISPR-Nuklease sowie mindestens eine Effektor-Domäne gezielt epigenetische Veränderungen, z.B. Methylierungen, Demethylierungen, Acetylierungen, De-Acetylierungen, Phosphorylierungen, De-Phosphorylierungen, oder Ubiquitinierungen in ein Histonprotein, oder ein beliebiges anderes Protein innerhalb eines Nukleosoms im Zellkern einer eukaryontischen Zelle von Interesse eingebracht werden. Hierdurch können, selbst wenn die hierfür eingesetzten CRISPR-Systeme nur transient in eine pflanzliche Zielstruktur von Interesse eingebracht und somit selbst nicht vererbt werden, gezielt strukturelle Anpassungen chromosomaler Regionen bewirkt werden, um veränderte Zustände der Aktivierung zu erreichen, wobei diese strukturellen Anpassungen dann potenziell vererbbar sind.

Die hierin offenbarten CRISPR-Systeme sowie insbesondere die hierin offenbarten Verfahren zur gezielten Veränderung mindestens einer meristematische Zelle sind insbesondere für die Genomeditierung von pflanzlichen Zellen oder Organismen geeignet, da durch die hohe Präzision Off-Target-Schnitte vermieden werden können, die oftmals letal für die Zielzellen sind, oder zu unerwünschten Nebenwirkungen führen.

In einer Ausführungsform kann die CRISPR-Nuklease-Komponente des CRISPR-Systems, oder eine Variante, umfassend Nickasen oder Nuklease-Null-Varianten, oder ein aktives Fragment davon stabil in einem Pflanzengenom integriert vorliegen. Die Expression der CRISPR-Nuklease kann unter der Kontrolle eines pflanzenzspezifischen Promotors stehen, wobei der Promotor ein konstitutiver Promotor, ein gewebespezifischer Promotor oder ein induzierbarer Promotor, z.B. ein Temperatur-, Stress-, Entwicklungsstadiums- oder chemisch-induzierbarer Promotor sein kann. In der Abwesenheit einer weiteren essenziellen Komponente des CRISPR-Systems, d.h. einer künstlichen gRNA oder einer crRNA wird die Cas-Nuklease nicht dazu in der Lage sein, DNA zu schneiden und/oder zu erkennen, sodass die schiere Anwesenheit der Cas-Nuklease keinen oder nur geringe Einflüsse auf die pflanzliche Zelle von Interesse und ihren Metabolismus hat. Daher ist es ein Vorteil der hierin offenbarten Verfahren für die Pflanzenzüchtung und - Entwicklung, dass Zelllinien oder transgene Pflanzenzellen hergestellt und propagiert werden können, die konstitutiv oder induzierbar eine Cas-Nuklease, oder eine Variante oder ein katalytisch aktives Fragment davon, exprimieren können, ohne dass dies negative Folgen auf Zell-Integrität oder - Lebensfähigkeit hätte. Um die Aktivität einer CRISPR-Nuklease, sei es wie oben geschildert stabil integriert oder transient bereitgestellt, zu erzielen, ist als weiterer Sicherheitsmechanismus stets die Anwesenheit einer gRNA oder einer crRNA notwendig, die durch eine Vielzahl von Verfahren in eine pflanzliche Zielstruktur umfassend mindestens eine meristematische Zelle von Interesse stabil oder transient eingeführt werden kann. Die gRNA kann etwa als transkribierbares DNA-Konstrukt in Form eines genetischen Konstrukts wie eines Vektors in die Zelle eingebracht werden, wobei die gRNA dann, unter der Kontrolle eine adäquaten Promotors, konstitutiv oder induzierbar transkribiert und somit funktionsfähig bereitgestellt werden kann. Alternativ kann die gRNA auch direkt als RNA in eine pflanzliche Zielstruktur von Interesse eingebracht werden. CRISPR-Nuklease und gRNA können somit simultan oder zeitversetzt eingebracht werden, wobei es bevorzugt ist, dass gRNA und CRISPR-Nuklease derart räumlich und zeitlich bereitgestellt werden, dass die weniger stabile RNA und die Protein-CRISPR-Nuklease in stöchiometrisch idealer Zusammensetzung in dem Zellkompartiment von Interesse interagieren können. Sofern das Ziel der gezielten Veränderung eine RNA ist, so ist das Kompartiment von Interesse das Zytoplasma einer Zielzelle. Sofern die Nukleinsäure-Zielregion von Interesse genomische DNA oder das Nukleosom ist, so ist das Kompartiment von Interesse der Zellkern einer pflanzlichen Zielstruktur, umfassend mindestens eine meristematische Zelle. In dieser Ausgestaltung kann es notwendig sein, dass gRNA und/oder CRISPR-Nuklease mit geeigneten Kernlokalisationssequenzen funktional verknüpft sind, damit die CRISPR-Moleküle bzw. der CRISPR-Komplex aus gRNA und CRISPR-Nuklease sowie optional damit assoziierte Effektor-Domänen ihren Wirkort erreichen können. In einer anderen Ausführungsform, sofern die Nukleinsäure-Zielregion in einem Organell, insbesondere Plastiden, lokalisiert ist, kann die Anwesenheit von Plastidenlokalisationssequenzen, z.B. mitochondrialen Lokalisationssequenzen oder Chloroplastenlokalisationssequenzen notwendig sein, um die CRISPR-Werkzeuge gemäß der vorliegenden Offenbarung an den Wirkort zu dirigieren.

Eine gRNA gemäß der vorliegenden Offenbarung kann ein einzelnes Molekül darstellen, oder in Form von zwei separaten RNAs, die crRNA und/oder tracrRNA entsprechen, verwendet werden oder vorliegen.

Rekombinant wie hierin verwendet bedeutet eine Nukleinsäure- oder Aminosäuresequenzabfolge, wie sie so insbesondere in ihrer Gesamtheit nicht natürlich vorkommt. Ferner umfasst der Begriff rekombinant auch solche Nukleinsäure- oder Aminosäuresequenzabfolgen, wie sie zwar bezüglich ihrer Nukleinsäure- oder Aminosäuresequenz so natürlich vorkommen, jedoch durch eine gezielte Modifikation oder Synthese gewonnen wurden, wie z.B. synthetisch gewonnene Nukleinsäure- oder Aminosäuresequenzen oder durch biotechnologische, z.B. fermentative Verfahren gewonnene Nukleinsäure- oder Aminosäuresequenzen, welche so zwar in der Natur vorkommen, jedoch gezielt in einem anderen als dem Ursprungsorganismus produziert wurden.

Der Begriff Epigenetik oder epigenetisch, wie hierein verwendet, beschreibt die strukturelle Anpassung chromosomaler Regionen, um veränderte Zustände der Aktivierung zu kodieren, zu signalisieren, zu konservieren und potentiell an die Nachkommen einer Zelle zu vererben. Demnach werden über Veränderungen, die nicht in der genomischen DNA selbst kodiert sind, strukturelle und potentiell vererbbare Veränderungen erzielt.

Wann immer die vorliegende Offenbarung auf Sequenzhomologien oder Sequenzidentitäten von Nukleinsäure- oder Proteinsequenzen in Form von Prozentangaben Bezug nimmt, beziehen sich diese Angaben auf Werte, wie sie unter Verwendung von EMBOSS Water Pairwise Sequence Alignments (Nucleotide)
(http://www.ebi.ac.uk/Tools/psa/emboss_water/nucleotide.html) für Nukleinsäuresequenzen bzw, EMBOSS Water Pairwise Sequence Alignments (Protein)
(http://www.ebi.ac.uk/Tools/psa/emboss_water/) für Aminosäuresequenzen berechnet werden können. Bei vom European Molecular Biology Laboratory (EMBL) European Bioinformatics Institute (EBI) zur Verfügung gestellten Tools für lokale Sequenzalignments verwenden einen modifizierten Smith-Waterman Algorithmus (siehe
http://www.ebi.ac.uk/Tools/psa/ und Smith, T.F. & Waterman, M.S. "Identification of common molecular subsequences" Journal of Molecular Biology, 1981 147 (1):195-197). Ferner wird hierbei bei der Durchführung des jeweiligen paarweisen Alignments zweier Sequenzen unter Verwendung des modifizierten Smith-Waterman Algorithmus auf die vom EMBL-EBI derzeit angegebenen Default Parameter Bezug genommen. Diese lauten (i) für Aminosäuresequenzen: Matrix = BLOSUM62, Gap open penalty = 10 und Gap extend penalty = 0,5 sowie (ii) für Nukleinsäuresequenzen: Matrix = DNAfull, Gap open penalty = 10 und Gap extend penalty = 0,5.

Im Sinne der vorliegenden Erfindung werden unter "homologen Sequenzen" oder "Homologe" oder vergleichbaren Termini Nukleinsäuresequenzen verstanden, welche den gleichen phylogenetischen Ursprung haben. Vorzugsweise üben Proteine, die durch diese Nukleinsäuresequenzen kodiert werden, die gleiche Funktion aus. Homologe Nukleinsäuresequenzen weisen mindestens 70%, vorzugsweise mindestens 75%, mindestens 80%, mindestens 85% oder mindestens 90%, besonders bevorzugt mindestens 95%, mindestens 96%, mindestens 97%, mindestens 98% oder mindestens 99% Sequenzidentität auf.

Nukleinsäure-Zielregion, wie hierin verwendet, bezieht sich auf jegliche genomische sowie extrachromosomale DNA oder RNA, insbesondere mRNA, eines Zielorganismus oder einer Zielzelle, deren Modifikation erwünscht ist und die durch die hierin offenbarten Verfahren und Konstrukte bewirkt werden kann und ist bewusst nicht auf Gen-Regionen, d.h. Regionen, welche die Information zur Transkription einer mRNA tragen, beschränkt. Diese Zielregionen sind somit natürliche oder endogene Zielregionen, wobei die Begriffe endogen und natürlich in diesem Kontext austauschbar verwendet werden. Darüber hinaus ist der Begriff Nukleinsäure-Zielregion nicht auf eine endogene Sequenz beschränkt. Im Fall, dass eine künstliche Nukleinsäure-Zielregion zuvor in eine Zielzelle einer Zielstruktur von Interesse eingebracht wurde, kann sich der Begriff Nukleinsäure-Zielregion somit auch auf eine künstlich eingebrachte Nukleinsäure-Zielregion beziehen.

Komplementär oder Komplementarität, wie hierin verwendet, beschreibt die Beziehung zwischen zwei DNA- oder RNA-Nukleinsäureregionen, welche auf Grund ihrer Nukleobasen nach dem Schlüssel-und-Schloss Prinzip zueinander passen und Wasserstoffbrücken untereinander eingehen (hybridisieren). Hierbei gelten nach der Watson-Crick Basenpaarung die Basen Adenin und Thymin/Uracil bzw, Guanin und Cytosin als komplementär. Auch weitere Nicht-Watson-Crick-Paarungen, wie Reverse-Watson-Crick-, Hoogsteen-, Reverse-Hoogsteen und Wobble-Paarung sind vom Begriff komplementär umfasst, sofern die entsprechenden Basenpaare Wasserstoffbrücken zueinander eingehen, d.h. zwei unterschiedliche Nukleinsäurestränge miteinander auf Grund ihrer Komplementarität hybridisieren können.

Unter "Hybridisieren" oder "Hybridisierung" wird ein Vorgang verstanden, bei dem sich ein einzelsträngiges Nukleinsäuremolekül an einen weitestgehend komplementären Nukleinsäurestrang anlagert, d.h. Basenpaarungen eingeht. Standardverfahren zur Hybridisierung sind beispielsweise in Sambrook et al. 2001 beschrieben. Vorzugsweise wird darunter verstanden, dass mindestens 60%, weiter bevorzugt mindestens 65%, 70%, 75%, 80% oder 85%, besonders bevorzugt 90%, 91%, 92%, 93%, 94%, 95%, 96%, 97%, 98% oder 99% der Basen der Nukleinsäuresequenz eine Basenpaarung mit dem weitestgehend komplementären Nukleinsäurestrang eingehen. Die Möglichkeit einer solchen Anlagerung hängt von der Stringenz der Hybridisierungsbedingungen ab. Der Begriff "Stringenz" bezieht sich auf die Hybridisierungsbedingungen. Hohe Stringenz ist dann gegeben, wenn eine Basenpaarung erschwert ist, niedrige Stringenz, wenn eine Basenpaarung erleichtert ist. Die Stringenz der Hybridisierungsbedingungen hängt beispielsweise von der Salzkonzentration bzw. lonenstärke und der Temperatur ab. Generell kann die Stringenz durch eine Erhöhung der Temperatur und/oder eine Erniedrigung des Salzgehaltes erhöht werden. Unter "stringenten Hybridisierungsbedingungen" sind solche Bedingungen zu verstehen, bei denen eine Hybridisierung überwiegend nur zwischen homologen Nukleinsäuresequenzen stattfindet. Der Begriff "Hybridisierungsbedingungen" bezieht sich dabei nicht nur auf die bei der eigentlichen Anlagerung der Nukleinsäuren herrschenden Bedingungen, sondern auch auf die bei den anschließenden Waschschritten herrschenden Bedingungen. Stringente Hybridisierungsbedingungen sind beispielsweise Bedingungen, unter denen überwiegend nur solche Nukleinsäuren hybridisieren, die mindestens 70%, vorzugsweise mindestens 75%, mindestens 80%, mindestens 85% oder mindestens 90%, besonders bevorzugt mindestens 95%, mindestens 96%, mindestens 97%, mindestens 98% oder mindestens 99% Sequenzidentität aufweisen. Stringente Hybridisierungsbedingungen sind beispielsweise: Hybridisieren in 4 x SSC bei 65 °C und anschließendes mehrfaches Waschen in 0,1 x SSC bei 65 °C für insgesamt etwa 1 Stunde. Der hier verwendete Begriff "stringente Hybridisierungsbedingungen" kann auch bedeuten: Hybridisieren bei 68 °C in 0,25 M Natriumphosphat, pH 7,2, 7 % SDS, 1 mM EDTA und 1 % BSA für 16 Stunden und anschließendes zweimaliges Waschen mit 2 x SSC und 0,1 % SDS bei 68 °C. Bevorzugt findet eine Hybridisierung unter stringenten Bedingungen statt.

### Detaillierte Beschreibung

Die vorliegende Erfindung betrifft ein Verfahren zur Herstellung einer Maispflanze wie in Anspruch 1 definiert.

Meristematische Zellen gehören einem Gewebetyp innerhalb einer Pflanze an, welcher als Meristem oder auch Bildungsgewebe bezeichnet wird. Wie auch Stammzellen in tierischen Organismen besitzen pflanzliche meristematische Zellen als undifferenzierte Zellen die Fähigkeit, sich je nach Umwelteinfluss zu jeglichem spezialisierten Zelltyp zu differenzieren.

In allen Ausführungsformen können die einzelnen Komponenten simultan oder nacheinander auf demselben oder auf unterschiedlichen Konstrukten eingebracht werden. In einigen Ausführungsformen der transienten Einbringung kann es vorteilhaft sein, zunächst das Konstrukt einzubringen, welches eine oder mehrere Proteinkomponenten des Systems, d.h. CRISPR Nuklease, Variante oder katalytisch aktives Fragment davon und optional eine Effektor-Domäne, trägt. Optional, wenn es sich um ein DNA-Konstrukt handelt, kann dieses mindestens eine Konstrukt dann zunächst von der Zelle translatiert werden. Zeitversetzt können dann die Konstrukte eingebracht werden, die gRNA und die optionalen weiteren DNA-Reparatur-Matrizen und/oder Effektor-Domänen tragen. Hierdurch kann sichergestellt werden, dass die instabilere gRNA unmittelbar mit der CRISPR Nuklease von Interesse interagieren kann und kein Abbau der gRNA eine wirksame DNA Editierung unterbindet.

Gemäß der vorliegenden Erfindung wird daher ein spezielles Verfahren bereitgestellt, welches gezielt die geringe Population an meristematischen Zellen in einer Pflanze als pflanzliche Zielstruktur entweder direkt oder indirekt ansteuern kann. Die mindestens eine meristematische Zielzelle kann direkt oder indirekt angesteuert werden, d.h. mindestens ein rekombinantes Konstrukt gemäß der vorliegenden Offenbarung kann direkt in die mindestens eine meristematische Zielzelle eingebracht werden bzw. das mindestens eine rekombinante Konstrukt kann mit Hilfe eines geeigneten Vektors in eine beliebige pflanzliche Zelle oder ein beliebiges pflanzliches Gewebe eingebracht werden, wobei das mindestens eine rekombinante Konstrukt im Anschluss zu der pflanzlichen Zielstruktur transportiert werden kann. Dies erfolgt etwa durch die systemische Ausbreitung von mindestens einem durch einen Vektor in eine pflanzliche Zelle oder in ein pflanzliches Gewebe eingeführten rekombinanten Konstrukt.

Eine pflanzliche Zielstruktur, wie hierin verwendet, umfasst mindestens eine pflanzliche meristematische Zelle, welche als Gewebe, Pflanzenmaterial, als ganze Pflanze oder als isolierte Zelle vorliegen kann, wobei die pflanzliche meristematische Zelle zudem mindestens eine Nukleinsäure-Zielregion enthält. Die in der pflanzlichen Zielstruktur enthaltene mindestens eine Nukleinsäure-Zielregion umfasst DNA- und RNA-Sequenzen und kann innerhalb der Zielstruktur chromosomal oder extrachromosomal vorliegen. Die zielgerichteten CRISPR basierten Verfahren zur Veränderung einer Nukleinsäure-Zielregion von Interesse können daher bei der Modifikation genomischer DNA, umfassend die epigentische Modifikation von genomischer DNA, oder der Modifikation von plastidärer oder mitochondrialer DNA zum Einsatz kommen, ebenso wie bei der Modifikation, etwa in Form von Silencing, von RNA.

In einem Aspekt der vorliegenden Erfindung, welcher sich auf die Einführung einer gezielten Nukleinsäure-Veränderung in eine nicht chromosomale Zielstruktur bezieht, umfasst der Begriff pflanzliche Zielstruktur, wie hierin verwendet, mindestens eine pflanzliche Zelle, welche als Gewebe, Pflanzenmaterial, als ganze Pflanze oder als isolierte Zelle vorliegen kann, wobei die pflanzliche Zelle zudem mindestens eine Nukleinsäure-Zielregion, umfassend DNA und RNA, enthält.

Gemäß der vorliegenden Erfindung wird mindestens eine Nukleinsäure-Zielregion in einer pflanzlichen meristematischen Zelle als Zielstruktur durch transient eingeführte CRISPR/Cas-Werkzeuge und/oder gegebenenfalls weitere Effektor-Domänen verändert. Da die so veränderte mindestens eine meristematische Zelle die gezielte Veränderung in der Nukleinsäure-Zielregion durch die anschließende Zellteilung und Differenzierung unmittelbar und direkt an ihre Nachkommen weitergeben kann bedarf das Verfahren gemäß der vorliegenden Erfindung keiner weiteren Kreuzungs- und Selektionsschritte, um eine Pflanze, Pflanzenmaterial oder eine pflanzliche Zelle mit der erwünschten gezielten Veränderung bereitzustellen. Vielmehr können aus den embryonalen oder ebenso aus sekundären Meristemen, wie z.B. Pollen oder Ovarien, optional unter Durchführung einer Selbstung oder einer Fremdbefruchtung, pflanzliche Organismen oder pflanzliche Zielstrukturen gewonnen werden, welche die gezielt eingeführte Veränderung tragen.

Dabei bietet das Verfahren gemäß der vorliegenden Erfindung den weiteren Vorteil, dass die CRISPR/Cas-Werkzeuge und/oder gegebenenfalls weitere Effektor-Domänen nur transient in die pflanzliche Zielstruktur, d.h. eine meristematische Zelle oder ein meristematisches Gewebe, eingebracht werden, sodass keine stabile Integration der CRISPR/Cas Werkzeuge wie CRISPR Nuklease und gRNA und ggf. regulatorischen Sequenzen sowie ggf. weiterer Effektor-Domänen in die endogenen chromosomalen oder endogenen extrachromosomalen Nukleinsäuren der pflanzlichen Zielstruktur erfolgt.

Gemäß der vorliegenden Offenbarung wurde gefunden, dass durch die Ausnutzung des Wirkmechanismus der RNA-dirigierten DNA-Modifikation der CRISPR-Cas Werkzeuge auch weitere Effektor-Domänen, gemäß den hierin bereitgestellten Verfahren, eingesetzt werden können, wodurch sich das Spektrum der gezielten Genom Editierung verbreitern lässt. Es können entweder die CRISPR Nuklease Variante oder das katalytisch aktive Fragment davon oder die gRNA oder beide davon mit einer Effektor-Domäne verknüpft werden.

Eine Effektor-Domäne wie hierin verwendet umfasst DNA- bzw. RNA-modifizierende oder DNA- bzw. RNA-bindende Polypeptide oder Nukleinsäuren, umfassend alle Arten von monomeren, dimeren oder multimeren Nukleasen, wie z.B. TALE Nukleasen, Meganukleasen, Zink-Finger-Nukleasen, Ribonuklease, Desoxyribonukleasen, Exonukleasen, Endonukleasen und Restriktionsendonukleasen vom Typ I, II, III oder IV und dergleichen und umfassend Nickasen, Aktivatoren und Repressoren der Transkription, Phosphatasen, Glykosylasen, oder Enzyme, welche epigenetische Veränderungen bewirken können, wie beispielsweise Acetylasen, Methylasen, Methyltransferasen, Proteine, welche methylierte DNA binden können, oder Histon-Deacetylasen, Aptamere, umfassend einzelsträngige DNA- oder RNA-Sequenzen sowie Peptide, fluoreszierende Proteine, Markernukleinsäure- oder Markeraminosäuresequenzen, und dergleichen, und Kombinationen davon. Im Bezug auf Enzyme oder Polypeptide allgemein, umfasst der Begriff "Effektor-Domäne" auch eine katalytische Domäne oder Kern-Domäne des jeweiligen Enzyms oder Polypeptids, z.B. einem Bindeprotein, wobei die katalytische Domäne bzw. Kern-Domäne nach wie vor dazu in der Lage ist, die enzymatische oder die Bindefunktion des jeweiligen nativen Enzyms oder Polypetids zu erfüllen. Der Entwurf derartiger trunkierter Domänen und deren Anpassung auf die gewünschte Funktion hin ist dem Fachmann auf dem Gebiet bekannt.

Hierin werden Verfahren und Konstrukte bereitgestellt, bei welchen gRNA und/oder CRISPR Nuklease oder das katalytisch aktive Fragment davon bereits verknüpft mit einer weiteren Effektor-Domäne als oder auf einem rekombinanten Konstrukt bereitgestellt werden. Die gRNA und/oder die CRISPR Nuklease, umfassend mindestens eine Effektor-Domäne, werden dann auf mindestens einem rekombinanten Konstrukt in eine Zielstruktur eingebracht, um dort nach Transkription und optional Translation zu einem funktionsfähigen Komplex assemblieren zu können.

In einer weiteren Ausführungsform wird ein Verfahren bereitgestellt, bei welchem die mindestens eine gRNA sowie die mindestens eine CRISPR Nuklease oder das katalytisch aktive Fragment davon und/oder mindestens ein weitere Effektor-Domäne separat auf unterschiedlichen rekombinanten Konstrukten bereitgestellt werden. Gemäß dieses Verfahrens kann die gRNA Komponente als DNA oder RNA bereitgestellt werden, die CRISPR Nuklease oder Variante oder das katalytisch aktive Fragment davon kann als DNA oder RNA oder als Polypeptidsequenz bereitgestellt werden, und die Effektor-Domäne kann als DNA oder RNA oder als Polypeptidsequenz bereitgestellt werden. Die gRNA und die CRISPR Nuklease, optional umfassend mindestens eine Effektor-Domäne, können somit *in vitro* vorassembliert und dann in eine Zielstruktur eingebracht werden.

Gemäß einer Ausführungsform der vorliegenden Erfindung, welche die simultane Einbringung mindestens einer gRNA sowie mindestens einer CRISPR Nuklease Variante oder eines katalytisch aktiven Fragments davon zusammen mit mindestens einer Effektor-Domäne umfasst, kann die Effektor-Domäne mit der gRNA bzw. der CRISPR Nuklease Variante oder dem katalytisch aktiven Fragment davon durch einen Nukleinsäure- oder Aminosäure-Linker verknüpft sein, um eine ideale Anordnung der Domänen zueinander und dadurch deren Funktionalität durch adäquate Flexibilität der Domänen zueinander zu gewährleisten.Gemäß einer Ausführungsform ist es bevorzugt, dass zur Herstellung einer gezielt optimierten Pflanze, eines Pflanzenmaterials oder einer pflanzlichen Zelle neben einer gRNA und einer CRISPR Nuklease, etwa einer Cas- oder einer Cpf1-Nuklease, die unabhängig voneinander eine Effektor-Domäne umfassen können, auch eine DNA-Reparatur-Matrize bereitgestellt wird. Diese Ausführungsform ist speziell bevorzugt, sofern eine CRISPR Nuklease, oder ein katalytisch aktives Fragment davon zum Einsatz kommt, welches dazu in der Lage ist, die Einführung eines gezielten DNA-Doppelstrangbruchs in eine Nukleinsäure-Zielregion von Interesse zu katalysieren. Die zusätzliche Bereitstellung einer DNA-Reparatur-Matrize, entweder in der Form von einzelsträngiger oder doppelsträngiger DNA, kann den natürlichen und fehleranfälligen NHEJ Reparaturmechanismus einer pflanzlichen Zelle überwinden, um hierdurch eine noch höhere Präzision der Genom Editierung sowie die Möglichkeit der gezielten Einführung von Insertionen, Mutationen oder Deletionen zu bewirken. Die DNA-Reparatur-Matrize kann hierbei in der Form eines rekombinanten Konstrukts bereitgestellt werden, entweder separat oder auf demselben Konstrukt, welches zur Einbringung der gRNA und/oder der CRISPR Nuklease verwendet wird. Alternativ kann die DNA-Reparatur-Matrize direkt in eine Zielzelle oder Zielstruktur von Interesse eingebracht werden, durch Transfektion oder Transformation. Üblicherweise ist eine DNA-Reparatur-Matrize derart gestaltet, dass diese linke und rechte Homologiearme umfasst, die die Position flankieren, die von einer CRISPR Nuklease gespalten wird. Die beiden Homologiearme können eine Länge von mehreren hundert, z.B. mindestens 100, mindestens 200, mindestens 300, mindestens 400 oder mindestens 500 Basenpaaren (bp) bis hin zu 1 Kilobase (kb) oder mehr umfassen. Eine Homologieregion, d.h. eine Region der Sequenzübereinstimmung, kann mindestens 5-10, 5-15, 5-20, 5-25, 5-30, 5-35, 5-40, 5-45, 5- 50, 5-55, 5-60, 5-65, 5- 70, 5-75, 5- 80, 5-85, 5-90, 5-95, 5-100, 5-200, 5-300, 5-400, 5-500, 5-600, 5-700, 5-800, 5-900, 5-1000, 5-1 100, 5-1200, 5-1300, 5-1400, 5-1500, 5-1600, 5-1700, 5-1800, 5-1900, 5-2000, 5-2100, 5-2200, 5-2300, 5-2400, 5-2500, 5-2600, 5-2700, 5-2800, 5-2900, 5-3000, 5-3100 oder mehr Basen umfassen, damit die Homologieregion ausreichend Homologie besitzt, um eine homologe Rekombination mit der korrespondierenden genomischen Region zu gestatten. Ausreichende Homologie in diesem Kontext bedeutet, dass zwei Polynukleotide ausreichend strukturelle Ähnlichkeit verfügen und somit als Substrate für eine homologe Rekombination dienen können. Demnach kann auch der Grad der Homologie der jeweiligen Homologiearme einer DNA-Reparatur-Matrize zu der entsprechenden Nukleinsäure-Zielregion variieren. Allgemein ist bei kürzerer Länge der Homologieregion ein höherer Homologiegrad erforderlich, um eine adäquate Anlagerung komplementärer Nukleinsäure-Sequenzen zu erzielen. Der Homologiegrad, d.h. die Sequenzidentität kann demnach mindestens etwa 50%, 55%, 60%, 65%, 70%, 71 %, 72%, 73%, 74%, 75%, 76%, 77%, 78%, 79%, 80%, 81 %, 82%, 83%, 84%, 85%, 86%, 87%, 88%, 89%, 90%, 91 %, 92%, 93%, 94%, 95%, 96%, 97%, 98%, 99% oderr 100% betragen. Zudem umfasst die DNA-Reparatur-Matrize ein zentral lokalisiertes Konstrukt, das eine einzubringende Sequenz, etwa ein Transgen, oder eine einzubringende Modifikation trägt. Der Erfolg der Einbringung einer gezielten Veränderung in eine Nukleinsäure-Zielstruktur kann im Anschluss durch (quantitative) PCR Verfahren überprüft werden. Über eine PCR durch Primer spezifisch für die beiden Homologiearme kann ein entsprechendes Konstrukt amplifiziert werden, dessen Sequenz sodann bestimmt werden kann, um einerseits festzustellen, ob die Reparatur durch die zelleigene NHEJ Maschinerie, oder durch homologe Rekombination, assistiert durch die DNA-Reparatur-Matrize, erfolgte.

Gemäß einer weiteren Ausführungsform ist es vorgesehen, dass zunächst mindestens ein(e) erste(s) Pflanze, Pflanzenmaterial oder eine pflanzliche Zelle bereitgestellt wird, die mindestens eine CRISPR Nuklease, bevorzugt eine Cas-Nuklease oder eine Cpf1-Nuklease, wobei die CRISPR Nuklease transient integriert ist, umfasst. Diese Ausführungsform ist speziell vorteilhaft, sofern diese(s) mindestens erste Pflanze, Pflanzenmaterial oder eine pflanzliche Zelle später mit mindestens einer zweiten Pflanze gekreuzt werden soll, wobei die zweite Pflanze, bzw. mindestens eine pflanzliche meristematische Zelle davon, eine gRNA umfasst, die mit der Cas-Nuklease der ersten Pflanze interagieren und dadurch eine gezielte Genom-Editierung bewirken kann.Die erfolgreiche Einbringung einer gezielten Veränderung in eine Nukleinsäure-Zielregion von Interesse gemäß der vorliegenden Erfindung kann vom Fachmann leicht unter Zuhilfenahme von Methoden wie Polymerase-Kettenreaktion und dergleichen verifiziert werden, zumal die Nukleinsäure-Zielregion von Interesse, und damit die Region, an welche sich mögliche PCR-Primer anlagern können, bekannt ist und bereits für das Design einer gRNA und/oder einer DNA-Reparatur-Matrize relevant waren.

Aktivatoren und Repressoren, welche gemäß der vorliegenden Erfindung zum Einsatz kommen können, umfassen vorzugsweise SEQ ID NOs:1-4 sowie Sequenzen mit mindestens 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%,90%, 91%, 92%, 93%, 94%, 95%, 96%, 97%, 98% oder 99% Sequenzhomologie zu diesen Sequenzen, welche trotz Modifikation noch die gleiche Funktion erfüllen wie die Sequenzen mit den entsprechenden SEQ ID NOs.

Eine gezielte Veränderung gemäß der vorliegenden Offenbarung kann demnach (i) den Austausch von mindestens einem Nukleotid einer Nukleinsäure-Zielregion; (ii) die Deletion von mindestens einem Nukleotid einer Nukleinsäure-Zielregion; (iii) die Insertion von mindestens einem Nukleotid einer Nukleinsäure-Zielregion; (iv) die gezielte epigenetische Veränderung der Region, die für die Regulation von mindestens einer Nukleinsäure-Zielregion verantwortlich ist; (v) die Bindung und/oder Visualisierung an/von mindestens ein(em) Nukleotid einer Nukleinsäure-Zielregion oder (vi) die Interaktion mit und/oder die Spaltung von mindestens einer RNA-Nukleinsäure-Zielregion, oder jegliche Kombination von (i) bis (vi) umfassen. Die Verfahren gemäß der vorliegenden Erfindung können insbesondere für die präzise und zeiteffiziente Merkmalsentwicklung in einer Pflanze, in Pflanzenmaterial oder in einer pflanzlichen Zelle zum Einsatz kommen.

In einer weiteren Ausführungsform des ersten Aspekts der vorliegenden Erfindung wird ein Verfahren zur Herstellung einer Pflanze, eines Pflanzenmaterials oder eine pflanzlichen Zelle bereitgestellt, bei welchem mindestens eine pflanzliche Zielstruktur, umfassend mindestens eine meristematische Zelle, mindestens ein gRNA, mindestens eine CRISPR-Nuklease oder ein katalytisch aktives Fragment davon und/oder eine Effektor-Domäne sowie mindestens eine DNA-Reparatur-Matrize bereitgestellt, wobei die gezielte Veränderung der Nukleinsäure-Zielregion von Interesse darin besteht, dass mindestens eine heterologe, d.h. nicht-endogene Sequenz, die ein Gen umfasst, das ausgewählt ist aus der Gruppe bestehend aus einem Reporter-Gen, einem Selektionsmarker, einem Resistenz gegenüber Krankheit vermittelnden Gen, einem Herbizid-Resistenz vermittelnden Gen, einem Resistenz gegenüber Insekten oder Nematoden vermittelnden Gen, einem Gen, das in den Kohlenhydratmetabolismus involviert ist, einem Gen, das in den Fettsäuremetabolismus involviert ist, einem Gen, das in den Aminosäuremetabolismus involviert ist, einem Gen, das beteiligt an der Pflanzenentwicklung ist, einem Gen, das an der Regulation des Pflanzenwachstums beteiligt ist, einem Gen, das daran beteiligt ist, die Ausbeute eines Pflanzenmaterials von Interesse zu verbessern, einem Gen, das daran beteiligt ist, Resistenz gegenüber Trockenheit zu vermitteln, einem Gen, das daran beteiligt ist, Kälteresistenz zu vermitteln, einem Gen, das daran beteiligt ist, Hitzeresistenz zu vermitteln, einem Gen, das daran beteiligt ist, Resistenz gegenüber einem Salz oder Salzen zu vermitteln, oder einem Gen, das eine funktionale RNA kodiert, wobei die funktionale RNA ausgewählt ist aus der Gruppe bestehend aus einer miRNA, einer siRNA, oder einer anderen RNA, die eine Inverted-Repeat-Struktur ausbilden kann, z.B. einem ddRNAi-Konstrukt, das sowohl einen Sinn- als auch einen Anti-Sinn (sense und anti-sense) Strang sowie einen den Sinn- und den Anti-Sinn-Strang verbindenden Hairpin-Loop kodiert, in das Genom einer pflanzlichen Zielstruktur von Interesse umfassend mindestens eine meristematische Zelle eingebracht wird.

Darüber hinaus eignen sich die Verfahren gemäß der vorliegenden Offenbarung, um einen komplexen Merkmalslokus zu bilden. Ein komplexer Merkmalslokus stellt ein chromosomales Segment dar, welches über mindestes zwei veränderte Nukleinsäure Regionen verfügt und in eine Nukleinsäure-Zielregion gemäß der vorliegenden Offenbarung, in einem Schritt oder sequentiell, integriert werden kann, wobei die mindestens zwei veränderten Nukleinsäure Regionen miteinander genetisch verknüpft sind. Die mindestens zwei veränderten Nukleinsäure Regionen stammen hierbei entweder beide von einem endogenen Pflanzenlokus ab, oder die Veränderung bezeichnet eine Mutation oder Deletion chromosomaler DNA, oder die mindestens zwei veränderten Nukleinsäure Regionen stellen transgene Sequenzen dar, oder eine Kombination davon. Da die DNA-Reparatur-Matrize gemäß der vorliegenden Offenbarung dazu geeignet ist, ein zentral lokalisiertes Konstrukt einer Länge von mindestens 1, 2, 3, 4, 5, 6, 7, 8, 9, 10, 20, 30, 40, 50, 60, 70, 80, 90, 100, 200, 300, 400, 500, 600, 700, 800, 1000, 2000, 3000, 4000 bp oder mehr verfügen kann, sind die Verfahren gemäß der vorliegenden Offenbarung dazu geeignet, einen komplexen Merkmalslokus in eine pflanzliche Zielstruktur, umfassend mindestens eine meristematische Zelle, einzubringen und dadurch die Verwendung eines CRISPR Systems sowie einer DNA-Reparatur-Matrize unmittelbar ein komplexes Merkmal in eine pflanzliche Zielstruktur mit hoher Präzision und Effizienz einzubringen. Da gemäß den Verfahren der vorliegenden Erfindung unmittelbar mindestens eine Pflanze, mindestens ein Pflanzenmaterial oder mindestens eine pflanzliche Zelle gewonnen werden kann, wird die Möglichkeit eröffnet, in kurzer Zeit gezielt veränderte und zur weiteren Kreuzung, Züchtung oder zur weiteren gezielten Veränderung geeignete pflanzliche Zellen, Pflanzenmaterial oder Pflanzen zur Verfügung zu stellen. In einer Ausführungsform kann etwa eine erste fertile Pflanze, die ein erste gezielte Veränderung in ihrem Genom enthält, mit einer zweiten Pflanze, die eine zweite gezielte Veränderung in ihrem Genom enthält miteinander gekreuzt werden, sodass die gezielten Veränderungen physikalisch verknüpft, d.h. Teil desselben Nukleinsäure-Moleküls, werden können, wobei mindestens die erste oder die zweite, oder beide Pflanzen gemäß der Verfahren der vorliegenden Offenbarung gewonnen wurden. Durch die intrinsischen Eigenschaften des CRISPR Systems und Kenntnis um PAM Motive sowie das Zusammenspiel einer künstlichen gRNA mit einer CRISPR Nuklease können gezielt auch künstliche Erkennungsstellen in Form eines veränderten Nukleinsäure-Zielregion in einen Lokus eingebracht werden, um anschließend einen verschachtelten komplexen Lokus, der mehr als eine gezielte Veränderung umfasst, zu erzeugen.

In einer anderen Ausführungsform kann ein komplexer Merkmalslokus auch in einem Schritt direkt in eine pflanzliche Zielstruktur umfassend mindestens eine meristematische Zelle, eingebracht werden. Da das CRISPR System gemäß der vorliegenden Offenbarung dahingehend skalierbar ist, dass es mehrere CRISPR Nukleasen, mehrere, ggf. spezfisch auf die CRISPR Nuklease und/oder eine Nukleinsäure-Zielregion von Interesse abgestimmte gRNAs, sowie mindestens eine DNA-Reparatur-Matrize, die neben geeigneter Homologiearme mindestes ein Merkmal von Interesse, das in das Genom einer pflanzlichen Zielstruktur integriert werden soll, umfasst, kann somit durch die hierein offenbarten Verfahren nicht nur ein Merkmal gezielt beeinflusst werden. Vielmehr ist es möglich, eine komplexes, genotypisches Merkmal, das sich in mindestens einem phänotypischen Merkmal niederschlägt, stabil in eine pflanzliche Zielstruktur von Interesse einzubringen.

In einer Ausführungsform gemäß der Verfahren der vorliegenden Offenbarung wird daher ein Verfahren zur Herstellung eines komplexen Merkmals-Locus in einer Pflanze, einem Pflanzenmaterial oder einer pflanzlichen Zelle offenbart, wobei das Verfahren die folgenden Schritte umfasst: (a) Auswählen einer genomischen Nukleinsäure-Zielregion von Interesse in einer Pflanze, wobei die genomische Nukleinsäure-Zielregion mindestens eine erste Nukleinsäure-Zielsequenz und eine zweite Nukleinsäure-Zielsequenz umfasst; (b) Inkontaktbringen von mindestens einer pflanzlichen Zielstruktur, umfassend mindestens eine meristematische Zelle, mit mindestens einer ersten gRNA, einer zweiten gRNA, und optional mindestens einer DNA-Reparatur-Matrize, und mindestens einer CRISPR-Nuklease oder einem katalytisch aktivem Fragment davon, wobei die erste und die zweite gRNA und die mindestens eine CRISPR-Nuklease oder das katalytisch aktive Fragment davon einen Komplex bilden können, der es der mindestens einen CRISPR-Nuklease gestattet einen Doppel-, oder im Fall einer Nickase einen Einzelstrangbruch, in mindestens eine erste und eine zweite Nukleinsäure-Zielregion einzuführen, wobei optional die mindestens eine gRNA oder die mindestens eine CRISPR-Nuklease zudem eine Effektor-Domäne umfasst, oder mit mindestens einer Effektor-Domäne assoziiert sein kann; (c) Identifizieren einer Zelle aus Schritt (b), die mindestens eine erste gezielte Veränderung an der ersten Nukleinsäure-Zielsequenz sowie eine zweite Veränderung an einer zweiten Nukleinsäure-Zielsequenz umfasst; und optional (d) Gewinnen einer ersten fertilen Pflanze von der mindestens einen meristematischen Zelle aus Schritt (c), wobei die fertile Pflanze die erste gezielte Nukleinsäureveränderung und die zweite gezielte Nukleinsäureveränderung umfasst, wobei die erste gezielte Nukleinsäureveränderung und die zweite gezielte Nukleinsäureveränderung physikalisch verknüpft, d.h. auf demselben Nukleinsäurestrang lokalisiert sind.

In einer anderen Ausführungsform umfasst das Verfahren ein Verfahren zur Herstellung eines komplexen Merkmals-Locus bei dem mindestens zwei veränderte Nukleinsäure-Zielsequenzen in einer genomischen Nukleinsäure-Zielregion von Interesse, in einer Pflanze, einem Pflanzenmaterial oder einer pflanzlichen Zelle modifiziert werden, die folgenden Schritte: (a) Auswählen einer genomischen Zielregion in einer Pflanze, einem Pflanzenmaterial oder einer pflanzlichen Zelle, umfassend mindestens eine meristematische Zelle, wobei die genomische Zielregion eine erste Nukleinsäure-Zielsequenz und eine zweite Nukleinsäure-Zielsequenz umfasst; (b) Inkontaktbringen der mindestens einen pflanzlichen Zelle, umfassend mindestens eine meristematische Zelle mit einer ersten gRNA, einer CRISPR-Nuklease oder einem katalytisch aktiven Fragement davon und optional einer ersten Donor-DNA in der Form einer DNA-Reparatur-Matrize, wobei die erste gRNA und die erste CRISPR-Nuklease oder das katalytisch aktive Fragment davon einen Komplex bilden können, der es der CRISPR-Nuklease gestattet, einen Doppelstrangbruch in die erste Nukleinsäure-Zielregion einzuführen, wobei die gRNA und/oder die CRISPR-Nuklease optional eine Effektor-Domäne umfassen können, oder mit einer Effektor-Domäne assoziiert sein können; (c) Identifizieren der mindestens einen meristematischen Zelle aus (b), die die erste gezielte Veränderung in der ersten Nukleinsäure-Zielsequenz umfasst; (d) Gewinnen einer ersten fertilen Pflanze aus der Zelle gemäß Schritt (c), wobei die erste fertile Pflanze die erste gezielte Veränderung umfasst; (e) Inkontaktbringen mindestens einer Pflanze, eines Pflanzenmaterials oder einer pflanzlichen Zelle, umfassend mindestens eine meristematische Zelle, mit einer zweiten gRNA, einer zweiten CRISPR-Nuklease oder einem katalytisch aktiven Fragment davon, und optional einer zweiten Donor-DNA in der Form einer DNA-Reparatur-Matrize; (f) Identifizieren einer Zelle aus Schritt (e), wobei die Zelle mindestens eine zweite gezielte Veränderung in einer zweiten Nukleinsäure-Zielsequenz umfasst; (g) Gewinnen einer zweiten fertilen Pflanze aus der Zelle gemäß Schritt (f), wobei die zweite fertile Pflanze die zweite gezielte Veränderung umfasst; und (h) Erhalten von fertilen Nachkommen von der zweiten fertilen Pflanze aus Schritt (g), wobei die fertilen Nachkommenpflanzen die erste sowie die zweite gezielte Veränderung in einer Nukleinsäure-Zielregion von Interesse umfassen, wobei die erste gezielte Veränderung und die zweite gezielte Veränderung physikalisch verknüpft sind.

Die hierin offenbarten Werkzeuge und Verfahren stellen daher wertvolle Werkzeuge dar, um zielgerichtet und mit hoher Effizienz, durch die Nutzung von CRISPR-Werkzeugen sowie der gezielten homologen Rekombination als Reparaturmechanismus eine Genomeditierung in höheren Pflanzen zu erzielen. Insbesondere kann durch die hierin offenbarten Verfahren die Umgehung des natürlichen fehleranfälligen DNA-Reparaturmechanismus *non-homologous endjoining* (NHEJ), der oftmals zu Mutationen oder chromosomalen Deletionen führt, auskompetitiert werden.

Ausgewählte Merkmale, welche gemäß der vorliegenden Offenbarung in eine Pflanze eingeführt oder in Form einer gezielten Veränderung durch Genomeditierung in einer Pflanze bewirkt werden können, umfassen, ohne darauf beschränkt zu sein, Resistenzen, umfassend Resistenzen gegenüber Herbiziden sowie Schädlingen, umfassend prokaryontische und eukaryontische Schädlinge sowie Viren, z.B. Bakterien, Pilze, Protozoen, pflanzenpathogene Viren, Nematoden, Insekten oder andere tierische Organismen, die Erzielung einer erhöhten Ausbeute, wobei sich die Ausbeute auf jegliches erwünschte Pflanzenprodukt beziehen kann, z.B. eine erhöhte Ausbeute an Samen, Früchten, Kohlenhydraten, Proteinen oder Fetten, oder weiterer pflanzlicher Stoffwechselprodukte, umfassend weitere Primärmetabolite oder Sekundärmetabolite, und dergleichen. Ein Ziel der Genomeditierung kann etwa das endogene 5-Enolpyruvylshikimat-3-phosphat-Synthase (EPSPS) Gen sein. Natürlicherweise katalysiert EPSPS die Umwandlung von Phosphoenolpyruvat und 3-Phosphoshikimat in Phosphat sowie 5-Enolpyruvylshikimat-3-phosphat durch die Einführung von gezielten Mutationen in dieses endogene Gen kann ein mutiertes EPSPS-kodierendes Gen erhalten werden, welches Resistenz gegenüber dem Herbizid N-Phosphonomethylglycin, oder jeglichem Salz davon, vermittelt.

Darüber hinaus können die Verfahren gemäß der vorliegenden Offenbarung verwendet werden, um gezielt Merkmale in eine Pflanze einzuführen, oder um unerwünschte Merkmale gezielt zu modifizieren. So besteht etwa großer Bedarf nach Lebensmitteln, Lebensmittelprodukten, die einen geringen Anteil an Acrylamid enthalten. Acrylamid, das als karzinogen eingestuft wird, entsteht als unerwünschtes Nebenprodukt der sogenannten Maillard-Reaktion aus den Aminosäuren Asparagin und Glutamin bei der Reaktion mit reduzierenden Zuckern wie Aldosen (z.B. Glucose) oder Acyloinen bei Temperaturen ab ca. 170° C. Etwa in stärkereichen Pflanzenprodukten, etwa Kartoffelprodukten, besteht daher ein großes Interesse daran, den Gehalt an potenziell acrylamidbildenden Edukten, etwa Asparagin, zu reduzieren, um hierdurch sicherere Lebensmittel bereitzustellen. Durch die hierin offenbarten Verfahren kann daher zielgerichtet eine Kartoffelpflanze erstellt werden, die dahingehend gezielt verändert wurde, dass Gene des Asparagin-Metabolismus gezielt beeinflusst werden, etwa durch die Beeinflussung eines Asparagin-Synthetase-Gens oder eines weiteren Gens, das im Asparagin-Metabolismus involviert ist.

Ein weiteres endogenes Zielgen von Interesse im Genom einer pflanzlichen Zelle, über dessen Modifikation eine Herbizidtoleranz bzw. -resistenz vermittelt werden kann, ist das Aceto-hydroxysäure-Synthase-(AHAS)-Gen. AHAS-Inhibitor-Herbizide stellen weltweit ein wichtiges Herbizid in der Landwirtschaft dar. Durch die Modifikation von mindestens einem Alleles eines endogenen AHAS-Gens durch die hierin offenbarten Verfahren, kann daher gezielt eine herbizidtolerante bzw. resistente Pflanzenzelle erzeugt werden, aus der durch den hierin offenbarten Ansatz der Targetierung einer meristematischen Zelle innerhalb kurzer Zeit eine fertile Pflanze, oder Pflanzenmaterial oder eine pflanzliche Zelle davon, gewonnen werden kann.

Von immer größerer Bedeutung werden auch Pflanzen, insbesondere Nutzpflanzen zur Energie- und Nahrungsmittelproduktion, welche resistent gegenüber diversen Umwelteinflüssen sind. Zu diesen Umwelteinflüssen gehören Hitze, Trockenheit, Kälte, Beschaffenheit des Bodens und damit verbunden des Nährstoffangebots, Salinität und dergleichen. Folglich besteht ein großes Bedürfnis danach, Pflanzen bereitzustellen, die auch sich ändernden und oftmals nicht in optimalen Umweltbedingungen für das optimale Wachstum entsprechenden Einflüssen gedeihen können. Demnach sind auch derartige Merkmale Eigenschaften, die gemäß den Verfahren der vorliegenden Erfindung gezielt in eine Pflanze, insbesondere ein Nutzpflanze, eingebracht werden können, bzw. die durch gezielte Veränderungen von mindestens einer Nukleinsäure-Zielregion in mindestens einer pflanzlichen Zielstruktur von Interesse hervorgebracht werden können.

Speziell im Hinblick auf die Optimierung agronomisch relevanter Merkmale in einer Pflanze kann es zudem von Interesse sein, die endogenen Sequenzen zu verändern und dadurch gezielt zu steuern, die in einer Pflanze als regulatorische Sequenzen, umfassend Promotoren, fungieren.

In einer Ausführungsform gemäß der vorliegenden Erfindung wird daher ein Verfahren zur Herstellung einer Pflanze, eines Pflanzenmaterials oder einer pflanzlichen Zelle, die mindestens eine gezielte Veränderung in mindestens einer meristematischen Zelle von Interesse umfasst, wobei die gezielte Veränderung die Veränderung eines endogenen Promotors ist. Die gezielte Veränderung des Promotors kann den Austausch des Promotors, oder eines Fragments davon gegen einen anderen Promotor oder ein Fragment davon umfassen, wobei der Promotor-Austausch in einer beliebigen der folgenden Kombinationen resultiert: eine erhöhte Promotor-Aktivität, eine erhöhte Promotor-Gewebespezifität, eine erhöhte Pathogen-induzierbare Promotor-Aktivität, eine erniedrigte Promotor-Aktivität, eine erniedrigte Promotor-Gewebespezifität, eine erniedrigte Pathogen-induzierbare Promotor-Aktivität, ein neue Promotor-Aktivität, eine induzierbare Promotor-Aktivität, eine Pathogen-induzierbare Promotor-Aktivität, ein erweitertes Spektrum der möglichen Genexpression, welche durch den Promotor gesteuert wird, eine Modifikation der zeitlichen, örtlichen oder vom Entwicklungsstadium abhängigen Genexpression einer Nukleinsäure-Zielregion, hier eines pflanzlichen Gens von Interesse, wodurch etwa nur in einem spezifischen Entwicklungsstadium aktiver Promotor ebenfalls in einem anderen Entwicklungsstadium oder, örtlich betrachtet, in einem anderen Gewebe aktiv sein kann, oder eine Mutation von DNA-Bindungselementen, und/oder die Deletion oder das Hinzufügen von DNA-Bindungselementen. Der Promotor oder das Fragment davon, das gemäß der Verfahren der vorliegenden Offenbarung modifiziert werden soll, kann ein Promotor oder Fragment davon sein, der in der pflanzlichen Zelle von Interesse endogen ist, es kann sich aber ebenso um einen künstlichen Promotor oder um einen transgenen Promotor handeln, welcher in einer pflanzlichen Zielstruktur von Interesse, welche mindestens eine meristematische Zelle umfasst, anwesend ist. Bevorzugt ist der Promotor oder das Fragment davon, das verändert werden soll, in das chromosomale oder extrachromosomale Genom einer pflanzlichen Zielstruktur von Interesse, umfassend mindestens eine meristematische Zelle, integriert. Der zu verändernde Promotor oder das Fragment davon kann jedoch auch auf einem extrachromosomalen, nicht genomisch integrierten Konstrukt, z.B. einem Plasmid, vorliegen.

Weitere interessante Merkmale stellen solche endogenen Gene dar, welche relevante Stoffwechsel-, Informations-, und/oder Signaltransduktionsproteine kodieren, z.B. Kinasen, Transkriptionsfaktoren, Zink-Finger- oder Hitzeschockproteine. Eine gezielte Veränderung dieser Gene und damit der kodierten Proteine erlaubt einen Eingriff in eine Vielzahl physiologischer Prozesse und eröffnet daher die Möglichkeit, gezielt Stoffwechselprozesse zu steuern. Weiterhin stellen diese Gene und die dazugehörigen regulatorischen DNA-Elemente und die Regionen, die regulierende Proteine kodieren, interessante Nukleinsäure-Zielregionen dar, die für Fertilität und/oder Sterilität einer Pflanze verantwortlich sind.

Weitere biotisch und abiotisch bedingte Faktoren, die Reaktionsmöglichkeiten, auf welche gemäß der Verfahren der vorliegenden Offenbarung durch gezielte Veränderung einer Zielfpflanze von Interesse modifiziert werden können umfassen Nährstoffmangel, Reaktion auf die Exposition gegenüber toxischen Metallen hin, Spurenelemente, Qualität, insbesondere Qualität der Samen bzw des Getreides, optimierter Nährstoffgehalt, Stärkequalität und -quantität, die Größe der Samen oder Körner, den Gesamtkohlenhydratgehalt, umfassend Stärke, Saccharose und andere Mono-, Di- und Polysaccharide, Stickstofffixierung und -verwendung, Fettsäure- und/oder Ölgehalt und/oder Zusammensetzung der Fette/Öle, umfassend gesättigte und ungesättigte Fette, eine Erhöhung des Gehalts an Lysin, oder anderer Aminosäuren, oder Schwefel in einem Pflanzenprodukt, oder auch eine Kombination der genannten Merkmale. Beispielhafte Gene, die die Getreide Ausbeute erhöhen können sind etwa Ammonium-induzierbare Glutamat-Dehydrogenasen. Gene, die die Aminosäure-Biosynthese beeinflussen sind z.B. Anthranilat Synthasen (EC 4.1.3.27).

In einer anderen Ausführungsform kann die gezielt zu verändernde Nukleinsäure-Zielregion ein Promotor sein, wobei die gezielte Veränderung den Austausch eines nativen EPSPS1-Promotors gegen einen Pflanzen-Ubiquitin-Promotor umfasst.

In einer weiteren Ausführungsform kann die gezielt zu verwendende Nukleinsäure-Zielregion ein Promotor sein, wobei die gezielte Veränderung des Promotors den Austausch eines endogenen NPK1-Promotors aus Mais gegen einen stress-induzierbaren RAB17 Mais-Promotor umfasst.

In einer Ausführungsform gemäß der vorliegenden Offenbarung kann die Nukleinsäure-Zielregion von Interesse ein Promotor sein, wobei der Promotor, der gezielt verändert werden soll, ausgewählt ist aus der Gruppe, umfassend *Zea mays* PEPC1-Promotor (Kausch et al, Plant Molecular Biology, 45:1-15, 2001), einen *Zea mays* Ubiquitin-Promotor (UBI1ZM PRO, siehe Christensen et al, Plant Molecular Biology 18:675 - 689, 1992), einen *Zea mays* Rootmet 2-Promotor, einen Actin-Promotor aus Reis (US-ACTIN PRO, McElroy et al, The Plant Cell, Ausgabe 2, 163 - 171, Februar 1990), einen Hirse RCC3-Promotor, einen *Zea mays* GOS2-Promotor, einen *Zea mays* ACO2-Promotor, oder einen Oleosin-Promotor aus *Zea mays.*

Da die hierin offenbarten Verfahren, wie oben geschildert, auch dazu geeignet sind, durch die Kombination eines CRISPR-Systems, insbesondere der Kombination mindestens einer spezifischen CRISPR-Nuklease, oder einer Variante oder einem aktiven Fragment davon, zusammen mit einer spezifischen gRNA sowie einer DNA-Reparatur-Matrize gezielte Insertionen in eine Nukleinsäure-Zielregion von Interesse einzuführen, richtet sich eine weitere Ausführungsform der vorliegenden Offenbarung auf die Bereitstellung eines Verfahrens zur Insertion eines Promotors oder eines Promotorelements in eine genomische Nukleinsäure-Zielregion von Interesse in einer pflanzlichen Zielstruktur umfassend mindestens eine meristematische Zelle, wobei die Promotor-Insertion zu einer beliebigen der folgenden phänotypischen Veränderungen führt: einer erhöhten Promotor-Aktivität, d.h. einer erhöhten Promotor-Stärke, einer erhöhten Promotor-Gewebespezifität, einer neuen Promotor-Aktivität, einer induzierbaren Promotor-Aktivität, einem erweiterten Spektrum der Genexpression desjenigen Gens, das von dem Promotor reguliert wird, oder das durch Einführung eines exogenen Promotors unter die Kontrolle dieses neu eingeführten Promotors gesetzt wird, einer Veränderung der zeitlichen, räumlichen oder der Entwicklungsstadium-abhängigen Genexpression, einer Mutation von DNA-Bindungselementen und/oder dem Hinzufügen von DNA-Bindungselementen. Ausgewählte Promotor-Elemente, die gemäß der Verfahren der vorliegenden Erfindung in eine pflanzliche Zielstruktur umfassend mindestens eine meristematische Zelle von Interesse eingeführt werden können, umfassen, ohne darauf beschränkt zu sein, Promotor-Kernelemente, wie z. B. CAAT-Box, eine CCAAT-Box, eine Pribnow-Box, ein Pathogeninduzierbarkeit vermittelndes Bindungselement wie eine W-Box, S-Box oder D-Box und/oder eine TATA-Box, regulatorische Sequenzen, die die Translation beeinflussen können, und/oder ein Repressor-System zur Erzielung einer induzierbaren Expression, wie z. B. ein Tetoperator/repressor/inducer element, oder ein Sulfonylharnstoff-repressor/operator/inducer-Element. Weitere regulierbare Promotor/Operator-Systeme, die zum Zwecke der vorliegenden Offenbarung in eine pflanzliche Zielstruktur von Interesse eingeführt werden können, sind dem Fachmann auf dem Gebiet bekannt. Ein beispielhafter Promotor, welcher als exogener Promotor in eine pflanzliche Zielstruktur von Interesse eingeführt werden kann, ist etwa der DRE-Promotor. Dieser Promotor wurde von Yamaguchi-Shinozaki und Shinozaki (1994), Plant Cell 6, 251 - 264 ursprünglich als cis-agierendes Promotorelement im Promotor des Trockenheit-Resistenzgens *rd29A* beschrieben, welches eine neun Basenpaare umfassende konservierte Kernsequenz enthält, TACCGACAT. Die Einführung eines DRE-Promotors in einen endogenen Promotor eines beliebigen Pflanzengens kann daher die induzierbare Expression des durch diesen Promotor regulierten Gens nach Trockenheit/Dürre-Stimulus vermitteln. Ein weiteres Beispiel sind etwa ABA-responsive Elemente, die eine (c/T) ACGTGGC Konsensus-Sequenz enthalten und die in zahlreichen ABA und/oder stress-regulierten Genen gefunden wurden (Busk & Pages (1998), Plant Mol. Biol. 37:425 - 435). Die Einfügung von 35 S Enhancer oder von MMV Enhancer in eine in einer pflanzlichen Zelle endogene Promotorregion kann ebenfalls die Expression des regulierten Gens erhöhen. Durch die gezielte und präzise Veränderung eines Promotors oder eines Teils davon gemäß der vorliegenden Offenbarung kann daher gezielt die Expression eines durch den Promotor regulierten Gens beeinflusst werden und die derart eingeführte gezielte Veränderung kann, da die primäre Zielzelle gemäß der vorliegenden Erfindung eine meristematische Zelle ist, unmittelbar an die Nachkommen vererbt werden, sodass eine fertile Pflanze oder Pflanzenmaterial oder eine pflanzliche Zelle davon gewonnen werden kann, welche die gewünschte Promotor-Veränderung in ihrem Genom trägt und darüber hinaus ein erwünschtes phänotypisches Merkmal, wie es sich durch die veränderte Ausprägung des durch den Promotor regulierten Gens ergibt, aufweist.

In einer weiteren Ausführungsform gemäß der vorliegenden Offenbarung wird ein Verfahren bereitgestellt, das auf die gezielte Veränderung eines Terminators unter Verwendung der hierin offenbarten Verfahren abzielt. Demnach kann die Nukleinsäure-Zielregion von Interesse in einer pflanzlichen Zielstruktur umfassend mindestens eine meristematische Zelle ein Terminator sein, wobei die Veränderung den Austausch des Terminators oder eines Fragments davon gegen einen anderen Terminator oder ein Fragment davon umfasst, wobei der Terminatoraustausch eine oder mehrere der folgenden phänotypischen Merkmalsänderungen nach sich ziehen kann: eine erhöhte Terminator-Aktivität, eine erhöhte Gewebespezifität des Terminators, eine erniedrigte Terminator-Aktivität, eine erniedrigte Gewebespezifität des Terminators, eine Mutation von DNA-Bindungselementen und/oder eine Deletion oder das Hinzufügen von DNA-Bindungselementen. Der Terminator (oder das Fragment davon), das gezielt verändert werden soll, kann ein Terminator (oder Fragment davon) eines endogenen Gens sein, es kann sich aber ebenso um einen künstlichen, bzw. chimären oder synthetischen Terminator, oder um einen transgenen Terminator handeln. Ebenso kann der Austausch-Terminator, also der Terminator oder das Fragment davon, der durch die hierin offenbarten Verfahren in das Genom einer pflanzlichen Zielstruktur von Interesse eingeführt werden soll, ein endogener Terminator, ein künstlicher Terminator, umfassend einen chimären Terminator, oder ein transgener Terminator sein. Beispielhafte Terminatoren können ausgewählt sein aus der Gruppe bestehend aus einem Mais ARGOS 8 oder SRTF18-Terminator, einem Tomaten-PIN-II-Terminator, einem Hirse-Aktin-Terminator, einem Hirse-SB-GKAF-Terminator, einem Reis T28-Terminator, einem AT-T9-Terminator oder einem GZ-W64-A-Terminator. Gemäß einer bevorzugten Ausführungsform der vorliegenden Erfindung wird das auszutauschende Terminator-Element durch die Kombination mindestens einer gRNA abgestimmt auf mindestens eine CRISPR-Nuklease sowie eine Nukleinsäure-Zielregion von Interesse zusammen mit einer DNA-Reparatur-Matrize verwendet, wobei das zentrale Element der DNA-Reparatur-Matrize als Donorsequenz für die Insertion eines Terminators oder Terminatorelements von Interesse in eine genomische Nukleinsäure-Zielregion einer pflanzlichen Zielstruktur umfassend mindestens eine meristematische Zelle dient.

In einer anderen Ausführungsform wird das hierin offenbarte gRNA/CRISPR-Nuklease/DNA-Reparatur-Matrizensystem in einer meristematischen Zielzelle angewandt, um hierdurch spezifisch einen Terminator oder ein Terminator-Element, wie es in einer pflanzlichen Zielstruktur von Interesse genomisch verankert vorliegt, zu deletieren.

Neben Promotoren und Terminatoren existieren im Genom von eukaryontischen Zellen noch weitere regulatorische Sequenzen, die von großer Bedeutung für die Regulation der Transkription eines Gens oder einer funktionalen RNA sind. In einer Ausführungsform gemäß der vorliegenden Offenbarung wird das hierin offenbarte CRISPR-System dafür genutzt, um derartige regulatorische Sequenzen gezielt zu verändern oder auszutauschen, um diese gezielte Veränderung oder den Austausch stabil im Genom einer pflanzlichen Zielstruktur von Interesse zu verankern, diesen über die initial modifizierte meristematische Zelle an die Nachkommen weiterzugeben und in dem somit erhaltenen Pflanzenmaterial oder pflanzlichen Zellen davon eine gezielte phänotypische Veränderung beobachten zu können. Beispielhafte regulatorische Sequenzen gemäß der vorliegenden Offenbarung umfassen, ohne darauf beschränkt zu sein, 3'UTR (nicht translatierte) Regionen, 5'UTR Regionen, transkriptionelle Aktivatoren, transkriptionelle Enhancer oder Repressoren, Repressoren der Translation, Splicing-Faktoren, miRNAs, siRNA, künstliche miRNAs, IncRNA, Promotorelemente, CaMV 35S enhancer, MMV enhancer Elemente, SECIS-Elemente, Polyadenylierungssignale und Polyubiquitinierungsstellen.

In einigen Ausführungsformen umfasst die Genomeditierung, d. h. die gezielte Veränderung einer Nukleinsäure-Zielregion, die gezielte Veränderung oder den Austausch von regulatorischen Elementen, was in einem oder mehreren der folgenden Effekte und/oder phänotypischen Ausprägungen resultiert: veränderte Protein-Translation, RNA-Spaltung, RNA Splicing, Termination der Transkription oder post-translationale Veränderungen. In einer Ausführungsform ist die Nukleinsäure-Zielregion von Interesse, die in einer meristematischen Zelle gezielt verändert werden soll, eine Polyubiquitinierungsstelle, wobei die gezielte Veränderung der Polyubiquitinierungsstelle in einer veränderten Rate des Protein-Abbaus eines Zielproteins von Interesse resultiert. Das Ubiquitin-Tag markiert Proteine, sodass diese anschließend im Proteasom oder durch einen Prozess, genannt Autophagie, abgebaut werden. Proteasom-Inhibitoren sind entsprechend dafür bekannt, dass sie Protein-Überproduktion bewirken können. Die gezielte Veränderung einer Nukleinsäure-Zielregion von Interesse, die ein Protein von Interesse kodiert, kann daher auch in mindestens einer Aminosäuremodifikation des Proteins von Interesse führen, wobei die Modifikation die anschließende Polyubiquitinierung des Proteins gestattet, d. h. eine post-translationale Veränderung, die in einer Veränderung des Protein-Abbaus bzw. der Protein-Abbaurate des Proteins von Interesse führt.

In einer Ausführungsform ist die genomische Sequenz von Interesse, die verändert werden soll, eine Polyubiquitinierungsstelle eines Mais-EPSPS-Gens, wobei die gezielte Veränderung der Polyubiquitinierungsstelle in einem erhöhten Proteingehalt resultiert, da das entsprechende Protein mit einer geringeren Rate abgebaut wird.

In einer weiteren Ausführungsform ist die genomische Nukleinsäure-Zielsequenz innerhalb einer meristematischen Zelle, die gezielt gemäß der Verfahren der vorliegenden Erfindung verändert werden soll, eine Intron-Stelle, wobei die gezielte Veränderung darin besteht, ein Intron-förderndes Motiv in das Intron einzufügen, was in einer Modulation (Erhöhung/Erniedrigung) der Transkriptionsaktivität desjenigen Gens resultiert, welches dieses Intron umfasst.

In einer weiteren Ausführungsform ist die Nukleinsäure-Zielregion von Interesse innerhalb des Genoms einer pflanzlichen Zielstruktur, die gezielt verändert werden soll, eine Intron-Stelle, wobei die gezielte Veränderung darin besteht, dass ein spezifisches Intron, z. B. ein Sojabohnen-EPSP1-lntron, gegen ein anderes Intron, z. B. ein Sojabohnenubiquitin-lntron-1 ausgetauscht wird.

In einer Ausführungsform gemäß der vorliegenden Offenbarung ist die Nukleinsäure-Zielsequenz von Interesse, die gezielt im Genom einer meristematischen Zelle einer Pflanze von Interesse verändert werden soll, eine Intron- oder UTR-Stelle, wobei die gezielte Veränderung darin besteht, dass mindestens eine micro RNA in diese Intron- oder UTR-Stelle eingefügt wird, wodurch die Expression des Gens, welches das Intron oder die UTR-Stelle umfasst, auch zur Expression dieser eingefügten micro RNA führt, was wiederum dazu führt, dass jegliches Zielgen von Interesse, sei es ein endogenes Pflanzengen oder das Gen eines Pflanzenschädlings, durch die so transkribierte micro RNA "gesiienced" werden kann, ohne die Genexpression des Gens zu beeinflussen, welches das Intron trägt. Gen-Silencing oder Gen-Stilllegung ist ein Vorgang, bei dem die Genexpression gemindert oder abgeschaltet wird. Hierbei erfolgt die Genregulation durch die Hemmung der Übertragung einer genetischen Information von der DNA auf die mRNA, oder der nachfolgenden Übersetzung (Translation) der auf der mRNA gespeicherten Information in ein Protein. Als post-transkriptionelles Gen-Silencing werden die Prozesse bezeichnet, die erst nach der Transkription der genetischen Information von der DNA auf die übertragende mRNA stattfinden. Diese Phänomene werden häufig als RNA-Interferenz oder RNAi bezeichnet, wobei es sich um regulatorische Prozesse unter Beteiligung spezifischer RNA-Moleküle, wie micro RNAs und siRNAs oder künstlicher ddRNAi-Hairpin-Konstrukte handelt. Das posttranskriptionelle Gen-Silencing kann zu einem intensivierten Abbau einer Ziel-mRNA von Interesse führen, wodurch die Bildung des Genprodukts (des Proteins) verhindert wird. Hierdurch können sowohl endogene als auch fremde Proteine, etwa durch einen Prozess genannt wirts-induziertes Gen-silencing (host-induced gen-silencing oder HIGS) gezielt stillgelegt oder in deutlich niedrigerer Frequenz translatiert werden.

In einer Ausführungsform wird das hierin offenbarte Verfahren zur gezielten Veränderung einer Nukleinsäure-Zielregion innerhalb einer pflanzlichen Zielstruktur umfassend mindestens eine meristematische Zelle unter Einsatz der Kombination einer CRISPR-Nuklease sowie einer gRNA und optional mindestens einer Effektor-Domäne dazu genutzt, gezielt einen Transkriptionsfaktor zu verändern, d.h. einen Transkriptionsfaktor zu mutieren, zu deletieren, oder einen Transkriptionsfaktor in eine Nukleinsäure-Zielregion von Interesse unter Einsatz eines geeigneten Donor-Konstrukts in der Form einer DNA-Reparatur-Matrize einzufügen. Beispielhafte Transkriptionsfaktoren sind der Zink-Finger-Transkriptionsfaktor oder der Faktor *tapetal development and function* (TDF; DE 10 2015 004 187 A1). Das Einfügen eines einzelnen Basenpaares in die einenTranskriptionsfaktor kodierende Sequenz kann zu einer Frame-Shift-Mutation führen, die wiederum ein neues Protein hervorbringt, das nach wie vor DNA-Bindungsaktivität zeigt, jedoch seine Transkriptions-Aktivierungsfähigkeit verloren hat. Demnach wird beispielsweise das mutierte Zink-Finger-Transkriptionsfaktor-Protein um die Bindung an Cytokinin-Oxidase-Gen-Promotoren kompetitieren und die Expression von Cytokinin-Oxidase blockieren. Die Reduktion der Cytokinin-Oxidase-Expression kann etwa in Reispflanzen das Cytokinin-Niveau erhöhen und das Rispenwachstum fördern, wohingegen in Mais das Ährenwachstum erhöht werden kann und allgemein in einer Vielzahl von Pflanzen die Ausbeute eines Pflanzenprodukts von Interesse erhöht werden kann. Das mutierte TDF dagegen kann z.B. in Weizen zu einer männlichen Sterilität führen, welche für die Erzeugung von hybriden Weizenpflanzen vorteilhaft eingesetzt werden kann.

In einer weiteren Ausführungsform können die Verfahren gemäß der vorliegenden Offenbarung dazu eingesetzt werden, gezielt Splice-Stellen in eine genomische Nukleinsäure-Zielregion von Interesse in einer pflanzlichen Zielstruktur umfassend mindestens eine meristematische Zelle gezielt zu verändern, oder alterative Splice-Stellen in die genomische Nukleinsäure-Zielregion von Interesse einzuführen. In eukaryontischen Zellen wird für die Synthese oder Expression von Proteinen mRNA verwendet, die von PrämRNA-Molekülen abstammt und anschließend einen Reifungsprozess durchläuft. Die PrämRNA-Moleküle werden gecapped, gespliced und anschließend durch Hinzufügen eines poly-A-Strange stabilisiert. Eukaryontische Zellen haben ein komplexes Verfahren für das sogenannte Splicing entwickelt, welches in alternativen Varianten eines ursprünglichen PrämRNA-Moleküls resultiert. In Maiszellen kann der Splicing-Prozess etwa durch Splice-Stellen an den Exon-Intron-Verbindungsstellen beinflusst werden. Ein Beispiel für eine kanonische Splice-Stelle ist AGGT. Sequenzen, welche Gene kodieren, können eine Vielzahl von alternativen Splice-Stellen enthalten, die die Gesamteffizienz des Prä-mRNA-Reifungsprozesses beeinflussen können und somit entscheidend die Proteinanhäufung in Zellen limitieren können. Hierin offenbarte gRNA/CRISPR-Nuklease-Paare können zusammen mit Effektor-Domänen und einer DNA-Reparatur-Matrize, welche dazu genutzt werden kann, ein spezifisches Modifikationstemplate in eine pflanzliche Zielstruktur von Interesse einzubringen, dazu eingesetzt werden, eine genomische Nukleinsäure-Zielregion von Interesse dahingehend zu modifizieren, dass eine kanonische Splice-Stelle hochpräzise an eine spezifische Position eingefügt oder erstellt wird. In einer Ausführungsform kann hierdurch beispielsweise ein pflanzliches EPSPS-Gen beeinflusst werden, wobei die gezielte Veränderung des Gens darin besteht, dass alternative Splice-Stellen derart verändert werden, dass diese gezielte Genomeditierung in einer erhöhten Produktion von funktionalen Gentranskripten und Genprodukten resultiert.

Sofern die hierin offenbarten Verfahren als Nukleinsäure-Zielregion ein endogenes Pflanzengen haben, kann durch die gezielte Veränderung eine oder mehrere der folgenden Effekte erzielt werden: eine erhöhte Protein-/Enzymaktivität, eine erhöhte Funktionalität eines Proteins von Interesse, eine erniedrigte Proteinaktivität, eine erniedrigte Proteinfunktionalität, eine ortsgerichtete Mutation, der Austausch einer Proteindomäne, ein Protein-knock-out, eine neue Proteinfunktionalität oder eine modifizierte Proteinfunktionalität.

In einer Ausführungsform kann der Protein-knock-out darin bestehen, dass ein Stop-Codon in die kodierende Sequenz von Interesse eingeführt wird.

In einer weiteren Ausführungsform kann der Protein-knock-out darin bestehen, dass ein Start-Codon einer kodierenden Sequenz von Interesse deletiert wird.

In einer weiteren Ausführungsform gemäß der vorliegenden Offenbarung können die hierin offenbarten Verfahren gezielt zum Silencing eines Gens von Interesse genutzt werden.

In einer Ausführungsform ist das Ziel des Gen-Silencing ein endogenes Pflanzengen, in einer anderen Ausführungsform ist das Ziel-Gen, dessen Expression gezielt verändert werden soll, kein endogenes Pflanzen-Gen, sondern das Gen eines Pflanzen-Pathogens, umfassend ein bakterielles Gen, ein eukaryontisches Gen, umfassend das Gen von Protozoen, Nematoden, Pilzen, Insekten, oder anderen tierischen Fraßfeinden oder Pathogenen von Pflanzen, oder ein virales Gen. Der als RNAi bezeichnete Prozess des Silencings von Genen findet im Zytoplasma eine Zielstruktur von Interesse statt, da hier die dafür benötigten Proteine und Proteinkomplexe in ihrer funktionalen Form vorliegen. Die hierin offenbarten Verfahren können somit gemäß zweier unterschiedlicher Ausführungsformen zum Einsatz kommen: (1.) Es können durch die hierin offenbarten Verfahren gezielt invertierte Genfragmente in eine Nukleinsäure-Zielregion von Interesse eingefügt werden. Diese Genfragmente können anschließend transkribiert werden, was zu einer doppelsträngigen RNA-Struktur, beispielsweise eine RNA-Hairpin-Struktur, führt, die anschließend ein endogenes oder exogenes Gen silencen kann. Alternativ kann gemäß dieser ersten Ausführungsform, wie oben ausgeführt, auch gezielt eine Nukleinsäure-Sequenz in eine genomische Nukleinsäure-Zielregion eingebracht werden, die für eine miRNA oder eine siRNA als funktionale RNA kodiert, wobei das siRNA- oder miRNA-Konstrukt anschließend das Gen-Silencing oder die Gen-Stilllegung mediiert. (2.) In einer zweiten Ausführungsform können die hierin offenbarten CRISPR-Nukleasen sowie die dazugehörigen gRNAs derart verändert werden, dass das künstliche CRISPR-System spezifisch für RNA als Nukleinsäure-Zielstruktur ist. Hierzu können auch weitere Effektor-Domänen entweder mit der gRNA und/oder der modifzierten CRISPR-Nukleasen von Interesse assoziiert sein. Dieser Ansatz ist insbesondere vorteilhaft, wenn keine genomische Zielregion gezielt verändert werden soll, sondern wenn vielmehr direkt RNA im Rahmen eines *Gene silencing-*Ansatzes direkt verändert werden soll.

In einer weiteren Ausführungsform eignen sich die hierin offenbarten Verfahren zur Unterstützung der Merkmalskartierung im Zuge der Pflanzenzüchtung. Bezüglich qualitativer Merkmale können die hierin offenbarten Verfahren dahingehend verwendet werden, um gezielt Kandidaten-Gene in den identifizierten chromosomalen Regionen zu eliminieren, um basierend hierauf zu bestimmen, ob die Deletion eines Gens die Expression eines Merkmals von Interesse beeinflusst oder nicht. Im Falle von quantitativen Merkmalen wird die Expression eines Merkmals von Interesse durch multiple quantitative Merkmals-Loci oder *quantitative trait loci* (QTL) von unterschiedlicher und stark variierender Größe, Komplexität und statistischer Signifikanz, die auch über mehrere Chromosomen verstreut im Genom einer Pflanze lokalisiert sein können, gesteuert. Ein QTL ist daher ein Abschnitt eines Chromosoms, oder ein Abschnitt mehrerer Chromosomen, der einen Einfluss auf die Ausprägung eines bestimmten quantitativen phänotypischen Merkmals von Interesse hat. Anders als diskrete Merkmale, in Pflanzen beispielsweise die Blütenfarbe, die in mehreren verschiedenen, voneinander abgegrenzten Zuständen vorliegen, sind quantitative oder stetige Merkmale ohne Abstufung auf einer kontinuierlichen Skala messbar. Im Falle eines negativen Einflusses von QTL-Regionen, die ein komplexes Merkmal bestimmen, können die hierin offenbarten Verfahren daher in einer Ausführungsform verwendet werden, um ganze chromosomale Regionen innerhalb einer pflanzlichen Zielstruktur umfassend mindestens eine meristematische Zelle von Interesse durch Marker-gestütztes Mapping zu eliminieren, um dadurch spezifische Regionen für die selektive Deletion, oder auch eine Umlagerung, zu markieren.

In einer weiteren Ausführungsform der vorliegenden Offenbarung können die hierin offenbarten Verfahren genutzt werden, um eine genomische Region von Interesse, die von zwei unterschiedlichen Nukleinsäure-Zielregionen gemäß der vorliegenden Offenbarung flankiert wird, durch zwei unabhängige gRNA/ CRISPR-Nukleasepaare, optional unter Einsatz einer DNA-Reparatur-Matrize, zu verändern. Diese Modifikation kann entweder simultan oder nacheinander erfolgen. Bevorzugt erfolgt das Herausschneiden simultan, oder gleichzeitig, und das dadurch bewirkte Deletionsereignis kann anschließend, optional durch den Einsatz einer DNA-Reparatur-Matrize, unter Verknüpfung der zwei chromosomalen Enden ohne die deletierte Nukleinsäure-Zielregion von Interesse, repariert werden.

In einer alternativen Ausführungsform kann eine Zielregion von Interesse durch Inversionen, Mutation in den Schnittstellen oder Duplikationen einer Region von Interesse modifiziert werden.

Beispielhafte Herbizidresistenz-Proteine oder Gene gemäß der vorliegenden Offenbarung umfassen die Acetolactat-Synthase (ALS)Inhibitoren, insbesondere sofern das Herbizid vom Sulfonylharnstoff-Typ ist, Gene, die für eine Resistenz gegenüber Herbiziden kodieren, die die Wirkung von Glutamin-Synthetase inhibieren, wie zum Beispiel Phosphinothricin oder BASTA, Glyphosat, z.B. EPSPS-Gene und die GAT-Gene, HPPD-Inhibitoren, z.B. die HPPD-Gene und dergleichen. So kodiert etwa das barGen Resistenz gegenüber dem Herbizid BASTA, wohingegen das *nptII* Gen Resistenz gegenüber den Antibiotika Kanamycin und Geneticin (G418) vermittelt und die ALS-Gen-Mutanten, die Resistenz gegenüber dem Herbizid Chlorsulfuron kodieren, bzw. vermitteln.

Beispielhafte Gene gemäß der vorliegenden Offenbarung, die Resistenz gegenüber Erkrankungen oder Pflanzenpathogenen vermitteln, können Resistenz gegenüber Pflanzenschädlingen wie dem Maiswurzelbohrer, dem Rebenfallkäfer oder Larven davon, dem europäischen Maiszünsler und dergleichen vermitteln. Krankheitsresistenzgene und/oder Insekten-Resistenzgene, wie zum Beispiel Lysozyme oder Cecropine für den Schutz vor Mikroorganismen, oder Proteine, wie zum Beispiel Defensine, Glukanasen oder Kitenasen zum Schutz vor Pilz-Pathogenen, oder *Bacillus thuringiensis* Endotoxine, Protease-Inhibitoren, Kollagenasen, Lektine oder Glycosidasen zur Kontrolle von Nematoden oder Insekten.

Darüber hinaus können die Verfahren gemäß der vorliegenden Offenbarung - wie bereits oben angedeutet, dafür verwendet werden, um männlich- oder weiblich-sterile Pflanzen zu erzeugen. Die Bereitstellung von männlich-sterilen Maispflanzen kann von großem Vorteil sein, da eine derartige Pflanze keine manuelle oder mechanische Entfernung der Tassel, das heißt der männlichen Blütenstände, die Pollen produzieren, erfordern, was zeit- und kostenintensiv sein kann. Beispielhafte männliche Sterilitätsgene sind zum Beispiel MS26, MS45, oder MSCA1. Maispflanzen können sowohl durch Selbst- als auch durch Fremdbestäubungstechniken gezüchtet werden. Die Maispflanze verfügt über männliche Blüten, die sich auf dem Tassel befinden, und über weibliche Blüten, die auf der Ähre lokalisiert sind, wobei sich männliche wie weibliche Blüten auf derselben Pflanze befinden. Daher kann eine Maispflanze sowohl durch Selbst- als auch durch Fremd- oder Kreuz-Bestäubung vermehrt werden. Züchtungsprogramme kombinieren wünschenswerte Merkmale von zwei oder mehreren in Zuchtlinien, oder von verschiedenen Quellen, in sogenannte Züchtungspools, aus denen neue Inzuchtlinien oder DH (Doppelhaploiden)-Linien hervorgehen, die durch Selbstung und anschließende Selektion von erwünschten Phänotypen entwickelt werden. Eine hybride Maissorte ist eine Kreuzung aus zwei solchen Inzucht- oder DH-Linien, wobei jeder der beiden parentalen Inzucht- oder DH-Linien ein oder mehrere wünschenswerte Charakteristika trägt, die der anderen parentalen Linie fehlt, oder die die andere Linie komplementieren können. Die neuen Inzuchten oder DHs werden dann mit anderen Inzucht- bzw. DH-Linien gekreuzt und die Hybriden aus diesen Kreuzungen werden untersucht, um solche Pflanzen mit potenziellem wirtschaftlichem und agronomischem Interesse zu identifizieren. Die Hybrid-Nachkommen der ersten Generation (sowie Nachkommen der ersten Generation allgemein) werden als F1 bezeichnet. Die F1 Hybride ist stärker und robuster als ihre Eltern. Dieser Effekt, auch bezeichnet als Heterosis kann sich in mannigfaltiger Weise äußern, etwa einem verstärkten vegetativen Wachstum oder einer erhöhten Ausbeute. Hybride Maissamen können unter Verwendung eines männlich-sterilen Systems unter Einsatz von manueller oder maschinengestützter Tasselentfernung erzeugt werden. Als Konsequenz der Entfernung der männlichen Tassel können die weiblichen Blütenstände einer Inzuchtlinie nur mit Pollen von einer männlichen Inzuchtlinie von Interesse befruchtet werden. Die resultierenden Samen sind daher Hybride (F1) und werden Hybrid-Pflanzen hervorbringen. Jedoch lässt es sich oftmals nicht leicht kontrollieren, dass sich, gerade in Feldversuchen, weibliche Pflanzen selbst bestäuben. Als Ergebnis werden dann Samen einer weiblichen Inzuchtlinie zusammen mit Hybriden-Samen geerntet. Wie oben bereits herausgestellt, sind die Samen einer weiblichen Inzucht- bzw. DH-Linie wirtschaftlich nicht so interessant wie die F1 Samen, da kein Heterosis-Effekt auftritt. Daher besteht in der Pflanzenzüchtung ein großer Bedarf nach männlich sterilen Pflanzen, die zur Herstellung von Hybridensaatgut für Pflanzen von agronomischem Interesse, wie zum Beispiel Mais oder Weizen, hergestellt werden und dies idealerweise durch geringe Arbeits- und Produktionskosten bewerkstelligt werden kann. Mutationen, die männliche Sterilität, etwa in Maispflanzen oder Weizenpflanzen, verursachen, wurden im Stand der Technik durch eine Vielzahl von Verfahren, wie zum Beispiel Röntgenstrahlen, oder UV-Bestrahlung, Chemikalienbehandlung, oder durch die Insertion von transponierbaren Elementen (Chaubal et al, 2000 Am. J. Bot. 87: 1193-1201). Nach wie vor besteht jedoch ein großer Bedarf nach neuen Genen, die männliche Fertilität in einer Pflanze von Interesse beeinflussen sowie verlässlichen Verfahren, um diese Gene, beziehungsweise eine gezielte Veränderung von Interesse, punktgenau in das Genom einer Pflanze von Interesse einzuführen. Beispielhafte Gene, die für männliche Sterilität verantwortlich sind, umfassen das aborted microspores (AMS)Gen von Arabidopsis, das Arabidopsis MS1-Gen, dass NEF1-Gen, das Arabidopsis AtGPAT1-Gen, die Arabidopsis dde2-2-Mutation, das Arabidopsis faceless pollen-1-Gen (flp1), das Arabidopsis male meiocyte death 1 Gen, das Tapetum-Spezifische Zink-Finger-Gen (TAZ1), das Tapetum Determinant 1 Gen und *tapetal development and function* (TDF) Gen.

Da sich die hierin offenbarten Verfahren sowohl zur stabilen als auch zur transienten Integration einer gezielten Veränderung in eine Nukleinsäure-Zielregion einer pflanzlichen Zielstruktur umfassend mindestens eine meristematische Zelle eignen, kann hierdurch beispielsweise auch direkt eine männlich oder weiblich sterile Pflanze oder Pflanzenmaterial gewonnen werden, da die gezielte Veränderung, wie sie gemäß der Verfahren der vorliegenden Erfindung in eine meristematische Zelle eingeführt werden können, direkt und unmittelbar an die Nachkommen dieser Zelle weitergegeben werden. Unter Einsatz der hierin offenbarten Technologien *in vivo* kann daher ohne weitere Kreuzung eine männlich- oder weiblich-sterile Pflanze, insbesondere eine Maispflanze, gewonnen werden.

In einer Ausführungsform gemäß der vorliegenden Offenbarung wird ein Verfahren bereitgestellt, das dazu geeignet ist, eine Pflanze, ein Pflanzenmaterial oder eine pflanzliche Zelle zu selektionieren oder zu bestimmen, die das mindestens eine gezielte Veränderung in einer Nukleinsäure-Zielregion, umfassend eine genomische Zielregion oder eine RNA-Zielregion umfasst, wobei das Verfahren die folgenden Schritte umfasst:
a) Erhalten einer ersten Pflanze, die mindestens eine CRISPR-Nuklease, oder eine Variante oder ein katalytisch aktives Fragment davon, in mindestens einer meristematischen Zelle umfasst, wobei die CRISPR-Nuklease dazu in der Lage ist, einen Doppel- oder Einzelstrangbruch in eine genomische oder in eine RNA-Nukleinsäure-Zielregion von Interesse einzuführen;
b) Erhalten einer zweiten Pflanze, die mindestens eine gRNA umfasst, die dazu in der Lage ist, einen Komplex mit der CRISPR-Nuklease, der Variante oder dem katalytisch aktiven Fragment davon aus Schritt a) zu bilden,
c) Kreuzen der ersten Pflanze aus Schritt a) mit der zweiten Pflanze aus Schritt b);
d) Überprüfen der Nachkommen oder der Zellen davon aus Schritt c) auf Veränderungen in einer Nukleinsäure-Zielregion von Interesse hin und
e) Auswählen einer Nachkommenpflanze, eines Pflanzenmaterials oder einer pflanzlichen Zelle, die die erwünschte gezielte Veränderung in mindestens einer Nukleinsäure-Zielregion von Interesse umfasst.

In einer weiteren Ausführungsform dieses Selektionsverfahrens gemäß der vorliegenden Offenbarung umfasst die gRNA und/oder die CRISPR-Nuklease zudem mindestens eine Effektor-Domäne, die mit der gRNA und/oder die CRISPR-Nuklease assoziiert ist oder assoziieren kann und/oder im Fall, dass die gRNA und/oder die CRISPR-Nuklease sowie die mindestens eine Effektor-Domäne auf einem rekombinanten Konstrukt bereitgestellt werden, eine für eine Effektor-Domäne kodierende Sequenz. Die Effektor-Domäne kann kovalent oder nicht-kovalent mit der gRNA und/oder die CRISPR-Nuklease assoziiert oder verknüpft sein.

In einer weiteren Ausführungsform gemäß der vorliegenden Offenbarung wird ein Verfahren zur Selektion einer Pflanze, eines Pflanzenmaterials oder einer pflanzlichen Zelle von Interesse bereitgestellt, die/das eine gezielt veränderte Nukleinsäure-Zielregion, entweder in ihrem Genom oder in ihrem Transkriptom, d.h. der Gesamtheit an aller zu einem bestimmten Zeitpunkt in einer Zelle transkribierten Gene oder funktionaler RNAs, umfasst, wobei das Verfahren die folgenden Schritte umfasst:
(a) Erhalten einer ersten Pflanze, die mindestens eine CRISPR-Nuklease oder eine Variante oder ein katalytisch aktives Fragment davon umfasst, die dazu in der Lage ist, einen Doppelstrangbruch, einen Einzelstrangbruch und/oder eine spezifische DNA-Bindung von/an einer Nukleinsäure-Zielregion zu bewirken;
(b) Erhalten einer zweiten Pflanze, die eine gRNA umfasst, wobei die gRNA dazu in der Lage ist, einen Komplex mit der CRISPR-Nuklease, der Variante oder dem katalytisch aktivem Fragment davon zu bilden, wobei die Cas-Nuklease, die Variante oder das katalytisch aktive Fragment davon sowie die gRNA direkt oder in der Form von mindestens einem rekombinanten Konstrukt bereitgestellt werden, und wobei die gRNA und/oder die CRISPR-Nuklease, die Variante oder das katalytisch aktive Fragment davon mit mindestens einer Effektor-Domäne oder einer für eine Effektor-Domäne kodierenden Sequenz assoziiert sind oder assoziieren können; und wobei die erste Pflanze aus (a) oder die zweite Pflanze aus (b) zudem eine DNA-Reparatur-Matrize umfasst, die als zentralen Bestandteil mindestens eine Donor-DNA umfasst, wobei die DNA-Reparatur-Matrize unmittelbar, etwa durch Transformation oder Transfektion, oder rekombinant in der Form von mindestens einem rekombinanten Konstrukt in die erste oder die zweite Pflanze, das Pflanzenmaterial oder die pflanzliche Zelle eingebracht wurde, bzw. eingebracht wird;
(c) Kreuzen der ersten Pflanze aus Schritt (a) mit der zweiten Pflanze aus Schritt (b) und optional Bereitstellen von mindestens einer gRNA und/oder einer DNA-Reparatur-Matrize, sofern diese nicht stabil in das Genom der ersten und/oder der zweiten Pflanze integriert wurde;
(d) Beurteilen der Nachkommen der Pflanze aus Schritt (c), oder der pflanzlichen Zellen davon, dahingehend, ob eine gezielte Veränderung in der mindestens einen Nukleinsäure-Zielregion von Interesse beobachtet werden kann, und
(e) Auswählen oder Selektieren einer Nachkommenpflanze, eines Pflanzenmaterials oder einer pflanzlichen Zelle davon, die/das eine gewünschte Insertion eingefügt in die mindestens eine Nukleinsäure-Zielregion von Interesse umfasst, wobei die Insertion über die Donor-DNA als Teil der DNA-Reparatur-Matrize eingefügt wurde.

Die hierin offenbarten Verfahren sind daher geeignet um damit eine hochpräzise Gen-Targetierung eines Transgens von Interesse zu vermitteln und/oder dazu, komplexe transgene Merkmals-Loci herzustellen, da wie oben erläutert, gemäß der Verfahren der vorliegenden Offenbarung auch multiple Transgene, entweder simultan oder nacheinander, in eine pflanzliche Zielstruktur von Interesse, umfassend mindestens eine meristematische Zelle, eingebracht werden können. Ein komplexer transgener Merkmals-Locus bezeichnet einen genomischen Locus, der eine Vielzahl von Transgenen trägt, die genetisch miteinander verknüpft sind. Durch das Einfügen unabhängige Transgene innerhalb von 0,1, 0,2, 0,3, 0,4, 0,5, 1, 2 oder sogar bis zu 5 Centimorgan (cM) voneinander können die Transgene als einziger genetischer Locus ausgebildet werden (siehe zum Beispiel US 2013/0263324 A1). Ein Centimorgan bezeichnet hierbei den Abstand zwischen zwei verlinkten Genen, Markern, Nukleinsäure-Zielregionen oder Loci oder einem beliebigen Paar davon, wobei 1% der Meiose-Produkte rekombinant sind. Daher ist 1 Centimorgan ein Äquivalent für eine Distanz, die 1% durchschnittlicher Rekombinationshäufigkeit zwischen den zwei verknüpften Genen, Markern, Nukleinsäure-Zielregionen, Loci, oder jedem beliebigen Paar davon entspricht. Nach Selektion der Pflanze des Pflanzenmaterials oder der pflanzlichen Zelle von Interesse, die/das das Transgen von Interesse umfasst, können diejenigen Pflanzen, die mindestens ein Transgen enthalten, dann gekreuzt werden, um eine F1-Pflanze, Pflanzenmaterial oder pflanzliche Zelle hervorzubringen, die beide Transgene enthält. Die Nachkommen dieser F1-Pflanzen würden dann in 1/500 Nachkommen die zwei unterschiedlichen Transgene rekombiniert auf demselben Chromosom umfassen. Der komplexe Locus kann dann als einziger genetischer Locus mit beiden transgenen Merkmalen für die weitere Züchtung verwendet werden. Dieses Verfahren kann so oft wie erwünscht gemäß der hierin offenbarten Verfahren wiederholt werden, um so viele Merkmale wie möglich oder erwünscht in einem komplexen Locus zu sammeln. Anschließend können dann chromosomale Intervalle, die mit einem Phänotyp oder Merkmal von Interesse korrelieren, identifiziert werden. Dem Fachmann auf dem Gebiet steht eine Vielzahl von Verfahren zur Verfügung, um chromosomale Intervalle identifizieren zu können. Die Grenzen solcher chromosomalen Intervalle werden derart gezogen, um Marker zu umfassen, die mit dem Gen verknüpft sind, das das Merkmal von Interesse kontrolliert. Mit anderen Worten werden die chromosomalen Intervalle derart gezogen oder definiert, dass jeder beliebige Marker, der innerhalb des Intervalls liegt, umfassend die terminalen Marker, die die Grenzen des Intervalls definieren, als Marker verwendet werden können. In einer Ausführungsform kann das chromosomale Intervall mindestens einen QTL oder mehr als einen QTL umfassen. Eine stark ausgeprägte Nähe der multiplen QTLs in demselben Intervall kann jedoch die Korrelation von einem spezifischen Marker mit einem spezifischen QTL in der Diagnostik verschleiern, da ein Marker eine Verknüpfung zu mehr als einem QTL zeigen kann. Umgekehrt ist es manchmal, für den Fall, dass zwei Marker in enger Nachbarschaft Segregation mit dem erwünschten phänotypischen Merkmal zeigen, unklar, ob jeder dieser Marker denselben QTL identifiziert, oder ob zwei unterschiedliche QTLs identifiziert werden. Ferner wird eine Pflanze, ein Pflanzenmaterial oder eine pflanzliche Zelle offenbart, die gemäß einem der oben geschilderten Verfahren gemäß des ersten Aspekts der vorliegenden Erfindung erhältlich ist, oder erhalten werden kann.

Verfahren zur Anzucht und Kultivierung der Mikroorganismen und Viren, welche gemäß der vorliegenden Offenbarung als Vektoren eingesetzt werden können, sind dem Fachmann auf dem Gebiet bekannt.

In einer Ausführungsform wird das rekombinante Konstrukt gemäß der vorliegenden Offenbarung mit Hilfe mindestens eines Vektors oder eines Vektorsystems in die pflanzliche Zielstruktur eingebracht.

In einer anderen Ausführungsform wird das rekombinante Konstrukt gemäß der vorliegenden Offenbarung direkt ohne einen zusätzlichen Vektor in die Zielzelle eingebracht, vorzugsweise durch mechanische Verfahren, durch Transfektion oder durch Ausnutzung von Endozytose. Ebenfalls vorgesehen, gemäß einer Ausführungsform der vorliegenden Erfindung, ist die Einbringung von mindestens einem rekombinanten Konstrukt in eine pflanzliche Zielstruktur. Vektoren und Vektorsysteme der vorliegenden Offenbarung umfassen solche, die ausgewählt sind aus der Gruppe bestehend aus SEQ ID NOs:12-15 und 25-38, sowie Sequenzen mit mindestens 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%,90%, 91%, 92%, 93%, 94%, 95%, 96%, 97%, 98% oder 99% Sequenzhomologie zu diesen Sequenzen, welche trotz Modifikation noch die gleiche Funktion erfüllen wie der/das jeweils nicht modifizierte Vektor oder Vektorsystemmit der entsprechenden SEQ ID NO. Die genannten Vektoren und Vektorsysteme können etwa Codon-optimierte oder trunkierte rekombinante Konstrukte umfassen, oder sie können gezielte Punktmutationen enthalten, um ihre Aktivität in unterschiedlichen Zielzellen zu gewährleisten. Die Sequenz gemäß SEQ ID NO:31 ist eine Hybridsequenz, bei der der Bereich zwischen den *BcII* und der *BssHII*-Schnittstellen des Fescue Segments RNA3 des Brom-Mosaik-Virus (s. NCBI: DQ530425) durch den korrespondierenden Abschnitt des R_BMV_RNA3_SI13'A/G (Hema & Kao 2004, Journal of Virology) Fragmentes ersetzt wurde. Ferner wird gemäß der vorliegenden Offenbarung auch ein *Agrobacterium spp.* als Vektor angesehen und kann alleine oder in Kombination mit weiteren Einbringungsmitteln bzw. Vektoren verwendet werden. Gemäß einer Ausführungsform werden die o.g. Vektoren und Vektorsysteme gemäß SEQ ID NOs:12-15 und 25-38 oder Sequenzen mit o.g. Sequenzhomologie hierzu als Gerüststruktur verwendet, um die rekombinanten Konstrukte, umfassend mindestens eine gRNA sowie mindestens eine CRISPR Nuklease und/oder mindestens eine Effektor-Domäne in eine pflanzliche Zielstruktur einzubringen. Die hierfür erforderlichen molekularbiologischen Methoden und Verfahren sind dem Fachmann auf dem Gebiet bekannt.

Rekombinante Konstrukte gemäß der vorliegenden Offenbarung umfassen solche, die ausgewählt sind aus der Gruppe, bestehend aus SEQ ID NOs: 23 und 24 sowie Sequenzen mit mindestens 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%,90%, 91%, 92%, 93%, 94%, 95%, 96%, 97%, 98% oder 99% Sequenzhomologie zu diesen Sequenzen, welche trotz Modifikation noch die gleiche Funktion erfüllen wie das jeweils nicht modifizierte rekombinante Konstrukt mit der entsprechenden SEQ ID NO. Die genannten rekombinanten Konstrukte können etwa Codon-optimierte oder trunkierte Sequenzen umfassen, oder sie können gezielte Punktmutationen enthalten, um ihre Aktivität oder Bindungseigenschaften in unterschiedlichen Zielzellen zu gewährleisten oder zu modifizieren. In SEQ ID NO:23 entsprechen die Positionen 16239-16258 der Position für die jeweilige gRNA von Interesse, welche je nach Ziel-Nukleinsäuresequenz modifiziert werden kann und muss. In SEQ ID NO:24 entsprechen die Positionen 16645-16664 der Position für die jeweilige gRNA von Interesse, welche je nach Ziel-Nukleinsäuresequenz modifiziert werden kann und muss.

In einem Aspekt bezieht sich die vorliegende Offenbarung auf Verfahren zur Herstellung einer Pflanze, eines Pflanzenmaterials oder einer pflanzlichen Zelle, wobei das rekombinante Konstrukt ausgewählt ist aus SEQ ID NOs: 23 und 24, sowie Sequenzen mit mindestens 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%,90%, 91%, 92%, 93%, 94%, 95%, 96%, 97%, 98% oder 99% Sequenzhomologie zu diesen Sequenzen.

In einem weiteren Aspekt bezieht sich die vorliegende Offenbarung auf eine Pflanze, ein Pflanzenmaterial oder eine pflanzliche Zelle, erhältlich oder erhalten durch ein Verfahren umfassend das Einbringen eines rekombinanten Konstrukts gemäß SEQ ID NOs: 23 und 24, sowie Sequenzen mit mindestens 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%,90%, 91%, 92%, 93%, 94%, 95%, 96%, 97%, 98% oder 99% Sequenzhomologie zu diesen Sequenzen, in eine pflanzliche Zielstruktur, welche mindestens eine meristematische Zelle umfasst.

In noch einem weiteren Aspekt bezieht sich die vorliegende Offenbarung auf die Verwendung von mindestens einem rekombinanten Konstrukt gemäß SEQ ID NOs: 23 und 24, sowie Sequenzen mit mindestens 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%,90%, 91%, 92%, 93%, 94%, 95%, 96%, 97%, 98% oder 99% Sequenzhomologie zu diesen Sequenzen, zur gezielten Veränderung mindestens einer Nukleinsäure-Zielregion in einer pflanzlichen Zelle.

Zur Anwendung in der vorliegenden Erfindung eignen sich alle Cas-Polypeptidsequenzen sowie Cas-kodierende Nukleinsäuresequenzen, welche speziell auf den Einsatz in einer pflanzlichen Zelle hin optimiert wurden bzw. deren kodierende Konstrukte geeignete regulatorische Sequenzen tragen, welche die adäquate Transkription und/oder Translation in einer pflanzlichen Zelle im dafür vorgesehenen zellulären Kompartiment, umfassend den Zellkern, das Zytosol, ein Mitochondrium, ein Plastid, umfassend einen Chloroplasten, bewirken können. Ferner müssen die jeweiligen CRISPR Nukleasen in einer Ausführungsform über ihre intrinsische Nukleasefunktion verfügen. Als CRISPR Nuklease gemäß der vorliegenden Offenbarung kann daher auch ein katalytisch aktives Fragment abgeleitet von einer nativen CRISPR Nuklease zum Einsatz kommen, sofern das katalytisch aktive Fragment nach wie vor dieselbe enzymtisch katalytische Funktion erfüllt, wie das native Enzym, von dem es abgeleitet ist.

Alternativ können gemäß eines Aspekts der vorliegenden Offenbarung auch Cas-Nickasen oder katalytisch aktive Fragmente davon zum Einsatz kommen, d.h. Cas-Polypeptide, die dahingehende verändert wurden, dass sie nur noch einen DNA-Strang spalten und nicht wie eine native CRISPR Nuklease einen DNA-Doppelstrangbruch erzeugen. Dies bietet zum einen die Möglichkeit einer erhöhten Spezifität, da zur Erzielung eines Doppelstrangbruchs zwei rekombinante Konstrukte umfassend je eine Cas-Nickase zum Einsatz kommen müssen. Zum anderen bietet sich die Möglichkeit, einen gezielt versetzten Doppelstrangbruch anstelle eines "Blunt" Schnittes einzuführen.

Schließlich können gemäß eines Aspekts der vorliegenden Offenbarung auch Cas-null Nukleasen oder katalytisch aktive Fragmente davon, d.h. Varianten, welche über keine Nukleaseaktivität mehr verfügen, zum Einsatz kommen. Hiermit eröffnet sich die Möglichkeit, die CRISPR Nuklease zusammen mit einer weiteren Effektor-Domäne gemäß der vorliegenden Offenbarung, z.B. einem weiteren DNA- bzw. RNA-modifizierenden oder DNA- bzw. RNA-bindenden Polypeptid oder Nukleinsäuren, gemäß der vorliegenden Offenbarung zu verwenden, wodurch sich das Spektrum der Einführung gezielter Veränderungen in einer pflanzlichen Zielstruktur erweitert.

Es ist dem Fachmann auf dem Gebiet bekannt, gezielte Mutationen in die katalytische Domäne einer CRISPR Nuklease von Interesse einzuführen, um diese zu einer Nickase oder auch zu einer Endonuklease-null Variante "umzuprogrammieren".

Beispielhafte CRISPR Nukleasen oder katalytisch aktive Fragmente davon oder CRISPR Nuklease oder katalytisch aktive Fragmente davon kodierende Sequenzen zur Anwendung in der vorliegenden Offenbarung sind in SEQ ID NOs 16-22 und als UniProtKB/Swiss-Prot Datenbankzugang Q99ZW2.1 (SEQ ID NO: 39) offenbart und umfassen auch solche Sequenzen mit mindestens 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%,90%, 91%, 92%, 93%, 94%, 95%, 96%, 97%, 98% oder 99% Sequenzhomologie zu diesen Sequenzen, welche trotz Modifikation noch die gleiche Funktion erfüllen wie die jeweils nicht modifizierten Sequenzen mit der entsprechenden SEQ ID NO bzw. zugänglich unter der genannten Datenbankhinterlegungsnummer.

Noch ein weiterer Aspekt der vorliegenden Erfindung nutzt den dem CRISPR/Cas System zu Grunde liegenden Wirkmechanismus der RNA-gesteuerten DNA-Modifikation dahingehend, dass eine Effektor-Domäne anstelle von oder zusammen mit der CRISPR Nuklease durch eine gezielt angepasste gRNA an die gewünschte Position einer Nukleinsäure-Zielregion in einer pflanzlichen Zielstruktur dirigiert wird, sodass die Effektor-Domäne gezielt platziert werden kann, um die gewünschte Nukleinsäure Editierung zu bewirken.

In einer Ausführungsform dieses Aspekts ist die Nukleinsäure-Zielregion eine genomische DNA.

In einer weiteren Ausführungsform dieses Aspekts ist die Nukleinsäure-Zielregion eine mitochondriale oder plastidäre DNA, wobei das rekombinante Konstrukt eine Lokalisationssequenz umfasst, welche die Lokalisation des rekombinanten Konstrukts im entsprechenden Zielkompartiment, z.B. einem Mitochondrium oder einem Chloroplasten, umfasst.

In einer Ausführungsform umfasst die CRISPR Nuklease oder das katalytisch aktive Fragmente davon oder die die CRISPR Nuklease oder die das katalytisch aktive Fragmente davon kodierende Sequenz und/oder die Effektor-Domäne oder die die Effektor-Domäne kodierende Sequenz zusätzlich eine Sequenz ausgewählt aus einer Kernlokalisationssequenz, einer Plastidenlokalisationssequenz, vorzugsweise einer Mitochondrienlokalisationssequenz und einer Chloroplastenlokalisationssequenz. Eine Kernlokalisationssequenz kann etwa ausgewählt sein aus SEQ ID NO: 49-58, welche folgende Sequenzen offenbaren: Simian virus 40 (SV40) monopartit: MAPKKKRKV; A. *tumefaciens* VirD2 (pTiA6): KRPRDRHDGELGGRKRAR; *A. tumefaciens* VirD2 (pTiC58): KRPREDDDGEPSERKRER; *A. tumefaciens* VirE2 (pTiA6) #1: KLRPEDRYVQTERYGRR; A. *tumefaciens* VirE2 (pTiC58) #1: KLRPEDRYIQTEKYGRR; *A. tumefaciens* VirE2 (pTiA6) #2: KRRYGGETEIKLKSK; *A. tumefaciens VirE2* (pTiC58) #2: KTKYGSDTEIKLKSK;A. *rhizogenes* GALLS (pRi1724): KRKRAAAKEEIDSRKKMARH; *A. rhizogenes* GALLS (pRiA4): KRKRVATKEEIEPHKKMARR;A. *rhizogenes VirD2* (pRiA4): KRPRVEDDGEPSERKRAR.

Es kann ein oder es können mehrere Kernlokalisationssequenzen mit mindestens einer Effektor-Domäne kombiniert werden, welche bevorzugt gemeinsam auf einem Plasmidvektor vereint werden.

In einer weiteren Ausführungsform umfasst die gRNA oder die die gRNA kodierende Sequenz zusätzlich eine Sequenz ausgewählt aus einer Kernlokalisationssequenz, einer Plastidenlokalisationssequenz, vorzugsweise einer Mitochondrienlokalisationssequenz und einer Chloroplastenlokalisationssequenz.

In noch einer weiteren Ausführungsform dieses Aspekts ist die Nukleinsäure-Zielregion eine Ribonukleinsäure (RNA) in einem beliebigen pflanzlichen Kompartiment, z.B. dem Zytosol. Gemäß dieser Ausführungsform kann eine gezielt modifizierte gRNA bereitgestellt werden, welche dazu in der Lage ist, mit einer RNA-Zielstruktur zu interagieren. Die gRNA kann zudem eine weitere Effektor-Domäne, z.B. ein Aptamer, umfassen.

Dem Fachmann auf dem Gebiet ist bekannt, dass das Design der entsprechenden mindestens einen gRNA, welche zusammen mit mindestens einer CRISPR Nuklease und/oder mit mindestens einer Effektor-Domäne verwendet wird, von der Spezifität und speziell den Bindungs- und Erkennungseigenschaften der jeweils verwendeten CRISPR Nuklease und/oder der Effektor-Domäne sowie der Nukleinsäure-Zielregion, welche gezielt verändert werden soll, abhängt.

Wildtyp CRISPR Nukleasen, speziell vom Typ Cas9, erzeugen einen "blunt" Doppelstrangbruch in der Ziel-DNA-Sequenz, d.h. ohne Einzelstrang-DNA-Überhang. Darüber hinaus können diese Nukleasen auch Einzelnukleotid-Überhänge hinterlassen, was etwa durch versetzte ("offset") Spaltung der beiden Einzelstränge eines DNA-Doppelstranges geschieht. Hierdurch werden zelleigene DNA-Reparaturmechanismen der Zielzelle aktiviert, umfassend die sog. Nicht-homologe Reparatur (non-homologous end joining oder NHEJ). Dieser Mechanismus ist jedoch fehleranfällig, insbesondere, da hierdurch Insertionen und Deletionen (INDELs) an den Ort eines Doppelstrangbruches eingeführt werden können. Es kann also zur Etablierung von Mutationen an den Orten der Wiederzusammenführung der einzelnen DNA-Stränge kommen. Mittels NHEJ können etwa einfache oder mehrfache Gen-Knock-Outs mediiert werden, wobei die DNA-Stränge nach dem gezielten DNA-Bruch durch zelleigene Mechanismen mit einer geänderten Sequenz, die in einem Frameshift oder einer anderen Mutation resultiert, welche die funktionelle Expression von einem oder mehreren Genen von Interesse verhindern kann, wieder zusammengeführt werden. Ein weiterer Reparaturmechanismus ist die Homologie-gerichtete Reparatur (homology-directed repair (HDR) oder homologe Rekombination (HR)). Diese Mechanismen verwenden homologe DNA als Template oder Matrize, von dem/der Sequenzinformation kopiert werden kann, um hierdurch einen DNA Bruch zu reparieren. Durch die gezielte Bereitstellung einer DNA-Reparatur-Matrize, die über eine gewisse Länge über Homologie zu einer genomischen DNA Region, in der ein DNA Bruch induziert werden soll innerhalb einer Zielzelle von Interesse verfügt, kann mindestens eine präzise Editierung, Insertion oder ein Gen-Austausch erfolgen. Die so erzielten präzisen Veränderungen umfassen keine unerwünschten oder nicht-steuerbaren Mutationen, was bei jeglichem Gene-Editierungsansatz stets wünschenswert ist.. Beide Reparaturmechanismen, NHEJ und HDR/HR stellen natürlich vorkommende Mechanismen zur DNA-Reparatur dar, welche in jeder hierin offenbarten Zelle vorkommen.

In einer Ausführungsform gemäß aller Aspekte der vorliegenden Offenbarung wird daher eine DNA-Reparatur-Matrize bzw. ein Reparatur-Template bereitgestellt, das ortsgerichtet und präzise einen Einzel- oder Doppelstrangbruch, der zuvor durch eine CRISPR Nuklease, oder eine Variante oder ein katalytisch aktives Fragment davon, und/oder eine Effektor-Domäne in eine Nukleinsäure Zielregion von Interesse eingebracht wurde.

Entscheidend für die ortsgerichtete Einführung der Veränderung einer Nukleinsäure-Zielregion ist gemäß dem oben dargelegten Mechanismus des Typ-II CRISPR/Cas Systems die gezielte Auswahl und das gezielte Design der gRNA-Sequenzen, um die Spaltung von Off-Target Regionen anders als der Zielregion zu vermeiden. Die Identifikation geeigneter PAM-Motive in Abhängigkeit der verwendeten CRISPR/Cas Werkzeuge und optional weiterer Effektor-Domänen und die Verwendung dieser Information zum Design geeigneter rekombinanter Konstrukte ist dem Fachmann auf dem Gebiet bekannt.

Gemäß einer Ausführungsform der vorliegenden Offenbarung wird das Genom oder die extrachromosomale Nukleinsäure-Zielregion einer Zelle daher zunächst nach geeigneten PAM-Sequenzen hin durchsucht, um so gezielt eine passende gRNA entwerfen zu können.

Der Begriff guide RNA oder gRNA wie hierin verwendet bezieht sich auf ein einzelsträngiges oder doppelsträngiges oder partiell doppelsträngiges Nukleinsäuremolekül, welches aus natürlicher oder synthetischer RNA und/oder aus natürlicher oder synthetischer DNA bestehen kann und über die Funktion verfügt, einen Komplex mit einer CRISPR Nuklease oder einem katalytisch aktiven Fragment davon bilden zu können, wodurch die CRISPR Nuklease oder das katalytisch aktive Fragment davon erst in die Lage versetzt wird, eine Nukleinsäure-Zielregion zu erkennen. Zudem kann eine gRNA neben der CRISPR Nuklease-Interaktionsdomäne über die Funktion einer Erkennungsdomäne, zur gezielten Hybridisierung an ein komplementäres Ziel-Nukleinsäuremolekül von Interesse, verfügen. Somit umfasst eine gRNA eine crRNA und optional eine tracrRNA wie oben geschildert. Die gRNA kann daher ein synthetisches duales Molekül sein, das mehrere Funktionalitäten vereint, oder die gRNA kann ausschließlich eine Funktionalität umfassen. Die Länge der crRNA und/oder der tracrRNA kann im Bereich von 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40 oder mehr Nukleotiden liegen. Folglich können gRNAs eine intrinsische Hairpin-Region durch komplementäre Basenpaarung bilden, wodurch die natürliche tracrRNA/crRNA Hairpin-Struktur nachgeahmt wird (siehe Jinek et al., 2012 oben) und zudem je nach erwünschter Zielsequenz eine geeignete Erkennungsdomäne umfassen. Sofern als CRISPR Nuklease eine Cpf1 Nuklease gewählt wird, kann die gRNA aus einer crRNA bestehen, die keine tracrRNA verwandte Struktur umfasst (s. Zetsche *et al.*, 2015, supra). Demnach kann eine gRNA gemäß der vorliegenden Offenbarung eine oder mehrere Domänen umfassen. Die gRNA kann zusätzlich eine oder mehrere Abstands- oder Spacerregionen umfassen, die nicht der Interaktion mit einem Bindungspartner oder Zielmolekül, sondern der korrekten Faltung und Orientierung der gRNA oder der Verknüpfung einer crRNA und einer tracrRNA dienen. Dieser Spacer kann aus DNA und/oder RNA bestehen und eine Länge von mindestens 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 78, 79, 80, 81, 82, 83, 84, 85, 86, 87, 88, 89, 90, 91, 92, 93, 94, 95, 96, 97, 98, 99 oder 100 Nukleotiden umfassen. Eine gRNA gemäß der vorliegenden Offenbarung umfasst zumindest einen Teilbereich, der aus RNA besteht, wobei die RNA aus natürlichen oder synthetischen Nukleotiden gebildet sein kann. Wenn erwünscht, kann eine gRNA von Interesse, bevorzugt an Ihrem 5'- oder 3'-Ende, eine Modifikation tragen, wobei die Modifikation ausgewählt ist aus dem Hinzufügen einer Gruppe bestehend aus Acridin, Amin, Biotin, Cascade Blue, Cholesterin, Cy3 @, Cy5 @, Cy5.5@ Daboyl, Digoxigenin, Dinitrophenyl, Edans, 6-FAM, Fluoreszein, oder Derivate davon, 3'- Glyceryl, HEX, IRD-700, IRD-800, JOE, Phosphate, Psoralen, Rhodamin, ROX, Thiol (SH), Spacern, TAMRA, TET, AMCA-S", SE, BODIPY<0>, Marina Blue^{®}, Pacific Blue^{®}, Oregon Green^{®}, Rhodamine Green^{®}, Rhodamine Red^{®}, Rhodol Green^{®} und Texas Red^{®}, einer Locked Nucleic Acid (LNA), 5-methyl dC, 2,6-Diaminopurin, 2'-Fluor A, 2'-Fluor U, 2'-O-Methyl RNA, einem oder mehreren Phosphorothioat(en) als Rückgrat, eine Polyethylen-Glycol verknüpfung, oder einer kovalenten 5'-3' Verknüpfung, die in der Zirkularisierung resultiert, oder Kombinationen davon. Für spezifische Ausführungsformen mag es von Interesse sein, gezielt die Länge der gRNA zu reduzieren, um gRNAs umfassend weniger als 100 Nukleotide, weniger als 95 Nukleotide, weniger als 90 Nukleotide, weniger als 85 Nukleotide, weniger als 80 Nukleotide, weniger als 75 Nukleotide, weniger als 70 Nukleotide, weniger als 65 Nukleotide, weniger als 60 Nukleotide, weniger als 55 Nukleotide, weniger als 50 Nukleotide, weniger als 45 Nukleotide, weniger als 40 Nukleotide, weniger als 35 Nukleotide oder weniger als 30 Nukleotide zu designen, um durch die Verkürzung eine höhere Spezifität der CRISPR Nuklease zu erzielen. In anderen Ausführungsformen kann die gRNA gemäß der vorliegenden Offenbarung mindestens eine Effektor-Domäne umfassen, wie z.B. ein Aptamer, oder eine DNA- oder RNA modifizierendes Molekül, oder eine Bindestelle für ein Protein oder Peptid, um so die Funktionalität der gRNA zu erweitern.

In einer weiteren Ausführungsform gemäß der vorliegenden Offenbarung kann die mindestens eine gRNA zusätzlich mit mindestens einem Nukleinsäure-Molekül assoziiert werden, *in vitro* oder *in vivo,* welches der gerichteten DNA Reparatur nach Induktion eines Doppelstrangbruches durch eine CRISPR Nuklease dient. Diese (DNA)-Reparatur-Matrize, oder HDR-Matrize, kann als einzelsträngige und/oder doppelsträngige DNA, direkt oder in der Form mindestens eines rekombinanten Konstrukts, in eine Zielstruktur von Interesse eingebracht werden. Die Reparatur-Matrize erlaubt somit die zielgerichtete homologe Rekombination, wodurch die Spezifität wie auch Anwendungsbereich der Genom Editierung deutlich erweitert werden kann.

Gemäß der vorliegenden Offenbarung können gRNAs verwendet werden, welche speziell auf den Einsatz in einer pflanzlichen Zelle hin angepasst wurden.

Gemäß der vorliegenden Erfindung wurde zudem erarbeitet, dass eine beliebige gRNA wie hierin beschrieben zusätzlich an mindestens eine Effektor-Domäne, wie z.B. ein Aptamer, gekoppelt oder zusammen mit der Effektor-Domäne eingebracht werden kann, um so die Funktionalität der gRNA zu erweitern. Das rekombinante Konstrukt umfassend eine gRNA und mindestens eine Effektor-Domäne kann als DNA- oder RNA-Konstrukt in die pflanzliche Zielstruktur unter Einsatz eines geeigneten rekombinanten Konstrukts und/oder Vektors eingebracht werden. Die Effektor-Domäne kann zudem nicht nur aus einer Nukleinsäure bestehen, sondern auch als Polypeptid, oder eine dafür kodierende Sequenz, vorliegen.

In einer Ausführungsform wird die gRNA gekoppelt an die CRISPR Nuklease oder das katalytisch aktive Fragmente davon und/oder die Effektor-Domäne, z.B. das/die DNA- bzw. RNA-modifizierende oder das/die DNA- bzw. RNA-bindende Polypeptid oder Nukleinsäure, in die pflanzliche Zielstruktur eingebracht.

In einer weiteren Ausführungsform wird die gRNA als separates rekombinantes Konstrukt unabhängig von der CRISPR Nuklease und/oder die Effektor-Domäne, z.B. das DNA- bzw. RNA-modifizierende oder DNA- bzw. RNA-bindende Polypeptid oder Nukleinsäure, in die pflanzliche Zielstruktur eingebracht.

Die gRNA kann als DNA- oder als RNA-Molekül in die pflanzliche Zielstruktur eingebracht werden. So können die gRNAs gemäß einer Ausführungsform direkt in Form einer synthetischen Nukleinsäure, z.B. als RNA, wahlweise auch im Komplex mit einer CRISPR Nuklease oder einem katalytisch aktiven Fragmente davon, oder in einer anderen Ausführungsform in Form eines aktivierbaren und transkribierbaren rekombinanten DNA-Konstrukts in die Zielzelle eingebracht werden. Ferner kann gemäß der vorliegenden Offenbarung eine einzelne gRNA eingesetzt werden, oder es können duale oder multiple gRNAs in einem oder mehreren rekombinanten Konstrukt(en) gleichzeitig in eine Zelle eingeführt werden, wobei die gRNAs über die selbe oder individuelle regulatorische Sequenz(en) verfügt/verfügen. Die Auswahl geeigneter gRNAs zur Einbringung in eine Zielzelle kann gemäß dem unten näher erläuterten Aspekt gemäß der vorliegenden Offenbarung erfolgen, wobei dieser Aspekt ein *in vitro* Screening Verfahren zur Identifizierung einer gRNA oder einer für eine gRNA kodierenden Sequenz bereitstellt.

Da die Interaktionsdomäne einer klassischen CRISPR/Cas gRNA stets mit der gleichen CRISPR Nuklease interagieren wird, kann durch den Einsatz individueller gRNAs, welche als weitere Komponente eine unterschiedliche Erkennungssequenz tragen, in einem Ansatz ein Multiplexing, d.h. die gezielte Veränderung mehrerer Zielregionen in einem Ansatz erreicht werden. Erforderlich kann hierbei stets sein, dass sich anliegend an die oder innerhalb der Zielregion eine PAM-Sequenz befindet. Das Design einer geeigneten gRNA kann vom Fachmann auf dem Gebiet in Kenntnis der verwendeten CRISPR Nuklease, der Nukleinsäure-Zielregion, der Art der erwünschten Nukleinsäure-Veränderung, ausgewählt aus Mutation, Insertion oder Deletion, sowie der erwünschten Zielzelle *in silico* bestimmt werden. Die Wirksamkeit dieser gRNAs *in vivo* sowie mögliche Off-Target Effekte müssen jedoch für jede gRNA separat evaluiert werden. Zudem gilt es für nicht-etablierte Systeme, wie bei der transienten Transformation von pflanzlichen Meristemzellen, geeignete Vektoren und Verfahren zur Einbringung mindestens einer gRNA zusammen mit mindestens einer geeigneten CRISPR Nuklease und/oder mindestens einer Effektor-Domäne, z.B. eines/einer DNA- bzw. RNA-modifizierenden oder eines/einer DNA- bzw. RNA-bindenden Polypeptids oder Nukleinsäure, zu etablieren, um die konzertierte Aktivität beider Moleküle in der Zielzelle zu gewährleisten. Schwierig ist neben dem reinen Design und der Synthese bzw. Bereitstellung der gRNA jedoch noch die Tatsache, dass gerade pflanzliche Genome sehr komplex sind und es bis dato keine zuverlässigen Verfahren zur Vorabtestung gibt, welche eine Aussage darüber erlauben, ob eine gewählte gRNA im Zusammenspiel mit der erwünschten CRISPR Nuklease oder dem katalytisch aktiven Fragment davon eine Nukleinsäure-Zielregion in einer pflanzlichen Zelle tatsächlich wirksam modifizieren kann. In einer Ausführungsform der vorliegenden Erfindung beruhen die erfindungsgemäßen Verfahren und dadurch die hiermit erzeugten Pflanzen, Pflanzenmaterialien oder Zellen auf dem natürlich vorkommenden DNA-Reparaturmechanismus der Zielzelle.

In einer anderen Ausführungsform gemäß der Aspekte der vorliegenden Offenbarung wird die Reparatur eines Einzelstrang- oder Doppelstrang-DNA-Bruchs, welcher zuvor durch eine CRISPR Nuklease oder ein katalytisch aktives Fragment davon und/oder eine weitere Effektor-Domäne mediiert wurde, durch eine oder mehrere HDR Matrize(n) in Form einer DNA-Reparatur-Matrize geheilt, welche nicht natürlich vorkommt/vorkommen, sondern in die Zielzelle eingeführt wurde(n).

Im Rahmen der vorliegenden Offenbarung wird daher in einer Ausführungsform eine DNA-Reparatur-Matrize offenbart, welche optional zusammen mit oder zeitversetzt zu den CRISPR/ Konstrukten und/oder einer weiteren mindestens einen Effektor-Domäne in eine Zielzelle eingebracht werden kann, um gezielt einen HDR-Mechanismus zu induzieren und dadurch gezielte Nukleinsäuresequenzen an die Stelle des Doppelstrangbruchs einzufügen. Hierdurch können sowohl gezielte Genom-Editierungen, umfassend Knock-Ins, als auch die gezielte Reparatur der DNA-Läsion zur Verhinderung einer unerwünschten Mutation am Ort des DNA-Bruchs bewirkt werden. Ein Knock-In kann das gezielte Einfügen von mindestens einem Nukleotid, mindestens 2 Nukleotide, mindestens 3 Nukleotide, mindestens 4 Nukleotide, mindestens 5 Nukleotide, mindestens 6 Nukleotide, mindestens 7 Nukleotide, mindestens 8 Nukleotide, mindestens 9 Nukleotide, mindestens 10 Nukleotide, mindestens 15 Nukleotide, mindestens 20 Nukleotide, mindestens 50 Nukleotide, mindestens 100 Nukleotide, mindestens 200 Nukleotide, mindestens 500 Nukleotide oder mindestens 1.000 Nukleotide in die Ziel-Nukleinsäure in der pflanzlichen Zelle bedeuten. Darüber hinaus kann ein Knock-In auch die Enbringung einer ganzen Gen-Expressionskassette bedeuten, die bis zu 10.000 Nukleotide umfassen kann. Eine Genom Editierung kann auch den gezielte Austausch von mindestens einem Nukleotid gegen ein anderes Nukleotid bedeuten. Ferner kann ein Knock-In auch durch zwei, drei, vier oder mehr Austausche oder eine Kombination von Einfügen und Austauschen erzielt werden. Insertionen meinen das gezielte Einfügen von mindestens einem Nukleotid, mindestens 2 Nukleotide, mindestens 3 Nukleotide, mindestens 4 Nukleotide, mindestens 5 Nukleotide, mindestens 6 Nukleotide, mindestens 7 Nukleotide, mindestens 8 Nukleotide, mindestens 9 Nukleotide, mindestens 10 Nukleotide, mindestens 15 Nukleotide, mindestens 20 Nukleotide, mindestens 50 Nukleotide, mindestens 100 Nukleotide, mindestens 200 Nukleotide, mindestens 500 Nukleotide, mindestens 1000 Nukleotide, mindestens 2000 Nukleotide, mindestens 5000 Nukleotide, mindestens 10000 Nukleotide oder mindestens 15000 Nukleotide in die Ziel-Nukleinsäure in der pflanzlichen Zelle bedeuten. Eine Nukleinsäuresequenzen als Insertion kann eine Sequenz, oder einen Teil davon, einer Transkriptionsfaktorbindestelle, eine regulatorische Sequenz, eine Polypeptid-kodierende Sequenz, eine Intronsequenz, eine Sequenz kodierend für eine nicht-kodierende RNA (z.B. IncRNA), eine Expressionskassette, eine nicht-kodierende Sequenz, etwa zur Anwedung als Marker, insbesondere ein selektionierbarer Marker, z.B. ein Marker zur markergestützten Selektion im Rahmen der Züchtung, oder ein RNAi-Konstrukt. Ferner kann ein Knock-In hierbei auch herbeigeführt werden durch die Deletion von Sequenzabschnitten, welche die Funktionalität eines Gens stören (beispielsweise die Deletion von Transposoninsertionen). Die DNA-Reparatur-Matrize kann als einzelsträngige oder als doppelsträngige Nukleinsäure in die pflanzliche Zielstruktur von Interesse eingebracht werden.

In einer Ausführungsform wird eine Ziel-Nukleinsäure in einer pflanzlichen Zelle gezielt ausgeschaltet (Knock-Out), d.h. die Transkription und ggf. Translation der Nukleinsäure wird verhindert. Dies kann durch die gezielte Insertion, Mutation oder Deletion einer regulatorischen Sequenz wie Promotor- oder Terminatorsequenz einer Ziel-Nukleinsäure erfolgen oder durch die gezielte Mutation oder Deletion der Ziel-Nukleinsäure selbst oder Teile davon. Weiterhin kann auch durch gezielte Mutation oder Deletion, welche den Leseraster einer Ziel-Nukleinsäure verändern, oder durch gezielte Mutation oder Deletion von potentiellen Spleißsignalen ein Knock-Out erzielt werden. In einer Ausführungsform erfolgt dieser Knock-Out ohne Einsatz einer HDR Matrize durch den NHEJ Weg erzielt, in einer anderen Ausführungsform wird hierfür zusätzlich zu den CRISPR Konstrukten und/oder einer weiteren Effektor-Domäne eine HDR Matrize oder DNA-Reparatur-Matrize in die Zielzelle eingebracht. Eine gezielte Mutation ist beispielsweise ein Austausch von mindestens einem Nukleotid gegen ein anderes Nukleotid, vorzugsweise mit der Folge, dass das betroffene Codon dann für eine andere Aminosäure kodiert. Eine gezielt ausgeschaltete Ziel-Nukleinsäure in einer pflanzlichen Zelle weist mindestens eine gezielte Mutation oder Deletion, kann aber auch zwei, drei, vier oder mehr gezielte Mutationen und/oder Deletionen aufweisen.

In Ausführungsformen, gemäß derer Cas oder ein Cpf1 Gen oder eine andere Effektor-Nuklease in der Form von DNA auf einem entsprechenden Konstrukt in eine Zielzelle von Interesse eingebracht werden, kann das die Nuklease kodierende Gen einen geeigneten Promotor umfassen, der funktional mit der die Nuklease kodierenden Sequenz verknüpft ist, um deren Transkription zu verbessern. Die Promotoren können konstitutiv aktiv sein, oder es kann sich um induzierbare Promotoren handeln, die erst nach Zugabe/Einwirkung eines entsprechenden Stimulus (chemisch oder physikalisch, umfassend Licht, Temperatur etc.) aktiviert werden. Ebenfalls kann ein Konstrukt, welches eine gRNA kodiert, einen geeigneten Promotor umfassen. Geeignete Promotoren für pflanzliche Zellen gemäß der vorliegenden Offenbarung können ausgewählt sein aus der Gruppe bestehend aus, einem Mais-Ubi-Intron Promotor (SEQ ID NO:7), einem Mais U3 Promotor (SEQ ID NO:10), einem Pflanzen U6 Polymerase III Promotor, z.B. einem Weizen U6 Promotor (SEQ ID NO:8), einem von Reis abgeleiteten U6 Promotor (s. Mikami et al., Plant Mol. Biol. 2015, 88(6), 561-572), oder einem von *Arabidopsis thaliana* abgeleiteten U6-26 Promotor, einem Reis U3 Promotor (SEQ ID NO: 9) und einem Brachipodium EF1 Promotor (SEQ ID NO: 40), oder einem einfachen oder doppelten 35S Promotors abgeleitet von dem Blumenkohl-Mosaikvirus, umfassend u.a. einen 35SPPDK Promotor (s. Yoo et al. Nature Protocols 2, 1565-1572 (2007), die Promotoren sind jedoch nicht darauf beschränkt, da die Wahl des Promotors von der jeweiligen pflanzlichen Zelle von Interesse abhängt.

In noch einer weiteren Ausführungsform kann der natürliche NHEJ Mechanismus einer Pflanzenzelle bewusst durch Zugabe eines entsprechenden Inhibitors oder durch einen gezielten Knock-out oder Knock-down einer endogenen Nukleinsäuresequenz, die in den NHEJ Vorgang involviert ist, unterdrückt werden, wodurch die Einführung einer gezielten Veränderung in eine Ziel-Nukleinsäuresequenz erleichtert werden kann, da hierdurch der NHEJ Mechanismus einer Zelle reprimiert werden kann. In einer Ausführungsform der vorliegenden Offenbarung, bei welcher die pflanzliche Zielstruktur eine isolierte meristematische Zelle eines Keimlings / Pflanze oder eines pflanzlichen Embryos oder freigelegte meristematische Zelle einer Pflanze in einem späteren Entwicklungsstadium ist, werden die die meristematischen Zellen umfassenden pflanzlichen Zielstrukturen vor, während und nach der Einbringung des mindestens einen rekombinanten Konstrukts gemäß der vorliegenden Offenbarung derart kultiviert, dass einer Oxidation der isolierten Strukturen vorgebeugt wird. In einer Ausführungsform umfasst dies die Zugabe eines Antioxidans.

Die nachfolgende Tabelle 1 führt geeignete Medien zur Kultivierung unterschiedlicher pflanzlicher Zielstrukturen auf, welche meristematische Zellen umfassen. Weitere geeignete Reaktionsbedingungen wie Puffer, Zusätze, Temperatur- und pH-Bedingungen sowie ggf. weitere notwendige Zusätze können vom Fachmann auf dem Gebiet in Kenntnis eines hierin offenbarten Verfahren und Konstrukte, gemäß eines beliebigen Aspekts der vorliegenden Offenbarung leicht bestimmt werden.

**Tabelle 1: Medienzusammensetzungen zur Kultivierung unterschiedlicher pflanzlicher Zielstrukturen mit meristematischen Zellen (MS Medium = Murashige Skoog Medium; MS Salze = Murashige Skoog Salze (Toshio Murashige, Folke Skoog: A Revised Medium for Rapid Growth and Bio Assays with Tobacco Tissue Cultures. In: Physiologia Plantarum, Jg. 15 (1962), Heft 3, S. 473-497, ISSN 0031-9317, doi:10.1111/j.1399-3054.1962.tb08052.x).**

| Embryo | Embryo | Embryo Bombardment | Embryo Bombardment | Agro-Embryo | Agro-Embryo | Planzen-Meristem Exposition |
|---|---|---|---|---|---|---|
| MM1 (MM=Maturation Medium) - Reifungsmedium 1 | MM2-Reifungsmedium2 | MM1OSM (Osmotikum) | MM1MOD (MOD= modifiziert) | MM1Tim (Tim= Timentin) | MM1ACE | Meristem Peeling - Antioxidans |
| MS Salze | MS Medium | MS Salze | MS Salze | MS Salze | MS Salze | MS Salze |
| 30,8 g/l Saccharose | 3,4 g/l Saccharose | 30,8 g/l Saccharose | 30,8 g/l Saccharose | 30,8 g/l Saccharose | 30,8 g/l Saccharose | 95 mg/L Cystein |
| | | 36,4 g/l Sorbitol | 95 mg/l L-Cystein | 150mg/l Timentin | 19.62g/l ACE | 100 mg/L Ascorbinsäur e |
| | | 36,4 g/l Mannitol | 4,25mg/l Silbernitrat | | | |
| | | 95 mg L-Cystein | | | | |
| | | 4,25mg/l L-Silbernitrat | | | | |

Die transiente Einführung der hierin offenbarten Konstrukte in meristematische Zellen oder Gewebe bietet den Vorteil, dass sich aus diesen Reproduktionsgewebe entwickeln, über welche die gezielte Veränderung dann stabil an die nächste Generation weitergegeben werden kann, wobei die Folgegeneration frei von den zuvor eingeführten Konstrukten ist. Die hierin offenbarten Verfahren und Konstrukte erlauben ferner, an der derart veränderten Pflanze direkt Saatgut zu ernten, welches die stabile DNA-Veränderung trägt, ohne als Zwischenschritt über eine Zellkultur in Form von Kallusproduktion und -regeneration zu gehen, womit auch auf die hierfür notwendigen Selektions- und Regenrationsschritte sowie die hierfür erforderlichen Medien und Zusätze umgangen werden können.

In einer Ausführungsform umfasst die Nukleinsäure-Sequenz, welche zur gezielten Veränderung einer Nukleinsäure-Zielregion eingesetzt wird mindestens eine oder mehrere regulatorische Sequenzen.

In einer Ausführungsform umfasst das Nukleinsäure-Sequenz, welche zur gezielten Veränderung einer Nukleinsäure-Zielregion eingesetzt als regulatorische Sequenz mindestens einen oder mehrere Promotor(n), optional einen pflanzen- und gewebespezifischen, einen phänotypischen, einen konstitutiven oder induzierbaren Promotor, welcher geeignet zur Induktion der Transkription in einer gewünschten Zielzelle ist. Ein Promotor ist eine Nukleinsäureregion, welche an der Erkennung sowie der Bindung von RNA-Polymerasen sowie anderen Proteinen beteiligt ist, um die Transkription zu steuern. Geeignete Promotoren für entweder die gRNA oder die die CRISPR Nuklease oder die das katalytisch aktive Fragmente davon kodierende Sequenz sind dem Fachmann auf dem Gebiet wohlbekannt. Die Induktion eines induzierbaren Promotors kann durch Stimuli wie Temperatur, Chemikalien, pH, Licht, endogene Pflanzensignale, z.B. ausgeschüttet nach Verwundung der Pflanze, und dergleichen erfolgen. Beispielhafte Promotoren sind ausgewählt aus der Gruppe, bestehend aus SEQ ID Nos:5-11, und umfassen auch solche Sequenzen mit mindestens 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%,90%, 91%, 92%, 93%, 94%, 95%, 96%, 97%, 98% oder 99% Sequenzhomologie zu diesen Sequenzen, welche trotz Modifikation noch die gleiche Funktion erfüllen wie das jeweils nicht modifizierte Sequenz mit der entsprechenden SEQ ID NO. Weitere vorteilhafte Promotoren sind ausgewählt aus der Gruppe, bestehend aus Promotoren der Wall-Associated Kinasen (WAKs) 1 und 2 (siehe z.B. Wagner TA, Kohorn BD. Wall-Associated Kinases Are Expressed throughout Plant Development and Are Required for Cell Expansion. The Plant Cell. 2001;13(2):303-318 sowie z. B. NCBI Eintrag: NCBI Reference Sequence: NC_003070.9), einem Promotor des SCARECROW1 (*scr*1) Gens (siehe z. B. Tissue Specificity and Evolution of Meristematic WOX3 Function; Rena Shimizu, Jiabing Ji, Eric Kelsey, Kazuhiro Ohtsu, Patrick S. Schnable and Michael J. Scanlon Plant Physiology February 2009 vol. 149 no. 2 841-850 und sowie z.B. NCBI Eintrag: NCBI Reference Sequence: NC_003070.9), einem Promotor der FAF2- und FAF4-Gene (siehe z. B. The FANTASTIC FOUR proteins influence shoot meristem size in Arabidopsis thaliana Vanessa Wahl, Luise H Brand, Ya-Long Guo, Markus Schmid Wahl et al. BMC Plant Biology 2010, 10:285 http://www.biomedcentral.com/1471-2229/10/285 sowie z.B. NCBI Eintrag: NCBI Reference Sequence: NC_003070.9), einem Promotor des OSH1-Gens (siehe z. B. Sato et al. (1996) Proc.Natl. Acad. Sci. USA, 93: 8117-8122 sowie z.B. Genbank Eintrag: GenBank: CP002688.1 oder GenBank: AP008209.2) oder ein Promotor eines Metalloprotein-Gens, z.B. aus Reis (z.B. GenBank: BAD87835.1). Die Promotoren der vorliegenden Offenbarung können natürlich vorkommende, synthetische oder chimäre Promotoren, oder eine Kombination davon, sein. Ein bevorzugter Promotor gemäß der vorliegenden Offenbarung ist ein in einer pflanzlichen Meristemzelle aktiver Promotor oder ein in Plastiden einer pflanzlichen Zelle aktiver Promotor. In einer Ausführungsform umfasst die Nukleinsäure-Sequenz, welche zur gezielten Veränderung einer Nukleinsäure-Zielregion eingesetzt wird, zudem als regulatorische Sequenz mindestens einen Terminator.

In einer Ausführungsform umfasst die die Nukleinsäure- oder Aminosäuresequenz, welche zur gezielten Veränderung einer Nukleinsäure-Zielregion eingesetzt wird, umfassend eine gRNA und eine CRISPR Nuklease oder ein katalytisch aktives Fragment davon, oder eine dafür kodierende Sequenz, mindestens eine oder mehrere Kernlokalisationssequenz(en) (KLS), welche die Kernlokalisation der zur gezielten Veränderung einer Nukleinsäure-Zielregion verwendeten Nukleinsäuren und Polylpeptiden bewirken.

In einer Ausführungsform umfasst das rekombinante Konstrukt umfassend eine Nukleinsäure- oder Aminosäuresequenz, welche zur gezielten Veränderung einer Nukleinsäure-Zielregion eingesetzt wird, eine gRNA und eine CRISPR Nuklease oder ein katalytisch aktives Fragment davon, oder eine dafür kodierende Sequenz, eine oder mehrere Plastidenlokalisationssequenz(en) (PLS), zum Beispiel eine Mitochondrien- oder Chloroplastenlokalisationssequenz(en) (MLS oder CLS), welche die Lokalisation der zur gezielten Veränderung einer Nukleinsäure-Zielregion verwendeten Nukleinsäuren und Polypeptiden in den entsprechenden pflanzlichen Plastiden bewirkt.

In einer Ausführungsform umfasst die Nukleinsäure-Sequenz, welche für eine hierin offenbarte CRISPR Nuklease oder ein katalytisch aktives Fragment davon kodiert, oder eine hierin offenbarte CRISPR Nuklease oder ein katalytisch aktives Fragment davon zudem eine tag-Sequenz. Eine tag-Sequenz ist ein Nukleinsäure- oder Proteinabschnitt, welcher der CRISPR Nuklease oder der gRNA, oder der die CRISPR Nuklease oder die gRNA kodierenden Nukleinsäure-Sequenz vorangestellt und/oder nachgestellt und/oder innerhalb der Sequenz lokalisiert sein kann, um optional unter anderem deren Lokalisation, Visualisierung innerhalb einer Zielzelle zu ermöglichen. Besonders bevorzugt sind tag-Sequenzen ausgewählt aus der folgenden Liste: Polyhistidin(His)-Tag, Glutathione-S-transferase (GST)-tag, Thioredoxin-Tag, FLAG-tag, einem Tag mit Fluoreszenz-Eigenschaften, ausgewählt aus einem Grün-fluoreszierenden Protein (GFP)-tag, einem DsRed-tag, einem mCherry-tag und dergleichen, einem Streptavidin oder strep-Tag, Maltose-Bindeprotein(MBP)-Tag, Chloroplastentransitpeptid, Mitochondrientransitpeptid, ein Snap-Tag und/oder ein Sekretionstag.

In einer weiteren Ausführungsform werden Fusionskonstrukte vorgeschlagen, die in den Verfahren gemäß der vorliegenden Erfindung zum Einsatz kommen können. Diese Fusionskonstrukte umfassen zum einen Fusionsproteine und zum anderen Fusionsnukleinsäuren. Fusionsproteine können aus einer CRISPR-Nuklease, eine Variante oder einem katalytisch aktiven Fragment davon, bzw. der die CRISPR-Nuklease oder die Variante oder das katalytisch aktive Fragment davon kodierenden Sequenz als einem Element sowie optional einem der oben genannten Tags sowie einer Effektor-Domäne, oder einer die die Effektor-Domäne kodierenden Nukleinsäure-Sequenz bestehen. Hierdurch ist es möglich, die hierin offenbarten Effektor-Domänen und/oder ein oder mehrere, gleiche oder unterschiedliche, CRISPR-Nukleasen, Varianten oder katalytisch aktive Fragmente davon als physikalisch verknüpfte Einheit in eine pflanzliche Zielstruktur von Interesse einzubringen, oder sie in einer pflanzlichen Zielstruktur von Interesse zu exprimieren. Bevorzugt wird die Effektor-Domäne optional umfassend eine Linker-Aminosäure-Sequenz am N-oder C-Terminus der CRISPR-Nuklease oder der Variante oder des katalytisch aktiven Fragments davon fusioniert. Die optional anwesende Linker- Aminosäure-Sequenz, oder die diese Linker-Sequenz kodierenden Nukleinsäure-Sequenz, dient dazu, dass sich sowohl die CRISPR-Nuklease als auch die Effektor-Domäne, ohne sich gegenseitig sterisch zu beeinflussen, ideal positionieren können, sodass sowohl die Effektor-Domäne als auch die CRISPR-Nuklease ihre Aktivität entfalten können. Eine Linker- Aminosäure-Sequenz kann 1, 2, 3, 4, 5, 6, 7, 8, 9, 10, 11, 12, 13, 14, 15,16, 17, 18, 19, 20 oder mehr und bis zu 50 oder 100 Aminosäuren umfassen, und kann im Einzelfall auch länger sein. Darüber hinaus werden auch Fusions-Nukleinsäuren offenbart, wobei eine gRNA von Interesse mit einer Effektor-Domäne von Interesse kovalent verknüpft wird. Darüber hinaus werden auch nicht-kovalente Fusions-Nukleinsäuren offenbart, die sowohl eine gRNA als auch eine Effektor-Domäne und/oder eine DNA-Reparatur-Matrize umfassen, wobei die nicht-kovalente Verknüpfung in einer Assoziation der jeweiligen Komponenten durch Hybridisierung, d.h. durch Basenpaarung komplementärer Bereiche erfolgen kann. Darüber hinaus werden Fusions-Konstrukte offenbart, bei welche gRNA und/oder eine CRISPR-Nuklease von Interesse chemisch mit einer Effektor-Domäne von Interesse *in vitro* / ex *vivo* miteinander verknüpft und anschließend in eine pflanzliche Zielstruktur von Interesse umfassend mindestens eine meristematische Zelle eingebracht werden, wodurch diese Effektor-Moleküle bereits verknüpft vorliegen und somit die Verfügbarkeit innerhalb einer pflanzlichen Zelle von Interesse erhöht wird, wodurch die Effizienz der hierin offenbarten Verfahren erhöht werden kann. Darüber hinaus kann die Assoziierung einer gRNA mit einem Effektor-Molekül und/oder einer DNA-Reparatur-Matrize dazu führen, dass nicht nur die Effektor-Domäne und/oder die DNA-Reparatur-Matrize ihre Funktion im Rahmen der Genomeditierung erfüllen, sondern zudem auch die Stabilität einer gRNA erhöht werden kann. Gerade in Ausführungsformen, bei welchen die gRNA unmittelbar in eine pflanzliche Zielstruktur von Interesse, umfassend mindestens eine meristematische Ziel-Zelle, durch Transformation oder Transfektion eingefügt wird, kann dies den Abbau der gRNA bevor diese ihre Funktion ausüben kann durch zelluläre RNAsen deutlich erhöhen.

Wie auch die gRNA und/oder die CRISPR-Nuklease, umfassend unter anderem eine Cas-Nuklease oder eine Cpf1-Nuklease, können auch die oben beschriebenen Fusionskonstrukte, umfassend Fusions-Proteine und Fusions-Nukleinsäuren, und/oder gemischte Fusions-Proteine, umfassend Nukleinsäuren und Proteine, als rekombinante Konstrukte stabil oder transient in eine pflanzliche Zielstruktur von Interesse eingebracht werden, oder zumindest eines der Moleküle kann direkt als RNA, DNA, oder Protein in eine pflanzliche Zielstruktur von Interesse, umfassend mindestens eine meristematische Zelle, eingebracht werden. Beispielsweise kann ein Gen, das für eine CRISPR-Nuklease von Interesse kodiert, zunächst Codon-optimiert werden. Dieses Gen kann dann in Form eines rekombinanten Konstrukts transient in eine pflanzliche Zielstruktur von Interesse eingeführt werden. Alternativ kann die CRISPR-Nuklease auch *in vitro* translatiert und anschließend unmittelbar als Protein in eine pflanzliche Zielstruktur von Interesse eingefügt werden. In einer Ausführungsform kann dann eine gRNA direkt in eine pflanzliche Zielstruktur von Interesse in der Form von RNA eingeführt werden. Dies kann, wie unten näher erläutert, etwa durch Partikel-Beschuss oder sonstige Transfektionsverfahren erfolgen, die dem Fachmann auf dem Gebiet bekannt sind. Ebenso werden die oben geschilderten Fusionskonstrukte folglich entweder als rekombinantes Konstrukt oder direkt in eine Nukleinsäure-Zielregion von Interesse eingebracht.

In einer Ausführungsform gemäß der Verfahren der vorliegenden Erfindung, kann eine transiente Expression der hierin offenbarten CRISPR-Systeme, was mindestens eine gRNA, eine CRISPR-Nuklease, bevorzugt auch mindestens eine Effektor-Domäne und optional eine DNA-Reparatur-Matrize transient durch Partikelbeschuss in eine pflanzliche Zielstruktur von Interesse umfassend mindestens eine meristematische Zelle eingebracht werden. Hierzu können beispielsweise Gold- oder Wolfram-Partikel mit Polyethylenimin (PEI) beschichtet werden. Dazu werden die Gold-Partikel zunächst gewaschen und nach Zentrifugation und optionalem Waschen in Ethanol in Ethanol resuspendiert und bei - 20°C gelagert. Um die Partikel mit PEI (Sigma # P3143) zu beschichten, wurde die gewaschene Mischung aus Gold-Partikeln in Ethanol zentrifugiert und das Ethanol wurde verworfen. Die Partikel wurden dann einmal in ddH₂O gewaschen, um Restalkohol zu entfernen und es wurde dann eine 250 µl einer 0,25 mM PEI-Lösung hinzugefügt, gefolgt von einer Puls-Ultraschallbehandlung, um die Partikel zu suspendieren. Die verschlossenen Gefäße wurden dann in einer Trockeneis-/Ethanolmischung schockgefroren und die Suspension wurde dann über Nacht lyophylisiert. An diesem Punkt können die getrockneten beschichteten Gold-Partikel bei - 80°C für mindestens drei Wochen gelagert werden. Vor der weiteren Verwendung werden die Partikel dreimal mit jeweils 250 µl an 2,5 mM HEPES-Puffer, pH 7,1, gespült gefolgt von einer Puls-Ultraschallbehandlung und dann kurz gevortext, bevor sie zentrifugiert werden. Die Partikel wurden dann in einem finalen Volumen von 250 µl HEPES-Puffer suspendiert. Ein 25 µl aliquoter Partikel wurde in frische Reaktionsgefäße überführt, bevor die DNA-Anheftung erfolgte. Um nicht-beschichtete DNA an die Gold-Partikel anzuheften, wurden die Partikel Puls-Ultraschall behandelt und hierauf wurde ein Mikrogramm an DNA (in 5 µl Nuklease-freiem Wasser) hinzugefügt und die Mischung wurde vorsichtig einige Male auf- und abpipettiert, bevor sie für 10 Minuten bei Raumtemperatur inkubiert wurde. Die Partikel wurden kurz, in der Regel für 10 Sekunden, anzentrifugiert, der Überstand wurde entfernt und 60 µl frischer Ethanol wurden hinzugefügt. Die Partikel mit PEI-präzipitierter DNA wurden zweimal in 60 µl Ethanol gewaschen. Die Partikel wurden dann zentrifugiert und der Überstand wurde verworfen, wonach die Partikel in 45 µl Wasser resuspendiert wurden. Um eine zweite DNA (DNA-2) anzuheften, wurde eine Präzipitation unter Verwendung eines wasserlöslichen kationischen Lipid-Transfektionsreagenz verwendet. 45 µl der Partikel-DNA-Suspension, entsprechend den Gold-Partikeln an die die ersten DNA angeheftet worden war, wurden kurz mit Ultraschall behandelt und dann wurden 5 µl einer 10-Nanogramm/Mikroliter DNA-2 sowie 2,5 µl des wasserlöslichen kationischen Lipid-Transfektionsreagenz hinzugefügt. Die Lösung wurde auf einem Orbitalschüttler für 10 Minuten inkubiert und anschließend bei 10.000 g für eine Minute zentrifugiert. Anschließend wurde der Überstand entfernt und die Partikel wurden in 60 µl Ethanol resuspendiert. Die Lösung konnte dann auf Makroträger übertragen werden und die Gold-Partikel, an die die erste und die zweite DNA sequenziell angeheftet wurden, wurden dann in eine meristematische Zelle von Interesse unter Verwendung eines Standardprotokolls für den Partikel-Beschuss durch eine PDS-1000 Apparatur transfiziert. Standardprotokolle für eine PDS-1000 Apparatur sind vom Hersteller (Bio-Rad) erhältlich.

In einer Ausführungsform gemäß der vorliegenden Offenbarung umfasst das Verfahren zur Herstellung einer Pflanze, eines Pflanzenmaterials oder einer pflanzlichen Zelle zudem eine Screeningschritt. In diesem Schritt wird durch Verfahren zur Analyse der Nukleinsäuresequenz einer Zielregion, z.B. durch Polymerase-Kettenreaktion oder durch Sonden, überprüft, ob die Einbringung, Aktivierung und anschließende Reaktion des mindestens einen rekombinanten Konstrukts gemäß der vorliegenden Offenbarung zur erwünschten gezielten Veränderung einer Nukleinsäure-Zielregion führt. Verfahren, um dieses Screening durchzuführen, sind dem Fachmann auf dem Gebiet für jegliche pflanzliche Zielstruktur sowie Nukleinsäure-Zielregion bekannt. Jedoch existieren derzeit keine Standardverfahren, welche es erlauben würden, dass effektive Zusammenspiel einer gRNA, einer CRISPR Nuklease oder einem katalytischen Fragment davon sowie einer Nukleinsäure-Zielregion von Interesse bezüglich der tatsächlichen Wirksamkeit einer gRNA von Interesse für eine spezifische Nukleinsäure-Zielregion, insbesondere eine Nukleinsäure-Zielregion innerhalb einer pflanzlichen Zelle, in einem *in vitro* Screening Verfahren zu prüfen, um somit den Zeit- und Kostenaufwand bei der Anwendung von CRISPR/Cas Konstrukten, speziell in einem Hochdurchsatzverfahren, zu überprüfen. Zudem sind die meisten verfügbaren *in silico* Tools (siehe etwa https://www.dna20.com/eCommerce/cas9/input) spezialisiert auf *E. coli*, Hefe oder tierische Genome oder Modelpflanzen, nicht jedoch auf wichtige monokotyledone wie dikotyledone Nutzpflanzen, die sich oft signifikant von Modelpflanzen, etwa bezüglich der PAM-Verteilung in genomischen Regionen, unterscheiden.

In einem Aspekt der vorliegenden Offenbarung wird daher ein *in vitro* Screening Verfahren zur Identifizierung einer gRNA oder einer für eine gRNA kodierenden Sequenz in einem *in vitro* Assay, zur Identifizierung einer gRNA oder einer für eine gRNA kodierenden Sequenz, die geeignet ist, zusammen mit einer CRISPR Nuklease oder einem katalytisch aktiven Fragment davon eine Nukleinsäure-Zielregion in einer pflanzlichen Zelle gezielt zu modifizieren, umfassend die folgenden Schritte: (i) Bereitstellen einer oder mehrerer Nukleinsäure-Zielregion(en) einer Pflanze, eines Pflanzenmaterials oder einer pflanzlichen Zelle; (ii) Einfügen der einen oder der mehreren Nukleinsäure-Zielregion(en) in mindestens einem Vektor; (iii) Bereitstellen mindestens einer gRNA; (iv) Bereitstellen mindestens einer CRISPR Nuklease oder eines katalytisch aktiven Fragments davon; (v) Inkontaktbringen der mindestens einen gRNA und der mindestens einen CRISPR Nuklease oder des katalytisch aktiven Fragments davon mit dem mindestens einen Vektor *in vitro* unter geeigneten Reaktionsbedingungen, welche die Interaktion einer gRNA mit einer CRISPR Nuklease und dadurch die katalytische Aktivität der CRISPR Nuklease oder des katalytisch aktiven Fragments davon gestatten, wobei der mindestens eine Vektor jeweils mit genau einer gRNA und genau einer CRISPR Nuklease oder einem katalytisch aktiven Fragment davon in einem separaten Reaktionsansatz in Kontaktgebracht wird; (vi) Analyse der Reaktionsprodukte aus Schritt (v); und (vii) Identifizierung einer gRNA oder einer für eine gRNA kodierenden Sequenz, die dazu in der Lage ist, zusammen mit einer spezifischen CRISPR Nuklease oder einem katalytisch aktiven Fragment davon eine Nukleinsäure-Zielregion in einer pflanzlichen Zelle gezielt zu modifizieren.

Gemäß dieses Aspekts der vorliegenden Offenbarung ist der Begriff *in vitro* derart zu verstehen, dass die mindestens eine Nukleinsäure-Zielregion nicht innerhalb ihres natürlichen Milieus, d.h. einer pflanzlichen Zelle, vorliegt, sondern zunächst in einen geeigneten Vektor zum Zwecke des *in vitro* Screenings überführt wird. Durch dieses Vorab-Screening können dann innerhalb kurzer Zeit eine Vielzahl von Ergebnissen generiert werden, welche die Eignung mindestens einer gRNA im Zusammenspiel mit der entsprechenden CRISPR Nuklease oder dem katalytisch aktiven Fragment davon zur gezielten Modifikation einer Nukleinsäure-Zielregion innerhalb einer intakten pflanzlichen Zelle aufzeigen. Die so ermittelten Kandidaten können dann sowohl *in vitro* als auch *in vivo,* umfassend *in planta*, mit einer deutlich höheren Erfolgsquote verwendet werden.

In einer Ausführungsform ist das PCR-Amplifikat der Nukleinsäure-Zielregion gemäß dieses Aspekts abgeleitet von genomischer DNA, wobei die genomische DNA neben dem nukleären Genom auch das Genom von Plastiden, wie Mitochondrien und Chloroplasten, umfasst. In einer anderen Ausführungsform ist das PCR-Amplifikat der Nukleinsäure-Zielregion gemäß dieses Aspekts abgeleitet von pflanzlicher RNA.

Der mindestens eine Vektor gemäß dieses Aspekts der vorliegenden Offenbarung ist bevorzugt ein Plasmidvektor, es können jedoch auch alle weiteren hierin offenbarten Vektoren zum Einsatz kommen, welche für die Klonierung und stabile Aufrechterhaltung eines PCR-Amplifikats einer Nukleinsäure-Zielregion von Interesse geeignet sind. Die Klonierung einer oder mehrerer Nukleinsäure-Zielregion(en) in mindestens einen Vektor ist dem Fachmann auf dem Gebiet bekannt. Der Vektor kann idealerweise mehr als eine Zielregion von Interesse umfassen, sodass eine Vielzahl von Zielgenen von Interesse analysiert werden kann. Alternativ können auch mehrere Vektoren, die mindestens eine Nukleinsäure-Zielregion von Interesse umfassen, bereitgestellt werden.

Die gRNA zur Anwendung gemäß diesem Aspekt muss in aktiver Ribonukleinsäure-Form appliziert werden, d.h. die gRNA kann entweder synthetisch, optional zusätzlich modifiziert, bereitgestellt werden. Alternativ kann die gRNA auch rekombinant hergestellt werden, d.h. durch *in vitro* oder *in vivo* Transkription und optional durch einen Reinigungsschritt.

Die CRISPR Nuklease oder das katalytisch aktive Fragment davon wird als Aminosäuresequenz bereitgestellt. Hierfür kann eine kommerziell erhältliche CRISPR Nuklease oder eine Variante davon zum Einsatz kommen. Alternativ kann in einer anderen Ausführungsform die CRISPR Nuklease oder das aktive Fragment davon rekombinant produziert und optional isoliert und/oder gereinigt werden, bevor sie in den *in vitro* Screening Verfahren gemäß der vorliegenden Offenbarung zum Einsatz kommt.

In einer weiteren Ausführungsform liegt die bereitgestellte CRISPR Nuklease oder das aktive Fragment davon gekoppelt an mindestens eine Effektor-Domäne vor. Zu möglichen Effektor-Domänen und deren potentiellen Vorteilen und Anwendungsbereichen gilt das in dieser Offenbarung oben Gesagte entsprechend. Der Einbezug der Effektor-Domänen/Cas-Konstrukte bereits in einem *in vitro* Screening Verfahren bietet den Vorteil, dass mögliche unerwünschte, positive oder synergistische Effekte der jeweiligen Effektor-Domäne, die das Cas- oder Cpf1-Konstrukt sterisch wie chemisch/physikalisch beeinflusst, bereits in der Vorabtestphase analysiert werden können. Diese betrifft v.a. einen möglichen Effekt der mindestens einen Effektor-Domäne auf die gRNA-Cas-Interaktion sowie die anschließende Bindung und Modifikation an die Nukleinsäure-Zielregion von Interesse zusätzlich/alternativ zu dem eigentlichen Einsatzbereich der jeweiligen Effektor-Domäne.

Das Inkontaktbringen der mindestens einen gRNA und der mindestens einen CRISPR Nuklease oder des katalytisch aktiven Fragments davon mit dem mindestens einen Vektor *in vitro* erfolgt unter geeigneten Reaktionsbedingungen. In diesem Kontext sind Bedingungen zu verstehen, die sowohl die Bindung der gRNA an die jeweilige CRISPR Nuklease oder des katalytisch aktive Fragment davon als auch die Interaktion des gRNA/Cas Komplexes mit der Zielregion von Interesse sowie die katalytische Aktivität der CRISPR Nuklease oder das katalytisch aktiven Fragments davon gestatten. Geeignete Reaktionsbedingungen wie Puffer, Zusätze, speziell Kofaktoren, welche benötigt werden, Temperatur- und pH-Bedingungen sowie ggf. weitere notwendige Zusätze können vom Fachmann auf dem Gebiet in Kenntnis eines hierin offenbarten Verfahrens und hierin offenbarter Konstrukte, gemäß eines beliebigen Aspekts der vorliegenden Offenbarung leicht bestimmt werden.

Gemäß einer Ausführungsform erfolgt die Analyse der Reaktionsprodukte gemäß des *in vitro* Screening Verfahrens qualitativ. Nach einer weiteren Ausführungsform erfolgt die Analyse der Reaktionsprodukte gemäß des *in vitro* Screening Verfahrens quantitativ. Gemäß noch einer weiteren Ausführungsform erfolgt die Analyse der Reaktionsprodukte gemäß des *in vitro* Screening Verfahrens sowohl qualitativ als auch quantitativ.

In einer Ausführungsform erfolgt das *in vitro* Screening Verfahren als Hochdurchsatzverfahren, d.h. es können simultan mehrere gRNAs und/oder mehrere CRISPR Nukleasen oder die katalytisch aktiven Fragmente davon und/oder mehrere Nukleinsäure-Zielregionen auf einem oder auf mehreren Vektoren in separaten Reaktionsgefäßen getestet werden. Diese Hochskalierung ist ein besonderer Vorteil, um schnell eine Vielzahl von Daten über geeignete gRNA/Cas-Kandidatenpaare für die jeweilige mindestens eine Nukleinsäure-Zielregion von Interesse zu erhalten. Alternativ kann als Variable die Fragestellung analysiert werden, welche gRNA/Cas-Kandidatenpaare besonders effizient zusammenwirken, speziell, wenn der Einsatz neuer CRISPR Nukleasen oder katalytisch aktiver Fragmente davon untersucht wird. Als weitere Fragestellung kann leicht im hohen Durchsatz untersucht werden, ob das Hinzufügen einer Effektor-Domäne an eine CRISPR Nuklease oder das katalytisch aktive Fragment davon einen Einfluss auf gRNA/Cas-Interaktion oder die anschließende katalytische Aktivität der CRISPR Nuklease oder des katalytisch aktiven Fragments davon hat.

Gemäß der vorliegenden Offenbarung können die Vektoren und/oder rekombinanten Konstrukte zur gezielten Veränderung einer Nukleinsäure-Zielregion in einer pflanzlichen Zelle durch mechanische Verfahren, unter anderem Partikel-Beschuss, Mikroinjektion und Elektroporation, oder durch induzierte Endozytose, geeignete Vektoren, direkte Transfektion, und dergleichen eingebracht werden. In einer Ausführungsform der vorliegenden Offenbarung werden die Vektoren und/oder rekombinanten Konstrukte durch Partikelbeschuss in die Zielzelle oder pflanzliche Zielstruktur eingebracht. Hierbei werden die Vektoren zunächst z.B. auf Gold- oder Wolframpartikel präzipitiert und die Zielzelle/pflanzliche Zielstruktur wird dann mit den so erhaltenen ggf. weiter prozessierten Partikeln durch eine geeignete Apparatur bombardiert.

In einer weiteren Ausführungsform der vorliegenden Offenbarung werden die Vektoren und/oder rekombinanten Konstrukte durch Mikroinjektion in die Zielzelle oder pflanzliche Zielstruktur direkt oder indirekt eingebracht.

In einer anderen Ausführungsform der vorliegenden Offenbarung werden die Vektoren und/oder rekombinanten Konstrukte durch Besprühen mit anschließender Aufnahme, z.B. im Zuge einer viralen Infektion, oder Infiltration in die Zielzelle oder pflanzliche Zielstruktur eingebracht.

Gemäß einer Ausführungsform werden die Vektoren und/oder rekombinanten Konstrukte durch Partikel-Beschuss in eine meristematische Zelle eingebracht. Diese Methode eignet sich sowohl zur Einbringung von rekombinanten Konstrukten umfassend doppelsträngige Plasmid-DNA, lineare einzelsträngige oder doppelsträngige DNA, einzelsträngige oder doppelsträngige RNA, und von Polypeptiden sowie von Kombinationen davon bei allen Arten von pflanzlichen Meristemen in unterschiedlichen Entwicklungsstadien einer Pflanze. Als Trägermaterial für die rekombinanten Konstrukte können unter anderem Gold und Wolfram verwendet werden. In einer weiteren Ausführungsform gemäß der vorliegenden Offenbarung erfolgt die Einbringung der Vektoren und/oder der rekombinanten Konstrukte durch Mikroinjektion direkt in die Zielzelle oder das gewünschte Kompartiment einer Zielzelle.

Gemäß dieser weiteren Ausführungsform werden die Vektoren und/oder rekombinanten Konstrukte durch Mikroinjektion in eine meristematische Zelle eingebracht. Diese Art der Einbringung eignet sich für alle Arten von Meristemen (s. Beispiel 2 unten). Zudem eignet sich die Einbringung gemäß dieser Ausführungsform sowohl zur Einbringung von rekombinanten Konstrukten, umfassend doppelsträngige Plasmid-DNA, lineare einzelsträngige oder doppelsträngige DNA, einzelsträngige oder doppelsträngige RNA, und von Polypeptiden sowie von Kombinationen davon.

In noch einer weiteren Ausführungsform gemäß der vorliegenden Offenbarung erfolgt die Einbringung der Vektoren durch Elektroporation durch Hochspannungspulse.

In noch einer anderen Ausführungsform gemäß der vorliegenden Offenbarung erfolgt die Einbringung der Vektoren durch Endozytose, d.h. einen zelleigenen Mechanismus, durch welchen zellfremdes Material in die Zelle aufgenommen werden kann.

In einer Ausführungsform ist der Vektor ein viraler Vektor, welcher das mindestens eine rekombinante Konstrukt umfasst. Die Einbringung durch einen viralen Vektor bietet die Möglichkeit der Ausbreitung des Vektors und des umfassten mindestens einen rekombinanten Konstrukts. Geeignete virale Vektoren, welche zur Anwendung gemäß der vorliegenden Offenbarung eingesetzt oder modifiziert werden können, sind ausgewählt aus der Gruppe, umfassend , aber nicht beschränkt auf SEQ ID NOs: 12-15 und 25-38 und umfassen auch solche Sequenzen mit mindestens 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%,90%, 91%, 92%, 93%, 94%, 95%, 96%, 97%, 98% oder 99% Sequenzhomologie zu diesen Sequenzen. Es ist dem Fachmann auf dem Gebiet bekannt, dass die Sequenz eine natürlich vorkommenden Virus, welcher als viraler Vektor eingesetzt werden soll, entsprechend an das gewünschte einzubringende rekombinante Konstrukt sowie die pflanzliche Zielstruktur von Interesse angepasst werden muss. In einer weiteren Ausführungsform werden die rekombinanten Konstrukte durch *Agrobacterium spp.-*vermittelte Transformation, insbesondere *Agrobacterium tumefaciens-* oder *Agrobacterium rhizogenes*-vermittelte Transformation, in eine pflanzliche Zielstruktur eingebracht. Diese Art der Einbringung allgemein ist dem Fachmann auf dem Gebiet für unterschiedliche Zielpflanzen sowie unterschiedliche pflanzliche Zielstrukturen davon wohlbekannt.

Der Fachmann auf dem Gebiet weiß, dass die Wahl der Einbringungsart u.a. von der entsprechenden pflanzlichen Zielzelle sowie dem einzubringenden Konstrukt abhängen kann, weshalb je nach Zielzelle eine andere Einbringungsart erforderlich sein kann.

In einer weiteren Ausführungsform gemäß der vorliegenden Offenbarung erfolgt die Einbringung der Vektoren und/oder rekombinanten Konstrukte durch eine Kombination der oben genannten Einbringungsmethoden. So kann beispielsweise ein viraler Vektor, welcher das mindestens eine rekombinante Konstrukt von Interesse enthält, durch *Agrobacterium tumefaciens* als weiteren Vektor, oder etwa durch Partikel-Beschuss oder Mikroinjektion, in die pflanzliche Zielstruktur eingebracht werden.

In einem Aspekt der vorliegenden Offenbarung werden spezielle Verfahren zur Einbringung von Vektoren und/oder rekombinanten Konstrukten gemäß der vorliegenden Offenbarung in meristematische Pflanzenzellen und -gewebe offenbart. Für die Transformation oder Transfektion meristematischer Zellen und Gewebe ist deren Zugänglichkeit ein entscheidender Faktor, wobei sich die Zugänglichkeit der unterschiedlichen pflanzlichen Meristemtypen in den verschiedenen Entwicklungsstadien einer Pflanze erheblich unterscheiden.

In einer Ausführungsform gemäß der vorliegenden Offenbarung wird daher ein Verfahren zur Bereitstellung einer pflanzlichen Zielstruktur, umfassend mindestens eine meristematische Zelle, offenbart, wobei in die pflanzliche Zielstruktur mindestens ein rekombinantes Konstrukt gemäß der vorliegenden Offenbarung eingebracht werden kann. Das Verfahren gemäß dieser Ausführungsform umfasst als entscheidenden Schritt das Zugänglichmachen einer meristematischen Struktur von Interesse, welche noch nicht ausdifferenzierte meristematische Zellen umfasst. Handelt es sich bei der pflanzlichen Zielstruktur um Meristeme im Embryo, ist es wesentlich, einen pflanzlichen Embryo der richtigen Größe zu wählen und die tieferen, d.h. im Inneren liegenden, meristematischen Zellen als Ziel einer Transformation oder Transfektion mit mindestens einem rekombinanten Konstrukt gemäß der vorliegenden Offenbarung anzusteuern, da die in den äußeren Schichten befindlichen meristematischen Zellen bereits einen gewissen Grad der Differenzierung erreicht haben können und somit nicht mehr gemäß der vorliegenden Erfindung geeignet sind. Vorzugsweise sind die pflanzlichen Embryonen hinsichtlich ihrer Größe so auszuwählen, dass sie mit einem exponierteren Meristem ausgestattet sind. Für Mais-Embryonen bedeutet das, dass Embryonen von weniger als 1,5 mm maximalem Durchmesser, bevorzugt von weniger als 1 mm maximalem Durchmesser, besonders bevorzugt von weniger als 0,7 mm maximalem Durchmesser und ganz besonders bevorzugt von weniger als 0,5 mm maximalem Durchmesser in vorteilhafter Weise erfindungsgemäß eingesetzt werden können. Eine meristematische Zelle im Sinne der vorliegenden Erfindung ist somit eine meristematische Zelle, deren Differenzierungsgrad es noch gestattet, dass aus der Zelle nach er gezielten Veränderung einer Nukleinsäure-Region von Interesse noch alle gewünschten Typen von Pflanzenzellen, insbesondere solche Typen, aus welchen direkt oder indirekt eine fertile Pflanze regeneriert werden kann, entstehen können.

In einer weiteren Ausführungsform wird ein Verfahren zur Bereitstellung einer pflanzlichen Zielstruktur, umfassend mindestens eine meristematische Zelle, in die mindestens ein rekombinantes Konstrukt gemäß der vorliegenden Offenbarung eingebracht werden kann, offenbart, wobei es sich bei der meristematischen Zelle um eine Zelle eines Keimlings oder einer älteren Pflanze handelt.

Gemäß dieser Ausführungsform muss das Meristem vollständig oder nahezu vollständig freipräpariert werden. Zudem muss beachtet werden, dass die tiefer liegenden, d.h. im Inneren liegenden, meristematischen Zellen als Ziel einer Transformation oder Transfektion mit mindestens einem rekombinanten Konstrukt gemäß der vorliegenden Offenbarung angesteuert werden, da die in den äußeren Schichten befindlichen meristematischen Zellen bereits einen gewissen Grad der Differenzierung erreicht haben können und somit nicht mehr gemäß der vorliegenden Erfindung geeignet sind. Gemäß dieser Ausführungsform sind die freigelegten Meristeme einer Oxidation ausgesetzt. Um eine Schädigung der meristematischen Zellen zu verhindern, werden daher vorzugsweise geeignete antioxidative Schutzmaßnahmen angewendet, wie z.B. der Einsatz eines Anti-Oxidationsmittels oder weiterer schützender Maßnahmen, um eine weitere Entwicklung der pflanzlichen Zielstruktur umfassend mindestens eine meristematische Zelle, zu gewährleisten.

### Sequenzprotokoll - freier Text

Die folgenden Angaben geben die deutsche Übersetzung der im Sequenzprotokoll als freier Text (Numerische Kennzahl <223>) enthaltenen Angaben, jeweils aufgeführt für die entsprechende Sequenzkennzahl, wieder. Alle angeführten Sequenzen enthalten unter der Kennzahl <213> den Hinweis auf eine "Künstliche Sequenz".
SEQ ID NO:1
   <223> VP16 Aktivator umfassende Sequenz
SEQ ID NO:2
   <223> VP16 Aktivator
SEQ ID NO:3
   <223> VP64 Aktivator mit Glycin Serin Spacern
SEQ ID NO:23
   <223> Vektor1_TaU6
SEQ ID NO:24
   <223> Vektor1_ZmU3
SEQ ID NOs: 41 bis 48
   Protospacer Region guide RNA 14, Protospacer Region guide RNA 16, Protospacer Region guide RNA 37, Protospacer Region guide RNA 38, Protospacer Region guide RNA 39, Protospacer Region guide RNA 43, Protospacer Region guide RNA 18 bzw. Protospacer Region guide RNA 52.

### Beispiele

Die vorliegende Erfindung wird weiter durch die folgenden, nicht als limitierend anzusehenden, Beispiele erläutert.

### Beispiel 1: Herstellung von CRISPR/Cas-Konstrukten

Die Erstellung der Konstrukte erfolgt auf Grundlage der Publikation von Mali et al. 2013. Die Promotoren wurden gezielt gegen spezifische pflanzliche Promotoren ersetzt und die gRNA den jeweiligen Zielgenen angepasst.

### Konstrukte für monolotyledone Pflanzen:

Als Promotoren wurden unter anderem der Mais-Ubi-Intron Promotor (SEQ ID NO:7), der Mais U3 Promotor (SEQ ID NO:10), der Weizen U6 Promotor (SEQ ID NO:8), der Reis U3 Promotor (SEQ ID NO: 9) und der Brachipodium EF1 Promotor (SEQ ID NO: 40) verwendet. Ein beispielhaftes verwendetes Konstrukt hat die SEQ ID NO: 23 (Vektor1_TaU6 standard).

### Konstrukte für dicotyledone Pflanzen (nicht erfindungsgemäß):

Hier wurden als Promotoren ein Parsley-Ubi4 (SEQ ID NO:5) und ein Arabidopsis U6 Promotor (SEQ ID NO:6) verwendet. Ein beispielhaftes verwendetes Konstrukt hat die SEQ ID NO:24 (Vektor1_ZmU3 standard).

Die unterschiedlichen gRNAs wurden für die jeweiligen Zielgene spezifisch erstellt und in die entsprechende Position in den oben angegebenen Vektoren kloniert. Die Position der gRNA Sequenz entspricht den als "n" gekennzeichneten Nukleotiden in SEQ ID NOs: 23 und 24.

Um die Anzahl an unterschiedlichen gRNAs, die zur Erzielung eines Genome Editings in die Pflanzen eingebracht werden müssen, zu reduzieren, wurde ein *in vitro* Assay etabliert, um die Kandidaten-gRNAs zu testen und nur die geeignetsten Kandidaten in die Pflanze einzubringen.

Hierfür wurden zunächst potentiell geeignete gRNAs durch *in silico* Analyse definiert. Diese Definition ist, wie im einleitenden Teil der Beschreibung erwähnt, auf Grund der dualen Funktion der gRNA abhängig von der Nukleinsäure-Zielregion von Interesse, speziell einem PAM-Motiv, als auch der gewünschten CRISPR Nuklease oder dem katalytisch aktiven Fragment davon, welches zum Einsatz kommen soll.

Um die unterschiedlichen gRNAs für unterschiedliche Gene zu testen, wurden in einem ersten Schritt die Nukleinsäure-Zielregionen von Interesse, oder Teilbereiche davon, mittels PCR amplifiziert und in Standard-Vektoren kloniert. Standard-Vektoren in diesem Sinne bezeichnet kommerziell erhältliche Vektoren oder Vektorsysteme, welche durch dem Fachmann bekannte Mittel leicht an die Bedürfnisse des erwünschten Assays angepasst werden können, insbesondere, indem er als Rückgrat für die Klonierung von Nukleinsäuren von Interesse fungieren kann. Beispielhafte Vektoren können ausgewählt sein aus: pJet (Thermo Fisher Scientific, Inc, USA), pGEM-T (Promega BioSciences, LLC, USA) oder pBluescript (Addgene, USA). Diese Vektoren dienen in dem neu entwickelten *in vitro* Assay als Substrat. In einem zweiten Schritt wurden die verschiedenen gRNAs mittels *in vitro* Transkription (Invitrogen MAXlscript T7 Kit; Cat. No. AM1312M) hergestellt.

In einem *in vitro* Assay wurden anschließend die gRNAs getestet und die potenziell besten Kandidaten ausgewählt und für die weiteren *in planta* Versuche verwendet. Hierzu wurde u.a. als exemplarische Nukleinsäure-Zielregion von Interesse ein Maispflanzen A188 *hmg13-*Gen (HMG-transcription factor 13; s. Gen GRMZM2G066528 gemäß EnsemblePlants bzw. maize Genome Data Base). Dieses wurde mittels PCR amplifiziert und in passend in die multiple cloning site von pJET1.2 kloniert, zum einen der Teil umfassend Exon 3-5 (hmg-fw4 und hmg-re2, s. Figur 14), zum anderen der hmg-3'Teil (hmg-fw3 und hmg-re1, s. Figur 15). Hierauf wurden die Plasmide durch Verdau mit Pvul linearisiert und zur Verhinderung der Rezirkularisierung wurde das Vektor-Rückgrat dephosphoryliert. Das erhaltene Produkt wurde auf ein präparatives Gel aufgetragen, woraufhin das Produkt nach Gellauf extrahiert und gereinigt wurde. Anschließend wurde die Konzentration des erhaltenen linearisierten Vektors vermessen. Für einen gewöhnlichen Assay wurden ca. 3 µl eines 30 nM Vektors als Substrat für den Cas-Verdau, jeweils mindestens in Triplikaten durchgeführt, eingesetzt. Zur Bereitstellung der zu testenden gRNA Varianten wurden diese in den Vektor pEn-Chimera kloniert (s. exemplarisch Figur 16 für die gRNA14). Die Klonierung in diesen Vektor erfolgt gemäß molekularbiologischer Standardmethoden, wie im Folgenden beispielhaft geschildert. Die Sequenz von pEn-Chimera findet sich in SEQ ID NO: 59. Auf diesem befindet sich eine RNA Chimera, welche sich relativ einfach via Bbsl + Oligo spezifizieren lässt. Danach kann sie über eine Gateway LR Reaktion in den pDe-CAS9 transferiert werden. Die RNA Chimera ist unter der Kontrolle des Promotors AtU6-26. Der erhaltene Vektor wurde dann mit Ncol und *Xba*l verdaut, wobei das resultierende Fragment die gRNA von Interesse umfasst. Das erwünschte Fragment umfassend die gRNA wurde durch übliche Methoden Gel-separiert, extrahiert und gereinigt. Für die Assays wurde jeweils ca. 1 µg des erhaltenen Fragments als Template für *in vitro* Transkriptionen (Invitrogen MAXlscript T7 Kit; Cat. No. AM1312M) verwendet. Ein exemplarischer Ansatz umfasste: 10 µl Template (1 µg); 2 µl 10 x Transkriptionspuffer (Invitrogen); 1 µl 10mM ATP; 1 µl 10mM CTP; 1 µl 10mM GTP; 1 µl 10mM UTP; 2 µl T7-RNA Polymerase (Invitrogen); 4 µl H₂O. Der Ansatz wurde üblicherweise für 2 h bei 37°C inkubiert. Hierauf wurde 1 µl TURBO DNAse hinzugefügt und der Ansatz für weitere 15 min bei 37°C inkubiert. H₂O wurde auf ein Volumen von 100 µl hinzugefügt und die erhaltene RNA wurde nach Herstellerangaben aufgereinigt (Qiagen RNeasy Kit). Nach der Elution (2fach mit 50 µl H₂O) wurde das exakte Volumen sowie die Konzentration der erhaltenen RNA bestimmt. Für die weiteren Assays wurden jeweils ca. 15 ng/µl des *in vitro* Transkripts einer gRNA eingesetzt, was bei einer 140 Nukleotid-langen RNA etwa 300 nM entspricht. Im Anschluss wurde ein *in vitro* Verdau wie folgt durchgeführt: Der Reaktionsansatz betrug typischerweise 30 µl. Um eine optimale Spaltungseffizienz zu gewährleisten ist es wichtig, ein molares Verhältnis von Cas9 und gRNA zur jeweiligen Nukleinsäure-Zielregion im Verhältnis 10:10:1 oder höher einzuhalten. Als nächstes wurden 300nM der jeweiligen zu testenden gRNA bereitgestellt. Zudem wurden je 30 nM an Substrate DNA umfassend jeweils eine einzige Nukleinsäure-Zielregion, bereitgestellt. Der Reaktionsansatz wurde in der folgenden Reihenfolge zusammengefügt:

| Komponente | 30 µl Ansatz |
|---|---|
| Nuklease-freies Wasser | 20 µl |
| 10X Cas9 Nuklease Reaktionspuffer (NEB) | 3 µl |
| 300nM gRNA (~15 ng/µl) | 3 µl (30nM final) |
| 1 µM Cas9 Nuklease | 1 µl (∼30nM final) |
| Reaktionsvolumen | 27 µl |
| Vorinkubation für 10 min bei 37°C 30nM Substrat DNA | 3 µl (3nM final) |
| Gesamt-Reaktionsvolumen | 30 µl |

Hierauf wurde vorsichtig gemischt und kurz anzentrifugiert bevor der Ansatz für eine weitere Stunde bei 37°C inkubiert wurde. Optional erfolgte hierauf eine Behandlung mit Proteinase K durch Hinzufügen von 1 µl Enzyme und Inkubation für 15-30 min bei 37°C. Hierauf konnte die Fragmentanalyse erfolgen (s. Figur 8).

Die Ergebnisse für 10 ausgewählte gRNAs, hier exemplarisch im Zusammenspiel mit einer Cas9 Nuklease, finden sich in Figur 8. Aus diesem Resultat geht eindeutig hervor, dass es qualitative wie quantitative Unterschiede in der Effizienz der jeweiligen gRNA und der Partner-CRISPR Nuklease bezüglich der Spaltungseffizienz einer Nukleinsäure-Zielregion von Interesse gibt.

Weiterführende Experimente (Daten nicht gezeigt) wurden mit CRISPR Nukleasen anderen Ursprungs als S. *pyogenes* sowie mit Cas-Nuklease, welche mindestens eine Punktmutation tragen, z. B: eine Cas-Nickase, durchgeführt, um dadurch die Effizienz dieser weiteren Cas-Nukleasen auf eine pflanzliche Nukleinsäure-Zielregion von Interesse im Zusammenspiel mit der jeweiligen gRNA *in vitro* zu testen. Darüber hinaus wurden erste erfolgreiche Experimente *in vitro* durchgeführt, welche zeigen, dass Cas-Nuklease-gRNA Paare von Interesse, welche in einem Vorab-Screen identifiziert wurden, auch dazu geeignet sind eine RNA sowie pflanzliche mitochondriale bzw. plastidäre DNA gezielt zu modifizieren, was das breite Anwendungsspektrum des vorliegenden Assays belegt.

Um die breite Anwendbarkeit des Verfahrens für weitere wichtige Nutzpflanzen zu testen, wurde das wie oben geschildert neuartige *in vitro* Screening Verfahren unter Einsatz von Nukleinsäure-Zielregionen abgeleitet von weiteren Nutzpflanzen wie *Beta vulgaris*, *Brassica napus* und *Sorghum bicolor* ebenfalls durchgeführt.

Hierfür war es auf Grund der Besonderheit der pflanzlichen Genome notwendig, neue *in silico* Analysen durchzuführen, um geeignete Zielregionen und damit gRNAs definieren zu können. Zudem wurde in Rahmen der Weiterentwicklung des Verfahrens auch mit weiteren Cas-Nuklease, -Nickasen, Cpf-Endonukleasen und enzymatisch aktiven, davon abgeleiteten Fragmenten gearbeitet, die zudem eine Effektordomäne tragen. Weiterhin konnten auch alternative Cas-Proteine, oder Cas-Proteine mit z.B. Punktmutationen in das Assay eingesetzt werden und dadurch die Effizienzen der unterschiedlichen Cas-Proteine getestet werden. Vor allem auch in direktem Zusammenspiel mit den getesteten gRNAs.

Hierdurch sollten mehrere Fragestellungen beantwortet werden: (1.) Welche gRNAs zeigen besonders hohe Aktivität?; (2.) wie wirken sich Modifikationen in der gRNA, etwa unterschiedliche Längen des Protospacers oder von Missmatchen, aus?; (3.) welche Cas-Proteine interagieren am besten mit welchen gRNAs?; (4) welche CRISPR Nuklease, d.h. Cas-Nukleasen oder Cpf1-Nuklease, oder welche Mutationen in einer CRISPR Nuklease haben einen Effekt auf die enzymatische Wirkung des Enzyms?; und (5) beeinflusst die Ankopplung einer Effektor-Domäne und damit die Bereitstellung eines sterisch anspruchsvolleren Cas-Konstruktes die Interaktion mit der entsprechenden gRNA und damit die Effizient der gezielten Modifikation einer Nukleinsäure-Zielregion von Interesse? Im Zuge dieser weiterführenden Experimentserie stellte sich bis dato heraus, dass speziell die Reduktion einer CRISPR Nuklease auf eine katalytisch aktives Minimalfragment davon vorteilhaft im Hinblick auf die erzielte Spaltungseffizienz ist. Darüber hinaus wurde herausgefunden, dass die Anheftung von Effektor-Domänen an die CRISPR Nuklease bzw. die gRNA möglich ist. Gerade hier war jedoch das *in vitro* Screening unerlässlich, da die Effizienz dieser modifizierten CRISPR Nukleasen oder gRNAs wohl auf Grund der größeren sterischen Beanspruchung durch die Effektor-Domäne und dadurch bedingt einer erschwerten Interaktion von Cas und gRNA für die getesteten Paare geringer war. Dennoch wurden auch hier wirksame Cas-gRNA-Effektor-Domänen Paare identifiziert.

Überraschenderweise wurde gefunden, dass die Ergebnisse des *in vitro* Vorabtests, d.h. des Screenings, auch mit deren Wirksamkeit in Folgeversuchen korrelieren.

In einem weiteren Versuch wurden dann alle in Figur 8 gezeigten gRNAs auf ihre Effizienz bei der tatsächlichen ortsgerichteten Modifikation in einem pflanzlichen Meristem hin getestet. Im Ergebnis zeigte der *in vitro* Assay zur Beurteilung der Effizienz der eingesetzten gRNA hervorragend einsetzbar ist, denn nicht bei allen eingesetzten gRNAs kam es zu einem Schneiden in dem Template *in vivo* oder *in vitro.* Gerade bei den Cas-gRNA Paaren, welche sich im *in vitro* Screening Assay als besonders effizient erwiesen haben, bestätigte sich diese Effizient auch bei den Folgeversuchen, bei welchen mit pflanzlichem Material, entweder *in vitro* oder *in vivo* gearbeitet wurde. Als Ausgangspflanze für diese Folgestudien fungierte eine Maispflanze, und als Target speziell das Zielgen *hmg13.*

### Beispiel 2: Einbringung von CRISPR/Cas-Konstrukten

Die oben in Beispiel 1 beschriebenen Konstrukte wurden mittels unterschiedlicher Methoden in die Meristeme eingebracht. Grundlage dafür ist die Zugänglichkeit der Meristeme, die abhängig von dem verwendeten Material durch unterschiedliche Methoden gewährleistet wurde (siehe Beispiel 4)

Folgende Methoden wurden verwendet:
- Partikel-Beschuss:
   Partikel-Beschuss kann bei allen verwendeten Meristemen angewendet werden. Es wird mit dsPlasmid-DNA, linearer dsDNA, RNA und Protein sowie mit Viruspartikeln beschossen. Als Trägermaterial können u.a. Gold und Wolfram verwendet werden. Als Test wurden Beschüsse von Embryo-Meristemen (Fig. 5) und "Tassel"-Meristemen (Fig. 7) gemacht, Mit Hilfe des rot fluoreszierenden Proteins konnte nachgewiesen werden, dass es möglich ist, DNA durch Partikel-Beschuss in diese Zellen einzubringen. Wichtig dabei zu beachten ist, dass die geeigneten Beschuss-Einstellungen in Abhängigkeit des jeweiligen Materials verwendet werden. So kann ein stärkerer Beschuss zu einer erhöhten transienten Transfektion führen (für Bilder hierzu, siehe Figur 9), aber z.B. die Embryonen dadurch auch stark beschädigen, was eine Keimung und Entwicklung unmöglich macht. Es bedarf also in Abhängigkeit des Pflanzenmaterials von Interesse, das als Zielstruktur dient, gewisser Vorarbeiten, um die geeigneten Bedingungen des Partikel-Beschusses auf die jeweiligen Bedürfnisse des Experiments hin anzupassen.
   Die Etablierung geeigneter Beschussmethoden in Abhängigkeit vom verwendeten Pflanzenmaterial wie auch des erwünschten Effekts (transiente versus stabile Einbringung) bei gleichzeitig niedriger Verletzung des Pflanzengewebes oder der Zerstörung des einzubringenden Konstrukts ist daher unerlässlich.
- Mikroinjektion:
   Die Mikroinjektion kann bei allen Meristemen stattfinden, vorzugsweise unter Verwendung eines Mikroskops mit Mikromanipulator. Auf Grund der Größe bestimmter Meristemstrukturen wie präparierten "Tassel"- und "Ear"-Meristemen kann die Mikroinjektion aber auch unter mikroskopischer Kontrolle durchgeführt werden. Die Injektion kann über unterschiedliche Methoden stattfinden und wie schon beim Partikel-Beschuss (Beispiel 1) angegeben mit unterschiedlichen Molekülen. Zum einen werden dsPlasmid-DNA, linearer dsDNA, RNA und Protein, sowie Virus-Partikel in flüssiger Lösung durch eine Mikro- oder Nano-Kanüle in die meristematischen Zellen injiziert, zum anderen werden dsPlasmid-DNA, linearer dsDNA, RNA und Protein auf Mikro- oder Nano-Nadeln aufgebracht und durch anstechen der meristematischen Zellen mit den Nadeln übertragen.

Eine Weiterentwicklung dieser Technologie liegt in der Verwendung einer Kombination aus Silicon Carbide (SiC) Whiskern (z.B. Silar^{®} Silicon Carbide Whisker) und Mikroinjektion. Hierbei wird dsPlasmid-DNA, lineare dsDNA, RNA, Protein oder Viruspartikel auf die Silicon Carbide (SiC) Whisker präzipitiert und mittels Mikroinjektionskanüle in die Meristeme injiziert.

Dies bietet den Vorteil, dass man nicht nur eine einzelne meristematische Zelle transfizieren kann, sondern man durch die Verbreitung der Whisker die Möglichkeit besitzt, verschiedene Zellen parallel zu penetrieren. Da man mit der Kanüle nicht in die Zelle stechen muss und die Whisker deutlich kleiner sind, sind die Verletzungen der Zellen deutlich geringer.

Vascular Puncture Infection/Inokulation (VPI):
Die Vascular Puncture Infection oder Inokulation ist eine in Benavente, 2012 (Virus-Induced Gene Silencing in Diverse Maize Lines Using the Brome Mosaic Virus-based silencing vector) und Louie, 1995 (Louie R, 1995. Vascular puncture of maize kernels for the mechanical transmission of maize white line mosaic virus and other viruses of maize.

Phytopathology85: 139-143) beschriebene Methode, die dazu genutzt wird, Viren, Virenpartikel, Agrobakterien und nackte DNA in intakte Maiskörner einzubringen. Diese Technik ermöglicht das gezielte Einbringen in die Nähe des Embryos und des meristematischen Gewebes. Sie bietet den Vorteil, dass keine Präparationsschritte nötig sind und der angekeimte Same direkt verwendet werden kann. Dadurch wird eine Verletzung des Gewebes minimiert und die Entwicklung der Pflanze nur gering gestört. Dieses Verfahren wurde wie folgt modifiziert und eingesetzt: Samen, welche eine Nukleinsäure-Zielregion von Interesse enthalten, wurden in Wasser für 4 h bei 30°C eingeweicht. Die Samen wurden dann über Nacht in feuchten Tüchern bei Raumtemperatur inkubiert. Im Anschluss wird ein Plasmid oder ein Plasmidmix oder ein Virus von Interesse auf die Embryo tragende Seite des Samenkorns pipettiert. Üblicherweise wurde eine 100 µl Plasmidmischung vorbereitet in einer Konzentration von 37,5 µg/100µl oder 1,5 µg/4 µl je Plasmid. Mit Hilfe eines Einkerbungswerkzeugs wurde das Inokulum 1-2 mm in das Scutellum entlang des Embryos in Richtung Gefäßbündel bewegt. Haltestifte im Winkel von 45° zur Oberfläche des zu behandelnden Korns. Es erfolgten 2 Inokulationen in 1 mm Entfernung zum Embryo, um den Embryo nicht zu verletzen. Der Tropfen wurde dann auf dem Korn belassen.

### Beispiel 3: Transiente meristematische Transformation von Maiskeimlingen (nicht erfindungsgemäß) und/oder Embryonen/erfindungsgemäße Behandlung der meristematischen Gewebe

Die Zugänglichkeit der Meristeme in den einzelnen Stadien unterscheidet sich erheblich. So sind im Embryo (Fig. 1 und 2) die Meristeme relativ gut zugänglich, vorausgesetzt man verwendet Embryonen der richtigen Größe. Wichtig ist es, die tiefer gelegenen Zellen der Meristeme zu transformieren, da die oberen Zellen bereits eine gewisse Differenzierung durchlaufen haben und nicht mehr geeignet sind. Figuren 10 und 11 zeigen zwei Ansichten eines Mais-Embryos sowie darin mit Sternen gekennzeichnet die Lokalisation meristematischer Gewebe. Initial wurden diese Daten durch einen Fluoreszenzmarker visualisiert. Hieraus ist ersichtlich, dass durch die Bereitstellung des neu gefundenen Verfahrens die gezielte Targetierung pflanzlicher meristematischer Zellen und Gewebe ermöglicht wird. Dadurch wird ein neuartiges Vorgehen zur Einbringung von Nukleinsäurekonstrukten, z.B. Vektoren, aber speziell auch von RNAs und Aminosäuren in eine pflanzliche Zielzellenermöglicht. Das Anwendungsspektrum umfasst dabei eine Vielzahl möglicher Konstrukte zur gezielten gentechnischen Veränderung einer Pflanzenzelle, etwa CRISPR/Cas Konstrukte, virale Vektoren, RNAi Konstrukte und dergleichen, um hierdurch gezielte Knock-Ins, Knock-Outs oder gezielte Punktmutationen in der Nukleinsäure-Zielregion der pflanzlichen Zelle zu erzielen.

Meristeme in Keimlingen und älteren Pflanzen müssen vollständig frei präpariert werden, da sie schon von so vielen Schichten Gewebe umgeben sind, dass sie nicht für einen Beschuss oder eine Mikroinjektion zugänglich sind. Fig. 3 und 4 zeigen die präparierten Meristeme, die für die Transformation verwendet werden können. Auch hier gilt wie bei den Embryo-Meristemen, dass die oberen Zellen bereits eine gewisse Differenzierung durchlaufen haben und nicht mehr geeignet sind. Es müssen also die Zellen weiter innen in den Meristemen transformiert werden. Die frei präparierten Meristeme können sowohl horizontal als auch vertikal beschossen werden. Bei Detailstudien wurde herausgefunden, dass durch den vertikalen Beschuss die Trefferquote in den geeigneten meristematischen Regionen deutlich erhöht wird (siehe Fig. 11 und 12). Dies zeigt erneut auf, dass Partikel-Beschuss zwar eine bekannte und etablierte Methode darstellt, dessen wirksame Anwendung für eine spezifische Fragestelllung bei der Transformation von spezifischen Pflanzengeweben jedoch die Optimierung diverser Parameter (einzubringendes Konstrukt, Form und Stadium des zu transformierenden Materials, Druck, Orientierung etc.) erfordert.

Da die isolierten Meristeme frei liegen und dadurch einer starken Oxidation und eines daraus resultierenden Absterben ausgesetzt sind, wurden sie mit einem Anti-Oxidationsmittel, um eine weitere Entwicklung des Keimlings zur Pflanze zu ermöglichen.

Um die "Tassel"-Meristeme zugänglich zu machen wurde eine Methode entwickelt, um die Pflanze und die Meristeme möglichst wenig zu schädigen. Dazu wird auf der Höhe der "Tassel"-Meristeme eine Art Fenster durch die Blätter hindurch geschnitten (Fig. 6). Dies gewährleistet, dass die Blätter nicht absterben und sich die Pflanze ganz normal weiterentwickelt und, dass das Meristem von den restlichen Blättern geschützt bleibt. Zusätzlich schiebt das Meristem sehr zügig (innerhalb weniger Stunden/Tage) weiter nach oben, so dass es dann wieder vollständig geschützt ist. Dies reduziert die Wahrscheinlichkeit eines Oxidierens und damit eines Absterben des Meristems. Durch diese Methode ist es möglich, eine nahezu normale Blütenentwicklung zu gewährleisten und Pollen für eine Selbstung oder Bestäubung zu erhalten. Dies wiederum bietet den Vorteil, dass bereits an der Pflanze gezielt modifizierte reproduktive Zellen gewonnen werden können, was mühsame *in vitro* Kultivierungsschritte entbehrlich macht.

Die Transfektion findet dann mittels der oben angegebenen Methoden (s. Beispiel 2) statt. Die Embryonen keimen aus und Pflanzen werden kultiviert bis zur Selbstung und Ernte. Die Ergebnisse sind vergleichbar bei den Keimlingen und den adulten Pflanzen, nur ohne Keimung.

### Beispiel 4: Nachweis der erfolgten gezielten gentechnischen Veränderung

Der Nachweis ist über unterschiedliche Verfahren und zu unterschiedlichen Zeitpunkten möglich:
Die Anwesenheit der gewünschten gezielten Veränderung einer Nukleinsäure-Zielregion kann in frühen Phasen des Keimlings, der sich entwickelnden Pflanzen und der Pollen analysiert werden, so dass man bereits Hinweise auf die erfolgten Mutationen erhält. Ein eindeutiges Ergebnis erhält man aber nur, wenn man die Nachkommen der Selbstung analysiert, da dies den Nachweis für eine vererbte Mutation erbringt.
- Enrichment PCR:
   Diese Methode kommt zur Anwendung, wenn durch die gezielte Mutation eine Restiktionsenzymschnittstelle zerstört wird. Man verdaut dann isolierte genomische oder extrachromosomale DNA mit dem Enzym, welches an dieser Stelle schneidet, so dass Wildtyp-DNA geschnitten wird. Anschließend folgt eine PCR mit Primern die genomisch upstream und downstream der Restiktionsenzymschnittstelle liegen. Man bekommt im Idealfall also nur ein Produkt, wenn eine Mutation stattgefunden hat und die DNA an der Stelle nicht geschnitten wurde. Da der Verdau der genomischen oder der extrachromosomalen DNA in der Regel nicht 100% ist, wird das erhaltene PCR-Amplifikat erneut mit dem Enzym verdaut um sicherzustellen, dass eine Mutation stattgefunden hat und die Restiktionsenzymschnittstelle mutiert ist. Die unverdauten Fragmente werden anschließen kloniert und sequenziert, um eine genaue Analyse der Mutation durchzuführen. Sollte die Nukleinsäure-Zielregion eine RNA sein, kann diese zunächst durch dem Fachmann bekannte Verfahren in DNA umgeschrieben werden, bevor eine Enrichment PCR erfolgt.
- Sequenzierung:
   Sollte eine Enrichment PCR nicht möglich sein, wird über einen Next Generation Sequencing (NGS)-Ansatz die spezifische Region sequenziert und die erhaltenen Sequenzen hinsichtlich ihrer Mutationen untersucht.
- Sequenzierung ganzer Genome (WGS) zur Identifikation von Off-Target Effekten: Um auszuschließen, dass es zu unerwünschten Mutationen gekommen ist, wird von den Kandidaten mit den gewünschten Mutationen eine WGS durchgeführt. Zusätzliche Analysen sind der Abwesenheits-Nachweis von den verwendeten Konstrukten und Viren mittels spezifischer PCR- und qPCR-Systeme.

### Beispiel 5: Virale Vektoren (nicht erfindungsgemäß)

Viren bieten den Vorteil, dass sie als fertige Viruspartikel, aber auch als DNA oder RNA in eine pflanzliche Zielstruktur eingebracht werden können. Das Einbringen der Viren erfolgt über die unter Beispiel 2 angegebenen Delivery Methoden. Dadurch erzielt man ein gezieltes Einbringen in die jeweiligen meristematischen Zielregionen von Interesse.

Zusätzlich bieten Viren die Möglichkeit der Ausbreitung in den Zellen. Voraussetzung dafür ist es, diese Funktion durch die Modifikation ihrer RNA/DNA-Sequenz nicht zu zerstören. Dies hat den Vorteil, dass man zum einen nicht direkt das Meristem infizieren muss, oder es ausreichen kann, nur wenige Zellen zu infizieren und es trotzdem zur Ausbreitung in mehrere Zellen- oder Gewebetypen kommt.

Bei dieser Anwendung gibt es zusätzlich zu den unter Beispiel 2 beschriebenen Delivery Methoden noch weitere Möglichkeiten die Viren bzw. Viruspartikel einzubringen.

Viruspartikel, *in vitro* Transkripte der Viren, oder Agrobakterien, die eine für die Viren kodierende T-DNA tragen wurden über Einreiben in die Blätter, sowie über Infiltration (Vakuum und nicht-Vakuum) eingebracht um eine Primärinfektion zu erzeugen. Durch ein systemisches Ausbreiten erfolgt dann die Infektion der jeweiligen Zielzellen und Zielgewebe.

Zusätzlich wurde auch Pflanzensaft mit einem hohen Titer an Pflanzenviren für die Infektion verwendet. Dazu wurde entweder Tabak oder Spinat mit den Viren infiziert und anschließend der Pflanzensaft mit den Viren isoliert und für die Infektion der Maispflanzen verwendet.

Neben dem breiten Spektrum an Infektionsmöglichkeiten und der Fähigkeit der Ausbreitung bieten DNA-Viren den Vorteil, DNA-Templates für die HR zur Verfügung zu stellen. In diesem Fall wird durch die Replikation des Virus innerhalb einer oder mehrerer Zellen eine sehr große Menge an Template für die homologe Rekombination nach dem Einfügen des Doppelstrangbruches zur Verfügung gestellt. Dadurch kommt es mit einer erhöhten Frequenz zur homologen Rekombination und zum Einbau des Template Fragmentes.

In einer Versuchsreihe wurden daher unterschiedliche BMV (s. SEQ ID NOs: 25-31 oder DSMZ Hinterlegungsnummer: BMV Virus-Inoculum: PV-0945; Referenz für BMV-Plasmide (C13/F1+F2 & C13/F3-13m): Benavente et al., Maydica, Vol 57, No 3 (2012): "Virus-Induced Gene Silencing in Diverse Maize Lines Using the Brome Mosaic Virus-based silencing vector.") und BSMV (umfassend mindestens eine Sequenz ausgewählt aus SEQ ID NOs: 32-37 oder DSMZ Hinterlegungsnummer: BSMV Virus-Inoculum: PV-0330; Referenz für BSMV-Plasmide (pCaBS-α & pCaBS-β & pCa-ybLlC): Yuan, C., et al. (2011). PLoS One 6(10): e26468."A high throughput barley stripe mosaic virus vector for virus induced gene silencing in monocots and dicots.") Viruspartikel, -plasmide oder -plasmidmischungen in eine Pflanze oder pflanzliche Zelle von Interesse eingebracht. Es wurden u.a. *Nicotiana benthamiana,* Mais A188, Mais Va35 sowie *Spinacia oleracea* mit entsprechenden Viren, Plasmiden oder einem Plasmidmix infiziert. Es kam entweder eine Rub Inokulation, Vascular Puncture Infection/Inokulation oder *Agrobakterium* vermittelte Transformation zum Einsatz.

Für die Rub Inokulation wurde zur primären Inokulation eine DNA-Plasmidmischung, enthaltend ähnliche Konzentrationen an unterschiedlichen Plasmiden, vorbereitet. Beispielsweise wurde jedes Plasmid in einer Konzentration von 6 µg/µl verwendet. Die verschiedenen Plasmide gleicher Konzentration wurden dann im gleichen Volumenverhältnis gemischt. Pro Blatt wurden je 6µl Plasmidmix auf die Blattoberfläche getropft, auf die zuvor schon das Carborundum gestreut wurde. Der Plasmidmix wird dann mit den Fingern in die Blattoberfläche eingerieben. Alternativ kann ein Virus-infizierter Pflanzensaft als Ausgangsmaterial verwendet werden. Für die zweite Inokulation werden bei dieser Methode frische oder eingefrorene Virus-infizierte Pflanzenblätter in einem Homogenisator vermahlen und das erhaltene Pulver/Produkt wird in 3-4 ml Inokulationspuffer (0,2406 g KH₂PO₄ + 0,543 g Na₂HPO₄ in 500 ml deionisiertem Wasser) gelöst. Hierauf wird eine kleine Menge an Carborundum zu der Plasmidmischung oder dem Pflanzensaft hinzugefügt. Die Plasmidmischung oder der Pflanzensaft werden in die obere und die untere Blattoberfläche durch Reiben eingebracht, wobei dies dadurch erfolgt, dass ein oder mehrere Finger in das Inokulum getaucht werden und dann das Inokulum vorsichtig manuell auf mindestens ein Blatt aufgetragen wird, wobei das Blatt bevorzugt mit der anderen Hand gestützt wird. Die Rub Inokulation kann auch mit einer vorhergehenden Verletzung eines Pflanzenblattes (Inzision) kombiniert werden, wobei hierbei die Blätter von Interesse zunächst mit einem Skalpell eingeschnitten werden und die Rub Inokulation dann direkt in das verwundete Blatt erfolgt.

Für die *Agrobakterium* (Ab) vermittelte Transformation werden zunächst Ab Kulturen über Nacht bei 28°C in 30 ml flüssigem Luria-Broth umfassend ein geeignetes Antibiotikum, 10 mM MES und 200 µM ACE kultiviert, Am nächsten Tag werden die Übernachtkulturen bei 4.400 Upm für 15 min zentrifugiert. Der Überstand wird verworfen und das Pellet wird danach erneut bei 4.400 Upm für 2 min zentrifugiert. Der verbleibende Überstand wird verworfen und das Pellet in Resuspensionsmedium (5 ml H₂O, 10 mM MES, 10 mM MgCl₂ + 20µM ACE) resuspendiert. Die Optische Dichte OD₆₀₀ der Suspension wird unter Verwendung des Resuspensionsmedium auf 1,5 angepasst. Die verdünnte Ab Suspension wird dann für 4 h bei Raumtemperatur inkubiert. Die Infiltration der Ab Suspension erfolgt dann bevorzugt auf der Unterseite eines Blattes von Interesse, z.B. einem Blatt von *Nicotiana benthamiana,* wobei üblicherweise 2 Blätter je Pflanze inokuliert werden.

Die folgende Tabelle 2 zeigt beispielhafte Ergebnisse für ausgewählte Viren und Pflanzenspezies unter Verwendung unterschiedlicher Transformationsmethoden:

**Tabelle 2: Übersicht zu Virusinfektionsexperimenten (Wpl: weeks post infection)**

| **Virusmaterial** | **Infizierte Pflanzenspezies** | **Methode** | **Ergebnis** |
|---|---|---|---|
| BMV - Viruspartikel DSMZ | *N. benthamiana* | Rub + Carborundum | 2 Wpl: 2/2 Pflanzen mit systemischer BMV Infektion |
| BMV - Viruspartikel DSMZ | Mais A188 | Rub + Carborundum | 2 Wpl: 2/2 Pflanzen mit lokaler BMV Infektion |
| BMV - Tabaksaft infiziert mit Viruspartikeln DSMZ | *N. benthamiana* | Rub + Carborundum | 2 Wpl: 4/6 Pflanzen mit systemischer BMV Infektion |
| BMV - Tabaksaft infiziert mit Viruspartikeln DSMZ | Mais A188 | Rub + Carborundum | 2 Wpl: 3/4 Pflanzen mit lokaler BMV Infektion |
| BMV - Tabaksaft infiziert mit Viruspartikeln DSMZ | Mais Va35 | Rub + Carborundum | 2 Wpl: 1/6 Pflanzen mit lokaler BMV Infektion |
| BMV - Tabaksaft infiziert mit Viruspartikeln DSMZ | Mais Va35 | Blatt-Inzision + Rub + Carborundum | 2 Wpl: 1/2 Pflanzen mit systemischer BMV Infektion |
| BMV - Plasmide C13/F1 +F2 und C13/F3-13m | *N. benthamiana* | Ab Infiltration | 1 Wpl: 12/12 Pflanzen mit systemischer BMV Infektion |
| BMV - Plasmide C13/F1 +F2 und C13/F3-13m-GFP | *N. benthamiana* | Ab Infiltration | 5 Wpl: 12/12 Pflanzen mit systemischer BMV Infektion |
| BMV - Tabaksaft infiziert mit Plasmiden C13/F1+F2 & C13/F3-13m | Mais Va35 | Rub + Carborundum | 4 Wpl: 1/2 Pflanzen mit systemischer BMV Infektion |
| BMV - Tabaksaft infiziert mit PlasmidenC13/F1+F2 & C13/F3-13m-GFP | Mais Va35 | Rub + Carborundum | 4 Wpl: 3/4 Pflanzen mit systemischer BMV Infektion |
| BMV - Viruspartikel DSMZ | *Spinacia oleracea* | Rub + Carborundum | 2 Wpl: 5/5 Pflanzen mit lokaler BSMV Infektion; 3 davon auch systemisch |
| BMV - Viruspartikel DSMZ | Mais A188 | Rub + Carborundum | 2 Wpl: 4/6 Pflanzen mit lokaler BSMV Infektion |
| BMV - Spinatsaft infiziert mit Viruspartikeln DSMZ | *Spinacia oleracea* | Rub + Carborundum | 2 Wpl: 5/ Pflanzen mit systemischer BSMV Infektion |
| BSMV - Plasmide pCaBS-a & pCaBS-β & pCa-γLIC | *Spinacia oleracea* | Rub Plasmidmix + Carborundum | 2 Wpl: 11/11 Pflanzen mit lokaler BSMV Infektion |
| BSMV - Plasmide pCaBS-a & pCaBS-β & pCa-γLIC | *N. benthamiana* | Ab Infiltration | 2 Wpl: 14/14 Pflanzen mit systemischer BSMV Infektion |
| BSMV - Plasmid pCaBS-a & pCaBS-β & pCa-γLIC | Mais A188 | Vascular Puncture Inokulation | 2 Wpl: 1/15 Pflanzen mit systemischer BSMV Infektion |
| BMV - Viruspartikel DSMZ | Mais A188 | Vascular Puncture Inokulation | 2 Wpl: 1/12 Pflanzen mit systemischer BSMV Infektion |

Die weiße Hinterlegung in Tabelle 2 zeigt an, dass für dieses Experiment eine systemische Infektion erzielt werden konnte. Eine hellgraue Hinterlegung bezeichnet eine lokale Infektion, wohingegen eine dunkelgraue Hinterlegung eine niedrige Infektionsrate anzeigt.

Der Nachweis einer erfolgreichen Infektion erfolgte entweder durch ELISA oder mittels RT-PCR.

### Beispiel 6: 2-gRNA Strategie

Um gezielt genomische DNA anzusteuern und eine Nukleinsäure Zielregion von Interesse durch Verwendung einer CRISPR Nuklease spezifisch aus dem Genom zu exzidieren wurde eine sog. 2-gRNA Strategie etabliert (vgl. Fig. 17 A und B). Wie auch in Fig. 17 A verdeutlicht, wurde hierzu zunächst genomische DNA isoliert und mit einem Restriktionsenzym (RE) von Interesse, dessen Schnittstelle innerhalb des PCR Produkts von Interesse liegt, verdaut. Es kann jedes beliebige RE verwendet werden, das zwischen den beiden gRNA Ziel-Regionen schneiden kann. So erfolgt eine Anreicherung von potentiell editierter DNA, da dort der Bereich zwischen den gRNA Ziel-Regionen nicht mehr vorhanden ist und das gewählte Restriktionsenzym diese DNA nicht schneiden kann. Anschließend erfolgt eine PCR-Amplifikation mit Primern, die upstream und downstream der beiden gRNA Ziel-Regionen binden, d.h. sich unter geeigneten Reaktionsbedingungen unter Hybridisierung anlagern können. Gegebenenfalls kann einer erneute Re-PCR mit einem verschachtelten ("nested") Primerset durchgeführt werden. Nach erfolgreichem Editing entsteht ein kleineres PCR-Produkt verglichen mit dem Produkt aus nicht-editierter DNA (s. Fig. 17 B). Die Abbildung Fig. 17 B zeigt das Ergebnis der Analyse auf ein Editing hin nach Anwendung der 2-gRNA Strategie mit genomischer DNA einer Maispflanze. Aus Maispflanzen wurde die genomische DNA isoliert und das Zielgen hmg13-Gen (HMG-transcription factor 13; GRMZM2G066528) wurde mit PCR amplifiziert. Die Sequenz des HMG-transcription factor 13 Gens ohne ein Editing ist in SEQ ID NO: 60 gezeigt.

Die Nukleotidpositionen 1-98 der SEQ ID NO: 60 sowie die Nukleotidpositionen 912-1023 der SEQ ID NO: 60 entsprechen der Region des *hmg* Gens, die nach einem erfolgreichen Editing vorhanden bleibt. Nukleotidpositionen 82-101 der SEQ ID NO: 60 sowie Nukleotidpositionen 909-928 der SEQ ID NO: 60 sind jeweils die gRNA Ziel-Regionen.

Fig. 17 B stellt das Ergebnis einer Auftrennung in einem 1%igen Gel mit dem Standard-Laufparameter 100 V und Visualisierung über Ethidiumbromid-vermittelte Fluoreszenz in unterschiedlicher Kontrastwiedergabe dar. In den Spuren 1 und 2 ist das Ergebnis für nichteditierte Maispflanzen zu sehen, in der Spur 4 das Ergebnis nach erfolgreichem Editing. Das PCR-Produkt ist kleiner, da der Bereich zwischen den beiden gRNA Ziel-Regionen herausgeschnitten wurde. Somit stellt dieser Ansatz eine schnelle und effiziente Strategie dar, um eine erfolgreiche Genom Editierung experimentell bestätigen zu können.

SEQ ID NO: 61 zeigt das Ergebnis der Sequenzierung des kleinen PCR-Produkts nach *hmg*13 Editing mit der 2-gRNA Strategie. Zwischen den zwei Basen C und T an Positionen 98 und 99 der SEQ ID NO: 61hat die Deletion durch eine gezielte Editierung stattgefunden.

### Beispiel 7: Genom Editierung in Tabak (nicht erfindungsgemäß)

Als Ziel-Gen in *Nicotiana benthamiana* wurde NbTTG1 für die Genom-Editing Arbeiten ausgewählt, dessen Ortholog in *Arabidopsis thaliana* bei Funktionsunfähigkeit zu einem Trichome-Phänotyp führt. Für das entsprechende Arabidopsis Gen AtTTG1 (AT5G24520) sind Mutanten beschrieben:

| | |
|---|---|
| - ttg1(EMS-Mutante): | Keine Trichome auf der Blattoberfläche und am Stil. Gelbe Samen durch Fehlen von braunen Pigmenten. |
| - ttg1-13 (fast neutrons Mutante): | Keine Trichome, transparente Samenhülle, erhöhte Anzahl an Wurzelhaaren. |

Das Ortholog in *Nicotiana benthamiana* wurde über Sequenzvergleiche identifiziert und der genomische Lokus über PCR amplifiziert. Der entsprechende Ausschnitt ist in Figur 18 gezeigt. Anhand dieser Sequenz wurden passende gRNAs wie oben beschrieben ausgewählt. Die Komponenten für das Genome-Editing wurden über TRV (tobacco rattle virus) in die Pflanze eingebracht (s. Beispiel 8 unten). Auch hier kam die oben in Beispiel 6 skizzierte 2-gRNA Strategie zur Analyse eines erfolgreichen Editings zum Einsatz.

Wie in Tabelle 3 unten ersichtlich konnten durch verschiedene Kombination von je zwei gRNAs unterschiedlich große Deletionen im NbTTG1 Gen erzeugt werden. Für diesen Versuch kam eine Cas9 Nuklease zum Einsatz, der Ansatz kann jedoch für beliebige CRISPR Nukleasen eingesetzt werden.

**Tabelle 3:**

| gRNAs | Deletion |
|---|---|
| gRNA1 + gRNA4 | 232 bp |
| gRNA2 + gRNA4 | 216 bp |
| gRNA3 + gRNA4 | 206 bp |
| gRNA4 + gRNA5 | 446 bp |
| gRNA1 + gRNA3 | 25 bp |

### Beispiel 8: Tobacco rattle virus (TRV)-vermittelte Expression von CRISPR-Cas in Nicotiana benthamiana (nicht erfindungsgemäß)

Für die Blattinokulation von Tabak wurden zunächst *Agrobacterium* (Ab)-Kulturen über Nacht bei 28°C in 30 ml flüssigem Luria-Broth (LB)-Medium, welches ein selektives Antibiotikum enthielt, kultiviert. Am nächsten Tag wurden die Übernachtkulturen bei 4.400 Upm für 15 min zentrifugiert. Der Überstand wurde verworfen und das Pellet wurde danach erneut bei 4.400 Upm für 2 min zentrifugiert. Der verbleibende Überstand wurde verworfen und das Pellet in 5 ml Resuspensionsmedium (10 mM MES, 10 mM MgCl₂, 20 µM ACE) resuspendiert. Die optische Dichte bei 600 nm (OD₆₀₀) der Suspension wurde unter Verwendung des Resuspensionsmediums auf 0,8 angepasst. Die verdünnte Ab Suspension wurde dann für 4 h bei Raumtemperatur inkubiert. Die Infiltration der Ab Suspension erfolgte im Anschluss unter Zuhilfenahme einer Spritze ohne Kanüle auf der Unterseite eines Blattes von Interesse, z.B. einem Blatt von *Nicotiana benthamiana,* wobei üblicherweise 3 Blätter je Pflanze inokuliert wurden. Um die systemische Ausbreitungseffizienz von TRV zu visualsieren, wurden Blätter mit einem RFP/pTRV2 (rot fluoreszierender Marker + TRV als viraler Vektor) sowie als Kontrolle mit pZFN-tDT-nptll inokuliert. Wie aus Figur 19 hervorgeht, ist eine deutliche RFP-Fluoreszenz in den direkt inokulierten sowie in den nicht-inokulierten distalen Blätter detektierbar (ursprüngliche rote Fluoreszenz entspricht den hellen und/oder weißen Bereichen in Fig. 19). Das Konstrukt pZFN-tDT-nptll fungiert als Kontrolle, die ausschließlich die Expression des RFP in den inokulierten, jedoch nicht in den distalen Blättern erlaubt.

Darüber hinaus wurde bestätigt, dass durch dieses TRV-vermittelte Verfahren sogar meristematische Gewebe angesteuert werden können, was eine gezielte Veränderung dieses Gewebetyps durch die spezifischen CRISPR Verfahren erlaubt. Hierzu wurden *Nicotiana benthamiana* Pflanzen mit TRV infiziert, wobei das Konstrukt ein Gen, das ein rot fluoreszierendes Protein kodiert, z.B. tdT oder dergleichen, umfasst. Durch Detektion der roten Fluoreszenz durch geeignete Mittel (Fluoreszenzmikroskop, -binokular) kann verfolgt werden, wo in der Pflanze sich der TRV befindet. Hierzu kann es vorteilhaft sein, Fluoreszenzmarker mit einer hohen Intensität zu verwenden, da diese auch in tieferen Gewebeschichten gut detektiert werden können. In Fig. 20 A bis H sind Abbildungen zu sehen, die die Aufnahme eines Blütenmeristems, einer Blütenknospe, eines Stempels bzw. eines präparierten Stempels mit offen liegenden Ovarien zeigen. Alle Abbildungen demonstrieren die erfolgreiche Expression des Fluoreszenzmarkers in den jeweiligen pflanzlichen meristematischen Zellen bzw. Geweben als Zielstruktur und damit die Effizienz des gewählten Einbringungsverfahrens.

Schließlich wurden noch TRV-Titer in inokulierten und nicht-inokulierten Tabakblättern mittels Standard Doppel-Antikörper-Sandwich (DAS) ELISA quantifiziert._ _ _Als Ausgangsmaterial für den ELISA wurden 10-12 dpl Blattmaterial von jeder zu analysierenden Pflanze geerntet, wobei für jede Pflanze folgende Mischproben erstellt wurden: (i) Mischprobe aus je zwei TRV-inokulierten Blättern; (ii) Mischprobe aus je zwei nicht-inokulierten Blättern. Das geerntete Blattmaterial wurde gepresst und der aufgefangene Pflanzensaft in einer Verdünnung von 1:50 in dem DAS-ELISA eingesetzt. Der DAS-ELISA wurde unter Verwendung eines polyklonalen Antiserums aus Kaninchen durchgeführt. Das Antiserum wird von der Firma Loewe^{®} erworben und trägt die Bezeichnung "Tobacco Rattle Tobravirus BroadRange.TRV" (Cat. No. 07152S). Die Auswertung des ELISAs erfolgte 60 min nach Applikation des Substrates 4-Nitrophenylphospaht durch photometrische Messung der OD₄₀₅. So wurde etwa der TRV-Titer in *N*. *bethamiana* inokuliert mit (i) pTRV1 (= Negativkontrolle); (ii) pTRV1 + pTRV2-tDTco (= Positivkontrolle) und (iii) pTRV1 + pTRV2-Cas9 quantifiziert. Die Ergebnisse sind in Figur 21 dargestellt.

### Beispiel 9: Quantifizierung von CRISPR Werkzeugen

Beispielhaft wurden Cas9-Transkripte mittels RT-PCR nachgewiesen. Hierzu wurden als Ausgangsmaterial für den ELISA 10-12 dpl Blattmaterial von jeder zu analysierenden Pflanzen geerntet, wobei für jede Pflanze folgende Mischproben erstellt wurden: (i) Mischprobe aus je zwei TRV-inokulierten Blättern und (ii) Mischprobe aus je zwei nicht-inokulierten Blättern (s. Beispiel 8). Aus dem geernteten Blattmaterial wurde zunächst unter Verwendung eines RNeasy Mini Kit (Qiagen) RNA extrahiert. Jeweils 500 ng RNA wurden anschließend unter Verwendung des RevertAid H Minus First Strand cDNA Synthesis Kit (Thermo Fisher Scientific) in cDNA umgeschrieben. Die cDNA diente als Template in einer sich anschließenden PCR zum Nachweis von Cas9. Es wurden Cas9 spezifische Primer verwendet.

Aus Blattmaterial transgener Maispflanzen wurden zudem Proteinextrakte hergestellt und auf einem 4-20% SDS-PAGE Gradientengel aufgetrennt. Die Detektion der 160 kDa großen Cas9 erfolgt mit einem monoklonalen Antikörper von ActiveMotif (Catalog Nr. 61577). Die Dokumentation dieses Nachweissystems ist in Fig. 22 dargestellt.

Zur Quantifizierung von gRNAs und zur Analyse, ob ausgehend von einem Virus eine durch einen subgenomischen Promotor vermittelte Expression von gRNAs stattfindet, wurden SYBR-Green basierte quantitative RT-PCR Systeme etabliert. Bei Amplifikation mit gleicher PCR-Effizienz kann durch Vergleich der gRNA Menge mit dem Transkriptlevel viraler Proteine eine Quantifizierung vorgenommen werden. Dieses System ist in Figur 23 beispielhaft für Brome Mosic Virus (BMV) dargestellt.

### Beispiel 10: Virale Expressionssysteme (nicht erfindungsgemäß)

Neben den oben beschriebenen viralen Vektoren können die CRISPR Werkzeuge und Verfahren dieser Offenbarung ebenso in andere Pflanzensysteme viral eingebracht werden. Abhängig von der Zielpflanze von Interesse sowie der Art der Transformation und das zu infizierende Zielgewebe können unterschiedliche Methoden zum Einsatz kommen. Für Mais Endosperm Zellen als primärer Zielstruktur eignet sich etwa das System von Ugaki et al. (1991, Nucleic Acids Res., Replication of a geminivirus derived shuttle vector in maize endosperm cells). Basierend auf Wheat Dwarf Virus (WDV) als Vektor kann somit durch Protoplastentransformation von Mais Endospermkulturen eine infizierte Kultur erhalten werden. Zu diesem Zweck wird ein modifizierter Virus eingesetzt, der an Stelle des Coat Proteins (CP) eine Neomycin Phosphotransferase Gen II (*npt*ll) trägt. Für *Triticum* Zielpflanze kann ein transientes Replikationssystem mit dem Wheat Dwarf Virus als Cargo gemäß Matzeit et al. (1991, Nucleic Acids Res., 19(2), 371-377) verwendet werden. Hierbei werden Protoplasten abgleitet von *Triticum* Suspensionskulturen transfiziert. Erneut wird das CP Gen des Virus durch ein Markergen von Interesse ersetzt.

Darüber hinaus können *Maize Streak Virus* basierte Systeme zum Einsatz kommen, die dem Fachmann auf dem Gebiet bekannt und etwa in Palmer & Rybicki (2000, Archives of Virology, 146 (6), 1089-1104) beschrieben sind. Hierbei werden 3-Tage alte Setzlinge am coleoptilaren Knoten infiziert und es kann eine transiente Expression eines rekombinanten Konstrukts von Interesse erzielt werden. Durch den Austausch der viralen CP und MP Gene kann eine systemische Ausbreitung des Virus verhindert werden, sodass nur die ersten 2 oder 3 Blätter infiziert werden.

Wie oben ausgeführt eignet sich auch *Barley Stripe Mosaic Virus* (BSMV) als viraler Vektor. Das BSMV Genom wurde intensiv umgestaltet, um dadurch einen bekannten Pflanzenprotoplasten Vektor zu etablieren (vgl. Joshi et al., 1991, EMBO J., 9(9), 2663-2669.). Dieser Vektor, der gemäß Joshi et al. 1990 ein Luziferase (luc) Reportergen trägt, eignet sich zur Protoplastentransfektion von Mais- sowie Tabakprotoplasten.

BSMV (s. auch Manning et al., 2010, New Phytologist, 187 (4), 1034-1047), WDV, Wheat Strike Mosaic Virus (WSMV) (Choi et al., 2000, Plant J., 23(4), 547-55), Tomato Yellow Leaf Curl Virus (TYLCV) (Peretz et al., 2007, Plant Physiol., 145 (4), 12514-1263) und Brome Mosaic Virus (BMV) (French et al., 1986, Science 231(4743), 1294-7) eignen sich zudem als Systeme zur Transfektion von Protoplasten, Setzlingen, Petiolen und anderen Pflanzenzellen oder -geweben in Weizen, aber auch Gerste und Tabak, wie in der Literatur ausführlich beschrieben. Speziell BSMV Vektoren (s. Manning et al., 2000 supra) eignen sich in modifizierter Form als Vektoren zum Virus-induzierten Gen Silencing. Hierzu wurde das BSMV Genom durch ortsgerichtete Mutagenese durch Unterbindung der Expression des viralen Coat Proteins modifiziert. TYLCV (s. Peretz et al., supra oder EP 2 436 769 A1)) wurde dahingehend attenuiert und für die Verwendung als viraler Shuttle Vektor für Pflanzen sowie *E. coli* zugänglich gemacht, dass im viralen Coat Protein eine 60 Basenpaare umfassende Sequenz nahe dem N-Terminus des Gens deletiert wurde.

Spezifische Ansätze zur Zuckerrübentransformation basierend auf viralen Vektoren sind ebenfalls bekannt. Hierzu eignen sich insbesondere der Beat Curly Top Virus (BCTV) (Kim et al., Plant Mol. Biol., 2007, 64(1-2):103-12), der Beet Yellows Virus (BYV) (Prokhnevsky et al., Molecular Biotechnology, 57 (2), 101-110, 2015), der Beet Soil Borne Mosaic Virus (BSBMV) (Dach et al., 2015 ASSBT Proceedings Tagungsband 47. Jahrestreffen Arbeitskreis "Viruskrankheiten der Pflanzen", Section C), oder der Beet Necrotic Yellow Vein Virus (BNYVV) (Hamza et al., 2015, ASSBT Proceedings). Diese Vektoren eignen sich nicht nur als Vektoren für Zuckerrübe, sondern darüber hinaus für weitere Dicotyledone, z.B. Spinat.

Für Tabak als Modellpflanze sind ebenfalls viele Methoden zur Transformation mittels viraler Vektoren verfügbar. Viele dieser Verfahren basieren auf *Agrobacterium* vermittelter Inflitration. Geeignete Viren umfassen Tobacco Mosaic Virus (TMV), Potato Virus X (PVX), Cowpea Mosaic Virus (CPMV), Bean Yellow Dwarf Virus (BeYDV), Plum Pox Virus (PPV) (s. hierzu etwa Gleba et al., 2014 oder Salzar-Gonzalez et al., 2015, Plant Mol. Biol., 87:203-217). Darüber hinaus sind diverse weitere Systeme beschrieben, die als Virus etwa Cabbage Leaf Curl Virus (CaLCuV) (Yin et al., 2015, Nature Scientific Reports, 5:14926, 2015), Tobacco Rattle Virus (TRV) (Ali et al., 2015, Genome Biology, 16:238) oder Tobacco Yellow Dwarf Geminivirus (TYDV) (Dugdale et al., 2014, Nat. Protoc., 9(5), 1010-27) nutzen.

Alle o.g. Vektoren enthalten Klonierungsstellen zur Einführung von Zielgenen von Interesse. Spezifische Schnittstellen können jedoch auch einfach durch verfügbare Mutageneseverfahren in ein virales Genom von Interesse eingebracht werden.

### Beispiel 11: Optimierte Verfahren zum Fenstern von Pflanzen (nicht erfindungsgemäß)

Um die gezielte Einbringung der CRISPR Konstrukte und damit den Effekt des Genom Editings noch zu optimieren, wurden die in Beispiel 3 oben geschilderten Verfahren weiter verbessert. Das ursprüngliche Verfahren umfasste das Verschließen des künstlich eingeführten Fensters mit einem Verschluss, wie etwa einem speziellen Gewebepapier. Dies kann je nach Exposition jedoch mit dem Nachteil verbunden sein, dass das verletzte und offenliegende Pflanzengewebe leichter für Pilzinfektionen zugänglich ist, oder, dass ein Teil der exponierten Tassel, der beschossene Teil, zu stark mit Luft in Kontakt kommt, wodurch es zu einem Austrocknen der freigelegten Tassel-Strukturen oder der unreifen Blütenstände und daher der einzelnen Tassel-Verzweigungen kommen kann. Daher wurde in einem ersten Schritt das freigelegte und wie oben beschrieben transformierte Tassel Gewebe mit einem angefeuchteten Baumwoll-Pad oder -Gewebe abgedeckt. Hierdurch konnte die Austrocknung deutlich vermindert werden, jedoch ist auch dieses Verfahren anfällig für mögliche Pilzinfektionen. Um dieses Problem zu addressieren wurden Wachse oder Vaseline-ähnliche Substanzen auf die verwundete Stelle (nach Transformation) aufgetragen. Diverse Substanzen wurden getestet, umfassend Vaseline, Mischungen von natürlichen Wachsen mit Vaseline und weitere kommerziell erhältliche Produkte, die zur Wundheilung speziell bei Bäumen angeboten werden. Diese Vorgehensweise ist dem Fachmann, gerade auf dem Gebiet der Pfropfung, gut bekannt. Zusätzlich wurde die verwundete Stelle mit speziellem Grafting/Pfropfungsband oder Parafilm umwickelt, was das Abschließen der Wunde und damit den Schutz vor Pilzinfektionen signifikant verbesserte und sich die transformierten meristematischen Gewebe voll zu ihrer Reife entwickeln konnten. Durch diese optimierte Strategie konnte sich ein Großteil der Tassel in transformierter Form zu ihrer Reife hin entwickeln. Es wurden hiermit Erfolgsquoten von 75% und mehr erreicht, d.h. Events, bei denen sich das freigelegte und transformierte Tassel-Gewebe bis zur vollen Reife *in planta* entwickeln konnte.

### Beispiel 12: Agrobacterium Injektion (nicht erfindungsgemäß)

Um die Breite des möglichen Einsatzbereiches noch zu erweiteren, wurden die in Beispiel 3 skizzierten Verfahren derart abgeändert durchgeführt, dass anstelle des Partikelbeschusses *Agrobacterium* (Ab) vermittelte Transformation zu Einsatz kam. In einem Vorabversuch wurde hierzu zunächst die Suszeptibilität von unreifem Tassel Gewebe für Ab getestet. Hierzu wurde ein rot fluoreszierendes Protein *in vitro* in unreifes Tassel-Gewebe transformiert, das zuvor von der Pflanze isoliert worden war. Zum Zeitpunkt der Isolierung waren die entsprechende Pflanzen im V6-V7 Stadium und die Tassel hatten eine Länge von ca. 2-3 cm. Ab wurde auf eine OD600 von 1,0 eingestellt und die Tassel wurden für 10 min mit der Ab-Suspension inkubiert. 2 Tage nach der Infektion wurde die rote Fluoresezenz beobachtet. Mehrere rot fluoreszierende Punkte wurden in den Tasseln beobachtet, was die Eignung von Ab-Inflitration zur Transformation von Tassel-Gewebe belegte. In einem nächsten Schritt wurden dann Pflanzen im V6-V7 Stadium verwendet und die Pflanzen wurden wir oben beschrieben im Bereich des unreifen Tassel Gewebes gefenstert. Ab, welche ein rot fluoreszierendes Expressionskonstrukt enthielten, wurden in einer OD von 0,7 direkt in das Tassel-Gewebe injiziert. Es wurde ungefähr 100-200 µl der Ab-Suspension je Tassel injiziert. Der Ablauf des Fensterns sowie der Ab-Injektion ist in Figur 24 gezeigt. Hierauf wurden die Tassel mit Vaseline/Parafilm bedeckt, wie oben beschrieben, und die Entwicklung roter Fluoreszenz wurde 2 Tage nach der Injektion überwacht. Um eine zu intensives Wachstum von Ab zu unterbinden, wurde nach 2 bis 7 Tagen nach der initialen Injektion eine Antibiotikalösung (z.B. Timentin, Carbenicillin, Cefotaxim etc.) auf das infizierte Gewebe appliziert. Die behandelten Tassel konnten sich bis zur Reife weiterentwickeln und es wurde eine Selbstbestäubung durchgeführt. Molekulare Analysen in der T1 Generation bestätigten die erfolgreiche Transformation. Hiermit wurde bestätigt, dass ein *in planta* Verfahren dazu geeignet ist, meristematische Zellen *in planta* zu transformieren, ohne die weitere Entwicklung der Tassel zu beeinträchtigen, sodass die resultierenden Pollen direkt ohne langwierige (*in vitro*) Kultivierungsverfahren von der transformierten Pflanze gewonnen und unmittelbar zur Bestäubung benutzt werden konnten.

### Beispiel 13: Anwendbarkeit auf unterschiedliche Mais Genotypen

Die oben geschilderten Tassel Transformationsexperimente wurden für unterschiedliche Mais Genotypen getestet, nämlich A188, Va35 und A632. Für jeden Genotyp ist das Vegetationsstadium, in dem das Tasselgewebe transformiert werden kann, natürlicherweise unterschiedlich. Dies kann jedoch leicht bestimmt werden. In A188 ist das Stadium beispielsweise V6-V7, wohingegen A632 etwa im Stadium V7 bis V9 targetiert wurde. In allen diesen Genotypen war es möglich, die Tassel in geeigneter Weise freizulegen, d.h. die Pflanzen ohne diese oder das Tasselgewebe zu schädigen zu fenstern, und reife Pollenproduzierende Antheren zu erhalten.

### Beispiel 14: Embryo-Meristem Bombardierung

Um die hierein beschriebenen Verfahren noch weiter zu optimieren, wurde eine sog. Embryo-Meristem Bombardierung etabliert, die es erlaubt, dass Pflanzen effizient direkt aus unreifen Embryonen ohne zeitraubende und kontaminationsanfällige Zellkultur als Zwischenschritt gewonnen werden können. Hierzu wurde im Pipeline-Modus der Partikelbeschuss von Meristemregionen unreifer Embryonen für die Genotpen A188 sowie A632 durchgeführt. Etwa 100 Embryonen (Fig. 25 A) wurden mit CRISPR/Cas9 Konstrukten zusammen mit einem ein rot fluoreszierendes Protein exprimierenden Plasmid bombardiert. Einen Tag nach dem Beschuss wurde die Fluoreszenzentwicklung beobachtet (Fig. 25 B). Zahlreiche Embryonen zeigten Fluoreszenz und damit die erfolgreiche und funktionale Einbringung der CRISPR Konstrukte. Mit den erfolgreich transformierten Embryos wurde weiter gearbeitet. Nach Keimung wurden 25% der Pflänzchen auf molekularer Ebene analysiert. Alle übrigen Fluoreszenz-positiven Pflanzen wurden im Gewächshaus bis zur Reife wachsen gelassen. Sobald die Pflanzen das reproduktive Stadium erreichten, wurde eine Probe des Tassels sowie der Ähre entnommen und auf CRISPR/Cas9 Aktivität hin untersucht. Sobald, etwa durch PCR, ein erfolgreiches Ergebnis identifiziert wurde, wurden die Pflanzen zur Selbstbestäubung verwendet und die resultierenden Nachkommen wurden ebenfalls analysiert.

Die derart produzierten Pflanzen brachten für beide Genotypen Samen hervor und waren fertil. Die Pflanzen zeigten ein langsameres Wachstum sowie einen leicht verkümmerten Wuchs (Fig. 25 C), dennoch konnte durch dieses Verfahren ohne Weiteres eine fertile Pflanze hervorgebracht werden, deren Pollen unmittelbar in weitere Experimente einfließen konnten. Diese Art der Transformation stellt somit ebenfalls ein hocheffizientes Verfahren dar, um schnell und wirksam CRISPR Konstrukte oder die dadurch vermittelten Genom Editierungen in ein meristematisches Gewebe bzw. eine Zelle von Interesse einzubringen, um dann unmittelbar aus diesem Gewebe reproduktives Gewebe erhalten und weiterverwenden zu können.

### Beispiel 15: Meristemzugang in unterschiedlichen Pflanzenarten

Wie oben erwähnt ist wünschenswert, eine *in planta* Transformation für eine Vielzahl von unterschiedlichen Pflanzen bereitzustellen und dies mit den hierein offenbarten Verfahren zu kombinieren, sodass eine gezielte Veränderung einer Vielzahl von meristematischen Zielstrukturen durch die CRISPR Systeme erreicht werden kann. Gerade die transiente Einbringung von CRISPR Konstrukten von Interesse in eine pflanzliche meristematische Zielstruktur ist von großem Interesse, da hierdurch gezielt eine Nukleinsäure-Zielstruktur von Interesse verändert werden kann und sodann diese Veränderung, nicht jedoch die CRISPR Konstrukte selbst weitervererbt werden.

Die Gewebe, die sich *in planta* zu Fortpflanzungsorganen entwickeln können, sind begrenzt. Das wichtigste davon ist das Sprossmeristem. Dieses Meristem ist definiert durch die Gruppe von Zellen, die sich in alle vegetativen Organe und Zellen sowie Fortpflanzungsorgane und Zellen oberhalb des Bodens differenzieren können. Es besteht aus einer begrenzten Anzahl von Zellen, die (um)programmiert werden können, um sich in alle Organe einer Pflanze zu differenzieren. Dieses Meristem hat in der Regel eine Kuppelform. Die äußere Linie der Zellen, L1-Schicht genannt, bildet die Grundlage für alle epidermalen Gewebe. Die inneren Schichten (L2 und L3) des Meristems bilden den Rest der Organe aus und sind somit interessante Ziele zum Zwecke der vorliegenden Erfindung. Das Meristem bildet sich sehr früh in der Entwicklung des Embryos. Nach dem vegetativen Wachstum entwickelt sich das Meristem in das Meristem der Blüten, um die Fortpflanzungsorgane der Pflanzen zu generieren. Die Gewebe, die modifizierte Fortpflanzungsorgane hervorbringen können sind: (1) das Sprossmeristem des Embryos, (2) das Sprossmeristem von Pflänzchen oder von Pflanzen im vegetativen Stadium und (3) das Blütenmeristem oder die Blütenanlagen.

Wenn die genetische Information dieser Gewebe durch nicht-virale Ansätze (gene-gun, Mikroinjektion, Agrobacterium, etc.) modifiziert wird, kann es sein, dass nicht alle Zellen dieser Meristeme gezielt verändert werden. Als Folge sind einige der sich differenzierenden Pflanzenorgane modifiziert und einige behalten den Wildtyp-Genotyp bei. Somit enstehen Chimäre.

Eine Alternative, um eine Vielzahl unterschiedlicher Getreidepflanzen gezielt zu manipulieren ist es, Mikrosporen (unreife Pollen) oder Pollenkörner zu transformieren. Diese Gewebe können dann verwendet werden, um weitere Pflanzen zu bestäuben und modifizierte Nachkommen zu erhalten. Es gibt nur wenige Beispiele in der Literatur, die meisten von ihnen im Zusammenhang mit Bombardierung und transienter Expressionsanalyse der eingeführten Gene (Twell et al., 1989, Obert et al. 2008). Allerdings wird die Technologie auch weiterentwickelt, um dadurch gezielt Mikrosporen oder Pollen reifen zu lassen und durch anschließende Bestäubung modifizierte Nachkommen zu erhalten. Das Verfahren für diese Technologie ist sehr ähnlich für verschiedene Kulturpflanzen. Targeting von Mikrosporen kann direkt in unreifen Antheren erfolgen, oder durch Freisetzung der Mikrosporen in ein Kulturmedium. Diese Targetierung kann beispielsweise durch Beschuß oder Mikroinjektion erfolgen. Diese Technik wurde erfolgreich zur Herstellung von transgenen Tabakpflanzen (Touraev et al., 1997) und Baumwolle (Gounaris et al., 2005) verwendet. Wie für die Targetierung reifer Pollen kann frisch erhaltener Pollen durch Beschuss oder Schall (Eapen (2011)) behandelt werden und sofort für die Bestäubung von, z.B. Mais-Ähren (Horikawa et al., 1997) verwendet werden. Die Nachkommen können dann auf die Gegenwart von Transgenen oder gezielt eingeführte genomische Ereignisse hin analysiert werden.

### Beta vulgaris:

Für die Transformation meristematischer Gewebe in Zuckerrüben können unreife Embryonen, wie in Zhang et al (2008) beschrieben, erhalten werden. Blüten-Spikes wurden von im Gewächshaus herangezogenen Pflanzen 14 Tage nach der Anthese erhalten. Sie wurden in 30%igem Bleichmittel für 30 min sterilisiert. Unreife Embryonen (lEs) wurden isoliert und anschließend 4 Wochen lang auf festem MS-Medium mit verschiedenen Pflanzenwachstumsregulatoren kultiviert. Eine Abbildung eines derartigen unreifen Embryos findet sich in Figur 26. In diesen unreifen Embryonen können die apikalen Sprossmeristeme (wie bei Mikroinjektion) direkt gezielt behandelt werden, wobei mit Hilfe eines Mikroskops die Meristembereiche gezielt angesteuert werden. Alternativ können zufällige Targeting-Technologien, wie Bombardment, eingesetzt werden. Nach Targetierung erfolgt die weitere Reifung. Diese Embryoreifung erfolgt in einem Inkubator im Dunkeln in einem Temperaturbereich von ca. 20-30°C. Die Reifedauer beträgt etwa 1 bis 4 Wochen. Sobald der Embryo herangereift ist und beginnt zu keimen, wird er auf festes MS Medium im Licht transferiert, damit sich die Pflänzchen entwickeln können. Wenn diese Pflänzchen robust genug sind, werden sie nach einer Akklimatisierungsphase von ca. 1 bis 4 Wochen in Erde transferiert. Diese Pflanzen werden dann herangezogen und die Nachkommen werden analysiert.

Targetierung reifer Embryonen aus Zuckerrüben erfordert es, die harte Fruchthülle der Samen (Hermann et al. 2007) zu entfernen. Der Embryo befindet sich in der Mitte und das Sprossapikalmeristem ist erreichbar. Vor Entfernung des Perikarp muss eine Sterilisation des Samens durch Bleichen und unter Verwendung von Ethanol durchgeführt werden. Dann kann, mit Hilfe von Skalpellen oder anderen scharfen Werkzeugen, das Perikarp entfernt weden und der Embryo wird freigelegt. Dieser Embryo wird dann in einem geeigneten Medium für die spezifische Methode der Transformation von Interesse überführt. Das Meristem des reifen Embryos oder der ganze Embryo kann dann direkt unter Verwendung eines Mikroskops einer Transformation unterzogen werden, die in zufälliger Weise MeristemBereiche ansteuert. Nach einer Ruhephase von ca. 1 bis 10 Tagen in einem Inkubator in Dunkelheit (20-30°C) keimt der Embryo und die Pflänzchen können eingepflanzt werden. Die Zuckerrübenpflanze wird dann bis zur Reife herangezogen und die Nachkommen werden analysiert.

Das Sprossmeristem in Zuckerrübensprösslingen kann durch gezielte Schnitte in Meristembereiche targetiert weden (Artschwager 1926). Diese Arten von Sprossmeristemen wurden bereits gezielt, etwa durch Partikelbeschuss, targetiert. Vor der Prüfung der Genexpression wurden Partikelpenetrationsversuche durchgeführt. Transiente GUS-Expression wurde in den ersten und zweiten Zellschichten des Meristems detektiert. Sich teilende Zellen mit GUS-Aktivität zeigten, dass Zellen den Beschuß vital überstehen (Mahn et al. 1995). Zusätzlich wurde vorgeschlagen, dass Meristeme mit attenuierten Agrobacterium-Stämmen zur *Beta vulgaris* Transformation verwendet werden können (Kerns et al 1988). Hierbei können unterschiedliche Methoden (Mikroinjektion, Agrobacterium) und unterschiedliche pflanzliche Gewebe in unterschiedlichen Entwicklungsstadien verwendet werden. Zum Zwecke der vorliegenden Offenbarung wurde ein Bombardment von meristematischen Geweben aus Setzlingen, die *in vitro* vermehrt wurden, durchgeführt. Das Blattmaterial wurde entfernt, bis die meristematischen Gewebe exponiert waren. Die meristematischen Bereiche wurden dann vertikal eingeschnitten, oder die Regionen wurden vertikal ohne Schnitte bereitgestellt. Nach dem Beschuss mit einer Gen-Kanone wurden die Explantate *in vitro* belassen. Einen Tag nach der Bombardierung der Zellen wurde nachgewiesen, dass die Zellen beta-Glucuronidase-Aktivität zeigten, die als Marker in die Zellen eingeführt wurde, was belegt, dass meristematische Regionen von Zuckerrübe geeignet für Partikelbeschuss und damit für die Einbringung von Genome Editierungswerkzeugen, z.B. CRISPR Werkzeugen, sind.

Zudem kann der Blütenstand von Zuckerrüben gezielt verändert werden, während er sich entwickelt, entweder vor der Blütenbildung, oder direkt in den unreifen Blüten. Die Blüten können dann weiter reifen und nach der Bestäubung werden die Samen geerntet, und die Nachkommen werden analysiert. In *Beta vulgaris* besteht der Blütenstand aus einer offenen Hauptachse mit vielen geschlossen, dichasial und sympodial verzweigten Blütenständen. Die Endblüte jeder Blütenstand-Einheit und laterale Blüten verschmelzen zu einem späteren Entwicklungsstadium. Die fünf Staubblatt Primordien stammen voneinander ab, und sie entstehen im Laufe der Blütenentwicklung durch die Bildung eines (intra-) staminalen Rings aus einem ringförmigen interkalierenden Meristem (Olvera et al. 2008).

### Triticum aestivum:

Für Weizen als Zielpflanze wurde ein weiterer Ansatz entwickelt. Hierzu wurden unreife Körner aus unreifen Ähren 5 bis 20 Tage nach der Blüte gesammelt. Diese Kerne wurden durch Oberflächenbehandlung mit Bleichmittel und Ethanol sterilisiert. Die unreifen Embryonen wurden dann mit einem Skalpell unter einem Mikroskop extrahiert. Diese Embryonen zeigten ein in unterschiedlicher Ausprägung exponiertes Meristem. Diese Meristeme wurden dann verschiedenen Transformationsmethoden unterzogen, wie in Sautter et al. (1995) beschrieben. Abbildungen hierzu finden sich in Fig. 27 A und B. Nachdem die Embryonen derart behandelt worden waren, wurden sie weiter kultiviert, nämlich in einem Embryonenreifungsmedium, wie beschrieben in Matthys-Rochon et al. (1998). Die keimenden Pflänzchen (Fig. 27 C) wurden dann nach Akklimatisierung in das Gewächshaus überführt. Die Pflanzen wurden regulär herangezogen und die Nachkommen wurden anschließend analysiert.

Ebenso kann in Weizen das Sprossmeristem für Transformationen targetiert werden, wie in Sautter et. al 1995 beschrieben. Hierzu werden zur Produktion vegetativer apikaler Sprossmeristeme Samen durch Einweichen in 70% Ethanol für 2 min, gefolgt von Natriumhypochloritbehandlung und vier Wasserspülungen, sterilisiert und gewaschen. Die sterilen Samen werden dann in Glasröhrchen auf MS-Medium, supplementiert mit 100 mg/l Cefotaxim, 2% Saccharose und 0,8% Difco Agar, eingesät. Apikale Sprossmeristeme von 6-bis 10-Tage-alten Pflänzchen werden anschließend durch Entfernung der Koleoptilen und der ersten drei bis fünf Blätter freigelegt. Wurzeln werden sodann auf etwa 5 mm gestutzt. Die Explantate werden hierauf mit verschiedenen Saccharosekonzentrationen (Optimum: 10%) supplementiert und auf MS-Basismedium platziert (0,8% Agarose). Nach Partikelbeschuss wurden die Explantate nach Bombardierung zur weiteren Kultur auf MS-Agarose transferiert.

Des Weiteren können in Weizen auch die Blütenorgane targetiert werden. Das Sprossmeristem in Weizen differenziert sich sehr früh in der Entwicklung zu unreife Ähren bzw. Schösslingen, wenige Wochen nach der Aussaat. Diese unreifen Ähren sind an der Unterseite der Deichsel zu finden (s. Fig 28 linkes Bild). In diese unreifen Ähren wurde durch Einschneiden der Schösslinge ein Fenster eingebracht. Durch dieses Fenster können die meristematischen Gewebe mit verschiedenen Transformationstechniken erreicht werden. Nach der Transformation wurde die Wunde verschlossen und die transformierten Anlage wurde bis zur Reife der Samen weiter kultiviert (s. Fig 28 Mitte links und rechts sowie rechtes Bild) und die Nachkommen wurden anschließend analysiert. Alternativ können die unreifen Ährchen vom Blütenstand abgelöst, *in vitro* sterilisiert und gezielt gelöst werden. Die Reifung und Samenproduktion kann dann angelehnt an Barnabas et al 1992 *in vitro* durchgeführt werden.

Für Weizen wurde darüber hinaus gezeigt, dass die Targetierung unreifer Blütenstände unter Verwendung einer Gene Gun möglich ist (Leduc et al. 1994, Sautter et al. 1995). Nach der Bombardierung wurde gezeigt, dass die Zellen in dem derart behandelten Gewebe eingeführte Reportergene exprimieren und sich weiter teilen können.

### Brassica napus:

Auch für Raps wurde gezeigt, dass sich das apikale Sprossmeristem bereits im sog. "Herzstadium" entwickelt. Ein Transformationsverfahren für Raps in diesem Stadium, das auch mit den hierein offenbarten Verfahren zum Einsatz kommen kann, ist in Huang et al., 2009, beschrieben.

Darüber hinaus kann in Raps als Zielpflanze das Sprossmeristem auch in einem späteren Stadium nach Keimung, bzw. wenn die Pflanzen das 2-8 Blatt-Stadium erreicht haben, gezielt transformiert werden. Hierzu werden die Blattprimordien, die das Meristem bedecken, vorsichtig mit einem Skalpell abgezogen. Die freigelegten Meristeme werden dann bevorzugt mit einem Antioxidans behandelt, um es zu schützen. Im Anschluss können die meristematischen Regionen mittels verschiedener Transformationstechniken transformiert werden. Auch hier kann die Pflanze anschließend bis zur Reife der reproduktiven Organe weiter großgezogen werden und die erhaltenen Nachkommen können auf das Vorhandensein der gezielt eingeführten Veränderung hin analysiert werden.

Auch die Blüten von Raps können targetiert werden. Die Blüten in einem Rapsblütenstand werden kontinuierlich produziert. An der Spitze der Blütentraube werden neue Blüten produziert. Wenn die Blütenorgane von Raps transformiert werden sollen, können zwei Ansätze verfolgt werden. Einerseits können unreife Blüten *in situ* geöffnet und reproduktive Gewebe gezielt angesteuert werden. Nach der Behandlung werden nicht behandelte Blütenstände und Schoten entfernt und der Blütenstand wird sodann abgedeckt, um eine Selbstbestäubung zu fördern. Die Samen werden geerntet, und die Nachkommen analysiert. Im zweiten Ansatz werden alle differenzierten Blüten sorgfältig von der Blütentraube/Rispe entfernt und das Blütenmeristem wird exponiert belassen. Diese Meristeme werden dann durch verschiedene Transformationsarten behandelt. Meristeme werden dann bedeckt, damit die normale Entwicklung fortgesetzt werden kann. Alle Schoten/Hülsen wurden geerntet und die Nachkommen wurden entsprechend molekularbiologisch und phänotypisch getestet.

### Glycine max:

Zur Sojabohnen Transformation können ebenfalls gezielt meristematische Regionen angesteuert werden.

Das Sprossmeristem von Embryonen wurde wie in McCabe beschrieben (McCabe et al., 1988) belichtet und transformiert. Hierzu werden reife Samen von Soja (BR-16, Doko RC, BR-91 und Conquista) in 70% Ethanol oberflächensterilisiert (1 min) gefolgt von Eintauchen in 1% Natriumhypochlorit für 20 min und dann dreimaliger Spülung in sterilem destillierten Wasser. Die Samen wurden in destilliertem Wasser für 18-20 h eingeweicht. Die embryonalen Achsen wurden aus Samen herausgeschnitten und die apikalen Meristeme wurden durch Entfernung der primären Blätter exponiert. Die embryonalen Achsen wurden in Bombardement Medium positioniert (BM: MS (Murashige und Skoog 1962) Basalsalze, 3% Saccharose und 0,8% Phytagel Sigma, pH 5,7), apikal nach oben ausgerichtet in 5-cm-Kulturschalen mit 12 ml Kulturmedium. Sobald die von embryonalen Achsen abgeleiteten Triebe eine Länge von 2-3 cm erreicht hatten, wurde ein 1-mm-langer Abschnitt von der Basis jedes Blatt für die Analyse der GUS (beta-Glucuronidase) entnommen (McCabe et al. 1988). Die Triebe oder Sprosse, die die exogene DNA exprimierten, wurden einzeln in einen Plastiktopf übertragen, der 0,2 I autoklavierte gedüngte Erde (Vermiculit (1:1)) enthielt, und dann mit einer Plastiktüte mit einem Gummiband verschlossen abgedeckt im Gewächshaus gehalten. Nach 1 Woche wurde das Gummiband entfernt. Nach einer weiteren Woche wurde die Plastiktasche ebenfalls entfernt. Sobald die akklimatisierten Pflänzchen etwa 10 cm Länge erreicht hatten wurden sie in einen Topf mit 5 I gedüngter Erde überführt, bis sich Samen entwickeln konnten (McCabe et al. 1988). Sobald die Pflänzchen herangewachsen waren, wurden Blattproben für die Analysen entnommen. Die Pflanzen wurden weiter herangezogen, bis zur Reife, um die Nachkommen davon auf die gezielt eingeführte Änderung hin zu analysieren.

Alternativ wurde das Meristem der unreifen Embryonen gezielt zur Transformation angesteuert. Dazu wurden 5 bis 20 Tage nach der Blüte die Schoten geerntet und Embryonen zwischen dem Herz- oder Kotyledonenstadium wurden mit Hilfe eines Skalpells und einem Greifwerkzeug extrahiert. Diese Embryonen wurden auf Embryoreifungsmedium gebracht und das Sprossapikalmeristem wurde mit verschiedenen Abgabe- /Transformationsmethoden gezielt transformiert. Die Embryonen wurden dann in der Dunkelheit für 1 bis 10 Wochen bis zur vollständigen Reifung, wie in Buchheim et al. (1989) beschrieben, herangezogen und erhielten Licht für die Embryo-Keimung. Die derart herangezogenen Pflänzchen wurden bis zur Reife im Gewächshaus kultiviert. Die Samen wurden geerntet, und die Nachkommen wurden analysiert. Die gezielte Einbringung von rekombinanten Konstrukten in die Sprossmeristeme der keimenden Pflänzchen von Sojabohnen wurde wie beschrieben durchgeführt (Chee et al. 1989). Samen von *Glycine max* L. Merr. (Cv A0949) wurden für 15 min durch Eintauchen in eine 15%ige Clorox-Lösung sterilisiert, gefolgt von mehreren Spülungen mit sterilem destilliertem Wasser. Samen wurden für 18 bis 24 h auf sterilen angefeuchteten Papiertüchern in Petrischalen zur Keimung gebracht, bei 26° C im Dunkeln. Samenhüllen wurden entfernt, und eine der zwei Kotyledonen jedes gekeimten Samens wurde entfernt, und die Halbsamen mit der Sprossknospe des Keimlings (Plumula), dem Kotyledonen-Knoten und benachbartem kotyledonen Gewebe wurden über Nacht mit Flüssigkulturen vom avirulenten Agrobacterium-Stamm C58Z707, der das binäre Plasmid pGA482G enthält, inokuliert. Agrobacterium-Transformation kann auch durch andere Einbringungs- bzw. Transformationsverfahren ersetzt werden. Ein weiterer Ansatz ist etwa in Chowrira et al. 1995 beschrieben. Hier wird die Endknospe von Pflänzchen (7-10 Tage alt) durch Entfernen der umgebenden Blattgewebe exponiert. Fremd-DNA wird mit einer Spritze, enthaltend auch Lipofectin als Transfektionsagens, injiziert und danach wird das Meristem mit der Mischung elektroporiert. Die Pflanzen werden ohne Selektion bis zur Reife herangezogen und chimäre Pflanzen können im Anschluss auf diese Weise erhalten werden. Die Nachkommen davon werden dann analysiert.

Zugang zu den Narben der zu bestäubenden Blüten von Sojabohnen wurde wie in Shou et al (2002) beschrieben erlangt. Kurz gesagt, wurden alle Experimente am späten Nachmittag mit Blüten durchgeführt, die am selben Morgen auf natürliche Weise bestäubt worden waren. Zwei Petalen und eine Kiel-Petale wurden entfernt, um die Stigmen/Narben von Sojabohnen Blüten dem Licht zu exponieren belichten. Stigmen wurden an der Grenze zwischen dem Ovar und Stigma abgetrennt, und Plasmid-DNA (Konzentrationen von 25, 80, 100 oder 150 µg/ml) wurde auf die freiliegende Narbe aufgebracht. Die behandelten Blüten wurden durch Tagging identifiziert und unbehandelten Blüten und Knospen auf demselben Knoten wurden entfernt. Die Schoten, die sich aus den behandelten Blüten entwickelten wurden einzeln geerntet. Alternativ kann das Blütenmeristem des Sojabohnen-Blütenstandes gezielt transformiert werden, bevor er sich terminal entwickelt, indem die Primordien entfernt werden, oder, wenn die Blüten beginnen sich weiter zu entwickeln. Diese Exposition wurde durch Exzision der Primordien mit einem Skalpell erreicht. Sobald das Blütenmeristem transformiert worden ist, wird es abgedeckt. Nachdem sich der Blütenstand entwickeln konnte und es zur Selbstbestäubung gekommen ist, können die Schoten der behandelten Pflanzen geerntet, die Samen verarbeitet, und die Nachkommen können auf eine gezielte genomische Veränderung hin getestet werden.

### Gossypium sp.:

Für Baumwolle können Experimente gemäß der vorliegenden Offenbarung zur Einbringung einer gezielten Veränderung von Interesse durchgeführt werden, indem die Meristeme von Embryonen wie in Aragäo et al (2005) behandelt werden. Hierzu werden Samen (var. 7mH, CD-401, Antares und ITA94) von Hand geerntet und Flusen werden durch Säurebehandlung entfernt. Konzentrierte Schwefelsäure wurde hinzugefügt, und die Samen (3 ml/g Samen) werden kräftig für 1 min mit einem Glasstab gemischt. Samen werden dann sofort in 5 I Wasser überführt, dreimal mit destilliertem Wasser gespült und auf einem Papiertuch getrocknet. Reife Samen werden mit 70% Ethanol für 10 min, gefolgt von 1 min Behandlung in 2,5% Calciumhypochlorit oberflächensterilisiert und 3x in sterilem destillierten Wasser gespült. Dann wurden die Samen in destilliertem Wasser für 24 h eingeweicht, woraufhin die Samen 16 h bei Raumtemperatur im Dunkeln keimen konnten. Embryonale Achsen wurden aus Samen herausgeschnitten und Apikalmeristeme wurden durch Entfernen der Kotyledonen exponiert. Explantate wurden auf MS-Medium, enthaltend 3% Glucose überführt (5 mg/l Benzylaminopurin (BAP), 0,8% Phytagel (Sigma)). Der pH-Wert wurde vor dem Autoklavieren auf 5,7 eingestellt. Embryonale Achsen wurden wie oben beschrieben hergestellt und in Beschuss- Medium (MS Basalsalze Medium, 3% Glukose, 5 mg/l BAP und 0,8% Phytagel Sigma, pH 5,7), apikal nach oben, in 5 cm-Kulturschalen mit 12 ml Kulturmedium positioniert. Hierauf können die Meristeme transformiert oder transfiziert werden. Die behandelten Apikalmeristeme wurden im Dunkeln kultiviert. Die Meristeme, die Wachstum zeigten, wurden in eine Wachstumskammer überführt. Die Pflänzchen wurden dann in das Gewächshaus überführt und bis zur Reife kultiviert. Die Nachkommen wurden analysiert.

Alternativ wurden die Meristeme von Embryonen transformiert, wie in Rajasekaran (2013) beschrieben.

In einem weiteren Ansatz wurden die Meristeme von unreifen Embryonen *in vitro* nach dem Protokoll, beschrieben von Mauney (1961), targetiert. Das verwendete Kulturmedium war zusammengesetzt aus Whites Nährstoffmischung mit allen Bestandteilen + das Fünffache der üblichen Konzentration und Supplementen (40 mg/l Adeninsulfat, 250 mg/l Caseinhydrolysat, 150 ml/l Kokosmilch, und 7 g/l NaCl). Das Medium wurde mit 8 g/l Bacto-Agar und 20 g/l Saccharose als Kohlenhydratquelle, gehärtet. Das wichtigste Merkmal dieses Mediums für den Erfolg des Kulturverfahrens ist die Einstellung des osmotischen Druckes auf ein hohes Niveau, was durch Zugabe von 7 gm/l NaCl erreicht werden kann. Nachdem die Embryonen auf diesem Medium für 3-4 Wochen gewachsen waren, wurden sie in ein Medium mit mittlerem osmotischen Druck (3 g/l NaCl anstelle der 7 g/l) überführt und dann nach einer weiteren Wachstumsperiode von 2 Wochen in ein Medium ohne NaCl überführt. Die erfolgreich kultivierten Embryonen im letzten Medium keimten und wurden in Erde gepflanzt.

Um Sprossmeristem in Pflänzchen von Baumwolle zu transformieren, wurde in einem ersten Ansatz Meristeme wie in Zapata et al. (1999) transformiert. Samen wurden mit konzentrierter Schwefelsäure (1 h), bei 50 Upm in einem Rotationsschüttler (50% Clorox (1 h)) oberflächensterilisiert und mindestens dreimal mit sterilem doppelt-destilliertem Wasser gespült. Die Samen wurden dann auf 0,15% (Gew./Vol.) mit Gelrit verfestigtes Medium, pH 5,7, gebracht, das Murashige und Skoog (MS) anorganischen Salze (Murashige und Skoog 1962) und 2% Saccharose zur Keimung enthält. Die Samen wurden für etwa 3-4 Tage bei 28°C im Dunkeln inkubiert. Die Triebspitze wurden isoliert und dann in MS anorganischen Salzen (Murashige und Skoog 1962 mit 100 mg/l myo-inositol, 0,5 mg/l Thiamin/ HCl, 0,5 mg/l Nikotinsäure, 0,5 mg/l Pyridoxin/HCl, 3% Saccharose und 0,15% (Gew./Vol.) Gelrite bei pH 5.7 überführt. Nach der Isolierung kann die Abgabe eines rekombinanten Konstrukts von Interesse durchgeführt werden und anschließend kann das Pflanzenmaterial auf neues Medium transferiert werden. Die vitalen transformierten Apices werden auf frisches Medium übertragen. Danach werden die überlebenden Apices auf das gleiche Medium, jedoch ohne Kanamycin, überführt. Sobald sich Wurzeln bilden konnten, werden die Pflanzen mit Wurzeln (T0) in Erde überführt und im Gewächshaus zur Reife heranwachsen gelassen. Die Nachkommen können dann analysiert werden.

Alternativ wurden die Baumwoll-Meristeme wie in Keshamma et al. beschrieben transformiert. (2008). Hierzu werden Samen einer Zuchtlinie viz. NC-71, über Nacht in destilliertem Wasser eingeweicht und die Oberfläche wurde, zuerst mit 1% Bavastin für 10 Minuten und dann mit 0,1% HgCl₂ für einige Sekunden sterilisiert und anschließend wurde jeweils gründlich mit destilliertem Wasser gewaschen. Die Samen konnten später bei 30° C in Dunkelheit auf Petrischalen keimen. Zwei Tage alte Keimlinge wurden als Explantate verwendet. Die Keimlinge mit gerade entstehender Plumula/Sprossknospe wurden infiziert, indem sie die Kotyledonen getrennt wurden, ohne sie so zu beschädigen, sodass das Meristem sichtbar war. Dann kann eine Transfektions- bzw. Transformationsmethode von Interesse angewendet werden. Die Keimlinge wurden anschließend auf autoklaviertes Soilrite (Vermiculit-Äquivalent) übertragen, mit Wasser angefeuchtet und für die Keimung unter aseptischen Bedingungen bedeckt in einen Wachstumsraum, 5 Setzlinge pro Topf, gebracht. Nach 5 bis 6 Tagen wurden die Keimlinge in Töpfe mit Soilrite überführt und für mindestens 10 Tage wachsen gelassen, bevor sie in das Gewächshaus überführt werden konnten. Die maturen Pflanzen können dann analysiert werden.

In einem anderen Ansatz wurden Baumwollemeristeme nach dem Protokoll, beschrieben in McCabe et al. (1993), transformiert.

In noch einem anderen Ansatz können Baumwolleblüten nach dem Verfahren von der Gounaris et al. (2005) gezielt transformiert werden. Hierzu werden Baumwollpflanzen der Sorte Christina für die Transformation verwendet. Blüten, die als Pollen-Rezeptoren verwendet werden sollen, müssen hierzu zwei Tage vor dem voraussichtlichen Zeitpunkt der Dehiszenz von männlichen Keimzellen befreit werden. Am Morgen des Tages der Bestäubung, werden Spenderblüten mit intakten Staubblättern 1-2 Stunden nach dem Aufgehen gesammelt. Jede Spenderblüte kann dann mit einer Transformations- oder Transfektionsmethode von Interesse behandelt werden. Diese Blütenstände wurden zur weiteren Bestäubung von Rezeptorblüten (vorab: Entfernung der männlichen Keimzellen) verwendet. Die bestäubten Blüten können sich weiter entwickeln und Samen produzieren. Die Nachkommen können dann molekularbiologisch analysiert werden. In einem anderen Ansatz können Blütenmeristeme oder unreife Blüten gezielt genetisch modifiziert werden. Hierzu wird das Meristem, das den fruchtbildenden Zweig bildet, die Fruchtknospe und die unreife Blüte durch Entfernen der Primordien und der Brakteen exponiert. Auf die freiliegenden Gewebe kann dann eine Abgabemethode (Transformation/Transfektion, biologisch, chemisch oder mechanisch) von Interesse gemäß der vorliegenden Offenbarung angewendet werden. Danach wurden die behandelten Zonen bedeckt und weiter wachsen gelassen. Die Früchte wurden dann geerntet. Die Nachkommen können molekularbiologisch sowie optional phänotypisch analysiert werden.

### Oryza sativa:

Sofern die Zielpflanze von Interesse Reis ist, können die folgenden Methoden zum Einsatz kommen, um in eine meristematische Zielstruktur eine gezielte genomische Veränderung einzuführen.

Nach der Methode von Naseri et al. 2014 werden Reissamen (O. sativa var Hashemi) durch Einweichen in 90% Ethanol sterilisiert (1 min) und dreimal mit Wasser gewaschen. Sterile Samen werden für zwei Tage bei 22 °C auf nasse Baumwolle gelegt. In diesem Stadium erfolgte die Inokulation mit *A*. *tumefaciens* in embryonale apikale Meristeme der getränkten Samen, in den Bereich auf der Saatgutoberfläche, wo sich später ein Spross entwickeln wird. Die Oberfläche wird zweimal bis zu einer Tiefe von etwa 1 bis 1,5 mm mit einer Nadel (Φ 0,7 mm, zuvor in *A. tumefaciens* Inokulum getaucht) durchstochen. Die inokulierten Samen wurden dann in Flaschen, mit Aluminiumfolie bedeckt, auf Filterpapier auf nasses Perlit gelegt und neun Tage bei 23°C in Dunkelheit inkubiert. 70 bis 75% der inokulierten Samen keimten. Um *A. tumefaciens* abzutöten, wurden die Keimlinge bei Raumtemperatur in einer wässrigen Lösung (1000 ppm) von Cefotaxim für 1 h eingetaucht. Zur Wurzelbildung wurden die Keimlinge in Yoshida Lösung gegeben. Schließlich wurden die Keimlinge in Töpfe gepflanzt und bis zur Reifung (T0) unter nicht sterilen Bedingungen herangezogen. Die Selbstbestäubung und damit das Hervorbringen einer T1 Generation wurde ermöglicht.

In einem weiteren Ansatz wurden die Meristeme von unreifen Embryonen zur gezielten Veränderung mit den hierein offenbarten CRISPR Werkzeugen angesteuert. Unreife Samen wurden 3-12 Tage nach der Bestäubung geerntet. Die unreifen Embryonen werden in ein Reifungsmedium (Ko et al. 1983) gegeben, und die Transformations-/Transfektionsmethode von Interesse kann angewendet werden. Die Embryonen wurden wie in Ko et al. (1983) beschrieben zur Reifung gebracht. Die Samen wurden geerntet, und die Nachkommen wurden molekularbiologisch analysiert.

Meristeme von Reispflänzchen wurden wie in Muniz de Péadua et al (2001) behandelt. Hierzu wurden Reissamen oberflächensterilisiert und *in vitro* zur Keimung gebracht. Nach ca. 4 Tagen werden die Sprossapices aus dem distalen Teil des ersten Internodiums des Epicotyl und der Koleoptile herausgeschnitten. Nach Exposition kann ein Abgabesystem von Interesse angewendet werden. Die Wurzelbildung wird sodann ermöglicht. Die gewonnenen Pflänzchen wurden in das Gewächshaus überführt und weiter kultiviert und die Nachkommen wurden schließlich analysiert.

Zur Targetierung von Blütenorganen werden unreife Reis-Ährchen wie in Rod-in et al,. (2014) beschrieben behandelt. Die Blütenstände werden im Stadium 51 (Beginn der Rispenentwicklung: Spitze des Blütenstandes außerhalb der Hülse) gemäß der BBCH-Skala verwendet (Lancashire et al., 1991) und können dann behandelt und die erhaltenen Nachkommen können anschließend analysiert werden. Des Weiteren können die Blütenmeristeme angesteuert werden. Hierzu müssen die Meristeme durch Entfernen des umgebenden Gewebes freigelegt und anschließend transformiert, weiterkultiviert und die Nachkommen schließlich analysiert werden. Ferner können die unreifen Ährchen entweder vor Differenzierung, oder nachdem die umliegenden Primoridia entfernt wurden, durch Transformation oder Transfektion behandelt werden. Die behandelten Ährchen konnten sich dann weiter entwickeln. Die resultierenden Samen der entsprechenden Blüten wurden geerntet und die Nachkommen wurden auf gezielte Editing-Events hin untersucht (Itoh et al., 2005).

### Beispiel 16: Transiente Transformationsverfahren

Speziell für die *in planta* Transformation meristematischer Gewebe besteht ein großes Interesse daran, transiente Transformationsverfahren bereitzustellen, insbesondere für die Einführung von CRISPR Werkzeugen von Interesse, da hierdurch nur die gezielt einzuführende Veränderung, nicht jedoch die Werkzeuge selbst an die Nachkommen einer Zelle weitergegeben werden. Derartige kontrollierte und kontrollierbare Verfahren werden auf Grund regulatorischer Bestimmungen und bedingt durch Sicherheitsüberlegungen immer wichtiger auf dem Bereich der Pflanzenzüchtung. Transformationsverfahren, transient wie stabil, müssen natürlicherweise auf das zu transformierende Gewebe hin angepasst werden. Daher wurden die folgenden Versuche durchgeführt, die sich teils breit und allgemein, teils für spezifische Gewebe (Pollen, Meristem, Blüten etc.) anwenden lassen.

### Cas9:

Cas9 wurde von New England Biolabs (NEB), PNA BIO, ToolGen, LDBIOPHARMA oder ABM erworben haben, oder Cas9 wurde wie in Liu et al. beschrieben (2015) gereinigt.

### In-vitro-Transkription von sgRNAs:

Die in vitro-Transkription wurde wie beschrieben von Zuris et al. (2015) durchgeführt. Lineare DNA-Fragmente, die eine T7 Promotorbindungsstelle enthalten gefolgt von der 20-bp sgRNA Zielsequenz, wurden unter Verwendung der T7 RNA High Yield Synthesis Kit (NEB) gemäß den Anweisungen des Herstellers *in vitro* transkribiert. In vitro transkribierte RNA wurde mit Ethanol präzipitiert und durch Gelelektrophorese auf einem 10% Polyacrylamid Criterion TBE-Harnstoff-Gel (Bio-Rad) gereinigt. Ausgeschnittene Gelfragmente wurden in 420 µl 300 mM NaCl über Nacht auf einer Schüttleroberfläche bei 4°C extrahiert. Gel-gereinigte sgRNA wurde mit Ethanol ausgefällt und wieder in Wasser gelöst, und die sgRNA Konzentration wurde schließlich durch UV-Absorption quantifiziert. Die sgRNA kann dann schockgefroren und bei -80°C gelagert werden. Alternativ wurden gRNAs wie in Kim et al beschrieben (2014) erhalten. Hierzu wurde RNA in vitro durch T7-RNA-Polymerase-Run-off-Reaktionen unter Verwendung des MEGAshortscript T7-Kit (Ambion) transkribiert. Templates für sgRNA oder crRNA wurden durch Annlagerung und Verlängerung von zwei komplementären Oligonukleotiden erzeugt. Transkribiert RNA wurde durch Phenol:Chloroform-Extraktion, Chloroform-Extraktion und Ethanolfällung gereinigt. Gereinigte RNA wurde durch Spektrometrie quantifiziert.

Alternativ kann ein weiteres Protokoll (beschrieben in Ramakrishna et al. 2014) durchgeführt werden. Hierzu wurde die RNA *in vitro* durch Run-off-Reaktionen durch die T7-RNA-Polymerase transkribiert. Templates für die sgRNA Transkription wurden durch Annealing/Hybridisierung und Verlängerung von zwei komplementären Oligonukleotiden erzeugt. Transkribierte RNA wurde auf einem 8%igen denaturierenden Harnstoff-PAGE-Gel aufgetrennt. RNA wurde in Nuklease-freiem Wasser aufgenommen, und anschließend durch Phenol:Chloroform-Extraktion, Chloroform-Extraktion und Ethanolfällung gereinigt und gewonnen. Gereinigte RNA wurde durch Spektrometrie quantifiziert.

### Komplexierung Protein Cas9 und gRNA:

Wie in Zuris et al. (2015) beschrieben wurde für die Einführung von Cas9:sgRNA Komplexen 1 µl 200 µM) Cas9 Protein mit 2 µl 50 µM sgRNA gemischt und 5 min bei Raumtemperatur inkubiert, bevor der Komplex mit 3 µl, entweder RNAiMAX oder Lipofectamine 2000, gemischt und für weitere 30 min vor der Injektion inkubiert wurde. Alternativ wurde die Komplexierung wie von Kim et al 2014 beschrieben durchgeführt. Hierzu wurde Cas9 Protein (4,5-45 mg) mit in vitro transkribierter sgRNA (6-60 mg) vorgemischt. Cas9 Protein in Lagerungspuffer (20 mM HEPES pH 7,5, 150 mM KCI, 1 mM DTT und 10% Glycerin) wurde mit sgRNA in Nuclease-freiem Wasser gelöst, gemischt und 10 min bei Raumtemperatur inkubiert.

### Agrobacterium (Ab)

### Pollen-Transformation mit Ab:

Die Pollen-Transformation wurde wie in Li et al. (2004) beschrieben durchgeführt. Hierzu wurden Blüten mit frisch entwickelten und exponierten Antheren gesammelt. Ein Aliquot einer Ab-Lösung wurde in sterile 1,5 ml Röhrchen überführt und für 10 min bei 3.000 Upm zentrifugiert. Das Pellet wurde resuspendiert (in Pollen-Keimungsmedium mit ca. 50 mg Pollen/ml) und Vakuum (-80Pa) wurde für 30 min angelegt und dann langsam abgelassen. Die Suspension wurde anschließend bei 3.000 Upm für 5 min zentrifugiert und die Pellets, d.h. die Pollen, wurden unmittelbar zur Bestäubung eingesetzt.

### Sprossapikalmeristem-Transformation mit Ab:

Meristeme von Keimlingen wurden auf der Grundlage des Protokolls von Chee et al. 1989 transformiert. Hierzu wurden Inokulationen an drei verschiedenen Stellen durchgeführt, indem eine 30'/2-Gauge-Nadel in die Plumula, kotyledone Knoten und benachbarte Regionen eingeführt und etwa 30 ml der Ab-Zellen je Injektionspunkt injiziert wurden. Der Keimungsprozess der Ab-infizierten Samen wurde fortgesetzt, indem die Samen auf steriles angefeuchtetes Papier transferiert und weiter bei 26°C inkubiert wurden (im Dunkeln, für etwa 4 h). Zur vollen Entwicklung wurden die Keimlinge dann in den Boden eingebracht. Alternativ kann das Protokoll beschrieben in Keshamma et al. (2008) angewendet werden. Die Keimlinge werden hierbei, sobald die Plumula entsteht, durch Separieren der Kotyledonen, ohne diese zu zerstören, infiziert, so dass das Meristem sichtbar ist. Dann werden die Meristeme mit sterilen Nähnadel angestochen und anschließend in für 60 min in eine *Agrobacterium*-Kultur getunkt. Nach der Infektion wurden die Keimlinge kurz mit sterilem Wasser gewaschen und später auf autoklaviertes Soilrite übertragen.

### Partikelbeschuss:

Beschuss bzw. Bombardierung von Embryomeristemen wurde wie folgt durchgeführt: Embryonen im Koleoptilar- bzw. oder Herz-Stadium wurden 0-6 Stunden vor der Bombardierung in Embryo-Maturierungsmedium mit Osmotikum supplementiert, wie in Vain et al. (1993) beschrieben, platziert. Die Partikelaufbereitung und -vorbereitung wurde nach einer Routine-DNA-Fällung mit Calciumchlorid und Spermidin durchgeführt. Im Fall von Protein/RNA-Mischungen wurde das Protokoll von Martin-Ortigosa et al (2014) durchgeführt, wobei die Mischung zusammen mit den Goldpartikeln getrocknet oder gefriergetrocknet wird. Nach 16-24 h nach dem Beschuss wurden die Embryonen auf eine Reifungsmedium ohne Osmotikum gebracht.

### Beschuss von Antheren:

Beschuss von Antheren kann gemäß Twell et al., 1989, durchgeführt werden. Hierzu werden die Antheren von Pflanzen 1d vor Pollen-Freisetzung in 10% Clorox für 10 min oberflächensterilisiert und in sterilem destilliertem Wasser gespült. Die Antheren wurden quer mit einer sterilen Rasierklinge aufgeschnitten und 20 Antheren-Abschnitte wurden auf auf festes MSO-Medium mit einer Fläche von 4cm² mit exponierten Theken positioniert.

In einem anderen Ansatz wurden Antheren wie von Obert beschrieben (Obert et al. 2008) beschossen. Hierzu wurden Spikes/Ähren geerntet, sobald Mikrosporen im mittleren einkernigen Entwicklungsstadium waren. Zwei verschiedene Vorbehandlungen wurden in unseren Studien zusammen mit der Verwendung von nicht-vorbehandeltem Material, eingesetzt. Für eine Kälte-Vorbehandlung wurde das Pflanzenmaterial auf einem angefeuchteten Filterpapier in einem kalten Raum bei 5°C gelagert (Dedicova et al. 1999). Nach der Vorbehandlung von Material (14 Tage im Kühlraum), wurden die spezifischen Teile der Ähren, die Mikrosporen enthalten, im exakt geeigneten Stadium ausgewählt und als weiteres experimentelles Material verwendet. Das Material wurde oberflächensterilisiert (in 70% (Vol./Vol.) Alkohol) und und 3x mit sterilem destillierten Wasser gewaschen. Für die Mannitol Vorbehandlung wurden geeignete Teile der frischen Ähren, die Mikrosporen im richtigen Stadium enthalten, oberflächensterilisiert (in 70% Alkohol) und mit sterilem destilliertem Wasser dreimal gewaschen. Antheren wurden unter sterilen Bedingungen isoliert und zur Vorbehandlung in Flüssigmedium platziert (0,3 M Mannit oder Wasser) und in einem kalten Raum bei 5°C gelagert. Antheren wurden vor dem Beschuss entweder frisch isoliert oder sie wurden nach einer Vorbehandlung auf die Oberfläche eines Kultivierungsmediums (FHG Medien Kasha et al., 2001) platziert. Die Beschuss-Bedingungen lauteten: Abstand (Macroträger - Antheren in Petrischale) 9 cm; Druckeinstellungen von 650, 900 und 1.100 psi. Antheren-Kulturen wurden dann bei 26°C in der Dunkelheit in einer Gewebekulturwachstumskammer kultiviert.

In einem anderen Ansatz (Touraev et al. (1997)) werden einzellige Mikrosporen und Pollenkörner im mittleren bizellulären Stadium unmittelbar nach der Isolierung in dem jeweiligen Kulturmedium bombardiert. Die Suspension (0,7 ml), enthaltend ca. 5×10⁵ Zellen, wurde gleichmäßig auf auf einem sterilen Filterpapier (Whatman No. 1) platziert und in eine 10 cm Petrischale (Sterilin, Großbritannien) überführt. Das Helium-betriebene PDS-1000/He Partikelabgabesystem (Bio-Rad, USA) wurde für die biolistische Transformation verwendet. Der Beschuss wurde im Wesentlichen wie von Sanford et al. (1993) beschrieben durchgeführt. Plasmid-DNA wurde auf Goldpartikel (Bio-Rad, USA) mit einem mittleren Durchmesser von 1,1 µm präzipitiert. Jede Transformation umfasste drei Bombardierungen. Die beschossenen Mikrosporen bzw. mid-bizelluläre Pollenkörner wurden von dem Filterpapier abgewaschen und im jeweiligen Reifungsmedium inkubiert.

### Beschuss von Blüten:

Angelehnt an ein Protokoll von Twell et al., 1989, wurden Gruppen von 10 Blüten mit gebogenen Blütenblättern in intakter Form bombardiert, wobei der angeschnittene Blütenstengel in destilliertem Wasser suspendiert wurde.

### Beschuss von Pollen:

Angelehnt an ein Protokoll von Twell et al., 1989 wurden Pollen von reifen Blüten wurde in sterilen Mikrozentrifugenröhrchen gesammelt. Vor dem Beschuss wurden trockene Pollenproben in flüssigem MSO-Medium in einer Dichte von etwa 106 Körner pro ml suspendiert. Die Pollensuspension (1 ml) wurde sofort auf die Oberfläche einer 9 cm Petrischale, enthaltend mit Agar verfestigtes MSO-Medium, pipettiert, auf die zuvor ein steriles Whatman Nr. 1 Filterpapier mit einer Nylonmembran (Genescreen, NEN) gegeben worden war. Der Beschuss wurde innerhalb von 60 min nach der Überführung des Pflanzenmaterials auf das MSO-Medium durchgeführt. Die Fällung von Plasmid-DNA auf Wolfram-Mikroprojektile und der Beschuss erfolgten wie in Klein et al. beschrieben. Nach dem Beschuss wurden Petrischalen oder intakte Blüten in destilliertem Wasser bei 26°C im Licht inkubiert.

In einem anderen Ansatz wurde Pollen-Bombardement wie in Horikawa et al. (1997) beschrieben durchgeführt. Hierzu wurden reife Pollenkörner aus den extrudierten Tasseln gesammelt. Die folgenden Vorbereitungsschritte für den Beschuss wurden sehr schnell durchgeführt, da die Lebensfähigkeit der Pollen sich innerhalb kürzester Zeit verringert. Der Pollen wurde in flüssiges MS-Medium eingetaucht, das 30 g/l Saccharose (pH 5,8) enthielt. Die 4.0×10⁵ Pollenkörner (in 1 ml Medium) wurden auf die Oberfläche eines Stücks Mikrofilter (Porengröße 0,45 um, Fuji Film Co., Tokyo) durch Vakuumfiltration adsorbiert. Der Mikrofilter wurde auf 1%ige Agar-Platten in einer Petrischale in Vorbereitung für den Beschuß mit einer Partikelkanone platziert.

### Bestäubung mit den behandelten Pollen:

Für die Bestäubung mit den beschossenen Pollen wurde das Protokoll beschrieben in Touraev et al 1997 durchgeführt. Hierzu wurden reife Blüten kurz vor der Blüte mit noch geschlossenen Antheren einen Tag vor der Bestäubung emaskuliert. Die *in vitro* gereiften Pollen wurde mehrere Male in Medium GK ohne Quercetin gewaschen und dann in Tröpfchen von 3 µl auf Narben überführt. Es wurden solche Narben wurden für die Pipetten-Bestäubung ausgewählt, die ein gute Narbensekretproduktion zeigten. Um Kreuzbestäubung zu verhindern, wurden alle anderen Blütenknospen in der Klimakammer am Tag vor dem Öffnen entfernt. Reife Samenkapseln/-hülsen wurden nach 3-4 Wochen eingesammelt.

In einem anderen Ansatz wurde das in Horikawa et al. (1997) beschriebene Verfahren angewendet. Hierzu wurde Pollen in 1 ml flüssiges MS-Medium gebracht. Der Pollen wurde sofort durch Pipettieren auf die Fäden einer Ähre (zuvor: mit Ährenbeuteln bedeckt) zur Bestäubung verwendet, 3 Tage nach der Entwicklung der Fäden. Die Bestäubungsbehandlungen wurden für 20 Ähren durchgeführt. Als Kontrolle wurden Pollen mit einer Probe ohne DNA bestäubt.

### Beschuss mit HELIOS von Blüten:

Der Beschuss von Blüten oder Blütenstände wurden mit der Handpistole Helios von Bio-Rad nach den Anweisungen des Herstellers durchgeführt. Sobald die Blütenstände oder Blüten exponiert vorlagen, wurden sie mit 1 bis 5 Schüsse bei 50-300 psi Druck beschossen. Die exponierten Meristeme wurden dann bedeckt und der Blütenstand oder die Blüte konnte weiter reifen.

In einem anderen Ansatz wurde das Protokoll beschrieben von Gounaris et al 2005 durchgeführt. Blüten, die als Pollen-Rezeptoren dienen sollten wurden zwei Tage vor dem voraussichtlichen Zeitpunkt der Pollenbestäubung emaskuliert. Am Morgen des Tages der durchzuführenden Bestäubung wurden von der Spender-Blüte intakte Staubblätter 1-2 Stunden nach deren Aufgehen gesammelt. Jede Spender-Blüte wurde mit 4-5 Schüssen der Partikelkanone behandelt, während diese auf einer ebenen Fläche in einer Petrischale und mit einem Nylonnetz bedeckt da lag. Die Partikelkanone wurde unter einem Heliumdruck von 400 Pfund pro Quadratzoll (psi) betrieben und war mit einem Partikeldiffusionsschirm ausgestattet. Die Heliumgas-Reinheit betrug Klasse 4.5 (99,994%). Jeder bombardierte Blütenstand wurde verwendet, um ca. 15-20 emaskulierte Rezeptor-Blüten zu bestäuben. Die bestäubten Blüten konnten sich weiter entwickeln und sodann Samen produzieren.

### Mikroinjektion: DNA/RNA/Protein und Kombinationen

### Embryo Mikroinjektion:

Für die Embryo Mikroinjektion wurde das Verfahren beschrieben in Neuhaus et al., 1987, verwendet. Hierzu wurden auf einem Deckglas positionierte Embryonen einzeln unter optischer Kontrolle mit einer handgeführten Mikrokapillare, verbunden mit einem Silikonschlauch, ausgewählt und für die Mikroinjektion als ca. 2 µl Tröpfchen in Medium auf einen Objektträger übertragen (Spangenberg et al., 1986 a). Die Mikroinjektion wurde durchgeführt, indem die Embryoide mit einer Haltekapillare fixiert und in die jeweiligen Zelle mikroinjiziert wurden. Exogene DNA wurde als 1:1 Gemisch von linearisiert (durch Schneiden der Plasmide außerhalb der insertierten Gene) sowie supercoiled Moleküle zu ca. 0,5 µg/µl in 50 mM NaCl, 50 mM Tris-HC1 pH 7,8, injiziert.

### Mikroinjektion von Sprossmeristemen mit Agrobacterium (Ab):

Die Mikroinjektion von Sprossmeristemen mit Ab wurde wie von Sivakumar et al (2014) beschrieben durchgeführt. 100 µl der Kultur wurden in eine Insulinspritze aufgenommen und in das embryonale Sprossapikalmeristem von angekeimten Baumwollsamen mikroinjiziert. Die Kultur wurde 1-5 mal (0,5-1,0 mm Tiefe) mikroinjiziert, um die Wirkung der Anzahl der Mikroinjektionen in und um die embryonale Sprossapikalmeristem herum zu überprüfen. Überschüssige Bakterienkultur wurde durch Abtupfen der infizierten Samen auf sterilem Filterpapier (Whatman No. 1) entfernt. Die Samen wurden auf ½ Stärke MS-Medium für zwei Tage im Dunklen co-kultiviert. Nach Co-Kultivierung wurden die Setzlinge mit Cefotaxim (200 mg/L) gewaschen und in Antibiotika-Selektionsmedium, enthaltend Cefotaxim und Hygromycin-B, überführt.

### Mikroinjektion von DNA in Sprossmeristeme:

Die Mikroinjektion wurde wie in Lusardi et al (1994), beschrieben durchgeführt. Reife, getrocknete Samen wurden für 20 Sekunden mit absolutem Ethanol gewaschen gefolgt von Sterilisation mit kommerzieller Bleiche (2,5% NaClO), ergänzt mit 0,01% Tween 80 (20 Minuten unter Schütteln). Die Samen wurden dann vier bis fünf Mal mit sterilem destillierten Wasser gespült. Die Keimung wurde durch Inkubation der Samen in einer 9 cm Petrischale zwischen Filterpapiere mit sterilem destilliertem Wasser, bei 27 ° C, im Dunkeln für 3-4 Tage induziert. Während dieser Zeit brach das Spross durch das Kern-Integument und erreichte eine Länge von ca. 0,8 bis 1,0 cm. Zu diesem Zeitpunkt wurde der Spross aus dem Samen auf der Ebene des skutellaren Knotens entfernt. Unter dem Stereomikroskop wurden die Koleoptile und die fünf oder sechs embryonalen Keimblätter entfernt. Nach Präparation der embryonalen Blätter wurden die unbedeckten Apices, umgeben von zwei Blattanlagen, in verschiedenen Entwicklungsstadien exponiert. Die isolierten Apices wurden in 9 cm-Petrischalen in MS-Medium (Murashige und Skoog, 1962), supplementiert mit 2% Saccharose und verfestigt mit 0,8% Difco Bacto-Agar (Difco Lab., Detroit), kultiviert und mit einem 27°C/22°C Temperaturregime und einer 16/8 h hell/dunkel-Lichtperiode weiter herangezogen. Innerhalb von 10 Tagen waren normale Pflänzchen entwickelt. Während der nächsten 15-20 Tagen erreichten diese eine Größe, die ausreichend für die Übertragung in Töpfe und ins Gewächshaus war. Für die Mikroinjektion wurden die Plasmide für die Injektion in Injektionspuffer (10 mM Tris-HCl und 0,1 M EDTA, pH 7,5) gelöst. Der Injektionspuffer wurde durch einen 0,2 µm Einweg-Filtereinheit (Schleicher und Schuell, Deutschland) filtriert, um die Lösung zu sterilisieren und Partikelkontaminationen zu vermeiden. Alle Injektionen wurden unter sterilen Bedingungen durchgeführt. Die isolierten Sprossmeristeme von Mais wurden in 9 cm Petrischalen überführt, das MS-Medium wurde mit 2% Saccharose ergänzt und mit 0,8% Difco Bacto Agar verfestigt. Die Apices wurden auf dem Medium ausgerichtet, so dass die apikalen Kuppeln deutlich sichtbar waren. Die Zellen der L2 Schichten der Meristeme wurden mit einem Stereomikroskop (mit hoher Vergrößerung ausgestattet) mit einem Embryo Splitter-System von Research Instruments (UK) (bis zu 200-facher Vergrößerung; SV 8, Zeiss, Deutschland) injiziert. In einigen Experimenten wurde eine Koinjektion von FITC-Dextran verwendet, um die injizierten Zellen besser identifizieren zu können (Neuhaus et al., 1993; Schnorf et al., 1991). Der mechanische Mikromanipulator des Systems trägt eine Injektionskapillare (Spitzendurchmesser von weniger als 1 µm), die mit einem Mikroinjektor (Eppendorf 5242 Microinjector) verbunden ist, der etwa 3 pl in die Zellen in konstanten Volumina abgibt (Neuhaus et al., 1986. 1987; Schnorf et al., 1991). Für die Stabilität und die Bewegung der Apices während der Einspritzung wurde der zweite Manipulator des Embryonen Splitter-System verwendet. Zu diesem Zweck wurde dieser Manipulator auch mit einer Mikronadeln ausgestattet, um die Apikalmeristeme bewegen und fixieren zu können, sodass diese in der richtigen Position behandelt werden können.

### WHISKERS

Whisker-vermittelte Abgabe in die verschiedenen Meristeme wurde wie in Frame et al. (1994) beschrieben durchgeführt. Die freiliegenden Gewebe wurden mit 40 µl 5% Whisker Suspension und 25 µl an Plasmid-DNA behandelt. Der Inhalt der Reaktionsgefäße wurde zunächt leicht gemischt und dann entweder aufrecht in einem Mehrfachprobenkopf auf einem Vortex Genie II Vortex-Mischer (Scientific Industries Inc., Bohemia, NY) platziert, oder horizontal in den Halter eines Mixomat Amalgammischers (Degussa Kanada Lt.d, Burlington, Ontario) positioniert. Die Transformation wurde für 60 Sekunden durch Mischen mit voller Geschwindigkeit durchgeführt (Vortex Genie II), oder bei festgelegter Geschwindigkeit für 1 Sekunde (Mixomat).

Alternativ wurden Whisker zusammen mit DNA/RNA oder Protein-Mix-Whisker in die Pipette eines Mikromanipulators geladen und sie wurden in meristematische Gewebe markoinjiziert. Zell-penetrierende Peptide: DNA/RNA/Protein und Kombinationen davon

### Mischen von Zell-penetrierende Peptiden und Cas9 Protein und gRNA:

Für den Einsatz Zell-penetrierender Peptide wurde ein an Ramakrishna et al., 2014, angelehnetes Protokoll verwendet. Einen Tag nach dem Ausplattieren wurden die Zellen mit Opti-MEM und mit Cas9-M9R und sgRNA:9R entweder nacheinander oder gleichzeitig gewaschen. Der sgRNA:9R-Komplex wurde während einer 30 min Inkubation von 10 mg sgRNA und 30-50 mg 9R Peptid in 250 ml (für die sequentielle Behandlung) oder 100 ml (für die gleichzeitige Behandlung) von Opti-MEM-Medium bei Raumtemperatur gebildet.

### Embryo:

Tat-Peptide (Tat, Tat2, M-Tat) wurden für die Einbringung von GUS-Enzym in Weizen Embryonen eingesetzt. Tat-Peptid und GUS-Enzym wurden zunächst in getrennten Mikrozentrifugenröhrchen vorbereitet. Unmarkiertes Tat-Peptid (4 µg) wurde zu sterilem Wasser gegeben (Endvolumen: 100 µL). Ebenso wurde 1 µg des GUS-Enzyms (Sigma Aldrich) in sterilem Wasser zugegeben, um ein Endvolumen von 100 µL zu erreichen. Der Inhalt der beiden Röhrchen wurden zusammengemischt, was ein 4:1 Verhältnis von Peptid:Protein-Verhältnis in der Mischung ergab. Das Gemisch wurde 1 h bei Raumtemperatur inkubiert und dann zu den isolierten unreifen Embryonen (in einem 2 ml Mikrozentrifugenröhrchen) in Gegenwart oder Abwesenheit der permeabilisierenden Mittel (Toluol/Ethanol 1:40, Vol./Vol. bezogen auf das Gesamtvolumen des Peptid:Protein-Gemischs). Nach 1 h Inkubation bei Raumtemperatur wurden die Embryos zweimal mit dem Puffer gewaschen, einer Permeabilisierung und einer Trypsin-Behandlung unterzogen (1:1 (Vol./Vol.) Permeabilisierungs-Puffer), für 5 min bei Raumtemperatur. Die Embryonen wurden zweimal mit Permeabilisierungs-Puffer gewaschen, gefolgt von einer histochemischen GUS-Analyse der Embryonen. Für die Abgabe wurde 1 µg des GUS-Enzyms durch den Chariot Proteintransduktion Kit (Active Motif, Carlsbad, CA, USA), den Herstellerangaben folgend, transfiziert. Permeabilisierte und nicht-permeablisierte Embryonen wurden mit Chariot-GUS-Komplex für 1 h inkubiert. Alle Post-Inkubationsschritte waren die gleichen wie die für die Tat-Peptide beschriebenen.

### Transformation von Mikrosporen:

Transformation von Mikrosporen unter Verwendung Zell-penentrierender Peptide wurde gemäß Shim et al. (2012) durchgeführt. Die Mikrosporen Extraktion wurde gemäß Eudes und Amundsen (2005) durchgeführt, und alle Schritte der Isolierung von Mikrosporen wurden unter Verwendung von NPB-99 Flüssigmedium durchgeführt (Zheng et al. 2001; Eudes und Amundsen 2005). Nach dem Waschen der Mikrosporen mit NPB-99, wurden 2-3 ml Mikrosporenlösung auf 2 bis 3 ml 30% Percoll-Lösung, enthaltend 400 mM Mannitol und 10 mM MES, pH 7,0, geschichtet. Mikrosporen wurden für 5 min bei 100 × g bei 4°C zentrifugiert. Die Zellen, die eine Bande an der Percoll/NPB-99-Schnittstelle gebildet hatten, wurden in ein neues 15 ml Zentrifugenröhrchen mit NPB-99 auf 15 ml verdünnt und dann erneut zentrifugiert. Der Überstand wurde dekantiert und die Mikrosporen wurden in etwa 1 ml NPB-99-Medium resuspendiert. Die Mikrosporenkonzentration wurde unter Verwendung eines Hämozytometers bestimmt und auf 2,5×10⁵ Zellen/ml eingestellt. Fünf Behandlungen, einschließlich der Kontrolle, wurden auf Mikrosporen-Suspensionen derselben Extraktion aufgebracht, und zwar wie folgt: T1, Kontrollbehandlung, bestehend aus 200 µl sterilem Wasser; T2, 1 µg an dsDNA in 100 µl sterilem Wasser wurde zu 4 µg Tat2, verdünnt in 100 µl sterilem Wasser, hinzuzugegeben und sanft gemischt, was in einem 1:4-Verhältnis von dsDNA zu Tat2 (dsDNATat2) resultierte; T3, 1 µg an dsDNA, verdünnt in 100µl sterilem Wasser, und 6 µl Chariot (Active Motif, Carlsbad, CA), in 100 µl sterilem Wasser verdünnt, wurden zusammengemischt (dsDNA-Pep1); T4, 4 µg RecA (MJS Biolynx, Brockville, Kanada; # UB70028Z) in 50 µl sterilem Wasser und 1 µg dsDNA in 50 µl sterilem Wasser wurden für 15 min zusammen gemischt und 6 µl Chariot in 100 µl sterilem Wasser wurde zu der dsDNA-RecA Lösung hinzugegeben, um ein Endvolumen von 200 µl in einem 2 ml Mikrozentrifugenröhrchen zu erhalten (dsDNA-RecA-Pep1); T5, µg RecA in 50 µl sterilem Wasser und 1 µg dsDNA in 50 µl sterilem Wasser wurden zusammen 15 min gemischt. 4 µg Tat2 in 100 µl sterilem Wasser wurde zu der dsDNA-RecA-Lösung gegeben, um auf ein Endvolumen von 200 µl in einem 2 ml Mikrozentrifugenröhrchen zu kommen (dsDNA-RecA-Tat2). Nach der Inkubation für 15 min bei Raumtemperatur (RT) wurden 5 µl Lipofectamin (Invitrogen, Carlsbad, CA; # 11.668 bis 019) zu allen Ansätzen zugesetzt und es wurde für weitere 5 min bei RT inkubiert. Die Mischungen wurden dann sofort zu 50.000 pelletierten Mikrosporen in 2 ml Mikrozentrifugenröhrchen hinzugegeben und für 15 min inkubiert. Dann wurden 100 µl NPB-99 pro Röhrchen zugegeben und es wurde für 45 min bei RT inkubiert. Transfizierter Mikrosporen wurden pelletiert, der Überstand wurde entfernt und die Zellen wurden zweimal mit NPB-99 gewaschen. Dann wurde 1 ml NPB-99 pro Röhrchen an Mikrosporen zugegeben, es wurde vorsichtig gemischt und Aliquote von 500 µl wurden in 35-mm-Petrischalen, enthaltend 3 ml NPB-99 + 10% Ficoll (Sigma, St. Louis, MO; F4375 ; NPB-99-10F) und 100 mg/l des Antibiotikums Cefotaxim (Sigma;. # C7039), pipettiert.

### Elektroporation

### Pollen-Transformation:

Pollen Transformation durch Elektroporation wurde nach dem Protokoll, das von Shi et al (1996), etabliert wurde, durchgeführt. Hierzu wurden reife Pollen, keimende Pollen oder Pollen ohne Exin-Schicht mit einer Feldstärke von 750-1250 V/cm mit einem konstanten Impuls von 13 ms elektroporiert.

### Ultraschall-Behandlung

### Pollen-Beschallung:

Pollen Transformation durch Ultraschallbehandlung wurde wie von Wang et al (2000) beschrieben durchgeführt. Hierzu wurden 0,3 g frische Pollen morgens gesammelt und in 20 ml einer Lösung mit 5% Saccharose mit etwa 10 ug der Plasmid-DNA von Interesse gemischt. Die Lösung wurde mit Ultraschall behandelt, sowohl bevor als auch nachdem Plasmid-DNA hinzugefügt wurde. Bei Verwendung eines JY92-II Ultraschallgeräts von Ningbo Xinzi Scientific Instrument Institute waren die verwendeten Parameter für die Beschallung: Schallintensität von 300 W, Behandlungen 8-mal für jeweils 5s und 10s-Intervalle. Dann wurde mit den behandelten Pollen angeclippte Mais-Seide bestäubt.

### Zitationsliste Beispielteil:

Aragäo, F. J., et al. (2005). "Germ line genetic transformation in cotton (Gossypium hirsutum L.) by selection of transgenic meristematic cells with a herbicide molecule." Plant Science 168(5): 1227-1233 / Record #: 2408
Artschwager, E. (1926). "Anatomy of the vegetative organs of the sugar beet." J. agric. Res 33: 143-176 / Record #: 2352
Barnabas, B., et al. (1992). "In vitro pollen maturation and successful seed production in detached spikelet cultures in wheat (Triticum aestivum L.)." Sexual Plant Reproduction 5(4): 286-291 / Record #: 2374
Buchheim, J. A., et al. (1989). "Maturation of Soybean Somatic Embryos and the Transition to Plantlet Growth." Plant Physiology 89(3): 768-775 / Record #: 2402
Chee, P. P., et al. (1989). "Transformation of soybean (Glycine max) by infecting germinating seeds with Agrobacterium tumefaciens." Plant Physiology 91(3): 1212-1218 / Record #: 2392
Chowrira, G., et al. (1995). "Electroporation-mediated gene transfer into intact nodal meristemsin planta." Molecular Biotechnology 3(1): 17-23 / Record #: 2393
Eapen, S. (2011). "Pollen grains as a target for introduction of foreign genes into plants: an assessment." Physiology and Molecular Biology of Plants 17(1): 1-8 / Record #: 2384
Frame, B. R., et al. (1994). "Production of fertile transgenic maize plants by silicon carbide whisker-mediated transformation." The Plant Journal 6(6): 941-948 / Record #: 1755
Gounaris, Y., et al. (2005). "Pollen-mediated genetic transformation of cotton with the Arabidopsis thaliana hmgr cDNA using the particle gun." Journal of Food Agriculture and Environment 3(2): 157-160 / Record #: 2330
Grandjean, O., et al. (2004). "In Vivo Analysis of Cell Division, Cell Growth, and Differentiation at the Shoot Apical Meristem in Arabidopsis." The Plant Cell 16(1): 74-87 / Record #: 2387
Hermann, K., et al. (2007). "1-Aminocyclopropane-1-carboxylic acid and abscisic acid during the germination of sugar beet (Beta vulgaris L.): a comparative study of fruits and seeds." Journal of Experimental Botany 58(11): 3047-3060 / Record #: 2370
Horikawa, Y., et al. (1997). "Transformants through pollination of mature maize (Zea mays L.) pollen delivered bar gene by particle gun." Journal of Japanese Society of Grassland Science (Japan) / Record #: 2383
Huang, Y., et al. (2009). "Probing the endosperm gene expression landscape in Brassica napus." BMC Genomics 10(1): 256 / Record #: 2377
Itoh, J.-I., et al. (2005). "Rice Plant Development: from Zygote to Spikelet." Plant and Cell Physiology 46(1): 23-47 / Record #: 2416
Keshamma, E., et al. (2008). "Tissue culture-independent in planta transformation strategy: an Agrobacterium tumefaciens-mediated gene transfer method to overcome recalcitrance in cotton (Gossypium hirsutum L.)." Journal of Cotton Science 12(3): 264-272 / Record #: 2405
Kim, S., et al. (2014). "Highly efficient RNA-guided genome editing in human cells via delivery of purified Cas9 ribonucleoproteins." Genome Research 24(6): 1012-1019 / Record #: 2288
Ko, S.-W., et al. (1983). "A simplified method of embryo culture in rice of Oryza sativa L." Bot. Bull. Acad. Sin 24: 97-101 / Record #: 2415
Krens, F. A., et al. (1988). "Transformation and regeneration in sugar beet (Beta vulgaris L.) induced by 'shooter' mutants of Agrobacterium tumefaciens." Euphytica 39(3): 185-194 / Record #: 1426
Leduc, N., et al. (1994). "Gene transfer to inflorescence and flower meristems using ballistic micro-targeting." Sexual Plant Reproduction 7(2): 135-143 / Record #: 2345
Li, X., et al. (2004). "Improvement of cotton fiber quality by transforming the acsA and acsB genes into Gossypium hirsutum L. by means of vacuum infiltration." Plant Cell Reports 22(9): 691-697 / Record #: 2419
Liu, J., et al. (2015). "Efficient delivery of nuclease proteins for genome editing in human stem cells and primary cells." Nat. Protocols 10(11): 1842-1859 / Record #: 2421
Lusardi, M. C., et al. (1994). "An approach towards genetically engineered cell fate mapping in maize using the Lc gene as a visible marker: transactivation capacity of Lc vectors in differentiated maize cells and microinjection of <i>Lc</i> vectors into somatic embryos and shoot apical meristems." The Plant Journal 5(4): 571-582 / Record #: 82
Mahn, A., et al. (1995). "Transient gene expression in shoot apical meristems of sugarbeet seedlings after particle bombardment." Journal of Experimental Botany 46(10): 1625-1628 / Record #: 2367
Martin-Ortigosa, S., et al. (2014). "Proteolistics: a biolistic method for intracellular delivery of proteins." Transgenic Research: 1-14 / Record #: 2260
Matthys-Rochon, E., et al. (1998). "In vitro development of maize immature embryos: a tool for embryogenesis analysis." Journal of Experimental Botany 49(322): 839-845 / Record #: 2285
Mauney, J. R. (1961). "The Culture In vitro of Immature Cotton Embryos." Botanical Gazette 122(3): 205-209 / Record #: 2409
McCabe, D. E., et al. (1993). "Transformation of elite cotton cultivars via particle bombardment of meristems." Nature Biotechnology 11(5): 596-598 / Record #: 2404
McCabe, D. E., et al. (1988). "Stable transformation of soybean (Glycine max) by particle acceleration." Nature Biotechnology 6(8): 923-926 / Record #: 2391
Muniz de Péadua, V., et al. (2001). "Transformation of Brazilian elite Indica-type rice (Oryza sativa L.) by electroporation of shoot apex explants." Plant Molecular Biology Reporter 19(1): 55-64 / Record #: 2412
Naseri, G., et al. (2014). "In planta transformation of rice (Oryza sativa) using thaumatin-like protein gene for enhancing resistance to sheath blight." African Journal of Biotechnology 11(31) / Record #: 2411
Neuhaus, G., et al. (1987). "Transgenic rapeseed plants obtained by the microinjection of DNA into microspore-derived embryoids." Theoretical and Applied Genetics 75(1): 30-36 / Record #: 2123
Obert, B., et al. (2008). "Genetic transformation of barley microspores using anther bombardment." Biotechnology Letters 30(5): 945-949 / Record #: 2068
Olvera, H. F., et al. (2008). "Floral and Inflorescence Morphology and Ontogeny in Beta vulgaris, with Special Emphasis on the Ovary Position." Annals of Botany 102(4): 643-651 / Record #: 2372
Rajasekaran, K. (2013). Biolistic Transformation of Cotton Zygotic Embryo Meristem. T Transgenic Cotton. 958: 47-57 / Record #: 2403
Ramakrishna, S., et al. (2014). "Gene disruption by cell-penetrating peptide-mediated delivery of Cas9 protein and guide RNA." Genome Research 24(6): 1020-1027 / Record #: 2287
Ritchie, G. L., et al. (2007). "Cotton growth and development." from http://cotton.tamu.edu/mwg-internal/de5fs23hu73ds/progress?id=-gCnQaUKI-guBsRa08L1m_QGIEFw5-bHQ3iPfT8R-CI, / Record #: 2410
Rod-in, W., et al. (2014). "The floral-dip method for rice (Oryza sativa) transformation." Journal of Agricultural Technology 10(2): 467-474 / Record #: 2414
Sautter, C., et al. (1995). Ballistic microtargeting of visible marker genes to the shoot meristem of wheat. Gene Transfer to Plants, Springer: 152-156 / Record #: 2339
Sautter, C., et al. (1995). "Shoot apical meristems as a target for gene transfer by microballistics." Euphytica 85(1-3): 45-51 / Record #: 2373
Shi, H., et al. (1996). "Exine-detached pollen of Nicotiana tabacum as an electroporation target for gene transfer." Acta Botanica Sinica 38(8): 626-630 / Record #: 2417
Shim, Y.-S., et al. (2012). "dsDNA and protein co-delivery in triticale microspores." In Vitro Cellular & Developmental Biology - Plant: 1-10 / Record #: 1253
Shou, H., et al. (2002). "Irreproducibility of the soybean pollen-tube pathway transformation procedure." Plant Molecular Biology Reporter 20(4): 325-334 / Record #: 2399
Sivakumar, S., et al. (2014). "Optimization of factors influencing microinjection method for Agrobacterium - Mediated transformation of Embryonic Shoot Apical Meristem in Cotton (Gossypium hirsutum L. cv.SVPR-2)." International Journal of Current Biotechnology / Record #: 2420
Touraev, A., et al. (1997). "Plant male germ line transformation." The Plant Journal 12(4): 949-956 / Record #: 2380
Twell, D., et al. (1989). "Transient expression of chimeric genes delivered into pollen by microprojectile bombardment." Plant Physiology 91(4): 1270-1274 / Record #: 2316
Vain, P., et al. (1993). "Osmotic treatment enhances particle bombardment-mediated transient and stable transformation of maize." Plant Cell Reports 12(2): 84-88 / Record #: 20
Wang, J., et al. (2000). "Transgenic maize plants obtained by pollen-mediated transformation." Acta Botanica Sinica 43(3): 275-279 / Record #: 2418
Wiebold, W. J. (2012). "Arrested Development in the Soybean Field." from http://ipm.missouri.edu/IPCM/2012/10/Arrested-Development-in-the-Soybean-Field/ / Record #: 2400
Zapata, C., et al. (1999). "Transformation of a Texas cotton cultivar by using Agrobacterium and the shoot apex." Theoretical and Applied Genetics 98(2): 252-256 / Record #: 2407
Zhang, C.-L., et al. (2008). "Efficient somatic embryogenesis in sugar beet (Beta vulgaris L.) breeding lines." Plant Cell, Tissue and Organ Culture 93(2): 209-221 / Record #: 1461
Zuris, J. A., et al. (2015). "Cationic lipid-mediated delivery of proteins enables efficient protein-based genome editing in vitro and in vivo." Nat Biotech 33(1): 73-80 / Record #: 2303

## Patentansprüche

1. Verfahren zur Herstellung einer Maispflanze, umfassend die folgenden Schritte:
(i) Bereitstellen einer pflanzlichen Zielstruktur,
welche mindestens eine meristematische Zelle umfasst,
wobei die pflanzliche Zielstruktur ein reifer oder unreifer Maisembryo ist und die mindestens eine meristematische Zelle mindestens eine Nukleinsäure-Zielregion umfasst;
(ii) Bereitstellen mindestens einer gRNA oder Bereitstellen eines oder mehrerer rekombinanten Konstrukts/Konstrukte, wobei das bzw. die rekombinante(n) Konstrukt(e)
(a) mindestens eine gRNA oder eine für eine gRNA kodierende Sequenz, und
(b) optional mindestens eine regulatorische Sequenz und/oder eine Lokalisationssequenz und
(c) optional mindestens eine DNA-Reparatur-Matrize umfasst/umfassen, und Bereitstellen mindestens einer CRISPR Nuklease, bevorzugt einer Cas oder einer Cpf1 Nuklease, oder eines katalytisch aktiven Fragments davon und/oder einer Effektor-Domäne oder Bereitstellen eines oder mehrerer rekombinanten Konstrukts/Konstrukte,
wobei das bzw. die rekombinante(n) Konstrukt(e)
(A) mindestens eine CRISPR Nuklease, oder ein katalytisch aktives Fragment davon oder eine für eine CRISPR Nuklease oder eine für ein katalytisch aktives Fragment davon kodierende Sequenz und/oder mindestens eine Effektor-Domäne oder eine für eine Effektor-Domäne kodierende Sequenz, und
(B) optional mindestens eine regulatorische Sequenz und/oder eine Lokalisationssequenz umfasst/umfassen,
wobei die gRNA dazu in der Lage ist, sowohl an einen Abschnitt der Nukleinsäure-Zielregion zu hybridisieren als auch mit der CRISPR Nuklease oder dem katalytisch aktiven Fragment davon und/oder der Effektor-Domäne zu interagieren;
wobei, wenn die gRNA oder die die gRNA kodierende Sequenz und die CRISPR Nuklease oder das katalytisch aktive Fragment davon oder die die CRISPR Nuklease oder die das katalytisch aktive Fragment davon kodierende Sequenz und/oder die Effektor-Domäne oder die für eine Effektor-Domäne kodierende Sequenz durch ein oder mehrere rekombinante(s) Konstrukt(e) bereitgestellt werden, die gRNA oder die die gRNA kodierende Sequenz und die CRISPR Nuklease oder das katalytisch aktive Fragment davon oder die die CRISPR Nuklease oder die das katalytisch aktive Fragment davon kodierende Sequenz und/oder die Effektor-Domäne oder die für eine Effektor-Domäne kodierende Sequenz auf oder in dem gleichen, oder auf oder in unterschiedlichen rekombinanten Konstrukten lokalisiert sind;
(iii) Einbringen der gRNA, der CRISPR Nuklease oder des katalytisch aktiven Fragments davon und/oder der Effektor-Domäne oder des bzw. der rekombinanten Konstrukts/Konstrukte in die mindestens eine meristematische Zelle der pflanzlichen Zielstruktur;
(iv) Kultivieren der pflanzlichen Zielstruktur unter Bedingungen, welche die Aktivierung der eingeführten gRNA, der CRISPR Nuklease oder des katalytisch aktiven Fragments davon und/oder der Effektor-Domäne oder des bzw. der eingeführten rekombinanten Konstrukts/Konstrukte und dadurch eine gezielte Veränderung der Nukleinsäure-Zielregion in der mindestens eine meristematische Zelle der pflanzlichen Zielstruktur gestatten, um eine pflanzliche Zielstruktur, umfassend mindestens eine meristematische Zelle, die die gezielte Veränderung der Nukleinsäure-Zielregion umfasst, zu erhalten;
(v) Gewinnen einer Maispflanze aus der gezielt veränderten mindestens einen meristematischen Zelle,
wobei die Maispflanze direkt durch Zellteilung und Differenzierung, ohne als Zwischenschritt über eine Zellkultur in Form von Kallusproduktion und -regeneration zu gehen, aus der gezielt veränderten mindestens einen meristematischen Zelle gewonnen wird,
wobei die gewonnene Maispflanze die gezielte Veränderung der Nukleinsäure-Zielregion umfasst und
wobei in der Maispflanze aus Schritt (v) das bzw. die rekombinante(n) Konstrukt(e), welche(s)
(a) mindestens eine gRNA oder eine für eine gRNA kodierende Sequenz, oder
(b) mindestens eine CRISPR Nuklease, bevorzugt eine Cas oder eine Cpf1 Nuklease, oder ein katalytisch aktives Fragment davon oder eine für eine CRISPR Nuklease oder eine für ein katalytisch aktives Fragment davon kodierende Sequenz und/oder mindestens eine Effektor-Domäne oder eine für eine Effektor-Domäne kodierende Sequenz umfasst/umfassen, nicht chromosomal oder extrachromosomal integriert werden/sind.

2. Verfahren gemäß Anspruch 1, wobei in Schritt (ii) die gRNA oder die die gRNA kodierende Sequenz und/oder die CRISPR Nuklease, bevorzugt eine Cas oder eine Cpf1 Nuklease, oder das katalytisch aktive Fragment davon oder die die CRISPR Nuklease oder die das katalytisch aktive Fragment davon kodierende Sequenz und/oder die Effektor-Domäne oder eine für eine Effektor-Domäne kodierende Sequenz auf die Anwendung in einer pflanzlichen Zelle hin angepasst sind.

3. Verfahren gemäß einem der Ansprüche 1 oder 2, wobei zwischen Schritt (ii) und (iii) das Bereitstellen mindestens eines Vektors zur Einbringung des bzw. der rekombinanten Konstrukts/Konstrukte erfolgt.

4. Verfahren gemäß einem der vorangehenden Ansprüche, wobei zwischen Schritt (ii) und (iii) das Bereitstellen mindestens eines weiteren rekombinanten Konstrukts umfassend einen rekombinanten Nukleinsäure-Abschnitt zur gezielten Homologie-gerichteten Reparatur der Nukleinsäure-Zielregion in der pflanzlichen Zielstruktur oder Insertion in die Nukleinsäure-Zielregion in der pflanzlichen Zielstruktur und optional mindestens eines weiteren Vektors zur Einbringung des mindestens einen weiteren rekombinanten Konstrukts erfolgt.

5. Verfahren gemäß einem der vorangehenden Ansprüche, wobei der mindestens eine Vektor ausgewählt ist aus der Gruppe bestehend aus einem Virus, ausgewählt aus der Gruppe bestehend aus SEQ ID NOs:12-15 und 25-38, sowie Sequenzen mit mindestens 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%,90%, 91%, 92%, 93%, 94%, 95%, 96%, 97%, 98% oder 99% Sequenzhomologie zu diesen Sequenzen, oder einem Agens, das zur Transfektion einer Peptid- oder Polypeptidsequenz oder von Nukleinsäuresequenzen geeignet ist, oder einer Kombination davon.

6. Verfahren gemäß einem der vorangehenden Ansprüche, wobei die gRNA direkt als natürliche oder synthetische Nukleinsäure und/oder die CRISPR Nuklease, bevorzugt eine Cas oder eine Cpf1 Nuklease, oder das katalytisch aktive Fragment davon direkt als Polypeptid und/oder die Effektor-Domäne direkt als Nukleinsäure oder Polypeptid in die pflanzliche Zielstruktur eingebracht wird.

7. Verfahren gemäß einem der vorangehenden Ansprüche, wobei die gRNA oder die die gRNA kodierende Sequenz oder die CRISPR Nuklease, bevorzugt eine Cas oder eine Cpf1 Nuklease, oder das katalytisch aktive Fragment davon oder die die CRISPR Nuklease oder die das katalytisch aktive Fragment davon kodierende Sequenz oder die Effektor-Domäne oder die die Effektor-Domäne kodierende Sequenz zusätzlich eine Lokalisationssequenz umfasst, ausgewählt aus einer Kernlokalisationssequenz, einer Plastidenlokalisationssequenz, vorzugsweise einer Mitochondrienlokalisationssequenz und einer Chloroplastenlokalisationssequenz.

8. Verfahren gemäß einem der vorangehenden Ansprüche, wobei zusätzlich ein Inhibitor des endogen non-homologous end joining (NHEJ) Reparaturmechanismus in die pflanzliche Zielstruktur eingebracht wird.

9. Verfahren gemäß einem der vorangehenden Ansprüche, wobei das rekombinante Konstrukt ausgewählt ist aus SEQ ID NOs: 23 und 24, sowie Sequenzen mit mindestens 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%,90%, 91%, 92%, 93%, 94%, 95%, 96%, 97%, 98% oder 99% Sequenzhomologie zu diesen Sequenzen.

## Claims

1. A method of producing a corn plant comprising the following steps:
(i) providing a plant target structure,
comprising at least one meristematic cell,
wherein the plant target structure is a mature or immature corn embryo and the at least one meristematic cell comprises at least one nucleic acid target region;
(ii) providing at least one gRNA or providing one or more recombinant construct(s), wherein the recombinant construct(s) comprise(s)
(a) at least one gRNA or sequence encoding a gRNA, and
(b) optionally at least one regulatory sequence and/or a localization sequence and
(c) optionally at least one DNA repair template,
and providing at least one CRISPR nuclease, preferably a Cas or a Cpf1 nuclease, or a catalytically active fragment thereof and/or an effector domain, or providing one or more recombinant construct(s),
wherein the recombinant construct(s) comprise(s)
(A) at least one CRISPR nuclease, or a catalytically active fragment thereof, or a sequence encoding a CRISPR nuclease or a catalytically active fragment thereof, and/or at least one effector domain or a sequence encoding an effector domain, and
(B) optionally at least one regulatory sequence and/or one localization sequence, wherein said gRNA is capable of both hybridizing to a portion of the nucleic acid target region as well as to hybridize with the CRISPR nuclease or the catalytically active fragment thereof and/or to interact with the effector domain;
wherein, when the gRNA or the sequence encoding the gRNA and the CRISPR nuclease, or the catalytically active fragment thereof, or the CRISPR nuclease or the sequence encoding the catalytically active fragment thereof and/or the effector domain or the sequence encoding an effector domain is provided by one or more recombinant construct(s), the gRNA or the gRNA or the sequence encoding the gRNA and the CRISPR nuclease or the catalytically active fragment thereof or the CRISPR nuclease or the sequence encoding the catalytically active fragment thereof, and/or the effector domain or the sequence encoding an effector domain are located on or in the same, or on or in different recombinant constructs;
(iii) introducing the gRNA, the CRISPR nuclease or the catalytically active fragment thereof and/or the effector domain or the recombinant construct(s) into the at least one meristematic cell of the plant target structure;
(iv) culturing the plant target structure under conditions that promote the activation of the introduced gRNA, the CRISPR nuclease or the catalytically active fragment thereof and/or the effector domain or the introduced recombinant construct(s) and thereby enabling a targeted alteration of the nucleic acid target region in the at least one meristematic cell of the plant target structure to obtain a plant target structure comprising at least one meristematic cell comprising the targeted alteration of the nucleic acid target;
(v) obtaining a maize plant from said targeted altered at least one meristematic cell, whereby the corn plant is obtained directly by cell division and differentiation, without an intermediate step of a cell culture in the form of callus production and regeneration, from the specifically modified at least one meristematic cell,
wherein the obtained maize plant comprises the targeted modification of the nucleic acid target region, and
wherein in the maize plant of step (v) the recombinant construct(s), which(s) comprise(s)
(a) at least one gRNA or sequence encoding a gRNA, or
(b) at least one CRISPR nuclease, preferably a Cas or a Cpf1 nuclease, or a catalytically active fragment thereof, or a sequence encoding a CRISPR nuclease or a catalytically active fragment thereof, and/or at least one effector sequence and/or at least one effector domain or effector domain-encoding sequence, is/are not chromosomally or extra-chromosomally integrated.

2. The method according to claim 1, wherein in step (ii) the gRNA or the sequence encoding the gRNA and/or the CRISPR nuclease, preferably a Cas or a Cpf1 nuclease, or the catalytically active fragment thereof, or the sequence encoding the CRISPR nuclease or the catalytically active fragment thereof and/or the sequence and/or the effector domain or a sequence encoding an effector domain are adapted for use in a plant cell.

3. The method according to any one of claims 1 or 2, wherein the provision at least one vector for introducing the recombinant construct(s) occurs between step (ii) and (iii).

4. The method according to any one of the preceding claims, wherein between step (ii) and (iii) the provision of at least one further recombinant construct comprising a recombinant nucleic acid portion for targeted homology-directed repair of the nucleic acid targeting region in the plant target structure or insertion into the nucleic acid targeting region in the plant target structure, and optionally at least one further vector for inserting of the at least one further recombinant construct occurs.

5. The method according to any one of the preceding claims, wherein the at least one vector is selected from the group consisting of a virus, selected from the group consisting of SEQ ID NOs: 12-15 and 25-38, and from sequences having at least 66%, 67%, 68%, 69%, 70%, 71%, 72%, 73%,74%, 75%, 76%, 77%, 78%, 79%, 80%, 81%, 82%, 83%, 84%, 85%, 86%, 87%,88%, 89%,90%, 91%, 92%, 93%, 94%, 95%, 96%, 97%, 98%, or 99% sequence homology to these sequences, or an agent used to transfect a peptide or polypeptide sequence or nucleic acid sequences, or a combination thereof.

6. The method according to any one of the preceding claims, wherein the gRNA directly as a natural or synthetic nucleic acid and/or the CRISPR nuclease, preferably a Cas or a Cpf1 nuclease, or the catalytically active fragment thereof directly as a polypeptide and/or the effector domain directly as a nucleic acid or polypeptide is introduced into the plant target structure.

7. The method according to any one of the preceding claims, wherein the gRNA or the sequence coding for the gRNA or the CRISPR nuclease, preferably a Cas or a Cpf1 nuclease, or the catalytically active fragment thereof or the sequence encoding the CRISPR nuclease or the catalytically active fragment encoding sequence or the effector domain or the effector domain encoding sequence additionally comprises a localization sequence selected from a nuclear localization sequence, a plastid localization sequence, preferably a mitochondrial localization sequence and a chloroplast localization sequence.

8. The method according to any one of the preceding claims, wherein additionally an inhibitor of the endogenous non-homologous end joining (NHEJ) repair mechanism is introduced into the plant target structure.

9. The method according to any one of the preceding claims, wherein the recombinant construct is selected from SEQ ID NOs: 23 and 24, and from sequences having at least 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% sequence homology to these sequences.

## Revendications

1. Procédé de production d'une plante de maïs, comprenant les étapes suivantes consistant à:
(i) fournir une structure cible végétale,
qui comprend au moins une cellule méristématique,
dans lequel la structure cible végétale est un embryon de maïs mature ou immature et ladite au moins une cellule méristématique comprend au moins une région cible d'acide nucléique;
(ii) fournir au moins un ARNg ou fournir une ou plusieurs construction(s) recombinante(s),
dans lequel la ou les construction(s) recombinante(s)
(a) comprend/comprennent au moins un ARNg ou une séquence codant pour un ARNg, et
(b) en option au moins une séquence régulatrice et/ou une séquence de localisation et
(c) en option au moins une matrice de réparation d'ADN,
et fournir au moins une nucléase CRISPR, de préférence une nucléase Cas ou Cpf1, ou un fragment catalytiquement actif de celle-ci et/ou un domaine effecteur ou fournir une ou plusieurs construction(s) recombinante(s),
dans lequel la ou bien les construction(s) recombinante(s)
(A) comprend/comprennent au moins une nucléase CRISPR, ou un fragment catalytiquement actif de celle-ci ou une séquence codant pour une nucléase CRISPR ou une séquence codant pour un fragment catalytiquement actif de celle-ci et/ou au moins un domaine effecteur ou une séquence codant pour un domaine effecteur, et
(B) en option au moins une séquence régulatrice et/ou une séquence de localisation,
dans lequel l'ARNg est capable à la fois de s'hybrider à un segment de la région cible d'acide nucléique et d'interagir avec la nucléase CRISPR ou le fragment catalytiquement actif de celle-ci et/ou le domaine effecteur;
dans lequel, si l'ARNg ou la séquence codant pour l'ARNg et la nucléase CRISPR ou le fragment catalytiquement actif de celle-ci ou la séquence codant pour la nucléase CRISPR ou le fragment catalytiquement actif de celle-ci et/ou le domaine effecteur ou la séquence codant pour un domaine effecteur sont fournis par une ou plusieurs construction(s) recombinante(s), l'ARNg ou la séquence codant pour l'ARNg et la nucléase CRISPR ou le fragment catalytiquement actif de celle-ci ou la séquence codant pour la nucléase CRISPR ou le fragment catalytiquement actif de celle-ci et/ou le domaine effecteur ou la séquence codant pour un domaine effecteur sont localisés sur ou dans la même, ou sur ou dans différentes constructions recombinantes;
(iii) introduire l'ARNg, la nucléase CRISPR ou le fragment catalytiquement actif de celle-ci et/ou le domaine effecteur ou la ou bien les construction(s) recombinante(s) dans ladite au moins une cellule méristématique de la structure cible végétale;
(iv) cultiver la structure cible végétale dans des conditions qui permettent l'activation de l'ARNg introduit, de la nucléase CRISPR ou du fragment catalytiquement actif de celle-ci et/ou du domaine effecteur ou de la construction recombinante ou bien des constructions recombinantes introduite(s) et ainsi une modification ciblée de la région cible d'acide nucléique dans ladite au moins une cellule méristématique de la structure cible végétale pour obtenir une structure cible végétale comprenant au moins une cellule méristématique qui comprend la modification ciblée de la région cible d'acide nucléique;
(v) obtenir une plante de maïs à partir de ladite au moins une cellule méristématique modifiée de manière ciblée,
dans lequel la plante de maïs est obtenue à partir de ladite au moins une cellule méristématique modifiée de manière ciblée, directement par division cellulaire et différenciation, sans passer par une culture cellulaire sous forme de production et de régénération de cal en tant qu'étape intermédiaire,
dans lequel la plante de maïs obtenue comprend la modification ciblée de la région cible d'acide nucléique et
dans lequel, dans la plante de maïs de l'étape (v), la ou bien les construction(s) recombinante(s) qui
(a) comprend/comprennent au moins un ARNg ou une séquence codant pour un ARNg, et
(b) au moins une nucléase CRISPR, de préférence une nucléase Cas ou une nucléase Cpf1, ou un fragment catalytiquement actif de celle-ci ou une séquence codant pour une nucléase CRISPR ou une séquence codant pour un fragment catalytiquement actif de celle-ci et/ou au moins un domaine effecteur ou une séquence codant pour un domaine effecteur sont intégrés de manière non chromosomique ou extrachromosomique.

2. Procédé selon la revendication 1, dans lequel à l'étape (ii) l'ARNg ou la séquence codant pour l'ARNg et/ou la nucléase CRISPR, de préférence une nucléase Cas ou une nucléase Cpf1, ou le fragment catalytiquement actif de celle-ci ou la nucléase CRISPR ou la séquence codant pour le fragment catalytiquement actif de celle-ci et/ou le domaine effecteur ou une séquence codant pour un domaine effecteur sont adaptés à une utilisation dans une cellule végétale.

3. Procédé selon l'une quelconque des revendications 1 ou 2, dans lequel entre les étapes (ii) et (iii) se fait la fourniture d'au moins un vecteur d'introduction de la construction recombinante ou bien des constructions recombinantes.

4. Procédé selon l'une quelconque des revendications précédentes, dans lequel, entre les étapes (ii) et (iii), se fait la fourniture d'au moins une autre construction recombinante comprenant un segment d'acide nucléique recombinant pour la réparation dirigée par homologie de la région cible d'acide nucléique dans la structure cible végétale ou l'insertion dans la région cible d'acide nucléique dans la structure cible végétale et, en option, d'au moins un autre vecteur pour introduire ladite au moins une autre construction recombinante.

5. Procédé selon l'une quelconque des revendications précédentes, dans lequel ledit au moins un vecteur est sélectionné dans le groupe consistant en un virus sélectionné dans le groupe consistant en SEQ ID NOs : 12-15 et 25-38, et des séquences avec au moins 66 %, 67 %, 68 %, 69 %, 70 %, 71 %, 72 %, 73 %, 74 %, 75 %, 76 %, 77 %, 78 %, 79 %, 80 %, 81 %, 82 %, 83 %, 84 %, 85 %, 86 %, 87 %, 88 %, 89 %, 90 %, 91 %, 92 %, 93 %, 94 %, 95 %, 96 %, 97 %, 98 % ou 99 % d'homologie de séquence avec ces séquences, ou un agent qui est approprié pour transfecter une séquence peptidique ou polypeptidique ou des séquences d'acide nucléique, ou une combinaison de ceux-ci.

6. Procédé selon l'une quelconque des revendications précédentes, dans lequel l'ARNg est introduit directement sous forme d'acide nucléique naturel ou synthétique et/ou la nucléase CRISPR, de préférence une nucléase Cas ou une nucléase Cpf1, ou son fragment catalytiquement actif est introduit directement sous forme de polypeptide et/ou le domaine effecteur est introduit directement sous forme d'acide nucléique ou de polypeptide dans la structure cible végétale.

7. Procédé selon l'une quelconque des revendications précédentes, dans lequel l'ARNg ou la séquence codant pour l'ARNg ou la nucléase CRISPR, de préférence une nucléase Cas ou une nucléase Cpf1, ou le fragment catalytiquement actif de celle-ci ou la séquence codant pour la nucléase CRISPR ou la séquence codant pour le fragment catalytiquement actif de celle-ci ou le domaine effecteur ou la séquence codant pour le domaine effecteur comprend en sus une séquence de localisation choisie parmi une séquence de localisation nucléaire, une séquence de localisation plastidiale, de préférence une séquence de localisation mitochondriale et une séquence de localisation chloroplastique.

8. Procédé selon l'une quelconque des revendications précédentes, dans lequel, en sus, un inhibiteur du mécanisme de réparation endogène de jonction d'extrémités non homologues (NHEJ) est introduit dans la structure cible végétale.

9. Procédé selon l'une quelconque des revendications précédentes, dans lequel la construction recombinante est choisie parmi les SEQ ID NO : 23 et 24, et des séquences avec au moins 66 %, 67 %, 68 %, 69 %, 70 %, 71 % , 72% , 73%, 74%, 75%, 76%, 77%, 78%, 79%, 80%, 81%, 82%, 83%, 84%, 85%, 86%, 87%, 88 %, 89 %, 90 %, 91 %, 92 %, 93 %, 94 %, 95 %, 96 %, 97 %, 98 % ou 99 % d'homologie de séquence avec ces séquences.
